(19) Europäisches Patentamt
European Patent Office
Office européen des brevets

(11) EP 3 395 345 A1

(12) # EUROPEAN PATENT APPLICATION
published in accordance with Art. 153(4) EPC

(43) Date of publication:
**31.10.2018 Bulletin 2018/44**

(21) Application number: **16878960.0**

(22) Date of filing: **22.12.2016**

(51) Int Cl.:
***A61K 31/519*** *(2006.01)* ***A61K 31/167*** *(2006.01)*
***A61K 31/282*** *(2006.01)* ***A61K 31/337*** *(2006.01)*
***A61K 31/357*** *(2006.01)* ***A61K 31/4045*** *(2006.01)*
***A61K 31/4164*** *(2006.01)* ***A61K 31/436*** *(2006.01)*
***A61K 31/44*** *(2006.01)* ***A61K 31/454*** *(2006.01)*
***A61K 31/4545*** *(2006.01)* ***A61K 31/47*** *(2006.01)*
***A61K 31/4709*** *(2006.01)* ***A61K 31/473*** *(2006.01)*
***A61K 31/4747*** *(2006.01)* ***A61K 31/497*** *(2006.01)*
***A61K 31/502*** *(2006.01)* ***A61K 31/5025*** *(2006.01)*
***A61K 31/513*** *(2006.01)* ***A61K 31/52*** *(2006.01)*

(86) International application number:
**PCT/JP2016/088473**

(87) International publication number:
**WO 2017/111074 (29.06.2017 Gazette 2017/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.12.2015 JP 2015250705**
**30.09.2016 JP 2016194889**

(71) Applicant: **Taiho Pharmaceutical Co., Ltd.**
**Chiyoda-ku, Tokyo 101-8444 (JP)**

(72) Inventor: **ISHIDA, Keiji**
**Tsukuba-shi**
**Ibaraki 300-2611 (JP)**

(74) Representative: **Hoffmann Eitle**
**Patent- und Rechtsanwälte PartmbB**
**Arabellastraße 30**
**81925 München (DE)**

(54) **ANTITUMOR EFFECT POTENTIATOR COMPRISING PYRROLOPYRIMIDINE COMPOUND**

(57) Disclosed is an antitumor effect potentiator for one or more other compounds having an antitumor effect, the antitumor effect potentiator comprising a novel pyrrolopyrimidine compound.

EP 3 395 345 A1

## Description

Technical Field

Cross-Reference to Related Applications

The present application claims priority to the specification of Japan Patent Application No. 2015-250705 filed on December 22, 2015, and the specification of Japan Patent Application No. 2016-194889 filed on September 30, 2016, the entire contents of which are incorporated herein by reference.

Technical Field

The present invention relates to a potentiator for one or more other compounds having an antitumor effect, the potentiator comprising a novel pyrrolopyrimidine compound. The present invention also relates to an antitumor agent and a pharmaceutical composition comprising a combination of a novel pyrrolopyrimidine compound and one or more other compounds having an antitumor effect.

Background Art

A protein group called ubiquitin-like protein (Ubl), typified by ubiquitin, covalently binds to corresponding activating enzyme E1 and transferase E2 to be added to a target protein through a covalent bond, thereby exerting an influence on various characteristics such as enzyme activity, stability, intracellular localization, and the like of the target protein (Non-patent Literature 1).

Nedd8, a kind of Ubl, is activated by APPBP1-UBA3 heterodimer, which is a Nedd8-activating enzyme (NAE), in an ATP-dependent manner. The activated Nedd8 is subsequently transferred to E2 (Ubc12), and is then added to a series of target proteins called cullin. The conjugation of Nedd8 to a target protein is called neddylation. Neddylation with respect to cullin promotes the activity (ability to add ubiquitin to a ligase substrate) of cullin-RING ligases (CRL), which function by forming a complex with cullin family protein and adaptor protein. The protein group ubiquitinated by CRL is degraded in the proteasome. Many proteins are known as CRL substrates that regulate cell cycles and conduct intracellular signal transduction, and that are reported to have decreased expression in tumors; examples of such proteins include p27, p21, and phosphorylated Iκ-B (Non-patent Literature 2 and 3). More specifically, NAE, through Nedd8 activation, contributes to tumor cell growth and survival by facilitating ubiquitination and degradation by proteasome of the CRL substrate protein group.

Because of the physiological function of NAE, an NAE inhibitor has a characteristic property of simultaneously affecting a plurality of signaling pathways involved in the survival and growth of tumors. Thus, NAE inhibitors are expected to serve as a therapeutic agent having broad and effective antitumor effect potentiation action for other compounds having an antitumor effect. N-[(1S)-1-indanyl]-7-[(1R)-3α-hydroxy-4α-(sulfamoyloxymethyl)cyclopentyl]-7H-pyrrolo[2,3-d]pyrimidin-4-amine (hereinafter referred to as "MLN4924") and the like have been known as compounds that inhibit the Nedd8-activating function of NAE (Patent Literature 1). MLN4924 is a compound having a pyrrolopyrimidine skeleton, and is characterized by having a substituted amino group at 4-position. MLN4924 causes accumulation of the CRL substrate protein group through neddylation inhibition, thereby inducing cell growth arrest and apoptosis (Non-patent Literature 4). Currently, the development of MLN4924 as an antitumor agent has been advanced (Patent Literature 2); and, in addition to the use of MLN4924 alone, tests using a combination of MLN4924 and various other anticancer drugs have also been conducted (Non-patent Literature 5 and 6). However, a great deal of the MLN4924 administered is transferred to red blood cells in the blood; thus, an issue of decrease in plasma concentration from the concentration required for ensuring the original medicinal effects of MLN4924 has been identified (Non-patent Literature 7). Further, since MLN4924 inhibits the activity of carbonic anhydrase II, which is highly expressed even in normal organs, such as red blood cells, kidneys, brain, and eyes, there is a concern that MLN4924 induces side effects, specifically, electrolyte abnormality, hypotonia bulbi, metabolic acidosis, polyuria, urinary calculus, and dysesthesia (Non-patent Literature 8). Under such circumstances, there is a demand for a new agent for potentiating the antitumor effect of other compounds having an antitumor effect through NAE inhibition, the agent having NAE inhibitory activity, but having smaller carbonic anhydrase II inhibitory activity.

Citation List

Patent Literature

PTL 1: WO2006084281

PTL 2: WO2012061551

Non-patent Literature

NPL 1: Nature Rev Mol Cell Biol, 2009, 10 (5): 319-331.
NPL 2: Genes & Cancer, 20101; 1 (7): 690-699
NPL 3: Journal of Cellular Physiology, 2000, 183, 10-17
NPL 4: Nature, 2009, 9; 458 (7239): 732-6
NPL 5: Mol Cancer Ther, 2014, 13 (6); 1-11
NPL 6: Mol Cancer Ther, 2012, 11 (4): 942-951
NPL 7: 9th International ISSX Meeting Abstract, p. 108
NPL 8: Israel Medical Association Journal, 2003, 5, April, 260-263

Summary of Invention

Technical Problem

There are a wide variety of antitumor agents. They are roughly classified into alkylating agents, platinum-based antitumor agents, antimetabolites, topoisomerase inhibitors, microtubule inhibitors, antitumor antibiotics, molecular target drugs, and the like. In recent years, the combined use of multiple drugs, instead of the administration of a single antitumor agent, has been widely prevalent. However, antagonism may be exhibited depending on the combination of antitumor agents, and it is unknown what combination of antitumor agents potentiates their antitumor effects. Further, it is unpredictable whether the antitumor effects alone are potentiated, without the potentiation of toxicity.

Solution to Problem

The present inventors conducted extensive research on compounds having a pyrrolopyrimidine skeleton, and found that a compound represented by Formula (A) below, which is characterized by comprising a vinylene group, an ethynylene group, an arylene group, or a heteroarylene group at 5-position ($R_3$ in Formula (A)) of the pyrrolopyrimidine skeleton, has excellent antitumor effect potentiation action, without potentiation of toxicity, in combined use with one or more other compounds having an antitumor effect. With this finding, the inventors completed the present invention.

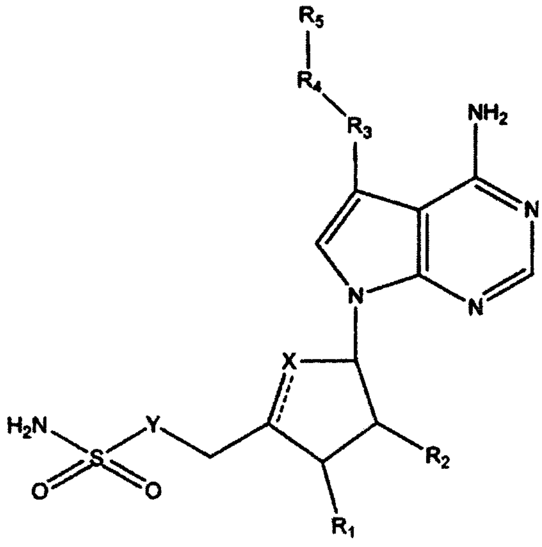

(A)

wherein:
------
is a single bond or a double bond;

X is -O-, $-CH_2-$, or -CH=;
Y is -NH- or -O-;
$R_1$ is hydrogen, fluorine, a hydroxy group, a cyano group, or an amino group;
$R_2$ is hydrogen, fluorine, a hydroxy group, a cyano group, or an amino group;
$R_3$ is a vinylene group, an ethynylene group, a C6-C14 arylene group, or a monocyclic or bicyclic heteroarylene group having at least one heteroatom selected from the group consisting of N, S, and O;
$R_4$ is a bond, a methylene group, or a C3-C7 cycloalkylidene group;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$;
$R_6$ is
halogen,
a hydroxy group,
a cyano group,
a C1-C6 alkyl group that may be substituted with one or more phenoxy groups,
a carbamoyl group,
a C1-C6 alkoxycarbonyl group,
a monocyclic or bicyclic unsaturated heterocycloalkyl group having at least one heteroatom selected from the group consisting of N, S, and O,
a monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group, an amino group,
a mono- or di-(C1-C4 alkyl) amino group that may be substituted with one or more hydroxy groups or phenyl groups,
a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
a benzyloxy group that may be substituted with one or more carbamoyl groups,
a C1-C6 alkylthio group,
a C1-C6 alkylsulfonyl group, or
an aminosulfonyl group,
when a plurality of $R_6$ are present, the plurality of $R_6$ may be the same or different.

Thus, the present invention provides the following items.

Item 1. An antitumor effect potentiator for one or more other compounds having an antitumor effect, the antitumor effect potentiator comprising a compound represented by Formula (A) above or a salt thereof.
Item 2. The antitumor effect potentiator according to Item 1, wherein in Formula (A), $R_1$ is hydrogen, fluorine, or a hydroxy group;

$R_2$ is hydrogen, fluorine, or a hydroxy group; and
$R_3$ is an ethynylene group, or a monocyclic or bicyclic heteroarylene group having 1 to 4 of at least one kind of heteroatom selected from the group consisting of N, S, and O.

Item 3. The antitumor effect potentiator according to Item 1 or 2, wherein in Formula (A), $R_1$ is a hydroxy group;

$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$; and

$R_6$ is halogen; a hydroxy group; a cyano group; a C1-C6 alkyl group that may be substituted with one or more phenoxy groups; a carbamoyl group; a C1-C6 alkoxycarbonyl group; a monocyclic or bicyclic unsaturated heterocycloalkyl group having at least one heteroatom selected from the group consisting of N, S, and O; a monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group; an amino group; a mono- or di-(C1-C4 alkyl) amino group that may be substituted with one or more

hydroxy groups or phenyl groups; a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O; a benzyloxy group that may be substituted with one or more carbamoyl groups; a C1-C4 alkylthio group; a C1-C4 alkylsulfonyl group; or an aminosulfonyl group (when a plurality of $R_6$ are present, the plurality of $R_6$ may be the same or different) .

Item 4. The antitumor effect potentiator according to any one of Items 1 to 3, wherein, in Formula (A), $R_1$ is a hydroxy group;

$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$; and

$R_6$ is fluorine; chlorine; a hydroxy group; a cyano group; a C1-C6 alkyl group that may be substituted with one or more phenoxy groups; a carbamoyl group; a C1-C6 alkoxycarbonyl group; a pyridinyl group that may have at least one member selected from the group consisting of halogen, hydroxy group and C1-C4 alkyl group as a substituent; an azetidinyl group; a hydroxyazetidinyl group; a thiomorpholinyl group; a dioxidothiomorpholinyl group; a methylpiperazinyl group; a hydroxypiperidinyl group; an oxopiperidinyl group; a piperidinyl group; a hydroxypyrrolidinyl group; an oxopyrrolidinyl group; a pyrrolidinyl group; a carboxylpyrrolidinyl group; a fluoropyrrolidinyl group; a morpholinyl group; a 9-oxa-3-azabicyclo[3.3.1]nonan-3-yl group; a 3-oxa-8-azabicyclo[3.2.1]octan-8-yl group; an amino group; a methylamino group; an ethylamino group; an isopropylamino group; a hydroxyethylamino group; a dimethylamino group; a phenylmethylamino group; a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O; a benzyloxy group that may be substituted with one or more carbamoyl groups; a C1-C4 alkylthio group; a C1-C4 alkylsulfonyl group; or an aminosulfonyl group (when a plurality of $R_6$ are present, the plurality of $R_6$ may be the same or different).

Item 5. The antitumor effect potentiator according to any one of Items 1 to 4, wherein, in Formula (A), $R_1$ is a hydroxy group;

$R_2$ is a hydroxy group;
$R_3$ is an ethynylene group;
$R_4$ is a bond;
$R_5$ is a C6-C10 unsaturated cycloalkyl group that may have one or more $R_6$; or a monocyclic or bicyclic unsaturated heterocycloalkyl group that has at least one heteroatom selected from the group consisting of N, S, and O, and that may have one or more $R_6$; and

$R_6$ is fluorine; chlorine; a hydroxy group; a cyano group; a methyl group; a 3-fluoropyrrolidinyl group; a morpholinyl group; a thiomorpholinyl group; a 3-hydroxyazetidinyl group; an azetidinyl group; an amino group; a N-methylamino group; a C1-C6 alkoxy group that may be substituted with one or more of either halogen or C3-C7 saturated cycloalkyl group; or a C1-C4 alkylthio group (when a plurality of $R_6$ are present, the plurality of $R_6$ may be the same or different).

Item 6. The antitumor effect potentiator according to any one of Items 1 to 5, wherein in Formula (A), Y is -NH-;

$R_1$ is a hydroxy group;
$R_2$ is a hydroxy group;
$R_3$ is an ethynylene group;
$R_4$ is a bond;
$R_5$ is a phenyl group or a naphthyl group that may have one or more $R_6$; or a monocyclic or bicyclic unsaturated heterocycloalkyl group that has at least one heteroatom selected from the group consisting of N, S, and O, and that may have one or more $R_6$;

$R_6$ is fluorine; a methyl group; 3-fluoropyrrolidinyl; 3-hydroxyazetidinyl; azetidinyl; an amino group; a N-methylamino group; a C1-C6 alkoxy group that may have one or more cyclopropyl groups; or a C1-C4 alkylthio group (when a plurality of $R_6$ are present, the plurality of $R_6$ may be the same or different) .

Item 7. The antitumor effect potentiator according to any one of Items 1 to 6, wherein the compound represented by Formula (A) or a salt thereof is at least one member selected from the group consisting of: 4-amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-(4-amino-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-(methylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-[(3R)-3-fluoropyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-4,6-difluoro-phenyl)ethynyl]pyrrolo[2, 3-d]pyrimidine;
4-amino-5-[2-[2,6-difluoro-4-(3-hydroxyazetidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[4-(azetidin-1-yl)-2,6-difluoro-phenyl]ethynyl]-7-[(2R,3R,4S,5R)-3,4-dihydroxy-5-[(sulfamoylamino)methyl]cyclopentyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-6-fluoro-phenyl)ethynyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-fluoro-6-propoxy-phenyl)ethynyl]pyrrolo[2,3-d]pyrimidine;
8-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine;
4-amino-7-[(2R,3R,4S,5R)-3,4-dihydroxy-5-[(sulfamoylamino)methyl]tetrahydrofuran-2-yl]-5-[2-(2-ethylsulfanyl-6-fluoro-phenyl)ethynyl]pyrrolo[2,3-d]pyrimidine;
4-amino-5-[2-[2-(cyclopropylmethoxy)-6-fluoro-phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(1R,2S,3R,4R)-2,3-dihydroxy-4-[(sulfamoylamino)methyl]cyclopentyl]-5-[2-(2-fluoro-6-methylsulfanyl-phenyl)ethynyl]pyrrolo[2,3-d]pyrimidine;
8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine;
4-amino-7-[(1R,4R,5S)-4,5-dihydroxy-3-[(sulfamoylamino)methyl]cyclopent-2-en-1-yl]-5-[2-(2-ethoxy-6-fluoro-phenyl)ethynyl]pyrrolo[2,3-d]pyrimidine; and
4-amino-7-[(1R,4R,5S)-4,5-dihydroxy-3-[(sulfamoylamino)methyl]cyclopent-2-en-1-yl]-5-[2-(2-fluoro-6-methylsulfanyl-phenyl)ethynyl]pyrrolo[2,3-d]pyrimidine; and salts of these compounds.

Item 8. The antitumor effect potentiator according to any one of Items 1 to 7, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

Item 9. The antitumor effect potentiator according to any one of Items 1 to 7, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

Item 10. An antitumor agent comprising a combination of the compound represented by Formula (A) or a salt thereof according to any one of Items 1 to 7, and one or more other compounds having an antitumor effect.

Item 11. The antitumor agent according to Item 10, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

Item 12. The antitumor agent according to Item 10, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

Item 13. Use of the compound represented by Formula (A) or a salt thereof according to any one of Items 1 to 7 for potentiating the antitumor effect of one or more other compounds having an antitumor effect.

Item 14. Use of the compound represented by Formula (A) or a salt thereof according to any one of Items 1 to 7 for producing an antitumor effect potentiator for one or more other compounds having an antitumor effect.

Item 15. Use of the compound represented by Formula (A) or a salt thereof according to any one of Items 1 to 7 for producing an antitumor agent comprising a combination of the compound or a salt thereof (A) and one or more other compounds having an antitumor effect.

Item 16. The use according to any one of Items 13 to 15, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

Item 17. The use according to any one of Items 13 to 15, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

Item 18. A product as a combined preparation for use simultaneously, sequentially, or at one or more intervals upon prevention and/or treatment of a tumor, the product comprising the compound represented by Formula (A) or a salt thereof according to any one of Items 1 to 7 and one or more other compounds having an antitumor effect.

Item 19. The compound represented by Formula (A) or a salt thereof according to any one of Items 1 to 7 for potentiating the antitumor effect of one or more other compounds having an antitumor effect.

Item 20. A pharmaceutical composition comprising the compound represented by Formula (A) or a salt thereof according to any one of Items 1 to 7 and a pharmaceutical carrier, the pharmaceutical composition being for potentiating the antitumor effect of one or more other compounds having an antitumor effect.

Item 21. An antitumor composition comprising the compound represented by Formula (A) or a salt thereof according to any one of Items 1 to 7, and one or more other compounds having an antitumor effect.

Item 22. A method for treating a tumor, comprising administering to a patient the compound represented by Formula (A) or a salt thereof according to any one of Items 1 to 7, and one or more other compounds having an antitumor effect, in combination.

Item 23. A method for potentiating the antitumor effect of one or more other compounds having an antitumor effect, the method comprising administering to a patient the compound represented by Formula (A) or a salt thereof according to any one of Items 1 to 7, and the one or more other compounds having an antitumor effect, in combination.

Item 24. A combination of the compound represented by Formula (A) or a salt thereof according to any one of Items 1 to 7, and one or more other compounds having an antitumor effect, for tumor treatment.

## Advantageous Effects of Invention

An antitumor agent comprising a pyrrolopyrimidine compound represented by Formula (A) above potentiates other compounds having an antitumor effect when used in combination with various antitumor agents comprising such other compounds. Thus, the present invention provides an antitumor effect potentiator for one or more other compounds having an antitumor effect, the antitumor effect potentiator comprising a pyrrolopyrimidine compound represented by Formula (A) above as an active ingredient; and an antitumor agent comprising a combination of a pyrrolopyrimidine compound represented by Formula (A) above and one or more other compounds having an antitumor effect.

Therefore, the antitumor effect potentiator for one or more other compounds having an antitumor effect according to the present invention, which comprises a pyrrolopyrimidine compound represented by Formula (A) above; and the antitumor agent comprising a combination of a compound represented by Formula (A) above or a salt thereof and one or more other compounds having an antitumor effect according to the present invention, are extremely useful for the prevention and/or treatment of tumors.

Fig. 1 is a graph showing tumor volume measurement values of the control group, the group administered with the reference example compound, the group administered with bortezomib, and the group administered with the combination.

Fig. 2 is a graph showing tumor volume measurement values of the control group, the group administered with the reference example compound, the group administered with gilteritinib, and the group administered with the combination.

Fig. 3 is a graph showing tumor volume measurement values of the control group, the group administered with the reference example compound, the group administered with doxorubicin, and the group administered with the combination.

Fig. 4 is a graph showing tumor volume measurement values of the control group, the group administered with the reference example compound, the group administered with rituximab, and the group administered with the combination.

Fig. 5 is a graph showing tumor volume measurement values of the control group, the group administered with the reference example compound, the group administered with rituximab, and the group administered with the combination.

Fig. 6 is a graph showing tumor volume measurement values of the control group, the group administered with the reference example compound, the group administered with azacytidine, and the group administered with the combination.

Description of Embodiments

The pyrrolopyrimidine compound represented by Formula (A) above has an action of potentiating the antitumor effect of one or more other compounds having an antitumor effect.

An embodiment of the present invention provides an antitumor effect potentiator for one or more other compounds having an antitumor effect, the antitumor effect potentiator comprising the compound represented by Formula (A) or a salt thereof.

The forms of "other compounds having an antitumor effect" are not limited as long as they are compounds that are different from the compound represented by Formula (A), and have an antitumor effect. Examples include low-molecular compounds or salts thereof, antibodies, proteins, nucleic acids (e.g., aptamers, antisense molecules, and siRNA), and the like.

The mechanism of action of "other compounds having an antitumor effect" that can be used in the present invention is not particularly limited. Usable compounds include one or more antitumor agents selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents (platinum complexes), microtubule inhibitors, alkylating agents, and anthracycline-based antitumor agents. The "other compounds having an antitumor effect" also include salts thereof.

Examples of kinase inhibitors include tyrosine kinase inhibitors and serine-threonine kinase inhibitors.

Examples of tyrosine kinase inhibitors include receptor tyrosine kinase inhibitors and non-receptor tyrosine kinase inhibitors; receptor tyrosine kinase inhibitors are preferable.

Examples of receptor tyrosine kinase inhibitors include ALK inhibitors, EGFR family inhibitors, FLT3 inhibitors, VEGFR inhibitors, c-kit inhibitors, multikinase inhibitors that inhibit multiple receptor tyrosine kinases, and the like.

Examples of ALK inhibitors include alectinib, crizotinib, and the like.

Examples of EGFR family inhibitors include EGFR inhibitors and HER2 inhibitors.

Examples of EGFR inhibitors include afatinib, erlotinib, gefitinib, osimertinib (which is also referred to as AZD9291), and the like.

Examples of HER2 inhibitors include lapatinib, ARRY380, and the like.

Examples of FLT3 inhibitors include quizartinib, gilteritinib, ASP2215, crenolanib, and the like.

Examples of VEGFR inhibitors include lenvatinib, pazopanib, sorafenib, sunitinib, regorafenib, and the like.

Examples of c-kit inhibitors include masitinib, and the like.

Examples of non-receptor tyrosine kinase inhibitors include BCR-ABL inhibitors, BTK inhibitors, and Janus kinase inhibitors (which are also referred to as "JAK kinase inhibitors").

Examples of BCR-ABL inhibitors include dasatinib, imatinib, ponatinib, and the like.

Examples of BTK inhibitors include ibrutinib and the like.

Examples of Janus kinase inhibitors include ruxolitinib and the like.

Examples of serine-threonine kinase inhibitors include PI3K inhibitors, Akt inhibitors, mTOR inhibitors, and PLK inhibitors.

Examples of PI3K inhibitors include duvelisib, idelalisib, and the like.

Examples of AKT inhibitors include AZD5363, MK2206, and the like.

Examples of mTOR inhibitors include everolimus, rapamycin, and the like.

Examples of PLK inhibitors include volasertib and the like.

Examples of apoptosis inducers include BCL-2 inhibitors, IAP antagonists, and the like.

Examples of BCL-2 inhibitors include ABT-199, ABT-737, and the like.
Examples of IAP antagonists include GDC-0152 and the like.
Examples of nuclear receptor modulators include HDAC inhibitors and steroids.
Examples of HDAC inhibitors include panobinostat, vorinostat (trade name: Zolinda, SAHA) and the like.
Examples of steroids include dexamethasone, prednisolone, and the like.
Examples of immunomodulators include lenalidomide, pomalidomide, thalidomide, rituximab, and the like.
Examples of nuclear export signal inhibitors include Exportin-1 inhibitors.
Examples of Exportin-1 inhibitors include KPT-330 and the like.
Examples of proteasome modulators include bortezomib, carfilzomib, ixazomib, and the like.
Examples of DNA-damaging agents include PARP inhibitors. Specific examples include olaparib and the like.
Examples of antimetabolites include fluorouracil, tegafur, a combination drug of tegafur and uracil, a combination drug of tegafur, gimeracil, and oteracil potassium, Furtulon, capecitabine, gemcitabine, cytarabine, 6-mercaptopurine, methotrexate, pemetrexed, fludarabine, trifluridine, a combination drug of trifluridine and tipiracil hydrochloride, azacitidine, and the like.
Examples of platinum-based antitumor agents include cisplatin, carboplatin, oxaliplatin, nedaplatin, and the like.
Examples of microtubule inhibitors include vinblastine, vincristine, vinorelbine, eribulin, paclitaxel, docetaxel, albumin-bound paclitaxel, and the like.
Examples of alkylating agents include trabectedin, ifosfamide, bendamustine, cyclophosphamide, dacarbazine, and the like.
Examples of anthracycline-based antitumor agents include daunorubicin, doxorubicin, pixantrone, and the like.
In one preferred embodiment, the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.
Of these, in order to obtain potentiation action that is equal to or greater than an slight synergism, bortezomib, carfilzomib, alectinib, crizotinib, afatinib, erlotinib, gefitinib, lapatinib, ARRY380, imatinib, quizartinib, lenvatinib, pazopanib, masitinib, ponatinib, ruxolitinib, everolimus, rapamycin, AZD5363, MK2206, idelalisib, duvelisib, olaparib, prednisolone, dexamethasone, lenalidomide, pomalidomide, thalidomide, KPT-330, ibrutinib, ABT-199, fluorouracil, gemcitabine, cytarabine, 6-mercaptopurine, pemetrexed, cisplatin, carboplatin, oxaliplatin, ifosfamide, doxorubicin, pixantrone, rituximab, azacitidine, and GDC-0152 are more preferable. In order to obtain potentiation action that is equal to or greater than a moderate synergism, bortezomib, carfilzomib, alectinib, afatinib, erlotinib, gefitinib, lapatinib, ARRY380, imatinib, quizartinib, lenvatinib, masitinib, ponatinib, ruxolitinib, everolimus, rapamycin, AZD5363, MK2206, idelalisib, duvelisib, olaparib, prednisolone, dexamethasone, lenalidomide, pomalidomide, thalidomide, KPT-330, ibrutinib, ABT-199, fluorouracil, gemcitabine, cytarabine, 6-mercaptopurine, pemetrexed, cisplatin, oxaliplatin, ifosfamide, doxorubicin, pixantrone, rituximab, azacitidine, and GDC-0152 are more preferable. In order to obtain potentiation action that is equal to or greater than a synergism, bortezomib, carfilzomib, alectinib, afatinib, erlotinib, gefitinib, lapatinib, ARRY380, imatinib, quizartinib, lenvatinib, masitinib, ponatinib, everolimus, rapamycin, AZD5363, MK2206, idelalisib, duvelisib, olaparib, prednisolone, dexamethasone, thalidomide, ABT-199, fluorouracil, gemcitabine, cytarabine, oxaliplatin, ifosfamide, rituximab, azacitidine, and GDC-0152 are further preferable. In order to obtain potentiation action that is equal to or greater than a strong synergism, lapatinib is particularly preferable.
"Additive" "slight synergism" "moderate synergism" "synergism" "strong synergism" and "very strong synergism" as used herein are known criteria commonly used in determining the presence or absence of the synergistic effect of a combined use (Pharmacol Rev., 2006; 58(3):621-81, BMC Complement Altern Med., 2013; 13:212, Anticancer Res, 2005; 25(3B):1909-17). More specifically, although it is not particularly limited, the presence or absence of the synergistic effect can be determined according to the combination index (CI) value, as described later in Example 1.
The "other compounds having an antitumor effect" of the present invention can be individually produced according to known methods, or may be commercially available products.
Each group shown in the aforementioned Formula (A) is specifically as follows.
In Formula (A), the "C6-C14 arylene group" represented by $R_3$ is a monocyclic or polycyclic bivalent aromatic hydrocarbon group having 6 to 14 carbon atoms. More specifically, examples of the C6-C14 arylene group include phenylene, naphthylene, and tetrahydronaphthylene. Phenylene and naphthylene are preferable.
In Formula (A), the "monocyclic or bicyclic heteroarylene group having at least one heteroatom selected from the group consisting of N, S, and O" represented by $R_3$ is a monocyclic or bicyclic heteroarylene group having 1 to 3 of at least one kind of heteroatom selected from the group consisting of N, S, and O. Specific examples include thiazolylene,

pyrazolylene, imidazolylene, thienylene, furylene, pyrrolylene, oxazolylene, isoxazolylene, isothiazolylene, thiadiazolylene, triazolylene, tetrazolylene, pyridylene, pyrazylene, pyrimidinylene, pyridazinylene, indolylene, isoindolylene, indazolylene, triazolopyridilene, benzimidazolylene, benzoxazolylene, benzothiazolylene, benzothienylene, benzofuranylene, purinylene, quinolylene, isoquinolylene, quinazolinylene, quinoxalylene, methylenedioxyphenylene, ethylenedioxyphenylene, dihydrobenzofuranylene, benzoxazinylene, dihydrobenzoxazinylene, chromanylene, thiochromanylene, 1,1-dioxythiochromanylene, dihydrobenzothienylene, and 1,1-dioxydihydrobenzothienylene. Preferable examples include a monocyclic heteroarylene group having 1 to 3 of at least one kind of heteroatom selected from the group consisting of N, S, and O. More preferable examples include a monocyclic 5-membered heteroarylene group having 1 or 2 of at least one kind of heteroatom selected from the group consisting of N, S, and O. Particularly preferable examples include thiazolylene, pyrazolylene, imidazolylene, thienylene, and oxazolylene.

In Formula (A), the "C3-C7 cycloalkylidene group" is a monocyclic saturated alkylidene group having 3 to 7 carbon atoms. Specific examples include the following.

Cyclopropylidene group

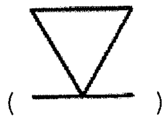,

cyclobutylidene group

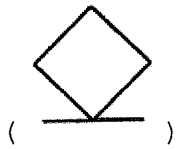,

cyclopentylidene group

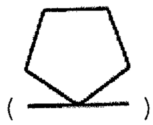,

cyclohexylidene group

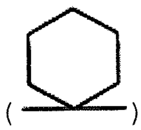,

and cycloheptylidene group

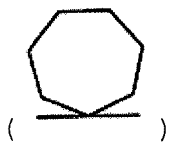

Preferable examples include a cyclopropylidene group.

In the present specification, the "cycloalkyl group" refers to a saturated or unsaturated monovalent cyclic hydrocarbon group. Unless otherwise specified, the term "cycloalkyl" encompasses monocyclic cycloalkyl, and polycyclic cycloalkyl such as bicyclic or tricyclic cycloalkyl.

In the present specification, the "heterocycloalkyl group" refers to a saturated or unsaturated monovalent heterocyclic group. Unless otherwise specified, the term "heterocycloalkyl" encompasses monocyclic heterocycloalkyl, and polycyclic heterocycloalkyl such as bicyclic or tricyclic heterocycloalkyl.

In Formula (A), the C3-C7 saturated cycloalkyl group of the "C3-C7 saturated cycloalkyl group that may have

one or more $R_6$" represented by $R_5$ refers to a cyclic saturated hydrocarbon group having 3 to 7 carbon atoms. Specific examples include cyclopropyl, cyclobutyl, cyclopentyl, cyclohexyl, and cycloheptyl. Preferable examples include cyclohexyl.

In Formula (A), the C6-C10 unsaturated cycloalkyl group of the "C6-C10 unsaturated cycloalkyl group that may have one or more $R_6$" represented by $R_5$ is a monocyclic or bicyclic unsaturated hydrocarbon group having 6 to 10 carbon atoms. Specific examples include phenyl, naphthyl, tetrahydronaphthyl, and 2,3-dihydroindenyl. Preferable examples include phenyl, naphthyl, and 2,3-dihydroindenyl.

In Formula (A), specific examples of the monocyclic or bicyclic unsaturated heterocycloalkyl group that has at least one heteroatom selected from the group consisting of N, S, and O of the "monocyclic or bicyclic unsaturated heterocycloalkyl group that has at least one heteroatom selected from the group consisting of N, S, and O, and that may have one or more $R_6$" represented by $R_5$ include hexamethyleneimino, imidazolyl, thienyl, furyl, pyrrolyl, oxazolyl, isooxazolyl, thiazolyl, isothiazolyl, pyrazolyl, triazolyl, tetrazolyl, pyridyl, pyrazyl, pyrimidinyl, pyridazinyl, indolyl, isoindolyl, indazolyl, methylenedioxy phenyl, ethylenedioxyphenyl, benzofuranyl, dihydrobenzofuranyl, benzimidazolyl, benzoxazolyl, benzothiazolyl, purinyl, quinolyl, isoquinolyl, quinazolinyl, quinoxalinyl, 1H-pyrazolo[4,3-b]pyridin-5-yl, 2,3-dihydro-1,4-benzoxazino, 1,1-dioxo-3,4-dihydro-2H-thiochromen-8-yl, 2,3-dihydrobenzothiophen-7-yl, and 1,1-dioxo-2,3-dihydrobenzothiophen-7-yl. Preferable examples include a monocyclic or bicyclic 5- to 10-membered unsaturated heterocycloalkyl group having 1 to 3 of at least one kind of heteroatom selected from the group consisting of N, S, and O. More preferable examples include thienyl, pyridyl, pyrazyl, quinolyl, isoquinolyl, 1H-pyrazolo[4,3-b]pyridin-5-yl, 2,3-dihydro-1,4-benzoxazino, 1,1-dioxo-3,4-dihydro-2H-thiochromen-8-yl, 2,3-dihydrobenzothiophen-7-yl, and 1,1-dioxo-2,3-dihydrobenzothiophen-7-yl.

In Formula (A), specific examples of the "halogen" represented by $R_6$ include fluorine, chlorine, bromine, and iodine. Preferable examples include fluorine and chlorine.

In Formula (A), the C1-C6 alkyl group of the "C1-C6 alkyl group that may be substituted with one or more phenoxy groups" represented by $R_6$ refers to a linear or branched alkyl group having 1 to 6 carbon atoms. Specific examples include methyl, ethyl, n-propyl, isopropyl, n-butyl, isobutyl, sec-butyl, tert-butyl, n-pentyl, and n-hexyl. Methyl is preferable.

In Formula (A), the C1-C6 alkoxy group of the "C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O" represented by $R_6$ refers to a linear or branched alkoxy group having 1 to 6 carbon atoms. Specific examples include methoxy, ethoxy, n-propoxy, isopropoxy, n-butoxy, isobutoxy, and tert-butoxy. Methoxy, ethoxy, n-propoxy, and isopropoxy are preferable.

In Formula (A), the halogen of the "C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O" represented by $R_6$ refers to the halogen listed above, and is preferably fluorine. The number of halogens as substituents is 1 to 3, preferably 2 or 3.

In Formula (A), the C3-C7 saturated cycloalkyl group of the "C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O" represented by $R_6$ refers to a saturated cycloalkyl group having 3 to 7 carbon atoms. Specific examples include cyclopropyl, cyclobutyl, cyclopentyl, cyclohexyl, and cycloheptyl. Cyclopropyl is preferable. The number of C3-C7 saturated cycloalkyl groups as substituents is preferably 1.

In Formula (A), the monocyclic or bicyclic unsaturated heterocycloalkyl group having at least one heteroatom selected from the group consisting of N, S, and O of the "C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O" represented by $R_6$ refers to the unsaturated heterocycloalkyl group described above. Preferable examples include a monocyclic unsaturated heterocycloalkyl group having 1 to 3 of at least one kind of heteroatom selected from the group consisting of N, S, and O. More preferable examples include a monocyclic 5- or 6-membered unsaturated heterocycloalkyl group having 1 or 2 of at least one kind of heteroatom selected from the group consisting of N, S, and O. Particularly preferable examples include pyrazolyl, triazolyl, and pyridyl. The number of unsaturated heterocycloalkyl groups as substituents is preferably 1.

In Formula (A), preferable examples of the "C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O" represented by $R_6$ include methoxy, difluoromethoxy, trifluoromethoxy, cyclopropylmethoxy, 3-pyridylmethoxy, pyrazol-1-ylmethoxy, ethoxy, 2,2,2-trifluoroethoxy, n-propoxy, and isopropoxy.

In Formula (A), preferable examples of the "benzyloxy group that may be substituted with one or more carbamoyl groups" represented by $R_6$ include benzyloxy and 3-carbamoyl benzyloxy.

In Formula (A), the mono- or di-(C1-C4 alkyl) amino group of the "mono- or di-(C1-C4 alkyl) amino group that

may be substituted with one or more hydroxy groups or phenyl groups" represented by $R_6$ refers to an amino group monosubstituted or disubstituted with a C1-C4 alkyl group among the above C1-C6 alkyl groups. Specific examples include methylamino, ethylamino, dimethylamino, diethylamino, methylethylamino, isopropylamino, butylmethylamino, and dimethylamino. Preferably examples include methylamino, ethylamino, dimethylamino, and isopropylamino.

In Formula (A), the "mono- or di-(C1-C4 alkyl) amino group that may be substituted with one or more hydroxy groups or phenyl groups" represented by $R_6$ is preferably methylamino, ethylamino, isopropylamino, hydroxyethylamino, dimethylamino, or phenylmethylamino (benzylamino).

In Formula (A), the "C1-C6 alkoxycarbonyl group" represented by $R_6$ refers to a carbonyl group substituted with the above alkoxy group. Specific examples include methoxycarbonyl, ethoxycarbonyl, n-propoxycarbonyl, isopropoxycarbonyl, 1-methylpropoxycarbonyl, n-butoxycarbonyl, isobutoxycarbonyl, tert-butoxycarbonyl, 2-methyl-butoxycarbonyl, neopentyloxycarbonyl, and pentan-2-yloxycarbonyl. Methoxycarbonyl is preferable.

In Formula (A), the "monocyclic or bicyclic unsaturated heterocycloalkyl group having at least one heteroatom selected from the group consisting of N, S, and O" represented by $R_6$ refers to the unsaturated heterocycloalkyl group described above, preferably a monocyclic 5- or 6-membered unsaturated heterocycloalkyl group having 1 or 2 of at least one kind of heteroatom selected from the group consisting of N, S, and O, and more preferably pyridyl.

In Formula (A), specific examples of the monocyclic or bicyclic saturated heterocycloalkyl group that has at least one heteroatom selected from the group consisting of N, S, and O of the "monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group" represented by $R_6$ include azetidinyl, pyrrolidinyl, piperidinyl, 2-oxo-1-pyrrolidinyl, 4-oxo-1-piperidinyl, piperazinyl, hexamethyleneimino, morpholino, thiomorpholino, 1,1-dioxo-thiomorpholino, homopiperazinyl, tetrahydrofuranyl, tetrahydropyranyl, 9-oxa-3-azabicyclo[3.3.1]nonan-3-yl, and 3-oxa-8-azabicyclo[3.2.1]octan-8-yl. Preferable examples include a monocyclic or bicyclic 5- to 10-membered saturated heterocycloalkyl group having 1 to 4 of at least one kind of heteroatom selected from the group consisting of N, S, and O. More preferable examples include azetidinyl, pyrrolidinyl, piperidinyl, piperazinyl, 2-oxo-1-pyrrolidinyl, 4-oxo-1-piperidinyl, morpholino, thiomorpholino, 1,1-dioxo-thiomorpholino, 9-oxa-3-azabicyclo[3.3.1]nonan-3-yl, and 3-oxa-8-azabicyclo[3.2.1]octan-8-yl.

In Formula (A), the halogen of the "monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group" represented by $R_6$ refers to the halogen listed above, preferably fluorine, chlorine, etc., and more preferably fluorine.

In Formula (A), examples of the C1-C6 alkyl group of the "monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group" represented by $R_6$ include, among the above alkyl groups, an alkyl group having 1 to 6 carbon atoms, preferably a methyl group.

In Formula (A), preferable examples of the "monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group" represented by $R_6$ include azetidinyl, 3-hydroxyazetidin-1-yl, pyrrolidinyl, 3-fluoropyrrolidin-1-yl, 3-hydroxypyrrolidin-1-yl, 3-carboxy-1-pyrrolidin-1-yl, piperidinyl, 4-oxo-1-piperidinyl, 3-hydroxy-1-piperidinyl, piperazinyl, 4-methylpiperazin-1-yl, 4-oxo-1-piperidinyl, morpholino, thiomorpholino, 1,1-dioxo-thiomorpholino, 9-oxa-3-azabicyclo[3.3.1]nonan-3-yl, and 3-oxa-8-azabicyclo[3.2.1]octan-8-yl.

In Formula (A), the "C1-C6 alkylthio group" represented by $R_6$ refers to a thio group having the above C1-C6 alkyl group, preferably a C1-C4 alkylthio group, more preferably a methylthio group or an ethylthio group.

In Formula (A), the "C1-C6 alkylsulfonyl group" represented by $R_6$ refers to a sulfonyl group having the above C1-C6 alkyl group, preferably a C1-C4 alkylsulfonyl group, more preferably a methylsulfonyl group or an ethylsulfonyl group.

Y in Formula (A) is -NH- or -O-, preferably -NH-.

$R_1$ in Formula (A) is hydrogen, fluorine, a hydroxy group, a cyano group or an amino group, preferably hydrogen, fluorine or a hydroxy group, further preferably a hydroxy group.

$R_2$ in Formula (A) is hydrogen, fluorine, a hydroxy group, a cyano group or an amino group, preferably hydrogen, fluorine, or a hydroxy group, further preferably hydrogen or a hydroxy group, particularly preferably a hydroxy group.

$R_3$ in Formula (A) is preferably an ethynylene group, or a monocyclic heteroarylene group having 2 of at least one kind of heteroatom selected from the group consisting of N, S, and O, more preferably an ethynylene group.

$R_4$ in Formula (A) is preferably a bond.

$R_5$ in Formula (A) is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$. Preferably, $R_5$ is a phenyl group or a naphthyl group that may be substituted with one or more $R_6$, or a monocyclic or bicyclic unsaturated heterocycloalkyl group that has at least one heteroatom selected from the group consisting of N, S, and O,

and that may have one or more $R_6$. More preferably, $R_5$ is a unsaturated heterocycloalkyl group or a phenyl group that may be substituted with one or more $R_6$, further preferably a phenyl group, thienyl group, pyridyl group, pyrazyl group, quinolyl group, isoquinolyl group, 1H-pyrazolo[4,3-b]pyridin-5-yl group, 2,3-dihydro-1,4-benzoxazino group, 1,1-dioxo-3,4-dihydro-2H-thiochromen-8-yl group, 2,3-dihydrobenzothiophen-7-yl group, or 1,1-dioxo-2,3-dihydrobenzothiophen-7-yl group that may be substituted with one or more $R_6$.

When $R_5$ is an unsaturated heterocycloalkyl group, $R_5$ is preferably a 2,3-dihydro-1,4-benzoxazinyl group, a 3,4-dihydro-2H-thiochromen-8-yl group, a 2,3-dihydrobenzothiophen-7-yl group or the like, more preferably a 2,3-dihydro-1,4-benzoxazinyl group. The unsaturated heterocycloalkyl groups listed above may be substituted with one or more $R_6$.

When $R_5$ has $R_6$, the number of $R_6$ is, for example, 1 to 5, preferably 1 to 3.

When $R_5$ has one or more $R_6$, $R_6$ is any of:

(i-1) halogen,
(i-2) hydroxy group,
(i-3) cyano group,
(i-4) C1-C6 alkyl group that may be substituted with one or more phenoxy groups,
(i-5) carbamoyl group,
(i-6) C1-C6 alkoxycarbonyl group,
(i-7) monocyclic or bicyclic unsaturated heterocycloalkyl group having at least one heteroatom selected from the group consisting of N, S, and O,
(i-8) monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group,
(i-9) amino group,
(i-10) mono- or di-(C1-C4 alkyl) amino group that may be substituted with one or more hydroxy groups or phenyl groups,
(i-11) C1-6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
(i-12) benzyloxy group that may be substituted with one or more carbamoyl groups,
(i-13) C1-C6 alkylthio group,
(i-14) C1-C6 alkylsulfonyl group, or
(i-15) aminosulfonyl group.

When $R_5$ has one or more $R_6$, $R_6$ in Formula (A) is more preferably any of:

(ii-1) halogen,
(ii-2) hydroxy group,
(ii-3) cyano group,
(ii-4) C1-C6 alkyl group that may be substituted with one or more phenoxy groups,
(ii-5) carbamoyl group,
(ii-6) C1-C6 alkoxycarbonyl group,
(ii-7) monocyclic 5- or 6-membered unsaturated heterocycloalkyl group having 1 or 2 of at least one kind of heteroatom selected from the group consisting of N, S, and O,
(ii-8) monocyclic or bicyclic 5- to 10-membered saturated heterocycloalkyl group that has 1 to 4 of at least one kind of heteroatom selected from the group consisting of N, S, and O, and that may be substituted with one or more of halogen, hydroxy group, carboxyl group, or C1-C6 alkyl group,
(ii-9) amino group,
(ii-10) mono- or di-(C1-C4 alkyl) amino group that may be substituted with one or more hydroxy groups or phenyl groups,
(ii-11) C1-6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
(ii-12) benzyloxy group that may be substituted with one or more carbamoyl groups,
(ii-13) C1-C4 alkylthio group,
(ii-14) C1-C4 alkylsulfonyl group, or
(ii-15) aminosulfonyl group.

When $R_5$ has one or more $R_6$, $R_6$ in Formula (A) is more preferably any of:

(iii-1) fluorine, chlorine
(iii-2) hydroxy group,
(iii-3) cyano group,
(iii-4) C1-C6 alkyl group that may be substituted with one or more phenoxy groups,
(iii-5) carbamoyl group,
(iii-6) C1-C6 alkoxycarbonyl group,
(iii-7) 3- to 7-membered saturated heterocycloalkyl group, in particular, 5- to 7-membered saturated heterocycloalkyl group,
(iii-8) azetidinyl group, hydroxyazetidinyl group, thiomorpholinyl group, dioxidothiomorpholinyl group, methylpiperazinyl group, hydroxypiperidinyl group, oxopiperidinyl group, piperidinyl group, hydroxypyrrolidinyl group, oxopyrrolidinyl group, pyrrolidinyl group, carboxylpyrrolidinyl group, fluoropyrrolidinyl group, morpholinyl group, 9-oxa-3-azabicyclo[3.3.1]nonan-3-yl group, 3-oxa-8-azabicyclo[3.2.1]octan-8-yl group,
(iii-9) amino group,
(iii-10) methylamino group, ethylamino group, isopropylamino group, hydroxyethylamino group, dimethylamino group, phenylmethylamino group,
(iii-11) C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
(iii-12) benzyloxy group that may be substituted with one or more carbamoyl groups,
(iii-13) C1-C4 alkylthio group,
(iii-14) C1-C4 alkylsulfonyl group, or
(iii-15) aminosulfonyl group.

When $R_5$ has one or more $R_6$, $R_6$ in Formula (A) is more preferably any of:

(iv-1) fluorine, chlorine
(iv-2) hydroxy group,
(iv-3) cyano group,
(iv-4) methyl group,
(iv-7) 3-fluoropyrrolidinyl group, morpholinyl group, thiomorpholinyl group, 3-hydroxyazetidinyl group, azetidinyl group,
(iv-8) azetidinyl group, pyrrolidinyl group, piperidinyl group, piperazinyl group, 2-oxo-1-pyrrolidinyl group, 4-oxo-1-piperidinyl group, morpholino group, thiomorpholino group, 1,1-dioxo-thiomorpholino group, 9-oxa-3-azabicyclo[3,3,1]nonan-3-yl group, and 3-oxa-8-azabicyclo[3,2,1]octan-8-yl group,
(iv-9) amino group,
(iv-10) methylamino group,
(iv-11) C1-C6 alkoxy group that may be substituted with one or more of either halogen or C3-C7 saturated cycloalkyl group, or
(iv-13) C1-C4 alkylthio group.

When $R_5$ has one or more $R_6$, $R_6$ in Formula (A) is more preferably any of:

(v-1) fluorine,
(v-4) methyl group,
(v-7) saturated heterocycloalkyl group selected from the group consisting of 3-fluoropyrrolidinyl, 3-hydroxyazetidinyl, and azetidinyl,
(v-9) amino group,
(v-10) methylamino group,
(v-11) C1-C6 alkoxy group that may have one or more cyclopropyl groups, or
(v-13) C1-C4 alkylthio group.

When a plurality of $R_6$ are present, the plurality of $R_6$ may be the same or different.
The compound represented by Formula (A) is preferably a compound having high enzyme inhibitory activity against NAE that can generally be tested by a known method, more preferably, a compound such that the concentration ($IC_{50}$) of the compound by which 50% of the enzyme can be inhibited is 0.03 $\mu$M or less, further preferably a compound having an $IC_{50}$ of 0.01 $\mu$M or less, particularly preferably a compound having an $IC_{50}$ of 0.003 $\mu$M or less.

The compound represented by Formula (A) is preferably a compound having high tumor growth inhibitory activity that can generally be tested by a known method, more preferably a compound such that the concentration ($IC_{50}$) of the compound by which 50% of tumor growth can be inhibited is 0.01 $\mu$M or less, particularly preferably a compound having an $IC_{50}$ of 0.003 $\mu$M or less.

Further preferable examples of the compound include a compound wherein in Formula (A),
$R_1$ is hydrogen, fluorine, or a hydroxy group;
$R_2$ is hydrogen, fluorine, or a hydroxy group; and
$R_3$ is an ethynylene group, or a monocyclic or bicyclic heteroarylene group having 1 to 4 of at least one kind of heteroatom selected from the group consisting of N, S, and O.

Further preferable examples of the compound include a compound wherein in Formula (A),
$R_1$ is a hydroxy group;
$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$; a C6-C10 unsaturated cycloalkyl group that may have one or more $R_6$; or a monocyclic or bicyclic unsaturated heterocycloalkyl group that has at least one heteroatom selected from the group consisting of N, S, and O, and that may be substituted with one or more $R_6$;
$R_6$ is
halogen,
a hydroxy group,
a cyano group,
a C1-C6 alkyl group that may be substituted with one or more phenoxy groups,
a carbamoyl group,
a C1-C6 alkoxycarbonyl group,
a monocyclic or bicyclic unsaturated heterocycloalkyl group having at least one heteroatom selected from the group consisting of N, S, and O,
a monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group, an amino group,
a mono- or di-(C1-C4 alkyl) amino group that may be substituted with one or more hydroxy groups or phenyl groups,
a C1-6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
a benzyloxy group that may be substituted with one or more carbamoyl groups,
a C1-C4 alkylthio group,
a C1-C4 alkylsulfonyl group, or
an aminosulfonyl group
(when a plurality of $R_6$ are present, the plurality of $R_6$ may be the same or different).

Further preferable examples of the compound include a compound wherein, in Formula (A), $R_1$ is a hydroxy group;
$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$; a C6-C10 unsaturated cycloalkyl group that may have one or more $R_6$; or a monocyclic or bicyclic unsaturated heterocycloalkyl group that has at least one heteroatom selected from the group consisting of N, S, and O, and that may have one or more $R_6$;
$R_6$ is fluorine,
chlorine,
a hydroxy group,
a cyano group,
a C1-C6 alkyl group that may be substituted with one or more phenoxy groups,
a carbamoyl group,
a C1-C6 alkoxycarbonyl group,
a pyridinyl group that may have at least one substituent selected from the group consisting of halogen, hydroxy group, and C1-C4 alkyl group,
a saturated heterocycloalkyl group selected from the group consisting of azetidinyl, hydroxyazetidinyl, thiomorpholinyl, dioxidothiomorpholinyl, methylpiperazinyl, hydroxypiperidinyl, oxopiperidinyl, piperidinyl, hydroxypyrrolidinyl, oxopyrrolidinyl, pyrrolidinyl, carboxylpyrrolidinyl, fluoropyrrolidinyl and morpholinyl,

an amino group,
a methylamino group,
an ethylamino group,
an isopropylamino group,
a hydroxyethylamino group,
a dimethylamino group,
a phenylmethylamino group,
a 9-oxa-3-azabicyclo[3.3.1]nonan-3-yl group,
a 3-oxa-8-azabicyclo[3.2.1]octan-8-yl group,
a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O, a benzyloxy group that may be substituted with one or more carbamoyl groups,
a C1-C4 alkylthio group,
a C1-C4 alkylsulfonyl group, or
an aminosulfonyl group
(when a plurality of $R_6$ are present, the plurality of $R_6$ may be the same or different).

Further preferable examples of the compound include a compound wherein in Formula (A),
$R_1$ is a hydroxy group;
$R_2$ is a hydroxy group;
$R_3$ is an ethynylene group;
$R_4$ is a bond;
$R_5$ is a C6-C10 unsaturated cycloalkyl group that may have one or more $R_6$, or
a monocyclic or bicyclic unsaturated heterocycloalkyl group that has at least one heteroatom selected from the group consisting of N, S, and O, and that may have one or more $R_6$; and
$R_6$ is
fluorine,
chlorine,
a hydroxy group,
a cyano group,
a methyl group,
a 3-fluoropyrrolidinyl group,
a morpholinyl group,
a thiomorpholinyl group,
a 3-hydroxyazetidinyl group,
an azetidinyl group,
an amino group,
a methylamino group,
a C1-C6 alkoxy group that may be substituted with one or more of either halogen or C3-C7 saturated cycloalkyl group, or a C1-C4 alkylthio group
(when a plurality of $R_6$ are present, the plurality of $R_6$ may be the same or different).

Further preferable examples of the compound include a compound wherein, in Formula (A), Y is -NH-;
$R_1$ is a hydroxy group;
$R_2$ is a hydroxy group;
$R_3$ is an ethynylene group;
$R_4$ is a bond;
$R_5$ is a phenyl group or a naphthyl group that may have one or more $R_6$, or
a monocyclic or bicyclic unsaturated heterocycloalkyl group that has at least one heteroatom selected from the group consisting of N, S, and O, and that may have one or more $R_6$; and $R_6$ is fluorine,
a methyl group,
3-fluoropyrrolidinyl,
3-hydroxyazetidinyl,
azetidinyl,
an amino group,
a methylamino group,
a C1-C6 alkoxy group that may have one or more cyclopropyl groups, or
a C1-C4 alkylthio group
(when a plurality of $R_6$ are present, the plurality of $R_6$ may be the same or different).

Specifically, preferable examples of the compound include:

4-amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-(4-amino-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-(methylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-[(3R)-3-fluoropyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-[4-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3-ethoxy-5-fluoro-phenyl]morpholine;
4-amino-7-[(2R,3R,4S,5R)-3,4-dihydroxy-5-[(sulfamoylamino)methyl]tetrahydrofuran-2-yl]-5-[2-(2-ethoxy-4,6-difluoro-phenyl)ethynyl]pyrrolo[2,3-d]pyrimidine;
4-[4-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3,5-difluoro-phenyl]thiomorpholine;
4-amino-5-[2-[2,6-difluoro-4-(3-hydroxyazetidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[4-(azetidin-1-yl)-2,6-difluoro-phenyl]ethynyl]-7-[(2R,3R,4S,5R)-3,4-dihydroxy-5-[(sulfamoylamino)methyl]cyclopentyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-6-fluorophenyl)ethynyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-fluoro-6-propoxy-phenyl)ethynyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5- [2- [2-fluoro-6-(2,2,2-trifluoroethoxy)phenyl]ethynyl]pyrrolo[2,3-d]pyrimidine;
8-[2-[4-amino-7-[(2R,3R,4S,5R)-3,4-dihydroxy-5-[sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine;
4-amino-7-[(2R,3R,4S,5R)-3,4-dihydroxy-5-[(sulfamoylamino)methyl]tetrahydrofuran-2-yl]-5-[2-(2-ethylsulfanyl-6-fluoro-phenyl)ethynyl]pyrrolo[2,3-d]pyrimidine;
4-amino-5-[2-[2-(cyclopropylmethoxy)-6-fluoro-phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(1R,2S,3R,4R)-2,3-dihydroxy-4-[(sulfamoylamino)methyl]cyclopentyl]-5-[2-(2-ethoxy-6-fluoro-phenyl)ethynyl]pyrrolo[2,3-d]pyrimidine;
4-amino-7-[(1R,2S,3R,4R)-2,3-dihydroxy-4-[(sulfamoylamino)methyl]cyclopentyl]-5-[2-(2-fluoro-6-methylsulfanylphenyl)ethynyl]pyrrolo[2,3-d]pyrimidine;
8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine;
4-amino-7-[(1R,4R,5S)-4,5-dihydroxy-3-[(sulfamoylamino)methyl]cyclopent-2-en-1-yl]-5-[2-(2-ethoxy-6-fluorophenyl)ethynyl]pyrrolo[2, 3-d]pyrimidine;
4-amino-7-[(1R,4R,5S)-4,5-dihydroxy-3-[(sulfamoylamino)methyl]cyclopent-2-en-1-yl]-5-[2-(2-fluoro-6-methylsulfanyl-phenyl)ethynyl]pyrrolo[2,3-d]pyrimidine;
[(2R,3S,4R,5R)-5-[4-amino-5-[2-(2,6-difluorophenyl)ethynyl]pyrrolo[2,3-d]pyrimidin-7-yl]-3,4-dihydroxy-tetrahydrofuran-2-yl]methylsulfamate; and salts of these compounds,
further preferably,
4-amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-(4-amino-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-(methylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-[(3R)-3-fluoropyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-4,6-difluoro-phenyl)ethynyl]pyrrolo[2,3-d]pyrimidine;
4-amino-5-[2-[2,6-difluoro-4-(3-hydroxyazetidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[4-(azetidin-1-yl)-2,6-difluoro-phenyl]ethynyl]-7-[(2R,3R,4S,5R)-3,4-dihydroxy-5-[(sulfamoylamino)methyl]cyclopentyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-6-fluoro-phenyl)ethynyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-fluoro-6-propoxy-phenyl)ethynyl]pyrrolo[2,3-d]pyrimidine;
8-[2-[4-amino-7-[(2R,3R,4S,5R)-3,4-dihydroxy-5-[sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine;
4-amino-7-[(2R,3R,4S,5R)-3,4-dihydroxy-5-[(sulfamoylamino)methyl]tetrahydrofuran-2-yl]-5-[2-(2-ethylsulfanyl-6-fluoro-phenyl)ethynyl]pyrrolo[2,3-d]pyrimidine;
4-amino-5-[2-[2-(cyclopropylmethoxy)-6-fluoro-phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(1R,2S,3R,4R)-2,3-dihydroxy-4-[(sulfamoylamino)methyl]cyclopentyl]-5-[2-(2-fluoro-6-methylsulfanyl-phenyl)ethynyl]pyrrolo[2,3-d]pyrimidine;
8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine;
4-amino-7-[(1R,4R,5S)-4,5-dihydroxy-3-[(sulfamoylamino)methyl]cyclopent-2-en-1-yl]-5-[2-(2-ethoxy-6-fluoro-phenyl)ethynyl]pyrrolo[2,3-d]pyrimidine;
4-amino-7-[(1R,4R,5S)-4,5-dihydroxy-3-[(sulfamoylamino)methyl]cyclopent-2-en-1-yl]-5-[2-(2-fluoro-6-methylsulfanyl-phenyl)ethynyl]pyrrolo[2,3-d]pyrimidine; and salts of these compounds.

The method for producing the compound of the present invention is described below.

The compounds of the present invention may be produced, for example, through the production methods below, or the methods described in the Reference Examples. However, the method for producing the compounds of the present invention is not limited to these reaction examples.

Production Method A

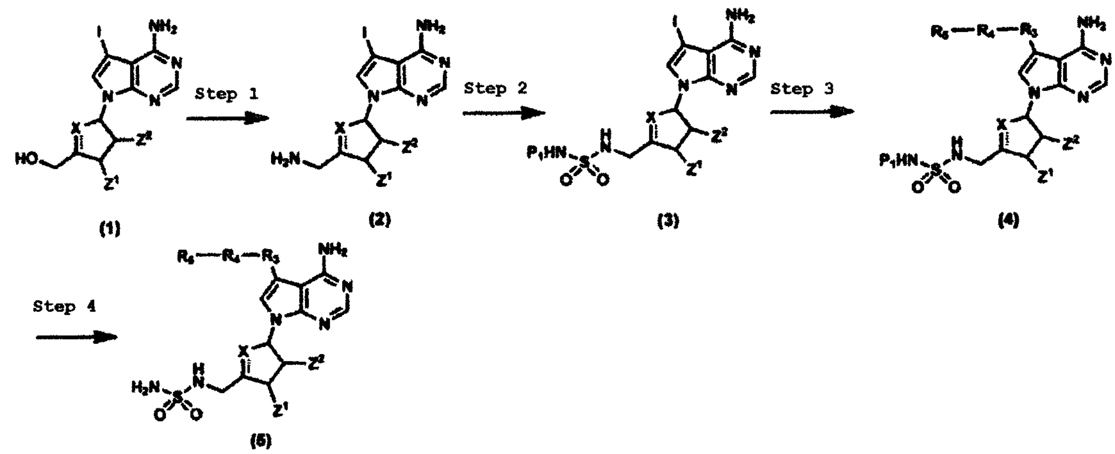


In the formula, $Z^1$ and $Z^2$ are the same or different, and each represents hydrogen, fluorine, a hydroxy group, an amino group, a cyano group, or a protector thereof.
$P_1$ is a protecting group of an amino group.
$R_3$ represents a vinylene group, an ethynylene group, a C6-C14 arylene group, or a monocyclic or bicyclic heteroarylene group having at least one heteroatom selected from the group consisting of N, S, and O.
$R_4$ represents a single bond, a methylene group, or a C3-C7 cycloalkylidene.
$R_5$ is a C3-C7 saturated cycloalkyl group that may be substituted with one or more $R_6$;
a C6-C10 unsaturated cycloalkyl group that may be substituted with one or more $R_6$; or
a monocyclic or bicyclic unsaturated heterocycloalkyl group that has at least one heteroatom selected from the group consisting of N, S, and O, and that may be substituted with one or more $R_6$.
$R_6$ is
halogen;
a hydroxy group;
a cyano group;
a C1-C6 alkyl group that may be substituted with one or more phenoxy groups;

a carbamoyl group, a C1-C6 alkoxycarbonyl group;
a C4-C7 unsaturated cycloalkyl group that may be substituted with one or more of halogen, hydroxy group, C1-C4 alkyl group, or carbamoyl group;
a monocyclic or bicyclic unsaturated heterocycloalkyl group having at least one heteroatom selected from the group consisting of N, S, and O;
a monocyclic or bicyclic saturated heterocycloalkyl group that has at least one heteroatom selected from the group consisting of N, S, and O, and that may be substituted with one or more of halogen, hydroxy group, oxo group, carboxyl group, dioxide group, or C1-C6 alkyl group;
an amino group;
a mono- or di-(C1-C4 alkyl) amino group that may be substituted with one or more hydroxy groups or phenyl groups;
a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O;
a benzyloxy group that may be substituted with one or more carbamoyl groups;
a C1-C4 alkylthio group;
a C1-C4 alkylsulfonyl group; or
an aminosulfonyl group.

When a plurality of $R_6$ are present, the plurality of $R_6$ may be the same or different.

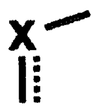

represents -O-, $-CH_2-$ or =CH-.

Step 1

This step produces Compound (2) using a compound represented by Formula (1) (in this specification, the compound represented by Formula (1) may be simply referred to as Compound (1); similarly, compounds represented by Formulas (2) to (30) may be simply referred to as Compounds (2) to (30) as a raw material, through a Mitsunobu reaction using a nitrogen nucleophile, and a subsequent deprotection reaction.

In Compound (1), when $Z^1$ and/or $Z^2$ represents a protector of a hydroxy group, examples of the protector include dimethyl acetal group, benzylidene acetal group, benzoyl group, and tert-butyldimethylsilyloxy group. $Z^1$ and $Z^2$ may form the structure below, or the like,

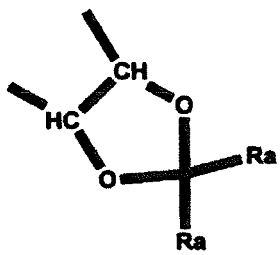

(wherein Ra are the same or different, and each represents hydrogen, methyl, ethyl, phenyl, cyclohexyl or cyclopentyl) together with the carbon atoms bound thereto. Examples of nitrogen nucleophiles include phthalimide. When phthalimide is used as a nitrogen nucleophile, the amount of phthalimide used is generally 1 to 10 moles, preferably 1 to 5 moles, per mole of Compound (1).

The Mitsunobu reaction can generally be performed by a known method, such as, for example, the method described in Synthesis, p. 1 (1981); or a similar method.

Examples of azodicarboxylic acid esters used for the Mitsunobu reaction include diethyl azodicarboxylate and diisopropyl azodicarboxylate. Such an azodicarboxylic acid ester can be generally used in an amount of 1 to 10 moles, and preferably 1 to 5 moles, per mole of Compound (1).

Examples of phosphine compounds used in the Mitsunobu reaction include triphenylphosphine and tributylphosphine, and the amount of phosphine compound used is generally 1 to 10 moles, preferably 1 to 5 moles, per mole of Compound (1).

Examples of the solvent include tetrahydrofuran, 1,2-dimethoxyethane, 1,4-dioxane, toluene, N,N-dimethylformamide, N,N-dimethylacetamide, dimethyl sulfoxide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C. The removal of the protecting group of a nitrogen nucleophile can generally be performed by a known method, such as the method described in Protective Groups in Organic Synthesis, T.W. Greene, John Wiley & Sons (1981); or a similar method.

The removal of the phthalimide group can be performed by using an isolated phthalimide intermediate or by directly using the Mitsunobu reaction solution, with hydrazine, hydroxylamine, methylamine, ethylamine, n-butylamine, etc., as a deprotection reagent. The amount of the deprotection reagent is typically an equimolar to excessive molar amount per mole of Compound (1).

Examples of the solvent include alcohol solvents (ethanol, methanol, etc.), acetonitrile, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 2

This step produces Compound (3) by reacting a sulfamoyl-introducing reagent with Compound (2).

The sulfamoyl-introducing reagent can be obtained from commercial suppliers, or can be produced through a known method using, for example, sulfamoyl chloride, 1-aza-4-azoniabicyclo [2.2.2]octane-4-ylsulphonyl(tert-butoxycarbonyl)azanide or the like. The amount of the sulfamoyl-introducing reagent used is generally 1 to 10 moles, preferably 1 to 5 moles, per mole of Compound (2).

Examples of the protecting group of an amino group include C1-C6 alkyl group, tert-butoxycarbonyl group, benzyloxycarbonyl group, acetyl group, and propionyl group.

Examples of bases include triethylamine, diisopropylethylamine, pyridine, imidazole, and DBU. When a base is used, the amount of the base is generally 1 to 30 moles, preferably 1 to 10 moles, per mole of Compound (2).

Examples of the solvent include acetonitrile, dichloromethane, chloroform, tetrahydrofuran, 1,2-dimethoxyethane, 1,4-dioxane, toluene, N,N-dimethyl formamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, and preferably from 0°C to 100°C.

Step 3

Using Compound (3) as a raw material, this step produces Compound (4) through a coupling reaction (Sonogashira coupling, Suzuki-Miyaura coupling, etc.). This step may be performed through multiple steps as necessary, and may suitably be combined with a protection reaction, a deprotection reaction.

For example, among various Compounds (4), a compound in which $R_3$ has an alkynylene group may be obtained through a coupling (Sonogashira) reaction, using Compound (3) and a compound: H-C≡C-$R_4$-$R_5$ (wherein $R_4$ and $R_5$ are as defined above) .

In this case, the compound: H-C≡C-$R_4$-$R_5$ (wherein $R_4$ and $R_5$ are as defined above) can be obtained from commercial suppliers, or can be produced through a known method. The amount of this compound is generally 1 to 10 moles, preferably 1 to 3 moles, per mole of Compound (3).

This step can generally be performed by a known method, for example, the method disclosed in Chemical Reviews, Vol. 107, p. 874 (2007). For example, this step can be performed in the presence of a transition metal catalyst and a base in a solvent that does not adversely affect the reaction.

Examples of transition metal catalysts usable in this step include palladium catalysts (e.g., palladium acetate, tris(dibenzylideneacetone)dipalladium, 1,1'-bis(diphenylphosphino)ferrocene-palladium(II) dichloride-dichloromethane complex, etc.). As necessary, a ligand (e.g., triphenylphosphine, tri-tert-butylphosphine, etc.) is added, and a copper reagent (e.g., copper iodide, copper acetate, etc.) is used as a cocatalyst. The amount of the transition metal catalyst varies depending on the type of catalyst. For example, the amount of the transition metal catalyst used is generally 0.0001 to 1 mole, preferably 0.01 to 0.5 moles, per mole of Compound (4). The amount of the ligand used is generally 0.0001 to 4 moles, preferably 0.01 to 2 moles, per mole of Compound (4). The amount of the cocatalyst used is generally 0.0001 to 4 moles, preferably 0.001 to 2 moles, per mole of Compound (4).

Further, a base may be added during the above reaction as necessary. Examples of bases include organic bases such as triethylamine, diisopropylethylamine, pyridine, lutidine, collidine, 4-dimethylaminopyridine, potassium tert-butyrate, sodium tert-butyrate, sodium methoxide, sodium ethoxide, lithium hexamethyldisilazide, sodium hexamethyldisilazide, potassium hexamethyldisilazide, and butyl lithium; and inorganic bases such as sodium hydrogen carbonate, sodium carbonate, potassium carbonate, cesium carbonate, sodium hydroxide, and sodium hydride. Among these, organic bases such as triethylamine and diisopropylethylamine are preferable. The amount of the base used is generally 0.1 to 50 moles, and preferably 1 to 20 moles, per mole of Compound (4).

The reaction solvent is not particularly limited, and any solvent that does not adversely affect the reaction can be used. Examples of the solvent include hydrocarbons (e.g., benzene, toluene, xylene, etc.), nitriles (e.g., acetonitrile etc.), ethers (e.g., dimethoxyethane, tetrahydrofuran, 1,4-dioxane, etc.), alcohols (e.g., methanol, ethanol, etc.), aprotic polar solvents (e.g., dimethylformamide, dimethylsulfoxide, hexamethyl phosphoramide, etc.), water, and mixtures thereof. The reaction time generally ranges from 0.1 to 100 hours, preferably 0.5 to 24 hours. The reaction temperature generally ranges from 0°C to the boiling temperature of the solvent, preferably 0 to 150°C.

Further, this step may also be performed through Suzuki-Miyaura coupling using Compound (3) and an organic boron compound (boric-acid compound, boric-acid ester, etc.) having a substituent $-R_3-R_4-R_5$ (wherein $R_3$, $R_4$, and $R_5$ are as defined above) .

In this method, the organic boron compound can be obtained from commercial suppliers, or can be produced through a known method. The amount of the organic boron compound used is generally 1 to 10 moles, preferably 1 to 3 moles, per mole of Compound (3).

In this method, the Suzuki-Miyaura coupling can generally be performed by a known method, such as the method described in Chemical Reviews, Vol. 95, p. 2457 (1995); or a similar method.

Examples of reaction catalysts used for the Suzuki-Miyaura coupling include tetrakis triphenylphosphine palladium (0), bis (triphenylphosphine)palladium(II) dichloride, and 1,1'-bis (diphenyl phosphino)ferrocene-palladium(II) dichloride-dichloromethane complex. The amount of the reaction catalyst used depends on the type of the catalyst. The amount of the catalyst used is generally 0.0001 to 1 mole, preferably 0.01 to 0.5 moles, per mole of Compound (3).

Examples of the solvent include hydrocarbons (e.g., benzene, toluene, xylene, etc.), nitriles (e.g., acetonitrile etc.), ethers (e.g., dimethoxyethane, tetrahydrofuran, 1,4-dioxane, etc.), alcohols (e.g., methanol, ethanol, etc.), aprotic polar solvents (e.g., dimethylformamide, dimethylsulfoxide, etc.), and water. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 4

This step produces Compound (5) by deprotecting the protected amino group of Compound (4). The deprotection can generally be performed by a known method, such as the method described in Protective Groups in Organic Synthesis, T.W. Greene, John Wiley & Sons (1981); or a similar method. An example of the protecting group is tert-butyloxycarbonyl. When a tert-butyloxycarbonyl group is used as a protecting group, the deprotection is preferably performed under acidic conditions. Examples of acids that can be used include hydrochloric acid, acetic acid, trifluoroacetic acid, sulfuric acid, methanesulfonic acid, tosic acid, and the like. The amount of the acid used is preferably 1 to 100 moles per mole of Compound (4).

Any solvent that does not adversely affect the reaction may be used for the reaction. Examples of the solvent include alcohols (e.g., methanol etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile etc.), ethers (e.g., dimethoxyethane, tetrahydrofuran, etc.), aprotic polar solvents (e.g., N,N-dimethylformamide, dimethylsulfoxide, hexamethyl phosphoramide, etc.), and mixtures thereof. The reaction time generally ranges from 0.1 to 100 hours, preferably 0.5 to 24 hours. The reaction temperature generally ranges from 0 to 120°C, preferably 0 to 90°C.

Compound (1) used as a raw material in Production Method A can be obtained from commercial suppliers, or can be produced through a known method. For example, Compound (7) in which $Z^1$ and $Z^2$ in Formula (1) represent a specific protector of a hydroxy group may be produced through Production Method B below.

Production Method B

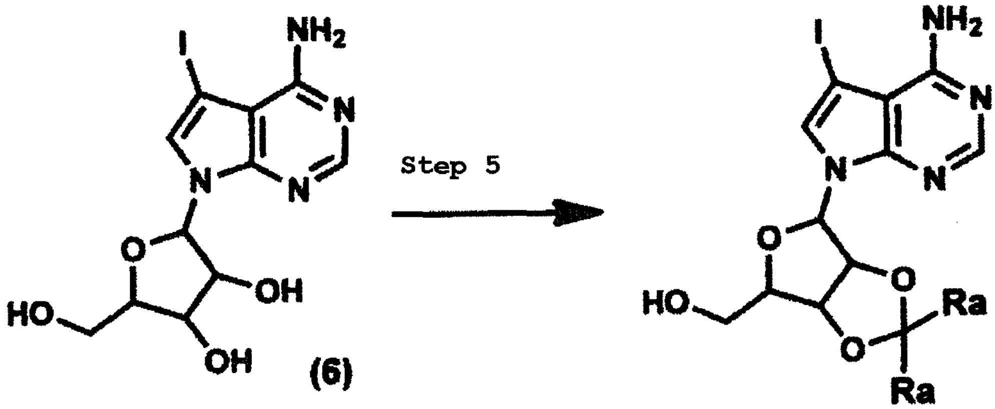


(6)

(7)

wherein Ra are the same or different, and each represents hydrogen, methyl, ethyl, phenyl, cyclohexyl, or cyclopentyl.

Step 5

This step produces Compound (7) by protecting two hydroxy groups among the hydroxy groups of Compound (6). Examples of the protection reagent include dialkoxy alkane and the like. The amount of the protection reagent used is generally 1 to 100 moles, preferably 1 to 10 moles, per mole of Compound (6).

The protection can generally be performed by a known method, such as the method described in Protective Groups in Organic Synthesis, T.W. Greene, John Wiley & Sons (1981); or a similar method.

Examples of the reaction catalyst include p-toluenesulfonic acid, methanesulfonic acid, pyridinium p-toluenesulfonates, perchloric acid, and sulfuric acid. When a reaction catalyst is used, the amount of the reaction catalyst depends on the type of the catalyst. For example, the amount of the reaction catalyst is generally 0.0001 to 1 mole, preferably 0.01 to 0.5 moles, per mole of Compound (6).

Examples of the solvent include acetonitrile, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Further, Compound (15) in which $Z^1$ and $Z^2$ in Formula (1) represent a specific protector of a hydroxy group may be produced through Production Method C below.

Production Method C

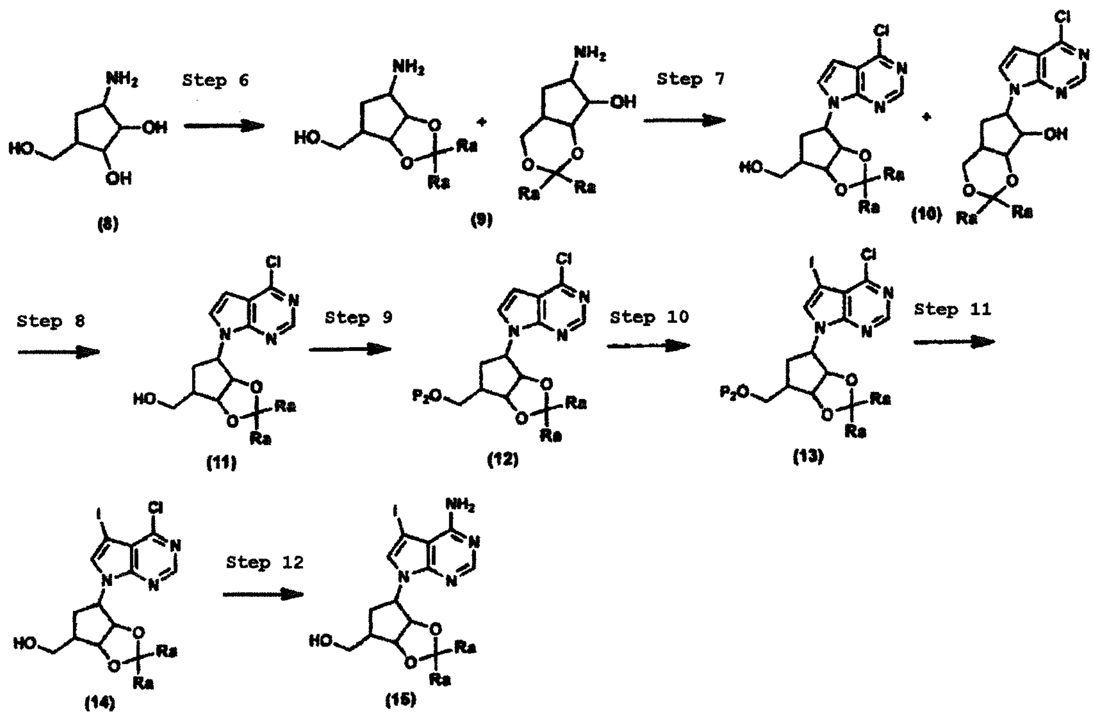


(8) (9) (10) (11) (12) (13) (14) (15)

wherein $P_2$ is a protecting group of a hydroxy group. Ra is as defined above.

Step 6

This step produces Compound (9) by protecting two hydroxy groups among the hydroxy groups of Compound (8). The protection reaction may be performed in the same manner as in Step 5.

Step 7

This step produces an isomer mixture of Compound (10), which is a pyrrolopyrimidine compound, by reacting an isomer mixture of Compound (9) and 2-(4,6-dichloro pyrimidin-5-yl)acetaldehyde in the presence of a base.
The amount of the 2-(4,6-dichloro pyrimidin-5-yl)acetaldehyde is generally 1 to 10 moles, preferably 1 to 3 moles, per mole of Compound (9).
The reaction can generally be performed by a known method, for example, the method disclosed in Tetrahedron Letters, 26 (16), 2001-2 (1985); or a similar method.
Examples of bases include triethylamine, diisopropylethylamine, pyridine, lutidine, collidine, and DBU. When a base is used, the amount of the base is generally 1 to 100 moles, preferably 1 to 20 moles, per mole of Compound (9).
Examples of the solvent include ethanol, 2-propanol, 2-butanol, acetonitrile, tetrahydrofuran, 1,2-dimethoxyethane, 1,4-dioxane, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 8

This step converts the isomer mixture of Compound (10) into Compound (11) consisting of only one of the isomers, in the presence of an acid catalyst.
Examples of acids include p-toluenesulfonic acid, methanesulfonic acid, pyridinium p-toluene sulfonate, perchloric acid, and sulfuric acid. The amount of the acid is generally 0.001 to 10 moles, preferably 0.01 to 2 moles, per mole of Compound (10).
Examples of the solvent include acetone, 2-butanone, acetonitrile, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 1 to 48 hours. The reaction temperature generally ranges from 0°C to the boiling temperature of the solvent, preferably 0°C to 100°C.

Step 9

This step protects the hydroxy group of Compound (11) using Compound $P_2$-Cl (wherein $P_2$ is a protecting group of a hydroxy group).

The reaction can generally be performed by a known method, such as the method described in Protective Groups in Organic Synthesis, T.W. Greene, John Wiley & Sons (1981); or a similar method.

The protecting group of a hydroxy group represented by $P_2$ in Compound $P_2$-Cl is not particularly limited insofar as it has a protecting function. Examples include lower alkyl groups such as methyl, ethyl, propyl, isopropyl, and tert-butyl; lower alkylsilyl groups such as trimethylsilyl and tert-butyldimethylsilyl; lower alkoxymethyl groups such as methoxymethyl and 2-methoxyethoxymethyl; tetrahydropyranyl; trimethylsilylethoxymethyl; aralkyl groups such as benzyl, p-methoxybenzyl, 2,3-dimethoxybenzyl, o-nitrobenzyl, p-nitrobenzyl, and trityl; and acyl groups such as formyl, acetyl, and trifluoroacetyl. In particular, methyl, methoxymethyl, tetrahydropyranyl, trimethylsilylethoxymethyl, tert-butyldimethylsilyl, and acetyl are preferable.

The compound can be obtained from commercial suppliers, or can be produced through a known method. The amount of the compound is generally 1 to 20 moles, preferably 1 to 5 moles, per mole of Compound (11).

Examples of the base include triethylamine, diisopropylethylamine, pyridine, lutidine, collidine, and DBU. The amount of the base is generally 1 to 20 moles, preferably 1 to 5 moles, per mole of Compound (11).

Examples of the solvent include acetonitrile, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 10

This step produces Compound (13) by reacting Compound (12) with iodosuccinimide, thereby introducing an iodine atom.

The iodination may be performed according to the method disclosed in WO2006/102079, or a similar method. The amount of the iodosuccinimide is generally 1 to 20 moles, preferably 1 to 5 moles, per mole of Compound (12).

Examples of the solvent include acetone, acetonitrile, ethyl acetate, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 11

This step produces Compound (14) by deprotecting the protected hydroxy group of Compound (13).

The deprotection can generally be performed by a known method, such as the method described in Protective Groups in Organic Synthesis, T.W. Greene, John Wiley & Sons (1981); or a similar method.

When a tert-butyldimethylsilyl group is used as a protecting group, tetrabutylammonium fluoride, for example, is used as a deprotection reagent. The amount of the reagent is preferably 1 to 10 moles, per mole of Compound (13).

Any solvent that does not adversely affect the reaction may be used for the reaction. Examples of the solvent include ethers (e.g., 1,2-dimethoxyethane, tetrahydrofuran, etc.), aprotic polar solvents (e.g., N,N-dimethylformamide, dimethylsulfoxide, hexamethyl phosphoryl amide, etc.), and mixtures thereof. The reaction time generally ranges from 0.1 to 100 hours, preferably 0.5 to 24 hours. The reaction temperature generally ranges from 0 to 80°C, preferably 0 to 50°C.

Step 12

This step produces Compound (15) by reacting Compound (14) with ammonia.

The amount of the ammonia used in this step is typically an equimolar to excessive molar amount per mole of Compound (14).

The reaction solvent is not particularly limited, and any solvent that does not adversely affect the reaction can be used. Examples of the solvent include water, methanol, ethanol, isopropanol, tert-butyl alcohol, tetrahydrofuran, 1,2-dimethoxyethane, 1,4-dioxane, N,N-dimethylformamide, N-methylpyrrolidin-2-one, dimethylsulfoxide, and mixtures thereof.

The reaction temperature is generally 0°C to 200°C, preferably from room temperature to 150°C. The reaction

time is generally 5 minutes to 7 days, preferably 30 minutes to 72 hours.

Production Method D

Further, Compound (19), which is a compound in which $Z^1$ in Formula (1) is a hydroxy group, $Z^2$ is hydrogen, and X is $CH_2$, may be produced through Production Method D below.

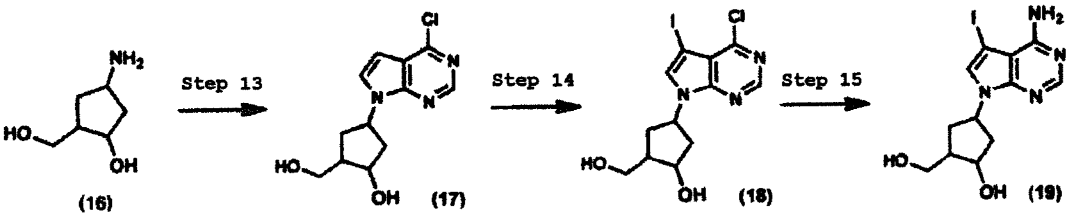

(16) (17) (18) (19)

Step 13

This step reacts Compound (16) with 2-(4,6-dichloro pyrimidin-5-yl)acetaldehyde. This reaction may be performed in the same manner as in Step 7.

Step 14

This step reacts the reaction product obtained in Step 13 with iodosuccinimide, thereby introducing an iodine atom. This reaction may be performed in the same manner as in Step 10.

Step 15

This step produces Compound (19) by reacting the reaction product obtained in Step 14 with ammonia. This reaction may be performed in the same manner as in Step 12.

Further, Compound (20), which is a compound in which $Z^1$ in Formula (1) is a hydroxy group, $Z^2$ is hydrogen, and X is O, is a publicly known compound.

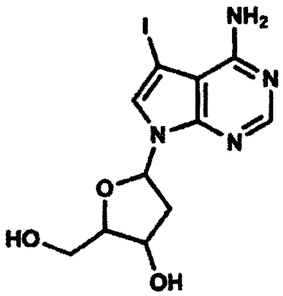

(20)

Production Method E

Further, among Compounds (A), Compound (27) in which $R_1$ and $R_2$ are hydroxy groups, and

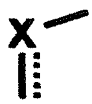

is =CH- may be produced through Production Method E below.

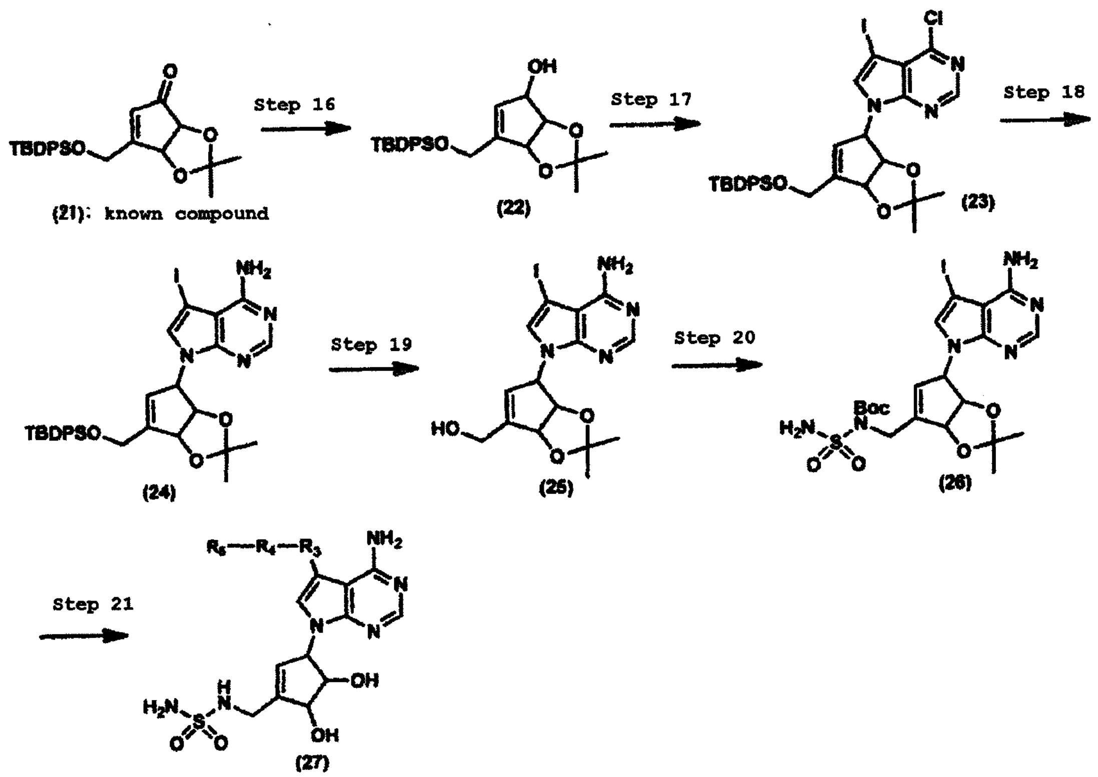


wherein $R_3$, $R_4$, and $R_5$ are as defined above.

Step 16

This step produces Compound (22) by reducing the carboxyl group of Compound (21). This step is performed in the presence of a reducing agent. In this step, the amount of the reducing agent is 1 to 20 moles, preferably 1 to 5 moles, per mole of Compound (21). Examples of the reducing agent include sodium borohydride, lithium aluminum hydride, borane reagent (e.g., diborane), and diisobutylaluminum hydride.

Examples of the solvent include methanol, ethanol, diethylether, tetrahydrofuran, 1,2-dimethoxyethane, 1,4-dioxane, and toluene. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 17

This step produces Compound (23) through a Mitsunobu reaction using Compound (22) as a raw material, and 4-chloro-5-iodo-7H-pyrrolo[2,3-d]pyrimidine as a nucleophilic agent.

This reaction may be performed in the same manner as in Step 1.

Step 18

This step produces Compound (24) by reacting Compound (23) with ammonia. This reaction may be performed in the same manner as in Step 12.

Step 19

This step produces Compound (25) by deprotecting the protected hydroxy group of Compound (24). This reaction may be performed in the same manner as in Step 11.

Step 20

This step produces Compound (26) through a Mitsunobu reaction using Compound (25) as a raw material, and

tert-butyl sulfamoyl carbamate as a nucleophilic agent.

This reaction may be performed in the same manner as in Step 1.

Step 21

This step produces Compound (27) using Compound (26) as a raw material, by deprotecting the protected amino group after a coupling reaction (Sonogashira coupling, Suzuki-Miyaura coupling, etc.). This reaction may be performed in the same manner as in Step 3 and Step 4.

Production Method F

Further, among Compounds (A), Compound (29) in which $R_1$ and $R_2$ are the same or different, and each represent hydrogen, fluorine, a hydroxy group, an amino group, a cyano group, or a protector thereof may be produced through Production Method F below.

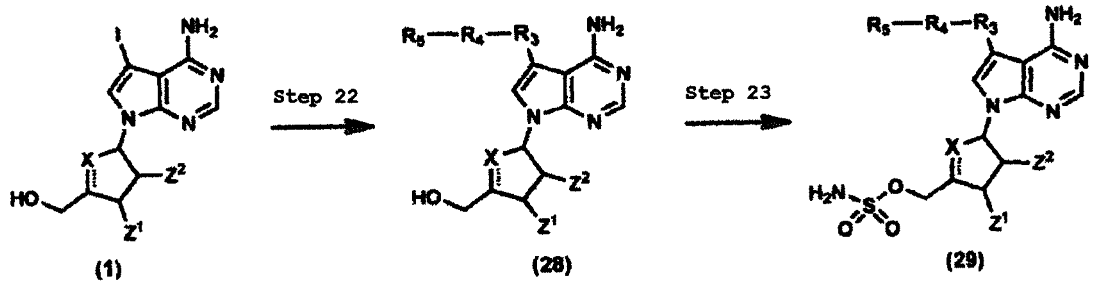


(1) (28) (29)

wherein X, $R_3$, $R_4$, $R_5$, $Z^1$, and $Z^2$ are as defined above.

Step 22

This step produces Compound (28) through a coupling reaction (Sonogashira coupling, Suzuki-Miyaura coupling, etc.) using Compound (1) as a raw material. This reaction may be performed in the same manner as in Step 3.

Step 23

This step produces Compound (29) by reacting Compound (28) with a sulfamoyl-introducing reagent.

This reaction may be performed in the same manner as in Step 2. This step may be performed through multiple steps as necessary, and may suitably be combined with a deprotection reaction.

Production Method G

Further, among Compounds (A), Compound (32) may be produced through Production Method G below.

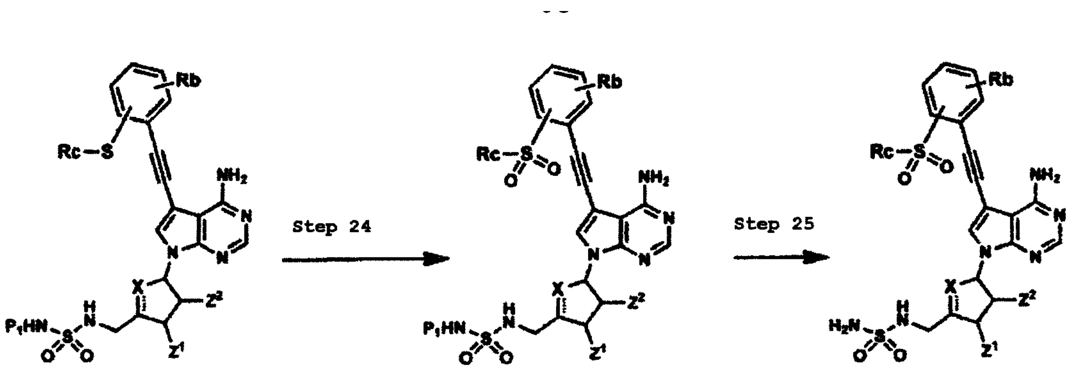


(30) (31) (32)

wherein $P_1$, $Z^1$, and $Z^2$ are as defined above. Rb is the same as $R_6$ above. Rc represents a substituted or unsubstituted

alkyl group.

Step 24

This step produces Compound (31) by oxidizing Compound (30). This step is performed in the presence of an oxidant. The amount of the oxidant used in this step is generally 1 to 20 moles, preferably 1 to 5 moles, per mole of Compound (30). Examples of the oxidant include oxone, m-chloroperbenzoic acid, hydrogen peroxide, and potassium permanganate.

Examples of the solvent include water, acetone, 2-butanone, acetonitrile, ethyl acetate, dichloromethane, chloroform, tetrahydrofuran, 1,2-dimethoxyethane, 1,4-dioxane, toluene, N,N-dimethylformamide, N,N-dimethylacetamide, and N-methylpyrrolidin-2-one. These solvents may be used alone, or in a mixture. The reaction time generally ranges from 0.1 to 100 hours, preferably 0.1 to 24 hours. The reaction temperature generally ranges from 0°C to the boiling temperature of the solvent, preferably 0°C to 100°C.

Step 25

This step produces Compound (32) by deprotecting the protected amino group of Compound (31). This reaction may be performed in the same manner as in Step 4.

The compounds thus-obtained through Production Methods A to G can be subjected to the subsequent step after or without isolation and purification by known separation and purification means, such as concentration, vacuum concentration, crystallization, solvent extraction, reprecipitation, and chromatography.

When the compound represented by Formula (A) or other compounds having an antitumor effect have isomers such as optical isomers, stereoisomers, regioisomers, and rotational isomers, any of the isomers and mixtures thereof is included within the scope of the compounds of the present invention. For example, when the compounds of the present invention have optical isomers, the optical isomer separated from a racemic mixture is also included within the scope of the compounds of the present invention. Each of such isomers can be obtained as a single compound by known synthesis and separation means (e.g., concentration, solvent extraction, column chromatography, recrystallization, etc.).

The compound represented by Formula (A) or other compounds having an antitumor effect can be easily isolated and purified by usual isolation and purification means. Examples of such means include solvent extraction, recrystallization, preparative reversed-phase high-performance liquid chromatography, column chromatography, preparative thin-layer chromatography, and the like.

The compound represented by Formula (A) or other compounds having an antitumor effect may be in the form of crystals. Single crystals and polymorphic mixtures are included within the scope of the compounds of the present invention. Such crystals can be produced by crystallization according to a crystallization method known *per se* in the art. The compounds of the present invention may be solvates (e.g., hydrates) or non-solvates. Any of such forms are included within the scope of the compounds of the present invention. Compounds labeled with an isotope (e.g., $^{3}H$, $^{14}C$, $^{35}S$, $^{125}I$) are also included within the scope of the compounds of the present invention.

A prodrug of the compound represented by Formula (A) or prodrugs of other compounds having an antitumor effect refer to a compound that can be converted to the compound through a reaction with an enzyme, gastric acid, or the like, under physiological conditions *in vivo,* i.e., a compound that can be converted to the compound by enzymatic oxidation, reduction, hydrolysis, or the like; or a compound that can be converted to the compound by hydrolysis with gastric acid or the like. Further, the prodrugs of the compounds may be compounds that can be converted to the compounds under physiological conditions, such as those described in "Iyakuhin no Kaihatsu [Development of Pharmaceuticals]," Vol. 7, Molecular Design, published in 1990 by Hirokawa Shoten Co., pp. 163-198.

The salt of the compound represented by Formula (A) or the salts of other compounds having an antitumor effect refer to a common salt used in the field of organic chemistry. Examples of such salts include base addition salts to a carboxyl group when the compound has a carboxyl group, and acid addition salts to an amino or basic heterocycloalkyl group when the compound has an amino or basic heterocycloalkyl group. The salt of the compound represented by Formula (A) or the salts of other compounds having an antitumor effect include pharmaceutically acceptable salts of the compound.

Examples of base addition salts include alkali metal salts such as sodium salts and potassium salts; alkaline earth metal salts such as calcium salts and magnesium salts; ammonium salts; and organic amine salts such as trimethylamine salts, triethylamine salts, dicyclohexylamine salts, ethanolamine salts, diethanolamine salts, triethanolamine salts, procaine salts, and N,N'-dibenzylethylenediamine salts.

Examples of acid addition salts include inorganic acid salts such as hydrochlorides, sulfates, nitrates, phosphates, and perchlorates; organic acid salts such as acetates, formates, maleates, fumarates, tartrates, citrates, ascorbates, and trifluoroacetates; and sulfonates such as methanesulfonates, isethionates, benzenesulfonates, and p-toluenesulfonates. The acid addition salts are preferably hydrochlorides.

The compound represented by Formula (A) is useful as an antitumor effect potentiator of or one or more other compounds having an antitumor effect. The compound represented by Formula (A) or one or more other compounds having an antitumor effect are also useful as an antitumor agent comprising the compound represented by Formula (A) and the one or more other compounds having an antitumor effect in combination. The type of malignant tumor to be treated is not particularly limited. Further, the organ from which the tumor is developed is not particularly limited. Examples include head and neck cancers, esophagus cancer, gastric cancer, colon cancer, rectum cancer, liver cancer, gallbladder cancer, cholangiocarcinoma, biliary tract cancer, pancreatic cancer, lung cancer, breast cancer, ovarian cancer, cervical cancer, endometrial cancer, renal cancer, bladder cancer, prostate cancer, testicular tumor, osteosarcoma, soft-tissue sarcoma, multiple myeloma, skin cancer, brain tumor, and mesothelioma. Preferably, the target cancer is colon cancer, rectum cancer, pancreatic cancer, lung cancer, prostate cancer, breast cancer, osteosarcoma, soft-tissue sarcoma, or skin cancer.

Further, examples of hematopoietic tumors include bone marrow tumors (e.g., lymphocytic leukemia, myelogenous leukemia, acute leukemia, chronic leukemia, and the like), and lymphoid tumors.

Examples of bone marrow tumors include myeloproliferative neoplasm (MPN), acute myelogenous leukemia (AML), related precursor neoplasm, acute lymphocytic leukemia, chronic myelogenous leukemia (CML), and myelodysplastic syndrome (MDS). Preferable examples include acute leukemia. Particularly preferable examples include acute myelogenous leukemia.

Examples of lymphoid tumors include precursor lymphoid tumor, mature B-cell tumor, mature T-cell tumor and NK-cell tumor, and Hodgkin's lymphoma. Preferable examples include precursor lymphoid tumors, mature B-cell tumor, mature T-cell tumor and NK-cell tumor. Lymphoid tumors that are not regarded as Hodgkin's lymphoma may be collectively referred to as non-Hodgkin's lymphoma.

Examples of precursor lymphoid tumors include B-lymphoblastic leukemia/lymphoma, T-lymphoblastic leukemia/lymphoma (ALL), blastic NK-cell lymphoma, and like blastic lymphoma. T-lymphoblastic leukemia/lymphoma is preferable.

Examples of mature B-cell tumor include chronic lymphocytic leukemia/small lymphocytic lymphoma (CLL/SLL), B-cell prolymphocytic leukemia (B-PLL), splenic marginal zone lymphoma (SMZL), hairy cell leukemia (HCL), Waldenstrom's macroglobulinemia (WM), plasma cell tumor, MALT lymphoma, follicular lymphoma, mantle cell lymphoma (MCL), B-cell lymphoma (diffuse large B-cell lymphoma (DLBCL), and Burkitt's lymphoma). Plasma cell tumor and B-cell lymphoma are preferable.

Preferable examples of plasma cell tumor include multiple myeloma.

Examples of mature T-cell tumor and NK-cell tumor include T-cell prolymphocytic leukemia (T-PLL), aggressive NK-cell leukemia/lymphoma, adult T-cell leukemia/lymphoma, and peripheral T-cell lymphoma not otherwise specified (PTCL-NOS).

Examples of Hodgkin's lymphoma include nodular lymphocyte-predominant Hodgkin's lymphoma, classical Hodgkin's lymphoma, nodular sclerosis classical Hodgkin's lymphoma, and mixed cellularity classical Hodgkin's lymphoma.

FAB classification has been hitherto known for use in the diagnosis and classification of hematopoietic tumors. In recent years, WHO classification has also been used. The compound represented by Formula (A) or one or more other compounds having an antitumor effect are useful for the various hematopoietic tumors classified by both FAB classification and WHO classification.

When the compound represented by Formula (A) or one or more other compounds having an antitumor effect are used as a pharmaceutical preparation, a pharmaceutical carrier can be added, if required, thereby forming a suitable dosage form according to prevention and treatment purposes. Examples of dosage forms include oral preparations, injections, suppositories, ointments, patches, eye drops, and the like. Of these, injections (intravenous injections etc.) are preferable. Such dosage forms can be formed by methods conventionally known to persons skilled in the art. Further, the compound represented by Formula (A) or one or more other compounds having an antitumor effect may be formulated as a single composition, or two or more compositions.

As the pharmaceutical carrier, various conventional organic or inorganic carrier materials used as preparation materials may be blended as an excipient, binder, disintegrant, lubricant, or coating agent in solid preparations; or as a solvent, solubilizing agent, suspending agent, isotonizing agent, pH adjuster, pH buffer, or soothing agent in liquid preparations. Moreover, pharmaceutical preparation additives, such as antiseptics, antioxidants, colorants, sweetening agents, flavoring agents, and stabilizers, may also be used, if required.

When liquid preparations for oral administration are prepared, a sweetening agent, a buffer, a stabilizer, a flavoring agent, and the like, may be added to the compounds of the present invention; and the resulting mixtures may be formulated into oral liquid preparations, syrups, elixirs, etc., according to an ordinary method.

When suppositories are prepared, pharmaceutically acceptable carriers known in the art, such as polyethylene glycol, lanolin, cacao butter, and fatty acid triglyceride; and as necessary, surfactants such as Tween 80®, may be added to the compounds of the present invention, and the resulting mixtures may be formulated into suppositories according

to an ordinary method.

When ointments are prepared, a commonly used base, stabilizer, wetting agent, preservative, and the like, may be blended into the compounds of the present invention, as necessary; and the obtained mixtures may be mixed and formulated into ointments according to an ordinary method.

Examples of bases include liquid paraffin, white petrolatum, white beeswax, octyl dodecyl alcohol, paraffin, and the like.

Examples of excipients include lactose, sucrose, D-mannitol, starch, crystalline cellulose, calcium silicate, and the like.

Examples of binders include hydroxypropyl cellulose, methyl cellulose, polyvinylpyrrolidone, candy powder, hypromellose, and the like.

Examples of disintegrators include sodium starch glycolate, carmellose calcium, croscarmellose sodium, crospovidon, low-substituted hydroxy propyl cellulose, partially pregelatinized starch, and the like.

Examples of lubricants include talc, magnesium stearate, sucrose fatty acid ester, stearic acid, sodium stearyl fumarate, and the like.

Examples of coating agents include ethyl cellulose, aminoalkyl methacrylate copolymer RS, hypromellose, sucrose, and the like.

Examples of solvents include water, propylene glycol, physiological saline, and the like.

Examples of solubilizing agents include polyethylene glycol, ethanol, $\alpha$-cyclodextrin, macrogol 400, polysorbate 80, and the like.

Examples of suspending agents include carrageenan, crystalline cellulose/carmellose sodium, polyoxyethylene hydrogenated castor oil, and the like.

Examples of isotonizing agents include sodium chloride, glycerin, potassium chloride, and the like.

Examples of pH adjusters and pH buffers include sodium citrate, hydrochloric acid, lactic acid, phosphoric acid, sodium dihydrogen phosphate, and the like.

Examples of soothing agents include procaine hydrochloride, lidocaine, and the like.

Examples of antiseptics include ethyl parahydroxybenzoate, cresol, benzalkonium chloride, and the like.

Examples of antioxidants include sodium sulfite, ascorbic acid, natural vitamin E, and the like.

Examples of coloring agents include titanium oxide, iron sesquioxide, Food Blue No. 1, copper chlorophyll, and the like.

Examples of sweetening agents and flavoring agents include aspartame, saccharins, sucralose, l-menthol, mint flavor, and the like.

Examples of stabilizers include sodium pyrosulfite, edetate sodium, erythorbic acid, magnesium oxide, dibutylhydroxytoluene, and the like.

Examples of preservatives include methyl parahydroxybenzoate, ethyl parahydroxybenzoate, propyl parahydroxybenzoate, and the like.

When a patch is prepared, the above-described ointment, cream, gel, paste, or the like, may be applied to an ordinary substrate according to an ordinary method.

As the substrate, woven fabrics or non-woven fabrics comprising cotton, staple fibers, or chemical fibers; and films or foam sheets of soft vinyl chloride, polyethylene, polyurethane, etc., are suitable.

The amount of the compound represented by Formula (A) or one or more other compounds having an antitumor effect to be incorporated in each of such dosage unit forms depends on the condition of the patient to whom the compound or one or more other compounds are administered, the dosage form, etc. In general, in the case of an oral agent, an injection, and a suppository, the amount of the compound of the present invention is preferably 10 $mg/m^2$ to 1000 $mg/m^2$ per dosage unit form.

The daily dose of the medicine in such a dosage form depends on the condition, body weight, age, gender, etc., of the patient, and cannot be generalized. For example, the daily dose of the compound for an adult (body weight: 50 kg) may be 13.9 to 1500 mg, and preferably 50 to 1000 mg; and is preferably administered in one dose, or in two to three divided doses, per day.

The amount of one or more other compounds having an antitumor effect to be incorporated in each of such dosage unit forms is suitably determined according to the type of antitumor agent used, the condition of a patient, the dosage form, etc. The daily dose of the medicine in such a dosage form as described above is also suitably determined.

When the compound represented by Formula (A) and one or more other compounds having an antitumor effect are formulated into two or more separate compositions, the method for administering the compositions is not particularly limited; and they may be administered simultaneously, sequentially, or at one or more intervals as long as the potentiation action of the present invention is attained. The order of administration of the compositions is also not particularly limited. An antitumor agent comprising the compound represented by Formula (A) may be administered before, simultaneously with, or after administration of antitumor agent(s) comprising one or more other compounds having an antitumor effect.

Another embodiment provides an antitumor agent comprising a combination of the compound represented by

Formula (A) and one or more other compounds having an antitumor effect.

The terms "combination" and "combined use" used in the present specification mean that the compound represented by Formula (A) and one or more other compounds having an antitumor effect can be administered simultaneously as a single composition or two separate compositions, or that the compound represented by Formula (A) and one or more other compounds having an antitumor effect can be administered sequentially or at one or more intervals as two separate compositions, as long as the potentiation action of the present invention is attained. The compound represented by Formula (A) may be administered before, simultaneously with, or after administration of one or more other compounds having an antitumor effect.

Another embodiment provides use of the compound represented by Formula (A) or a salt thereof for potentiating the antitumor effect of antitumor agent(s) containing one or more other compounds having an antitumor effect.

Another embodiment provides use of the compound represented by Formula (A) or a salt thereof for producing an antitumor effect potentiator for antitumor agent(s) containing one or more other compounds having an antitumor effect.

Another embodiment provides use of the compound represented by Formula (A) or a salt thereof for producing an antitumor agent comprising a combination of the compound or a salt thereof and one or more other compounds having an antitumor effect.

Another embodiment provides a pharmaceutical composition for preventing and/or treating a tumor, the composition comprising the compound represented by Formula (A) or a salt thereof, and one or more other compounds having an antitumor effect.

Another embodiment provides an antitumor effect potentiating method for one or more other antitumor agents, comprising administering to a patient the compound represented by Formula (A) or a salt thereof in an amount effective for prevention and/or treatment of a tumor.

Another embodiment provides a method for preventing and/or treating a tumor, comprising administering to a patient a combination of the compound represented by Formula (A) or a salt thereof and one or more other compounds having an antitumor effect in an amount effective for prevention and/or treatment of a tumor.

Another embodiment provide a product as a combined preparation for use simultaneously, sequentially, or at one or more intervals upon prevention and/or treatment of a tumor, the product comprising the compound represented by Formula (A) or a salt thereof and one or more other compounds having an antitumor effect.

## Examples

The following describes the present invention in more detail with reference to Reference Examples, Test Examples, and Examples. However, the scope of the present invention is not limited to these Examples.

Commercially available reagents were used in the Examples, unless otherwise specified.

For silica gel column chromatography, Purif-Pack (registered trademark) SI produced by Moritex Corp., KP-Sil (registered trademark) Silica prepacked column produced by Biotage, or HP-Sil (registered trademark) Silica prepacked column produced by Biotage was used.

For basic silica gel column chromatography, Purif-Pack (registered trademark) NH produced by Moritex Corp or KP-NH (registered trademark) prepacked column produced by Biotage was used.

For preparative thin-layer chromatography, Kieselgel TM 60F 254, Art. 5744 produced by Merck or $NH_2$ Silica Gel 60F254 Plate produced by Wako was used.

NMR spectra were measured by using AL400 (400 MHz; produced by JEOL), Mercury 400 (400 MHz; produced by Agilent Technologies, Inc.) model spectrometer, or Inova 400 (400 MHz; produced by Agilent Technologies, Inc.) model spectrometer equipped with an OMNMR probe (produced by Protasis). The measurement was carried out using tetramethylsilane as an internal standard when tetramethylsilane was contained in a deuterated solvent; otherwise, an NMR solvent was used as an internal standard. All the $\delta$ values are shown by ppm.

The microwave reaction was performed using Discover S-class produced by CEM Corporation, or Initiator produced by Biotage.

LCMS spectra were measured using an Acquity SQD (quadrupole) produced by Waters Corporation under the following conditions.

Column: YMC-Triart C18, 2.0 x 50 mm, 1.9 $\mu$m (produced by YMC)
MS detection: ESI positive
UV detection: 254 and 210 nm
Column flow rate: 0.5 mL/min
Mobile phase: Water/acetonitrile (0.1% formic acid)
Injection volume: 1 $\mu$L

Gradient (Table 1)

| Time (min) | Water (%) | Acetonitrile (%) |
|---|---|---|
| 0 | 95 | 5 |
| 0.1 | 95 | 5 |
| 2.1 | 5 | 95 |
| 3.0 | STOP | |

Preparative reversed-phase HPLC purification was performed using a preparative separation system available from Waters Corporation under the following conditions.

Column: Connected YMC-Actus Triart C18, 20 x 50 mm, 5 μm (produced by YMC) and YMC-Actus Triart C18, 20 x 10 mm, 5 μm (produced by YMC)
UV detection: 254 nm
MS detection: ESI positive
Column flow rate: 25 mL/min
Mobile phase: Water/acetonitrile (0.1% formic acid)
Injection volume: 0.1 to 0.5 mL

Abbreviations

s: Singlet
d: Doublet
t: Triplet
q: Quartet
m: Multiplet
brs: Broad Singlet
brm: Broad Multiplet
dd: Double Doublet
dt: Double Triplet
dq: Double Quartet
ddd: Double Double Doublet
DMSO-$D_6$: Deuterated dimethyl sulfoxide
$CDCl_3$: Deuterated chloroform
$CD_3OD$: Deuterated methanol
$PdCl_2$ (dppf) $CH_2Cl_2$: 1,1'-bis(diphenylphosphino)ferrocene-palladium(II) dichloride-dichloromethane complex
n-butyllithium: normal butyl lithium

Reference Example 1

4-Amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of [(3aR,4R,6R,6aR)-4-(4-amino-5-iodo-pyrrolo[2,3-d]pyrimidin-7-yl)-2,2-dimethyl-3a,4,6,6a-tetrahydrofuro[3,4-d][1,3]dioxol-6-yl]methanol

(2R,3R,4S,5R)-2-(4-Amino-5-iodo-pyrrolo[2,3-d]pyrimidin-7-yl)-5-(hydroxymethyl)tetrahydrofuran-3,4-diol (15 g, 34.8 mmol) was suspended at room temperature in acetone (120 mL) and 2,2-dimethoxypropane (24.4 mL). Thereafter, boron trifluoride diethyl etherate (27.8 mL, 6.3 eq) was added thereto dropwise in an ice bath with stirring so as to keep the internal temperature at 10°C or lower. The obtained mixture was stirred for 75 minutes in an ice bath, and a 5 M aqueous sodium hydroxide solution (60 mL) was then slowly added thereto dropwise so as to keep the internal temperature at 15°C or lower. After acetone was distilled off under reduced pressure, chloroform and water were added thereto, followed by stirring for about 5 minutes. The reaction solution was filtered through a celite bed to remove the generated insoluble matter. Thereafter, the aqueous layer was separated and extracted twice with chloroform. All of the organic layers were combined, washed with water and saturated saline, and dried over sodium sulfate, followed by distilling off

the solvent. The brown oil residue was suspended in hexane (50 mL), and stirred for 2 hours. The formed solid was then collected by filtration, followed by drying, thereby obtaining the title compound (10.7 g, 71%) as a light-brown solid. $^1$H-NMR ($CDCl_3$) δ: 8.23 (1H, s), 7.12 (1H, s), 6.40 (1H, d, J = 11.5 Hz), 5.76-5.74 (2H, brs), 5.69 (1H, d, J = 5.1 Hz), 5.24-5.22 (1H, m), 5.10-5.08 (1H, m), 4.49 (1H, s), 3.97-3.94 (1H, m), 3.78 (1H, t, J = 11.5 Hz), 1.63 (3H, s), 1.36 (3H, s). LCMS (ESI) m/z 433 $[M+H]^+$

Step 2: Synthesis of 7-((3aR,4R,6R,6aR)-6-(aminomethyl)-2,2-dimethyltetrahydrofuro[3,4-d] [1,3]dioxol-4-yl)-5-iodo-7H-pyrrolo[2,3-d]pyrimidin-4-amine

[(3aR,4R,6R,6aR)-4-(4-Amino-5-iodo-pyrrolo[2,3-d]pyrimidin-7-yl)-2,2-dimethyl-3a,4,6,6a-tetrahydrofuro[3,4-d][1,3]dioxol-6-yl]methanol (3.2 g, 7.4 mmol) and phthalimide (2.18 g, 14.8 mmol) were dissolved in tetrahydrofuran (30 mL), and triphenylphosphine (2.9 g, 11.1 mmol) was added thereto with stirring under ice-cooling. After triphenylphosphine was dissolved, diisopropyl azodicarboxylate (2.2 mL, 11.1 mmol) was added thereto dropwise with stirring under ice-cooling. After the reaction solution was stirred for 1.5 h under ice-cooling, the reaction solution was distilled off under reduced pressure, and ethanol (30 mL), water (9 mL), and hydrazine monohydrate (1.2 mL, 24.7 mmol) were added at room temperature to the residue. After being stirred under reflux overnight, the reaction solution was distilled off under reduced pressure. The residue was partitioned between ethyl acetate and saturated sodium bicarbonate solution. Then, the aqueous layer was separated and extracted with ethyl acetate. All of the organic layers were combined and dried over sodium sulfate, followed by distilling off the solvent. The residue was then purified by basic silica gel column chromatography (developing solvent: methanol/chloroform), thereby obtaining the title compound (3.23 g, quantitative) as a yellow solid.
$^1$H-NMR ($CDCl_3$) δ: 8.28 (1H, s), 7.19 (1H, s), 6.13 (1H, d, J = 3.2 Hz), 5.65-5.63 (2H, brs), 5.23 (1H, dd, J = 6.7, 3.2 Hz), 4.93 (1H, dd, J = 6.7, 4.0 Hz), 4.18-4.14 (1H, m), 3.04 (1H, dd, J = 13.4, 4.3 Hz), 2.93 (1H, dd, J = 13.4, 5.9 Hz), 1.61 (3H, s), 1.37 (3H, s). LCMS (ESI) m/z 432 $[M+H]^+$

Step 3: Synthesis of tert-butyl N-(((3aR,4R,6R,6aR)-6-(4-amino-5-iodo-7H-pyrrolo[2,3-d]pyrimidin-7-yl)-2, 2-dimethyltetrahydrofuro[3,4-d][1,3]dioxol-4-yl)methyl)sulfamoyl carbamate

7-((3aR,4R,6R,6aR)-6-(Aminomethyl)-2,2-dimethyltetrahydrofuro[3,4-d] [1,3]dioxol-4-yl)-5-iodo-7H-pyrrolo[2,3-d]pyrimidin-4-amine (3.23 g) was dissolved in chloroform (40 mL), and 1-aza-4-azoniabicyclo[2.2.2]octan-4-ylsulfonyl(tert-butoxycarbonyl)azanido: 1,4-diazabicyclo[2.2.2]octane monohydrochloride (Reference: Organic Letters, 2012, 10, 2626-2629) (6.2 g, 14.1 mmol) was added thereto at room temperature. After the reaction solution was stirred for 2 hours at room temperature, the precipitate was filtered off and washed with chloroform. After the filtrate was concentrated, the residue was purified by silica gel column chromatography (developing solvent: chloroform/methanol), thereby obtaining the title compound (4.0 g, 88%) as a milky-white solid.
$^1$H-NMR ($CDCl_3$) δ: 9.27-9.25 (1H, brs), 8.50 (1H, s), 7.08 (1H, s), 6.04-6.02 (2H, brs), 5.65 (1H, d, J = 4.7 Hz), 5.28 (1H, dd, J = 6.3, 4.7 Hz), 5.07 (1H, dd, J = 6.3, 2.2 Hz), 4.50 (1H, d, J = 2.2 Hz), 3.63-3.49 (2H, m), 1.61 (3H, s), 1.44 (9H, s), 1.35 (3H, s). LCMS (ESI) m/z 611 $[M+H]^+$

Step 4: Synthesis of Reference Example Compound 1

tert-Butyl N-(((3aR,4R,6R,6aR)-6-(4-amino-5-iodo-7H-pyrrolo[2,3-d]pyrimidin-7-yl)-2,2-dimethyltetrahydrofuro[3,4-d] [1,3]dioxol-4-yl)methyl)sulfamoyl carbamate (20 mg, 0.033 mmol), 2-ethynyl-1,3-difluorobenzene (9.0 mg, 0.066 mmol), bis(triphenylphosphine)palladium (II) dichloride (3 mg, 0.0043 mmol), copper iodide (1 mg, 0.0053 mmol), and diisopropylethylamine (0.011 mL, 0.066 mmol) were suspended in tetrahydrofuran (0.20 mL). After the reaction solution was stirred at 70°C for 2 hours, a mixed solution (0.60 mL) of trifluoroacetic acid/water = 4/1 was added thereto at room temperature, followed by stirring at room temperature overnight. After the solvent was distilled off, the residue was purified by basic silica gel column chromatography (developing solvent: methanol/chloroform), thereby obtaining the title compound (14 mg, 91%) as a yellow powder.
$^1$H-NMR ($CD_3OD$) δ: 8.25 (1H, s), 7.68 (1H, s), 7.45-7.37 (1H, m), 7.10-7.04 (2H, m), 5.86 (1H, d, J = 7.3 Hz), 4.86-4.81 (1H, m), 4.31-4.29 (1H, m), 4.27-4.25 (1H, m), 3.40-3.35 (2H, m). LCMS (ESI) m/z 481 $[M+H]^+$

Step 5: Synthesis of 4-amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d] pyrimidine hydrochloride

tert-Butyl N-(((3aR,4R,6R,6aR)-6-(4-amino-5-((2,6-difluorophenyl)ethynyl)-7H-pyrrolo[2,3-d]pyrimidin-7-yl-2,2-dimethyltetrahydrofuro[3,4-d] [1,3]dioxol-4-yl)methyl)sulfamoyl carbamate (8.05 g, 12.9 mmol) was dissolved in acetonitrile (120 mL), and concentrated hydrochloric acid (10.8 mL, 129 mmol) was added thereto at room temperature.

After the mixture was stirred at room temperature for 6 hours, acetonitrile (80 mL) was added thereto, followed by stirring at room temperature overnight. The precipitate was collected by filtration and washed with acetonitrile (80 mL), followed by drying, thereby obtaining the title compound hydrochloride (5.93 g, 88%) as a white solid.
$^1$H-NMR (DMSO-$D_6$) δ: 8.42 (1H, s), 8.25 (1H, s), 7.55 (1H, tt, J = 8.1, 7.7 Hz), 7.28 (2H, dd, J = 8.4, 8.1 Hz), 7.02 (1H, brs), 6.61 (1H, brs), 6.03 (1H, d, J = 6.6 Hz), 4.48 (1H, dd, J = 6.6, 5.1 Hz), 4.12-4.10 (1H, m), 4.06-4.03 (1H, m), 3.22 (1H, dd, J = 13.9, 5.5 Hz), 3.12 (1H, dd, J = 13.2, 5.5 Hz). LCMS (ESI) m/z 481 $[M+H]^+$.

Reference Example 2

4-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]-2-(o-tolyl)thiazole

tert-Butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1,3]dioxol-4-yl)methyl)sulfamoyl carbamate (300 mg, 0.491 mmol), 1,1'-bis(diphenylphosphino)ferrocene-palladium (II) dichloride-dichloromethane complex (40.1 mg, 0.049 mmol), and 4-(4,4,5,5-tetramethyl-1,3,2-dioxaborolan-2-yl)-2-(o-tolyl)thiazole (295 mg, 0.982 mmol) were suspended in a 2 M aqueous sodium carbonate solution (1.23 mL) and dimethoxyethane (5 mL), followed by stirring at 70°C for 17 hours. The reaction solution was partitioned between ethyl acetate and water, and the organic layer was washed with water and concentrated. The residue was dissolved in acetonitrile (1 mL), trifluoroacetic acid (0.5 mL), and water (0.1 mL), followed by stirring at room temperature overnight. After the reaction liquid was concentrated, the residue was purified by basic silica gel column chromatography (developing solvent: methanol/chloroform), thereby obtaining the target product (110 mg, 43%) as a yellowish white solid.
$^1$H-NMR (DMSO-$D_6$) δ: 8.10 (1H, s), 8.08 (1H, s), 8.06 (1H, s), 7.70 (1H, d, J = 7.3 Hz), 7.46-7.35 (3H, m), 6.60 (2H, s), 5.97 (1H, d, J = 6.6 Hz), 5.40 (1H, d, J = 6.6 Hz), 5.20 (1H, d, J = 4.8 Hz), 4.60 (1H, dt, J = 6.6, 5.5 Hz), 4.14-4.11 (1H, m), 4.08-4.04 (1H, m), 2.53 (3H, s). LCMS (ESI) m/z 518 $[M+H]^+$.

Reference Example 3

4-Amino-5-[2-(4-benzyloxyphenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 1-(benzyloxy)-4-ethynylbenzene was used in place of 2-ethynyl-1,3-difluorobenzene.
$^1$H-NMR ($CDCl_3$) δ: 8.29 (1H, s), 7.44-7.27 (8H, m), 6.96 (2H, d, J = 8.8 Hz), 5.69 (1H, d, J = 6.8 Hz), 5.10 (2H, s), 4.87-4.84 (1H, m), 4.36-4.33 (2H, m), 3.46-3.40 (4H, m). LCMS (ESI) m/z 551 $[M+H]^+$.

Reference Example 4

4-Amino-7-[(2R,3R,4S,5R)-3,4-dihydroxy-5-[(sulfamoylamino)methyl]tetrahydrofuran-2-yl]-5-[2-[5-(2-pyridyl)-2-thienyl]ethynyl]pyrrolo[2,3-d]pyrimidine

The title compound was obtained as in step 4 of Reference Example 1, except that 2-(5-ethynylthiophen-2-yl)pyridine was used in place of 2-ethynyl-1,3-difluorobenzene.
$^1$H-NMR ($CD_3OD$) δ: 8.49 (1H, d, J = 5.1 Hz), 8.25 (1H, s), 7.84-7.82 (2H, m), 7.64 (1H, s), 7.64 (1H, d, J = 4.1 Hz), 7.35 (1H, d, J= 4.1 Hz), 7.30-7.27 (1H, m), 5.87 (1H, d, J = 6.8 Hz), 4.87-4.84 (1H, m), 4.32-4.30 (1H, m), 4.28-4.25 (1H, m), 3.39-3.30 (2H, m). LCMS (ESI) m/z 528 $[M+H]^+$.

Reference Example 5

4-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]-2-(2-methoxyphenyl)thiazole

The title compound was obtained as in Reference Example 2, except that 2-(2-methoxyphenyl)-4-(4,4,5,5-tetramethyl-1,3,2-dioxaborolan-2-yl)thiazole was used in place of 4-(4,4,5,5-tetramethyl-1,3,2-dioxaborolan-2-yl)-2-(o-tolyl)thiazole.
$^1$H-NMR ($CD_3OD$) δ: 8.20 (1H, s), 8.17 (1H, dd, J = 7.7, 1.8 Hz), 7.88 (1H, s), 7.84 (1H, s), 7.49 (1H, dt, J = 1.1, 8.8 Hz), 7.24 (1H, d, J = 8.8 Hz), 7.15 (1H, t, J = 7.7 Hz), 5.98 (1H, d, J = 6.6 Hz), 4.84-4.80 (1H, m), 4.36 (1H, dd, J = 5.5, 2.9 Hz), 4.29-4.26 (1H, m), 4.08 (3H, s), 3.47-3.37 (2H, m). LCMS (ESI) m/z 534 $[M+H]^+$.

Reference Example 6

4-Amino-7-[(2R,3R,4S,5R)-3,4-dihydroxy-5-[(sulfamoylamino)methyl]tetrahydrofuran-2-yl]-5-[2-(1-naphthyl)ethynyl]pyrrolo[2,3-d]pyrimidine

The title compound was obtained as in step 4 of Reference Example 1, except that 1-ethynylnaphthalene was used in place of 2-ethynyl-1,3-difluorobenzene.
$^1$H-NMR ($CD_3OD$) δ: 8.36 (1H, d, J = 8.0 Hz), 8.26 (1H, s), 7.93-7.90 (2H, m), 7.77 (1H, d, J = 7.6 Hz), 7.73 (1H, s), 7.64-7.47 (3H, m), 5.90 (1H, d, J = 6.8 Hz), 4.87-4.84 (1H, m), 4.34-4.32 (1H, m), 4.30-4.25 (1H, m), 3.39-3.30 (2H, m). LCMS (ESI) m/z 495 $[M+H]^+$.

Reference Example 7

4-Amino-7-[(2R,3R,4S,5R)-3,4-dihydroxy-5-[(sulfamoylamino)methyl]tetrahydrofuran-2-yl]-5-(3-phenylprop-1-ynyl)pyrrolo[2,3-d]pyrimidine

The title compound was obtained as in step 4 of Reference Example 1, except that prop-2-yn-1-yl benzene was used in place of 2-ethynyl-1,3-difluorobenzene.
$^1$H-NMR ($CD_3OD$) δ: 8.18 (1H, s), 7.43 (1H, s), 7.40 (2H, d, J = 7.6 Hz), 7.33 (2H, dd, J = 7.6, 7.3 Hz), 7.24 (1H, t, J = 7.3 Hz), 5.80 (1H, d, J = 6.8 Hz), 4.87-4.80 (1H, m), 4.30-4.25 (1H, m), 4.24-4.20 (1H, m), 3.88 (2H, s), 3.39-3.30 (2H, m). LCMS (ESI) m/z 459 $[M+H]^+$.

Reference Example 8

4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]quinoline

The title compound was obtained as in step 4 of Reference Example 1, except that 4-ethynylquinoline was used in place of 2-ethynyl-1,3-difluorobenzene.
$^1$H-NMR ($CD_3OD$) δ: 8.83 (1H, d, J = 4.4 Hz), 8.40 (1H, d, J = 9.5 Hz), 8.28 (1H, s), 8.06 (1H, d, J = 8.0 Hz), 7.91 (1H, s), 7.84 (1H, dd, J = 8.8, 8.0 Hz), 7.76-7.73 (2H, m), 5.93 (1H, d, J = 6.6 Hz), 4.87-4.80 (1H, m), 4.35-4.30 (1H, m), 4.29-4.26 (1H, m), 3.40-3.30 (2H, m). LCMS (ESI) m/z 496 $[M+H]^+$.

Reference Example 9

4-Amino-7-[(2R,3R,4S,5R)-3,4-dihydroxy-5-[(sulfamoylamino)methyl]tetrahydrofuran-2-yl]-5-[2-[4-(phenoxymethyl)phenyl]ethynyl]pyrrolo[2,3-d]pyrimidine

The title compound was obtained as in step 4 of Reference Example 1, except that 1-ethynyl-4-(phenoxymethyl)benzene was used in place of 2-ethynyl-1,3-difluorobenzene.
$^1$H-NMR ($CD_3OD$) δ: 8.25 (1H, s), 7.61 (1H, s), 7.57 (2H, d, J = 8.3 Hz), 7.49 (2H, d, J = 8.3 Hz), 7.28 (2H, dd, J = 8.0, 7.8 Hz), 7.00 (2H, d, J = 7.8 Hz), 6.94 (1H, t, J = 8.0 Hz), 5.86 (1H, d, J= 6.8 Hz), 5.12 (2H, s), 4.85-4.75 (1H, m), 4.35-4.30 (1H, m), 4.28-4.25 (1H, m), 3.40-3.30 (2H, m). LCMS (ESI) m/z 551 $[M+H]^+$.

Reference Example 10

4-Amino-7-[(2R,3R,4S,5R)-3,4-dihydroxy-5-[(sulfamoylamino)methyl]tetrahydrofuran-2-yl]-5-[2-(1-phenylcyclopropyl)ethynyl]pyrrolo[2,3-d]pyrimidine

The title compound was obtained as in step 4 of Reference Example 1, except that (1-ethynylcyclopropyl)benzene was used in place of 2-ethynyl-1,3-difluorobenzene.
$^1$H-NMR ($CD_3OD$) δ: 8.21 (1H, s), 7.46 (1H, s), 7.43-7.40 (2H, m), 7.32 (2H, d, J = 8.0 Hz), 7.23-7.19 (1H, m), 5.82 (1H, d, J = 6.8 Hz), 4.82-4.79 (1H, m), 4.30-4.28 (1H, m), 4.25-4.21 (1H, m), 3.37-3.34 (2H, m), 1.54-1.50 (2H, m), 1.40-1.37 (2H, m). LCMS (ESI) m/z 485 $[M+H]^+$.

Reference Example 11

1-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]isoquinoline

The title compound was obtained as in step 4 of Reference Example 1, except that 1-ethynylisoquinoline was used in place of 2-ethynyl-1,3-difluorobenzene.
$^1$H-NMR (DMSO-$D_6$) δ: 8.56 (1H, d, J = 8.0 Hz), 8.45 (1H, d, J = 8.0 Hz), 8.23 (1H, s), 8.19 (1H, s), 8.07 (1H, d, J = 8.0 Hz), 7.90 (1H, d, J = 8.0 Hz), 7.88-7.78 (2H, m), 7.38-7.34 (1H, brs), 6.64 (2H, s), 5.99 (1H, d, J = 6.8 Hz), 5.48-5.46 (1H, brs), 5.29-5.25 (1H, brs), 4.63-4.59 (1H, m), 4.15-4.11 (1H, m), 4.10-4.06 (1H, m), 3.25-3.21 (1H, m), 3.18-3.12 (1H, m). LCMS (ESI) m/z 496 $[M+H]^+$.

Reference Example 12

4-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]-2-phenyl-oxazol

The title compound was obtained as in Reference Example 2, except that 2-phenyl-5-(4,4,5,5-tetramethyl-1,3,2-dioxaborolan-2-yl)oxazol was used in place of 4-(4,4,5,5-tetramethyl-1,3,2-dioxaborolan-2-yl)-2-(o-tolyl)thiazole.
$^1$H-NMR ($CD_3OD$) δ: 8.28 (1H, s), 8.07-8.00 (2H, m), 7.93 (1H, s), 7.51-7.49 (3H, m), 7.48 (1H, s), 6.05 (1H, d, J = 6.3 Hz), 4.77-4.71 (2H, m), 4.33 (1H, dd, J = 5.4, 3.2 Hz), 4.26-4.22 (1H, m), 3.45-3.35 (2H, m). LCMS (ESI) m/z 488 $[M+H]^+$.

Reference Example 13

5-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]quinoline

The title compound was obtained as in step 4 of Reference Example 1, except that 5-ethynylquinoline was used in place of 2-ethynyl-1,3-difluorobenzene.
$^1$H-NMR ($CD_3OD$) δ: 8.91 (1H, dd, J = 4.4, 1.7 Hz), 8.84-8.78 (1H, m), 8.26 (1H, s), 8.04 (1H, d, J = 8.5 Hz), 7.89 (1H, dd, J = 7.9, 1.1 Hz), 7.81 (1H, s), 7.80-7.76 (1H, m), 7.67 (1H, dd, J = 8.5, 4.4 Hz), 5.92 (1H, d, J = 6.8 Hz), 4.87-4.82 (1H, m), 4.35-4.33 (1H, m), 4.29-4.26 (1H, m), 3.45-3.36 (2H, m). LCMS (ESI) m/z 496 $[M+H]^+$.

Reference Example 14

4-Amino-7-[(2R,3R,4S,5R)-3,4-dihydroxy-5-[(sulfamoylamino)methyl]tetrahydrofuran-2-yl]-5-[2-[2-(trifluoromethoxy)phenyl]ethynyl]pyrrolo[2,3-d]pyrimidine

The title compound was obtained as in step 4 of Reference Example 1, except that 1-ethynyl-2-(trifluoromethoxy)benzene was used in place of 2-ethynyl-1,3-difluorobenzene.
$^1$H-NMR ($CD_3OD$) δ: 8.26 (1H, s), 7.68-7.62 (1H, m), 7.65 (1H, s), 7.50-7.46 (1H, m), 7.42-7.38 (2H, m), 5.86 (1H, d, J = 7.1 Hz), 4.86-4.81 (1H, m), 4.31-4.29 (1H, m), 4.27-4.25 (1H, m), 3.40-3.35 (2H, m). LCMS (ESI) m/z 529 $[M+H]^+$.

Reference Example 15

4-Amino-7-[(2R,3R,4S,5R)-3,4-dihydroxy-5-[(sulfamoylamino)methyl]tetrahydrofuran-2-yl]-5-[2-(2-methoxy-1-naphthyl)ethynyl]pyrrolo[2,3-d]pyrimidine

The title compound was obtained as in step 4 of Reference Example 1, except that 1-ethynyl-2-methoxynaphthalene was used in place of 2-ethynyl-1,3-difluorobenzene.
$^1$H-NMR ($CD_3OD$) δ: 8.23 (1H, s), 7.89-7.80 (2H, m), 7.66-7.57 (1H, m), 7.64 (1H, s), 7.58-7.54 (1H, m), 7.42-7.36 (2H, m), 5.90 (1H, d, J = 8.0 Hz), 4.83-4.81 (1H, m), 4.37-4.33 (1H, m), 4.27-4.25 (1H, m), 4.06 (3H, s), 3.40-3.35 (2H, m). LCMS (ESI) m/z 525 $[M+H]^+$.

Reference Example 16

4-Amino-7-[(2R,3R,4S,5R)-3,4-dihydroxy-5-[(sulfamoylamino)methyl]tetrahydrofuran-2-yl]-5-[2-(2,6-dimethoxyphenyl)ethynyl]pyrrolo[2,3-d]pyrimidine

The title compound was obtained as in step 4 of Reference Example 1, except that 2-ethynyl-1,3-dimethoxybenzene was used in place of 2-ethynyl-1,3-difluorobenzene.
$^1$H-NMR ($CD_3OD$) δ: 8.22 (1H, s), 7.50 (1H, s), 7.28 (1H, t, J = 8.5 Hz), 6.69 (2H, d, J = 8.5 Hz), 5.86 (1H, d, J = 6.8 Hz), 4.83-4.81 (1H, m), 4.32-4.30 (1H, m), 4.27-4.25 (1H, m), 3.91 (6H, s), 3.40-3.35 (2H, m). LCMS (ESI) m/z 505 $[M+H]^+$.

Reference Example 17

8-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]quinoline

The title compound was obtained as in step 4 of Reference Example 1, except that 8-ethynylquinoline was used in place of 2-ethynyl-1,3-difluorobenzene.
$^1$H-NMR ($CD_3OD$) δ: 8.99 (1H, dd, 4.1, 1.7 Hz), 8.33 (1H, dd, 8.5, 1.7 Hz), 8.22 (1H, s), 7.91 (1H, s), 7.90-7.82 (2H, m), 7.58-7.52 (2H, m), 5.86 (1H, d, J = 6.8 Hz), 4.87-4.85 (1H, m), 4.32-4.30 (1H, m), 4.27-4.25 (1H, m), 3.40-3.35 (2H, m). LCMS (ESI) m/z 496 $[M+H]^+$.

Reference Example 18

4-Amino-5-[2-[2-(difluoromethoxy)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 1-(difluoromethoxy)-2-ethynylbenzene was used in place of 2-ethynyl-1,3-difluorobenzene.
$^1$H-NMR ($CD_3OD$) δ: 8.24 (1H, s), 7.62 (1H, s), 7.58 (1H, d, J = 7.8, 1.7 Hz), 7.43-7.39 (1H, m), 7.28-7.23 (2H, m), 7.01 (1H, t, J = 7.2 Hz), 5.86 (1H, d, J = 6.8 Hz), 4.87-4.85 (1H, m), 4.32-4.30 (1H, m), 4.27-4.25 (1H, m), 3.40-3.35 (2H, m). LCMS (ESI) m/z 511 $[M+H]^+$.

Reference Example 19

4-Amino-7-[(2R,3R,4S,5R)-3,4-dihydroxy-5-[(sulfamoylamino)methyl]tetrahydrofuran-2-yl]-5-[2-(1H-pyrazolo[4,3-b]pyridin-5-yl)ethynyl]pyrrolo[2,3-d]pyrimidine

The title compound was obtained as in step 4 of Reference Example 1, except that 5-ethynyl-1H-pyrazolo[4,3-b]pyridine was used in place of 2-ethynyl-1,3-difluorobenzene.
$^1$H-NMR ($CD_3OD$) δ: 8.25 (1H, s), 8.23 (1H, s), 8.06 (1H, d, J = 8.8 Hz), 7.75 (1H, s), 7.64 (1H, d, J = 8.8 Hz), 5.89 (1H, d, J = 6.8 Hz), 4.87-4.85 (1H, m), 4.34-4.30 (1H, m), 4.27-4.25 (1H, m), 3.40-3.35 (2H, m). LCMS (ESI) m/z 486 $[M+H]^+$.

Reference Example 20

4-Amino-5-[2-(4-amino-2-fluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 4-ethynyl-3-fluoroaniline was used in place of 2-ethynyl-1,3-difluorobenzene.
$^1$H-NMR ($CD_3OD$) δ: 8.22 (1H, s), 7.48 (1H, s), 7.21-7.17 (1H, m), 6.46-6.41 (2H, m), 5.84 (1H, d, J = 6.8 Hz), 4.87-4.85 (1H, m), 4.32-4.28 (1H, m), 4.27-4.24 (1H, m), 3.40-3.35 (2H, m). LCMS (ESI) m/z 478 $[M+H]^+$.

Reference Example 21

4-Amino-7-[(2R,3R,4S,5R)-3,4-dihydroxy-5-[(sulfamoylamino)methyl]tetrahydrofuran-2-yl]-5-(2-indan-1-ylethynyl)pyrrolo[2,3-d]pyrimidine

The title compound was obtained as in step 4 of Reference Example 1, except that 1-ethynyl-2,3-dihydro-1H-indene was used in place of 2-ethynyl-1,3-difluorobenzene.
$^1$H-NMR ($CD_3OD$) δ: 8.19 (1H, s), 7.42 (1H, s), 7.42-7.38 (1H, m), 7.26-7.18 (3H, m), 5.81 (1H, d, J = 7.1 Hz), 4.84-4.79 (1H, m), 4.30-4.18 (3H, m), 3.40-3.35 (2H, m), 3.04-2.91 (2H, m), 2.62-2.54 (1H, m), 2.19-2.11 (1H, m). LCMS (ESI) m/z 485 $[M+H]^+$.

Reference Example 22

4-Amino-7-[(2R,3R,4S,5R)-3,4-dihydroxy-5-[(sulfamoylamino)methyl]tetrahydrofuran-2-yl]-5-[2-(2-methylsulfonylphenyl)ethynyl]pyrrolo[2,3-d]pyrimidine

The title compound was obtained as in step 4 of Reference Example 1, except that 1-ethynyl-2-(methylsulfonyl)benzene was used in place of 2-ethynyl-1,3-difluorobenzene.
$^1$H-NMR ($CD_3OD$) δ: 8.24 (1H, s), 8.07 (1H, dd, J = 7.7, 1.3 Hz), 7.79 (1H, dd, J = 7.7, 1.3 Hz), 7.77 (1H, s), 7.75-7.70 (1H, m), 7.62-7.57 (1H, m), 5.89 (1H, d, J = 6.8 Hz), 4.85-4.81 (1H, m), 4.33-4.29 (1H, m), 4.28-4.24 (1H, m), 3.40-3.35 (2H, m), 3.30 (3H, s). LCMS (ESI) m/z 523 $[M+H]^+$.

Reference Example 23

4-[4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3,5-difluoro-phenyl]morpholine

Step 1: Synthesis of 4-(4-ethynyl-3,5-difluorophenyl)morpholine

2-Ethynyl-1,3,5-trifluorobenzene (52 mg, 0.33 mmol) and cesium carbonate (163 mg, 0.50 mmol) were dissolved in N,N-dimethylformamide (0.50 mL). Morpholine (0.044 mL, 0.50 mmol) was added thereto at room temperature, followed by stirring at 80°C overnight. After the resulting mixture was air-cooled to room temperature, ethyl acetate (2.0 mL) and a saturated aqueous ammonium chloride solution (1.0 mL) were sequentially added thereto, and the mixture was partitioned into an aqueous layer and an organic layer. The organic layer was then sequentially washed with water and saturated saline, and dried over anhydrous sodium sulfate, followed by distilling off the solvent. The residue was purified by silica gel column chromatography (developing solvent: ethyl acetate/hexane), thereby obtaining 4-(4-ethynyl-3,5-difluorophenyl)morpholine (40 mg, 54%) as a white solid.
$^1$H-NMR ($CDCl_3$) δ: 6.37 (2H, d, J = 10.7 Hz), 3.83 (4H, dd, J = 5.7, 4.2 Hz), 3.40 (1H, s), 3.19 (4H, dd, J = 5.7, 4.2 Hz). LCMS (ESI) m/z 224 $[M+H]^+$

Step 2: Synthesis of Reference Example Compound 23

The title compound was obtained as in step 4 of Reference Example 1, except that 4-(4-ethynyl-3,5-difluorophenyl)morpholine was used in place of 2-ethynyl-1,3-difluorobenzene.
$^1$H-NMR ($CD_3OD$) δ: 8.23 (1H, s), 7.56 (1H, s), 6.63 (2H, d, J = 11.5 Hz), 5.85 (1H, d, J = 7.1 Hz), 4.85-4.79 (1H, m), 4.30 (1H, dd, J = 5.6, 2.4 Hz), 4.27-4.23 (1H, m), 3.80 (4H, t, J = 4.9 Hz), 3.42-3.32 (2H, m), 3.27-3.22 (4H, m). LCMS (ESI) m/z 566 $[M+H]^+$.

Reference Example 24

4-Amino-5-[2-(4-amino-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 4-ethynyl-3,5-difluoroaniline was used in place of 2-ethynyl-1,3-difluorobenzene.
$^1$H-NMR ($CD_3OD$) δ: 8.23 (1H, s), 7.52 (1H, s), 6.26 (2H, d, J = 10.2 Hz), 5.85 (1H, d, J = 6.8 Hz), 4.85-4.81 (1H, m), 4.33-4.29 (1H, m), 4.26-4.23 (1H, m), 3.40-3.34 (2H, m). LCMS (ESI) m/z 496 $[M+H]^+$.

Reference Example 25

4-Amino-5-[2-[2,6-difluoro-4-(methylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-ethynyl-3,5-difluoro-N-methylaniline

The title compound was obtained as in step 1 of Reference Example 23, except that methylamine was used in place of morpholine.
$^1$H-NMR ($CDCl_3$) δ: 6.11 (2H, d, J = 10.6 Hz), 4.22-4.14 (1H, brm), 3.39 (1H, s), 2.84 (3H, s). LRMS (ESI) m/z 168 $[M+H]^+$

Step 2: Synthesis of Reference Example Compound 25

The title compound was obtained as in step 4 of Reference Example 1, except that 4-ethynyl-3,5-difluoro-N-methylaniline was used in place of 2-ethynyl-1,3-difluorobenzene.
$^1$H-NMR ($CD_3OD$) δ: 8.24 (1H, s), 8.18 (1H, brs), 8.03 (1H, dd, J = 7.7, 1.1 Hz), 7.53 (1H, s), 7.48 (1H, t, J = 7.7 Hz), 6.22 (2H, d, J = 11.0 Hz), 5.86 (1H, d, J = 7.0 Hz), 4.86-4.81 (1H, m), 4.32 (1H, dd, J = 5.5, 2.2 Hz), 4.27-4.25 (1H, m), 3.43-3.34 (2H, m), 2.79 (3H, s). LCMS (ESI) m/z 510 $[M+H]^+$.

Reference Example 26

4-Amino-7-[(2R,3R,4S,5R)-3,4-dihydroxy-5-[(sulfamoylamino)methyl]tetrahydrofuran-2-yl]-5-[2-[4-(ethylamino)-2,6-difluoro-phenyl]ethynyl]pyrrolo[2,3-d] pyrimidine

Step 1: Synthesis of N-ethyl-4-ethynyl-3,5-difluoroaniline

The title compound was obtained as in step 1 of Reference Example 23, except that ethylamine was used in place of morpholine.
$^1$H-NMR ($CDCl_3$) δ: 6.10 (2H, d, J = 10.3 Hz), 4.07-4.00 (1H, brm), 3.38 (1H, s), 3.18-3.11 (2H, m), 1.27 (5H, t, J = 7.3 Hz). LRMS (ESI) m/z 182 $[M+H]^+$

Step 2: Synthesis of Reference Example Compound 26

The title compound was obtained as in step 4 of Reference Example 1, except that N-ethyl-4-ethynyl-3,5-difluoroaniline was used in place of 2-ethynyl-1,3-difluorobenzene.
$^1$H-NMR ($CD_3OD$) δ: 8.24 (1H, s), 8.07-8.00 (1H, m), 7.59-7.45 (1H, m), 7.57 (1H, s), 6.23 (2H, d, J = 11.0 Hz), 5.89 (1H, d, J = 7.0 Hz), 4.80 (1H, dd, J = 7.0, 5.5 Hz), 4.32 (1H, dd, J = 5.5, 2.6 Hz), 4.26 (1H, ddd, J = 4.0, 3.7, 2.6 Hz), 3.41 (1H, dd, J = 13.2, 3.7 Hz), 3.36 (1H, dd, J = 13.2, 4.0 Hz), 3.13 (2H, q, J = 7.3 Hz), 1.25 (3H, t, J = 7.3 Hz). LCMS (ESI) m/z 524 $[M+H]^+$

Reference Example 27

4-Amino-7-[(2R,3R,4S,5R)-3,4-dihydroxy-5-[(sulfamoylamino)methyl]tetrahydrofuran-2-yl]-5-[2-[3-(isopropylamino)phenyl]ethynyl]pyrrolo[2,3-d]pyrimidine

The title compound was obtained as in step 4 of Reference Example 1, except that 3-ethynyl-N-isopropylaniline was used in place of 2-ethynyl-1,3-difluorobenzene.
$^1$H-NMR ($CD_3OD$) δ: 8.24 (1H, s), 7.55 (1H, s), 7.11 (1H, t, J = 8.3 Hz), 6.79-6.76 (2H, m), 6.67-6.65 (1H, m), 5.86 (1H, d, J = 6.8 Hz), 4.84-4.79 (1H, m), 4.31 (1H, dd, J = 5.6, 2.4 Hz), 4.25 (1H, q, J = 3.2 Hz), 3.67-3.54 (1H, m), 3.39-3.35 (2H, m), 1.20 (6H, d, J = 6.3 Hz). LCMS (ESI) m/z 502 $[M+H]^+$.

Reference Example 28

4-Amino-5-[2-(5-amino-2-fluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 3-ethynyl-4-fluoroaniline was used in place of 2-ethynyl-1,3-difluorobenzene.

$^1$H-NMR (DMSO-$D_6$) δ: 8.18 (1H, s), 7.90 (1H, s), 7.38-7.33 (1H, m), 6.99 (1H, t, J = 9.3 Hz), 6.70 (1H, dd, J = 6.1, 2.9 Hz), 6.62-6.56 (3H, m), 5.92 (1H, d, J = 7.1 Hz), 5.40 (1H, d, J = 6.3 Hz), 5.23 (1H, d, J = 4.4 Hz), 5.16 (2H, s), 4.57 (1H, dd, J = 12.1, 6.7 Hz), 4.12-4.08 (1H, m), 4.07-4.03 (1H, m), 3.24-3.08 (2H, m). LCMS (ESI) m/z 478 $[M+H]^+$.

Reference Example 29

4-Amino-5-[2-[2,6-difluoro-4-[(3R)-3-fluoropyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of (3R)-1-(4-ethynyl-3,5-difluorophenyl)-3-fluoropyrrolidine

The title compound was obtained as in step 1 of Reference Example 23, except that (R)-3-fluoropyrrolidine was used in place of morpholine.
$^1$H-NMR ($CDCl_3$) δ: 6.07 (2H, d, J = 10.3 Hz), 5.38 (1H, d, J = 52.8 Hz), 3.59-3.38 (5H, m), 2.47-2.36 (1H, m), 2.27-2.06 (1H, m). LCMS (ESI) m/z 226 $[M+H]^+$

Step 2: Synthesis of Reference Example Compound 29

The title compound was obtained as in step 4 of Reference Example 1, except that (3R)-1-(4-ethynyl-3,5-difluorophenyl)-3-fluoropyrrolidine was used in place of 2-ethynyl-1,3-difluorobenzene.
$^1$H-NMR (DMSO-$D_6$) δ: 8.16 (1H, s), 7.84 (1H, s), 7.35-7.30 (1H, m), 6.58 (2H, s), 6.42 (2H, d, J = 11.2 Hz), 5.91 (1H, d, J = 7.1 Hz), 5.45 (1H, d, J = 52.7 Hz), 5.37 (1H, d, J = 6.3 Hz), 5.20 (1H, d, J = 4.4 Hz), 4.56 (1H, dd, J = 12.1, 6.7 Hz), 4.11-4.07 (1H, m), 4.06-4.01 (1H, m), 3.61-3.45 (3H, m), 3.41-3.33 (1H, m), 3.24-3.16 (1H, m), 3.14-3.06 (1H, m), 2.30-2.10 (2H, m). LCMS (ESI) m/z 568 $[M+H]^+$.

Reference Example 30

4-Amino-5-[2-[2,6-difluoro-4-[(3R)-3-hydroxypyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of (3R)-1-(4-ethynyl-3,5-difluorophenyl)-pyrrolidin-3-ol

The title compound was obtained as in step 1 of Reference Example 23, except that (R)-pyrrolidin-3-ol was used in place of morpholine.
$^1$H-NMR ($CDCl_3$) δ: 6.02 (2H, d, J = 10.5 Hz), 4.63-4.60 (1H, m), 3.50-3.43 (2H, m), 3.40-3.38 (1H, m), 3.33 (1H, dt, J = 3.3, 9.0 Hz), 3.22 (1H, d, J = 10.7 Hz), 2.22-2.11 (1H, m), 2.11-2.06 (1H, m). LCMS (ESI) m/z 224 $[M+H]^+$

Step 2: Synthesis of Reference Example Compound 30

The title compound was obtained as in step 4 of Reference Example 1, except that (3R)-1-(4-ethynyl-3,5-difluorophenyl)-pyrrolidin-3-ol was used in place of 2-ethynyl-1,3-difluorobenzene.
$^1$H-NMR (DMSO-$D_6$) δ: 8.17 (1H, s), 7.85 (1H, s), 7.36 (1H, dd, J = 7.7, 4.8 Hz), 6.60 (2H, s), 6.35 (2H, d, J = 11.0 Hz), 5.91 (1H, d, J = 7.0 Hz), 5.39 (1H, d, J = 6.2 Hz), 5.23 (1H, d, J = 4.4 Hz), 5.06 (1H, d, J = 3.7 Hz), 4.60-4.55 (1H, m), 4.42-4.37 (1H, m), 4.11-4.07 (1H, m), 4.06-4.02 (1H, m), 3.45-3.37 (2H, m), 3.24-3.18 (1H, m), 3.16-3.09 (2H, m), 2.07-1.99 (1H, m), 1.94-1.86 (1H, m). LCMS (ESI) m/z 566.3 $[M+H]^+$.

Reference Example 31

4-Amino-5-[3-(2,6-difluorophenyl)prop-1-ynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 1,3-difluoro-2-(prop-2-yn-1-yl)benzene was used in place of 2-ethynyl-1,3-difluorobenzene.
$^1$H-NMR ($CD_3OD$) δ: 8.20 (1H, s), 7.40 (1H, s), 7.36-7.30 (1H, m), 7.04-6.99 (2H, m), 5.79 (1H, d, J = 6.8 Hz), 4.80-4.69 (1H, m), 4.28-4.25 (1H, m), 4.22-4.20 (1H, m), 3.88 (2H, s), 3.39-3.32 (2H, m). LCMS (ESI) m/z 495 [M+H].

Reference Example 32

4-Amino-5-[2-[2,6-difluoro-4-(2-hydroxyethylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

2-((4-Ethynyl-3,5-difluorophenyl)amino)ethanol was obtained as in step 1 of Reference Example 23, except that 2-aminoethanol was used in place of morpholine, and the title compound was then obtained as in step 4 of Reference Example 1, except that the thus-obtained 2-((4-ethynyl-3,5-difluorophenyl)amino)ethanol was used in place of 2-ethynyl-1,3-difluorobenzene.
$^1$H-NMR (DMSO-$D_6$) δ: 8.20 (1H, s), 7.91 (1H, s), 7.38 (1H, s), 7.03 (2H, t, J = 9.5 Hz), 6.61 (2H, brs), 5.93 (1H, d, J = 6.6 Hz), 5.47-5.36 (1H, m), 5.34-5.18 (1H, m), 4.60 (1H, t, J = 10.0 Hz), 4.13-4.08 (1H, m), 4.08-4.04 (1H, m), 3.26-3.19 (1H, m), 3.16-3.08 (1H, m), 2.55 (2H, s). LCMS (ESI) m/z 540.3 $[M+H]^+$.

Reference Example 33

4-Amino-5-[2-[2,6-difluoro-4-(2-oxopyrrolidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-(4-ethynyl-3,5-difluorophenyl)pyrrolidin-2-one

The title compound was obtained as in step 1 of Reference Example 23, except that pyrrolidin-2-one was used in place of morpholine.
$^1$H-NMR ($CDCl_3$) δ: 7.35 (2H, d, J = 10.0 Hz), 3.82 (2H, t, J = 7.2 Hz), 3.47 (1H, s), 2.65 (2H, t, J = 8.2 Hz), 2.23-2.15 (2H, m). LCMS (ESI) m/z 222 $[M+H]^+$

Step 2: Synthesis of Reference Example Compound 33

The title compound was obtained as in step 4 of Reference Example 1, except that 1-(4-ethynyl-3,5-difluorophenyl)pyrrolidin-2-one was used in place of 2-ethynyl-1,3-difluorobenzene.
$^1$H-NMR (DMSO-$D_6$) δ: 8.18 (1H, s), 7.97 (1H, s), 7.64 (2H, d, J = 10.5 Hz), 7.33-7.30 (1H, brs), 6.58 (2H, s), 5.92 (1H, d, J = 7.3 Hz), 5.38 (1H, d, J = 6.8 Hz), 5.21 (1H, d, J = 4.1 Hz), 4.60-4.54 (1H, m), 4.08-4.07 (1H, m), 4.05-4.02 (1H, m), 3.84 (2H, t, J = 7.2 Hz), 3.27-3.18 (1H, m), 3.15-3.08 (1H, m), 2.56 (2H, t, J = 8.2 Hz), 2.10-2.02 (2H, m). LCMS (ESI) m/z 564 [M+H].

Reference Example 34

4-[4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3-ethoxy-5-fluoro-phenyl]morpholine

Step 1: Synthesis of 4-(3-ethoxy-4-ethynyl-5-fluorophenyl)morpholine

4-(4-ethynyl-3,5-difluorophenyl)morpholine (100 mg, 0.448 mmol) was dissolved in ethanol (3 mL). Sodium ethoxide (a 21 wt% ethanol solution, 0.168 mL, 0.448 mmol) was added thereto, followed by stirring for 0.5 hours in a sealed container at 160°C. After the resulting mixture was air-cooled to room temperature, ethyl acetate (5.0 mL) and a saturated ammonium chloride solution (2.0 mL) were sequentially added thereto, and the mixture was partitioned into an aqueous layer and an organic layer. The organic layer was then sequentially washed with water and saturated saline, and dried over anhydrous sodium sulfate, followed by distilling off the solvent. The residue was purified by silica gel column chromatography (developing solvent: hexane/ethyl acetate), thereby obtaining 4-(3-ethoxy-4-ethynyl-5-fluorophenyl)morpholine (60 mg, 54%) as a green solid.
$^1$H-NMR ($CDCl_3$) δ: 6.20 (1H, dd, J = 12.1, 2.2 Hz), 6.13 (1H, s), 4.12-4.07 (2H, m), 3.85-3.82 (4H, m), 3.41 (1H, s), 3.20-3.15 (4H, m), 1.46 (3H, t, J = 7.1 Hz). LCMS (ESI) m/z 250 $[M+H]^+$

Step 2: Synthesis of Reference Example Compound 34

The title compound was obtained as in Reference Example 1, except that 4-(3-ethoxy-4-ethynyl-5-fluorophenyl)morpholine was used in place of 2-ethynyl-1,3-difluorobenzene.
$^1$H-NMR (DMSO-$D_6$) δ: 8.17 (1H, s), 7.77 (1H, s), 7.41-7.36 (1H, m), 6.60 (2H, s), 6.48 (1H, d, J = 13.6 Hz), 6.42 (1H, s), 5.90 (1H, d, J = 7.0 Hz), 5.37 (1H, d, J = 6.6 Hz), 5.21 (1H, d, J = 4.4 Hz), 4.60-4.55 (1H, m), 4.20 (2H, q, J = 7.0

Hz), 4.12-4.07 (1H, m), 4.06-4.02 (1H, m), 3.74-3.69 (4H, m), 3.27-3.05 (6H, m), 1.36 (3H, t, J = 7.0 Hz). LCMS (ESI) m/z 592 $[M+H]^+$.

Reference Example 35

4-Amino-7-[(2R,3R,4S,5R)-3,4-dihydroxy-5-[(sulfamoylamino)methyl]tetrahydrofuran-2-yl]-5-[2-(2-ethoxy-4,6-difluorophenyl)ethynyl]pyrrolo[2,3-d]pyrimidine

Step 1: Synthesis of 1-ethoxy-2-ethynyl-3,5-difluorobenzene

The title compound was obtained as in step 1 of Reference Example 34, except that 2-ethynyl-1,3,5-trifluorobenzene was used in place of 4-(4-ethynyl-3,5-difluorophenyl)morpholine.
$^1$H-NMR ($CDCl_3$) δ: 6.48-6.39 (2H, m), 4.09 (2H, q, J = 7.0 Hz), 3.45 (1H, s), 1.47 (3H, t, J = 7.0 Hz).

Step 2: Synthesis of Reference Example Compound 35

The title compound was obtained as in Reference Example 1, except that 1-ethoxy-2-ethynyl-3,5-difluorobenzene was used in place of 2-ethynyl-1,3-difluorobenzene.
$^1$H-NMR (DMSO-$D_6$) δ: 8.18 (1H, s), 7.90 (1H, s), 7.36 (1H, s), 7.02-6.97 (2H, m), 6.61-6.58 (2H, m), 5.92 (1H, d, J = 7.1 Hz), 5.41-5.36 (1H, m), 5.24-5.21 (1H, m), 4.61-4.55 (1H, m), 4.24 (2H, q, J = 7.0 Hz), 4.11-4.08 (1H, m), 4.07-4.03 (1H, m), 3.25-3.07 (2H, m), 1.38 (3H, t, J = 7.0 Hz). LCMS (ESI) m/z 525 $[M+H]^+$.

Reference Example 36

4-Amino-7-[(2R,3R,4S,5R)-3,4-dihydroxy-5-[(sulfamoylamino)methyl]tetrahydrofuran-2-yl]-5-[3-(2-fluorophenyl)prop-1-ynyl]pyrrolo[2,3-d]pyrimidine

The title compound was obtained as in Reference Example 1, except that 1-fluoro-2-(prop-2-yn-1-yl)benzene was used in place of 2-ethynyl-1,3-difluorobenzene.
$^1$H-NMR ($CD_3OD$) δ: 8.20 (1H, s), 7.54 (1H, t, J = 8.0 Hz), 7.45 (1H, s), 7.33-7.25 (1H, m), 7.20-7.16 (1H, m), 7.13-7.08 (1H, m), 5.82 (1H, d, J = 6.8 Hz), 4.80-4.77 (1H, m), 4.30-4.28 (1H, m), 4.24-4.23 (1H, m), 3.90 (2H, s), 3.40-3.31 (2H, m). LCMS (ESI) m/z 477

[M+H].

Reference Example 37

4-[4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3,5-difluoro-phenyl]thiomorpholine Step 1: Synthesis of 4-(4-ethynyl-3,5-difluorophenyl)thiomorpholine

The title compound was obtained as in step 1 of Reference Example 23, except that thiomorpholine was used in place of morpholine.
$^1$H-NMR ($CDCl_3$) δ: 6.32 (2H, d, J = 11.0 Hz), 3.69-3.66 (4H, m), 3.38 (1H, s), 2.69-2.66 (4H, m). LCMS (ESI) m/z 240 $[M+H]^+$ Step 2: Synthesis of Reference Example Compound 37
The title compound was obtained as in Reference Example 1, except that 4-(4-ethynyl-3,5-difluorophenyl)thiomorpholine was used in place of 2-ethynyl-1,3-difluorobenzene.
$^1$H-NMR (DMSO-$D_6$) δ: 8.18 (1H, s), 7.87 (1H, s), 7.36-7.32 (1H, m), 6.78 (2H, d, J = 12.1 Hz), 6.59 (2H, s), 5.92 (1H, d, J = 7.0 Hz), 5.46-5.32 (1H, m), 5.30-5.16 (1H, m), 4.59-4.55 (1H, m), 4.11-4.07 (1H, m), 4.06-4.02 (1H, m), 3.78-3.72 (4H, m), 3.24-3.06 (2H, m), 2.63-2.58 (4H, m). LCMS (ESI) m/z 582 $[M+H]^+$.

Reference Example 38

4-Amino-5-[2-[2,6-difluoro-4-(3-hydroxy-1-piperidyl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-(4-ethynyl-3,5-difluorophenyl)piperidin-3-ol

The title compound was obtained as in step 1 of Reference Example 23, except that piperidin-3-ol was used

in place of morpholine.
$^1$H-NMR ($CDCl_3$) δ: 6.39 (2H, dt, J = 17.2, 3.2 Hz), 3.91-3.83 (1H, m), 3.49 (1H, dd, J = 12.7, 3.4 Hz), 3.38 (1H, s), 3.31-3.28 (1H, m), 3.12-3.00 (2H, m), 1.98-1.82 (3H, m), 1.68-1.56 (2H, m). LCMS (ESI) m/z 238 $[M+H]^+$

Step 2: Synthesis of Reference Example Compound 38

The title compound was obtained as in Reference Example 1, except that 1-(4-ethynyl-3,5-difluorophenyl)piperidin-3-ol was used in place of 2-ethynyl-1,3-difluorobenzene.
$^1$H-NMR (DMSO-$D_6$) δ: 8.20 (1H, s), 7.86 (1H, s), 7.36-7.32 (1H, m), 6.72 (2H, d, J = 12.1 Hz), 6.59 (2H, s), 5.92 (1H, d, J = 7.0 Hz), 5.38 (1H, d, J = 6.2 Hz), 5.22 (1H, d, J = 3.3 Hz), 4.88-4.83 (1H, m), 4.60-4.54 (1H, m), 4.11-4.08 (1H, m), 4.06-4.03 (1H, m), 3.68-3.51 (3H, m), 3.25-3.17 (1H, m), 3.15-3.07 (1H, m), 3.01-2.94 (1H, m), 2.84 (1H, dd, J = 12.6, 8.6 Hz), 1.89-1.83 (1H, m), 1.75-1.69 (1H, m), 1.48-1.34 (2H, m). LCMS (ESI) m/z 580 $[M+H]^+$.

Reference Example 39

4-Amino-5-[2-[2,6-difluoro-4-(3-hydroxyazetidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-(4-ethynyl-3,5-difluorophenyl)azetidin-3-ol

The title compound was obtained as in step 1 of Reference Example 23, except that azetidin-3-ol was used in place of morpholine.
$^1$H-NMR ($CDCl_3$) δ: 5.90 (2H, dq, J = 19.2, 3.4 Hz), 4.83-4.76 (1H, m), 4.17 (2H, dd, J = 8.4, 7.0 Hz), 3.75 (2H, dd, J = 8.4, 4.2 Hz), 3.38 (1H, s), 2.21 (1H, d, J = 6.2 Hz). LCMS (ESI) m/z 210 $[M+H]^+$

Step 2: Synthesis of Reference Example Compound 39

The title compound was obtained as in Reference Example 1, except that 1-(4-ethynyl-3,5-difluorophenyl)azetidin-3-ol was used in place of 2-ethynyl-1,3-difluorobenzene.
$^1$H-NMR (DMSO-$D_6$) δ: 8.17 (1H, s), 7.85 (1H, s), 7.37-7.32 (1H, m), 6.59 (2H, s), 6.24 (2H, d, J = 9.9 Hz), 5.91 (1H, d, J = 7.0 Hz), 5.75 (1H, d, J = 6.6 Hz), 5.38 (1H, d, J = 6.6 Hz), 5.22 (1H, d, J = 4.4 Hz), 4.60-4.54 (2H, m), 4.16-4.07 (3H, m), 4.06-4.02 (1H, m), 3.64 (2H, dd, J = 8.6, 4.6 Hz), 3.25-3.17 (1H, m), 3.14-3.05 (1H, m). LCMS (ESI) m/z 552 $[M+H]^+$.

Reference Example 40

4-Amino-5-[2-[2,6-difluoro-4-[(3R)-3-hydroxy-1-piperidyl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of (3R)-1-(4-ethynyl-3,5-difluorophenyl)piperidin-3-ol

The title compound was obtained as in step 1 of Reference Example 23, except that (R)-piperidin-3-ol was used in place of morpholine.
$^1$H-NMR ($CDCl_3$) δ: 6.39 (2H, d, J = 11.0 Hz), 3.91-3.84 (1H, m), 3.49 (1H, dd, J = 12.5, 3.3 Hz), 3.38 (1H, s), 3.33-3.27 (1H, m), 3.12-3.01 (2H, m), 1.98-1.85 (2H, m), 1.80 (1H, d, J = 6.2 Hz), 1.69-1.60 (1H, m). LCMS (ESI) m/z 238 $[M+H]^+$

Step 2: Synthesis of Reference Example Compound 40

The title compound was obtained as in Reference Example 1, except that (3R)-1-(4-ethynyl-3,5-difluorophenyl)piperidin-3-ol was used in place of 2-ethynyl-1,3-difluorobenzene.
$^1$H-NMR (DMSO-$D_6$) δ: 8.17 (1H, s), 7.86 (1H, s), 7.36-7.32 (1H, m), 6.72 (2H, d, J = 12.1 Hz), 6.59 (2H, s), 5.92 (1H, d, J = 7.0 Hz), 5.38 (1H, d, J = 6.6 Hz), 5.22 (1H, d, J = 4.4 Hz), 4.86 (1H, d, J = 4.4 Hz), 4.57 (1H, dd, J = 12.3, 6.8 Hz), 4.11-4.08 (1H, m), 4.06-4.03 (1H, m), 3.68-3.50 (3H, m), 3.23-3.18 (1H, m), 3.15-3.07 (1H, m), 3.01-2.94 (1H, m), 2.84 (1H, dd, J = 12.6, 8.6 Hz), 1.89-1.83 (1H, m), 1.75-1.69 (1H, m), 1.47-1.37 (2H, m). LCMS (ESI) m/z 580 $[M+H]^+$.

Reference Example 41

1-[4-[2-[4-amino-7-[(2R,3R,4S,5R)]-3,4-dihydroxy-5-[(sulfamoylamino)methyl]tetrahydrofuran-2-yl]ethynyl]-3,5-difluoro-phenyl]pyrrolidine-3-carboxylic acid

Step 1: Synthesis of 1-(4-ethynyl-3,5-difluorophenyl)pyrrolidine-3-carboxylic acid

The title compound was obtained as in step 1 of Reference Example 23, except that pyrrolidine-3-carboxylic acid was used in place of morpholine.
$^1$H-NMR ($CDCl_3$) δ: 6.06 (2H, d, J = 10.3 Hz), 3.61-3.52 (2H, m), 3.46-3.25 (4H, m), 2.38-2.33 (2H, m). LCMS (ESI) m/z 252 $[M+H]^+$ Step 2: Synthesis of Reference Example Compound 41

The title compound was obtained as in step 4 of Reference Example 1, except that 1-(4-ethynyl-3,5-difluorophenyl)pyrrolidine-3-carboxylic acid was used in place of 2-ethynyl-1,3-difluorobenzene.
$^1$H-NMR (DMSO-$D_6$) δ: 12.59 (1H, s), 8.17 (1H, s), 7.85 (1H, s), 7.36-7.32 (1H, m), 6.59 (2H, s), 6.39 (2H, d, J = 11.0 Hz), 5.91 (1H, d, J = 7.0 Hz), 5.38 (1H, d, J = 6.2 Hz), 5.22 (1H, d, J = 4.4 Hz), 4.59-4.55 (1H, m), 4.12-4.08 (1H, m), 4.06-4.02 (1H, m), 3.54-3.42 (2H, m), 3.24-3.07 (5H, m), 2.27-2.11 (2H, m). LCMS (ESI) m/z 594 $[M+H]^+$.

Reference Example 42

4-Amino-5-[2-[2,6-difluoro-4-(4-oxo-1-piperidyl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-(4-ethynyl-3,5-difluorophenyl)piperidin-4-one

The title compound was obtained as in step 1 of Reference Example 23, except that piperidin-4-one was used in place of morpholine.
$^1$H-NMR ($CDCl_3$) δ: 6.40 (2H, d, J = 11.0 Hz), 3.66 (4H, t, J = 6.0 Hz), 3.41 (1H, s), 2.57 (4H, t, J = 6.0 Hz). LCMS (ESI) m/z 236 $[M+H]^+$

Step 2: Synthesis of Reference Example Compound 42

The title compound was obtained as in step 4 of Reference Example 1, except that 1-(4-ethynyl-3,5-difluorophenyl)piperidin-4-one was used in place of 2-ethynyl-1,3-difluorobenzene.
$^1$H-NMR (DMSO-$D_6$) δ: 8.18 (1H, s), 7.88 (1H, s), 7.37-7.32 (1H, m), 6.86 (2H, d, J = 11.7 Hz), 6.60 (2H, s), 5.92 (1H, d, J = 7.0 Hz), 5.40 (1H, d, J = 6.2 Hz), 5.24 (1H, d, J = 4.0 Hz), 4.60-4.55 (1H, m), 4.11-4.08 (1H, m), 4.06-4.03 (1H, m), 3.73 (4H, t, J = 5.9 Hz), 3.25-3.18 (1H, m), 3.14-3.07 (1H, m), 2.44 (4H, t, J = 5.9 Hz). LCMS (ESI) m/z 578 $[M+H]^+$.

Reference Example 43

4-Amino-5-[2-[4-(azetidin-1-yl)-2,6-difluoro-phenyl]ethynyl]-7-[(2R,3R,4S,5R)-3,4-dihydroxy-5-[(sulfamoylamino)methyl]cyclopentyl]pyrrolo[2,3-d]pyrimidine

Step 1: Synthesis of 1-(4-ethynyl-3,5-difluorophenyl)azetidine

The title compound was obtained as in step 1 of Reference Example 23, except that azetidine was used in place of morpholine.
$^1$H-NMR ($CDCl_3$) δ: 5.86 (2H, d, J = 9.5 Hz), 3.93-3.89 (4H, m), 3.37 (1H, s), 2.45-2.37 (2H, m). LCMS (ESI) m/z 194 $[M+H]^+$ Step 2: Synthesis of Reference Example Compound 43

The title compound was obtained as in step 4 of Reference Example 1, except that 1-(4-ethynyl-3,5-difluorophenyl)azetidine was used in place of 2-ethynyl-1,3-difluorobenzene.
$^1$H-NMR (DMSO-$D_6$) δ: 8.17 (1H, s), 7.85 (1H, s), 7.36-7.33 (1H, m), 6.60 (2H, s), 6.20 (2H, d, J = 9.9 Hz), 5.91 (1H, d, J = 7.0 Hz), 5.38 (1H, d, J = 6.6 Hz), 5.22 (1H, d, J = 4.4 Hz), 4.57 (1H, q, J = 6.2 Hz), 4.11-4.07 (1H, m), 4.05-4.03 (1H, m), 3.91 (4H, t, J = 7.3 Hz), 3.25-3.17 (1H, m), 3.14-3.07 (1H, m), 2.37-2.30 (2H, m). LCMS (ESI) m/z 536 $[M+H]^+$.

Reference Example 44

4-Amino-7-[(2R,3R,4S,5R)-3,4-dihydroxy-5-[(sulfamoylamino)methyl]tetrahydrofuran-2-yl]-5-[2-(2-pyridyl)ethynyl]pyrrolo[2,3-d]pyrimidine

The title compound was obtained as in step 4 of Reference Example 1, except that 2-ethynylpyridine was used in place of 2-ethynyl-1,3-difluorobenzene.
$^1$H-NMR (DMSO-$D_6$) $\delta$: 8.12 (1H, s), 7.90 (1H, s), 7.78 (1H, t, J = 7.6 Hz), 7.60 (1H, d, J = 7.6 Hz), 7.33 (1H, dd, J = 7.6, 5.1 Hz), 7.28 (1H, dd, J = 7.6, 5.1 Hz), 6.53 (2H, s), 5.86 (1H, d, J = 7.0 Hz), 4.52 (1H, dd, J = 7.0, 5.7 Hz), 4.04 (1H, dd, J = 5.1, 2.5 Hz), 4.01-3.97 (1H, brm), 3.18-3.02 (2H, m). LCMS (ESI) m/z 446 $[M+H]^+$.

Reference Example 45

4-Amino-5-[2-(2-chlorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 1-chloro-2-ethynylbenzene was used in place of 2-ethynyl-1,3-difluorobenzene.
$^1$H-NMR (DMSO-$D_6$) $\delta$: 8.12 (1H, s), 7.88 (1H, s), 7.63-7.52 (1H, m), 7.54-7.53 (1H, m), 7.38-7.32 (2H, m), 7.27 (1H, dd, J = 7.6, 5.1 Hz), 6.53 (2H, s), 5.86 (1H, d, J = 7.0 Hz), 4.51 (1H, dd, J = 7.0, 5.1 Hz), 4.03 (1H, dd, J = 5.1, 2.5 Hz), 4.00-3.97 (1H, brm), 3.19-3.02 (2H, m). LCMS (ESI) m/z 479 $[M+H]^+$.

Reference Example 46

4-Amino-7-[(2R,3R,4S,5R)-3,4-dihydroxy-5-[(sulfamoylamino)methyl]tetrahydrofuran-2-yl]-5-[2-(2-fluorophenyl)ethynyl]pyrrolo[2,3-d]pyrimidine

The title compound was obtained as in step 4 of Reference Example 1, except that 1-ethynyl-2-fluorobenzene was used in place of 2-ethynyl-1,3-difluorobenzene.
$^1$H-NMR (DMSO-$D_6$) $\delta$: 8.11 (1H, s), 7.86 (1H, s), 7.58 (1H, t, J = 7.6 Hz), 7.43-7.37 (1H, m), 7.30-7.26 (2H, m), 7.21 (1H, t, J = 7.6 Hz), 6.52 (2H, s), 5.86 (1H, d, J = 7.0 Hz), 4.51 (1H, dd, J = 7.0, 5.1 Hz), 4.03 (1H, t, J = 2.5 Hz), 4.00-3.97 (1H, m), 3.17-3.02 (2H, m). LCMS (ESI) m/z 463 $[M+H]^+$.

Reference Example 47

4-Amino-7-[(2R,3R,4S,5R)-3,4-dihydroxy-5-[(sulfamoylamino)methyl]tetrahydrofuran-2-yl]-5-[2-(2-methoxyphenyl)ethynyl]pyrrolo[2,3-d]pyrimidine

The title compound was obtained as in step 4 of Reference Example 1, except that 1-ethynyl-2-methoxybenzene was used in place of 2-ethynyl-1,3-difluorobenzene.
$^1$H-NMR (DMSO-$D_6$) $\delta$: 8.10 (1H, s), 7.73 (1H, s), 7.36-7.29 (3H, m), 7.04 (1H, d, J = 8.2 Hz), 6.92 (1H, t, J = 7.6 Hz), 6.53 (2H, s), 5.84 (1H, d, J = 7.0 Hz), 4.50 (1H, dd, J = 7.0, 5.1 Hz), 4.03 (1H, dd, J = 5.1, 2.5 Hz), 3.84-3.84 (1H, brm), 4.00 (3H, s), 3.21-3.01 (2H, m). LCMS (ESI) m/z 475 $[M+H]^+$.

Reference Example 48

4-Amino-7-[(2R,3R,4S,5R)-3,4-dihydroxy-5-[(sulfamoylamino)methyl]tetrahydrofuran-2-yl]-5-[2-(4-dimethylaminophenyl)ethynyl]pyrrolo[2,3-d]pyrimidine

The title compound was obtained as in step 4 of Reference Example 1, except that 4-ethynyl-N,N-dimethylaniline was used in place of 2-ethynyl-1,3-difluorobenzene.
$^1$H-NMR (DMSO-$D_6$) $\delta$: 8.10 (1H, s), 7.73 (1H, s), 7.36-7.29 (3H, m), 7.04 (1H, d, J = 8.2 Hz), 6.92 (1H, t, J = 7.6 Hz), 6.53 (2H, s), 5.84 (1H, d, J = 7.0 Hz), 4.50 (1H, dd, J = 7.0, 5.1 Hz), 4.03 (1H, dd, J = 5.1, 2.5 Hz), 3.84-3.84 (1H, brm), 4.00 (3H, s), 3.21-3.01 (2H, m). LCMS (ESI) m/z 475 $[M+H]^+$.

Reference Example 49

4-Amino-5-[2-(2-cyanophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that 2-ethynylbenzonitrile was used in place of 2-ethynyl-1,3-difluorobenzene.
$^1$H-NMR (DMSO-$D_6$) δ: 8.12 (1H, s), 7.93 (1H, s), 7.87 (1H, d, J = 7.5 Hz), 7.74-7.68 (2H, m), 7.52 (1H, dt, J = 1.4, 7.5 Hz), 7.29 (1H, m), 6.53 (2H, s), 5.87 (1H, d, J = 6.8 Hz), 5.40 (1H, brs), 5.23 (1H, brs), 4.53 (1H, t, J = 5.5 Hz), 4.04 (2H, dd, J = 5.5, 2.7 Hz), 4.01-3.98 (2H, brm), 3.19-3.03 (2H, m). LCMS (ESI) m/z 470 $[M+H]^+$.

Reference Example 50

4-Amino-5-(3-cyclohexylpropyn-1-ynyl)-7-[(2R,3R,4S,5R)-3,4-dihydroxy-5-[(sulfamoylamino)methyl]tetrahydrofuran-2-yl]pyrrolo[2,3-d]pyrimidine

The title compound was obtained as in step 4 of Reference Example 1, except that prop-2-yn-1-yl cyclohexane was used in place of 2-ethynyl-1,3-difluorobenzene.
$^1$H-NMR (DMSO-$D_6$) δ: 8.05 (1H, s), 7.56 (1H, s), 7.27 (1H, dd, J = 7.5, 4.1 Hz), 6.51 (2H, s), 5.79 (1H, d, J = 6.8 Hz), 4.46 (1H, dd, J = 6.8, 5.5 Hz), 4.01 (1H, dd, J = 5.5, 2.1 Hz), 3.97-3.94 (1H, brm), 3.17-2.99 (2H, m), 2.32 (2H, d, J = 6.2 Hz), 1.76-1.72 (2H, brm), 1.65-1.60 (2H, brm), 1.58-1.54 (1H, brm), 1.49-1.41 (1H, brm), 1.23-0.93 (5H, m). LCMS (ESI) m/z 465 $[M+H]^+$.

Reference Example 51

4-Amino-7-[(2R,3R,4S,5R)-3,4-dihydroxy-5-[(sulfamoylamino)methyl]tetrahydrofuran-2-yl]-5-[2-(2-fluoro-6-methoxyphenyl)ethynyl]pyrrolo[2,3-d]pyrimidine

Step 1: Synthesis of 2-ethynyl-1-fluoro-3-methoxybenzene

2-Fluoro-6-methoxybenzaldehyde (2.0 g, 13 mmol) was dissolved in methanol (20 mL). Then, potassium carbonate (3.6 g, 26 mmol) was added thereto at room temperature, and dimethyl(1-diazo-2-oxopropyl)phosphonate (2.3 mL, 16 mmol) was added thereto under ice-cooling, followed by stirring under ice-cooling for 1 hour and at room temperature for another 1 hour. The reaction solution was partitioned with the addition of ethyl acetate, a saturated aqueous sodium hydrogen carbonate solution, and water, and the organic layer was washed with saturated saline. After being dried over sodium sulfate, the resulting product was filtered and concentrated, and the residue was purified by silica gel column chromatography (developing solvent: ethyl acetate/hexane), thereby obtaining 2-ethynyl-1-fluoro-3-methoxybenzene (1.6 g, 11 mmol, 81%) as a reddish brown solid.
$^1$H-NMR ($CDCl_3$) δ: 7.31-7.22 (1H, m), 6.76-6.65 (2H, m), 3.92 (3H, s), 3.53 (1H, s).

Step 2: Synthesis of Reference Example Compound 51

The title compound was obtained as in step 4 of Reference Example 1, except that 2-ethynyl-1-fluoro-3-methoxybenzene was used in place of 2-ethynyl-1,3-difluorobenzene.
$^1$H-NMR ($CD_3OD$) δ: 8.23 (1H, s), 7.57 (1H, s), 7.37-7.30 (1H, m), 6.90 (1H, d, J = 8.5 Hz), 6.82-6.78 (1H, m), 5.86 (1H, d, J = 6.8 Hz), 4.87-4.85 (1H, m), 4.32-4.27 (1H, m), 4.26-4.24 (1H, m), 3.97 (3H, s), 3.40-3.35 (2H, m). LCMS (ESI) m/z 493 $[M+H]^+$.

Reference Example 52

4-Amino-5-[2-(5-benzyloxy-2-pyridyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 5-(benzyloxy)-2-ethynylpyridine

The title compound was obtained as in step 1 of Reference Example 51, except that 5-(benzyloxy)picolinaldehyde was used in place of 2-fluoro-6-methoxybenzaldehyde.
$^1$H-NMR ($CDCl_3$) δ: 8.36 (1H, d, J = 2.9 Hz), 7.44-7.34 (5H, m), 7.20 (2H, dd, 8.8, 2.9 Hz), 5.13 (2H, s), 3.07 (1H, s).

LCMS (ESI) m/z 210 $[M+H]^+$

Step 2: Synthesis of Reference Example Compound 52

The title compound was obtained as in step 4 of Reference Example 1, except that 5-(benzyloxy)-2-ethynylpyridine was used in place of 2-ethynyl-1,3-difluorobenzene.
$^1$H-NMR ($CD_3OD$) $\delta$: 8.29 (1H, d, J = 2.9 Hz), 8.22 (1H, s), 7.65 (1H, s), 7.56 (1H, d, J = 8.0 Hz), 7.50-7.30 (6H, m), 5.86 (1H, d, J = 6.8 Hz), 5.20 (2H, s), 4.85-4.75 (1H, m), 4.35-4.20 (2H, m), 3.40-3.30 (2H, m). LCMS (ESI) m/z 552 $[M+H]^+$.

Reference Example 53

4-Amino-5-[2-(4-benzyloxy-2-methoxy-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-(benzyloxy)-1-ethynyl-2-methoxybenzene

The title compound was obtained as in step 1 of Reference Example 51, except that 4-(benzyloxy)-2-methoxybenzaldehyde was used in place of 2-fluoro-6-methoxybenzaldehyde.
$^1$H-NMR ($CDCl_3$) $\delta$: 7.48-7.32 (6H, m), 6.50-6.55 (2H, m), 5.07 (2H, s), 3.87 (3H, s), 3.24 (1H, s). LCMS (ESI) m/z 239 $[M+H]^+$ Step 2: Synthesis of Reference Example Compound 53
The title compound was obtained as in step 4 of Reference Example 1, except that 4-(benzyloxy)-1-ethynyl-2-methoxybenzene was used in place of 2-ethynyl-1,3-difluorobenzene.
$^1$H-NMR (DMSO-$D_6$) $\delta$: 8.16 (1H, s), 7.74 (1H, s), 7.46-7.32 (7H, m), 6.78-6.76 (1H, brs), 6.67 (1H, d, J = 8.0 Hz), 6.63-6.59 (2H, brs), 5.90 (1H, d, J = 6.3 Hz), 5.39 (1H, d, J = 6.1 Hz), 5.23 (1H, d, J = 4.1 Hz), 5.16 (2H, s), 4.61-4.55 (1H, m), 4.12-4.08 (1H, m), 4.06-4.04 (1H, m), 3.89 (3H, s), 3.26-3.15 (1H, m), 3.15-3.03 (1H, m). LCMS (ESI) m/z 581 $[M+H]^+$.

Reference Example 54

4-Amino-5-[2-(2,6-difluoro-4-hydroxy-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-ethynyl-3,5-difluorophenol

The title compound was obtained as in step 1 of Reference Example 51, except that 2,6-difluoro-4-hydroxybenzaldehyde was used in place of 2-fluoro-6-methoxybenzaldehyde.
$^1$H-NMR ($CDCl_3$) $\delta$: 6.43 (2H, d, J = 8.0 Hz), 5.60-5.40 (1H, brs.), 3.42 (1H, s).

Step 2: Synthesis of Reference Example Compound 54

The title compound was obtained as in step 4 of Reference Example 1, except that 4-ethynyl-3,5-difluorophenol was used in place of 2-ethynyl-1,3-difluorobenzene.
$^1$H-NMR ($CD_3OD$) $\delta$: 8.23 (1H, s), 7.58 (1H, s), 6.48 (2H, d, J = 9.5 Hz), 5.86 (1H, d, J = 6.8 Hz), 4.85-4.81 (1H, m), 4.33-4.29 (1H, m), 4.26-4.23 (1H, m), 3.40-3.34 (2H, m). LCMS (ESI) m/z 497 $[M+H]^+$.

Reference Example 55

4-Amino-7-[(2R,3R,4S,5R)-3,4-dihydroxy-5-[(sulfamoylamino)methyl]tetrahydrofuran-2-yl]-5-[2-(2-ethoxy-6-fluorophenyl)ethynyl]pyrrolo[2,3-d]pyrimidine

Step 1: Synthesis of 2-ethoxy-6-fluorobenzaldehyde

2-Fluoro-6-hydroxybenzaldehyde (50 g, 360 mmol) was dissolved in N,N-dimethylformamide (500 mL), and potassium carbonate (74 g, 540 mmol) and iodoethane (86 mL, 1.1 mol) were added thereto at room temperature, followed by stirring at room temperature overnight. The reaction solution was partitioned with the addition of ethyl acetate and water, and extracted with ethyl acetate. The organic layer was washed with water and saturated saline and dried over sodium sulfate, followed by filtration and concentration. The residue was purified by silica gel column chromatography (developing solvent: ethyl acetate/hexane), thereby obtaining 2-ethoxy-6-fluorobenzaldehyde (59 g, 98%) as a white

solid.
$^1$H-NMR ($CDCl_3$) δ: 10.47 (1H, s), 7.50-7.41 (1H, m), 6.77-6.67 (2H, m), 4.16 (2H, q, J = 6.8 Hz), 1.48 (3H, t, J = 6.8 Hz). LCMS (ESI) m/z 169 $[M+H]^+$

Step 2: Synthesis of 1-ethoxy-2-ethynyl-3-fluorobenzene

The title compound was obtained as in step 1 of Reference Example 51, except that 2-ethoxy-6-fluorobenzaldehyde was used in place of 2-fluoro-6-methoxybenzaldehyde.
$^1$H-NMR ($CDCl_3$) δ: 7.28-7.20 (1H, m), 6.74-6.63 (2H, m), 4.13 (2H, q, J = 7.1 Hz), 3.50 (1H, s), 1.47 (3H, t, J = 7.1 Hz).

Step 3: Synthesis of Reference Example Compound 55

The title compound was obtained as in step 4 of Reference Example 1, except that 1-ethoxy-2-ethynyl-3-fluorobenzene was used in place of 2-ethynyl-1,3-difluorobenzene.
$^1$H-NMR ($CD_3OD$) δ: 8.24 (1H, s), 7.58 (1H, s), 7.34-7.28 (1H, m), 6.88 (1H, d, J = 8.0 Hz), 6.82-6.74 (1H, m), 5.86 (1H, d, J = 7.0 Hz), 4.85-4.81 (1H, m), 4.33-4.29 (1H, m), 4.27-4.21 (3H, m), 3.40-3.34 (2H, m), 1.47 (3H, t, J = 7.0 Hz). LCMS (ESI) m/z 507 $[M+H]^+$.

Step 4: Synthesis of 4-amino-7-[(2R,3R,4S,5R)-3,4-dihydroxy-5-[(sulfamoylamino)methyl]tetrahydrofuran-2-yl]-5-[2-(2-ethoxy-6-fluoro-phenyl)ethynyl]pyrrolo[2,3-d]pyrimidine hydrochloride

The title compound hydrochloride was obtained as in step 5 of Reference Example 1.
$^1$H-NMR (DMSO-$D_6$) δ: 8.38 (1H,s), 8.13 (1H, s), 7.44-7.38 (1H, m), 7.01-6.91 (3H, m), 6.80-6.40(1H, brs), 6.01(1H, d, *J* = 6.8 Hz), 4.51-4.48 (1H, m), 4.24 (2H, q, *J* = 7.0 Hz), 4.11-4.09 (1H, m), 4.06-4.02 (1H, m), 3.24-3.19 (1H, m), 3.14-3.09 (1H, m), 1.38 (3H, t, *J* = 7.0 Hz). LCMS (ESI) m/z 507 [M+H]+

Reference Example 56

4-Amino-7-[(2R,3R,4S,5R)-3,4-dihydroxy-5-[(sulfamoylamino)methyl]tetrahydrofuran-2-yl]-5-[2-(2-fluoro-6-isopropoxy-phenyl)ethynyl]pyrrolo[2,3-d]pyrimidine

Step 1: Synthesis of 2-fluoro-6-isopropoxybenzaldehyde

The title compound was obtained as in step 1 of Reference Example 55, except that 2-iodopropane was used in place of iodoethane.
$^1$H-NMR ($CDCl_3$) δ: 10.44 (1H, s), 7.44 (1H, dt, J = 6.3, 8.5 Hz), 6.77 (1H, d, J = 8.5 Hz), 6.68 (1H, dd, J = 10.2, 8.5 Hz), 4.72-4.62 (1H, m), 1.41 (6H, d, J = 6.1 Hz).

Step 2: Synthesis of 2-ethynyl-1-fluoro-3-isopropoxybenzene

The title compound was obtained as in step 1 of Reference Example 51, except that 2-fluoro-6-isopropoxybenzaldehyde was used in place of 2-fluoro-6-methoxybenzaldehyde.
$^1$H-NMR ($CDCl_3$) δ: 7.22 (1H, dt, J = 6.6, 8.4 Hz), 6.71-6.65 (2H, m), 4.64-4.56 (1H, m), 3.47 (1H, s), 1.39 (6H, d, J = 6.2 Hz).

Step 3: Synthesis of Reference Example Compound 56

The title compound was obtained as in step 4 of Reference Example 1, except that 2-ethynyl-1-fluoro-3-isopropoxybenzene was used in place of 2-ethynyl-1,3-difluorobenzene.
$^1$H-NMR ($CD_3OD$) δ: 8.24 (1H, s), 7.57 (1H, s), 7.32-7.26 (1H, m), 6.89 (1H, d, J = 9.0 Hz), 6.78-6.73 (1H, m), 5.87 (1H, d, J = 6.8 Hz), 4.85-4.81 (1H, m), 4.79-4.71 (1H, m), 4.33-4.31 (1H, m), 4.27-4.24 (1H, m), 3.42-3.33 (2H, m), 1.41 (6H, d, J = 5.9 Hz). LCMS (ESI) m/z 521 $[M+H]^+$.

Reference Example 57

4-Amino-5-[2-(4-cyano-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-ethynyl-3,5-difluorobenzonitrile

The title compound was obtained as in step 1 of Reference Example 51, except that 3,5-difluoro-4-formylbenzonitrile was used in place of 2-fluoro-6-methoxybenzaldehyde.
$^1$H-NMR ($CDCl_3$) δ: 7.25 (2H, d, J = 8.0 Hz), 3.73 (1H, s).

Step 2: Synthesis of Reference Example Compound 57

The title compound was obtained as in step 4 of Reference Example 1, except that 4-ethynyl-3,5-difluorobenzonitrile was used in place of 2-ethynyl-1,3-difluorobenzene.
$^1$H-NMR ($CD_3OD$) δ: 8.26 (1H, s), 7.78 (1H, s), 7.58 (2H, d, J = 7.1 Hz), 5.88 (1H, d, J = 6.8 Hz), 4.85-4.78 (1H, m), 4.33-4.30 (1H, m), 4.27-4.24 (1H, m), 3.42-3.33 (2H, m). LCMS (ESI) m/z 506 $[M+H]^+$.

Reference Example 58

Methyl 4-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3,5-difluoro-benzoate

Step 1: Synthesis of methyl 4-ethynyl-3,5-difluorobenzimidate

3,5-Difluoro-4-formylbenzonitrile (200 mg, 1.2 mmol) was dissolved in methanol (3 mL). Then, potassium carbonate (331 mg, 24 mmol) was added thereto at room temperature, and dimethyl(1-diazo-2-oxopropyl)phosphonate (0.22 mL, 1.4 mmol) was added thereto under ice-cooling, followed by stirring under ice-cooling for 30 minutes and at room temperature for additional 2 hours and 30 minutes. The reaction solution was partitioned with the addition of ethyl acetate, a saturated aqueous sodium hydrogen carbonate solution, and water, and the organic layer was washed with saturated saline. After being dried over sodium sulfate, the resulting product was filtered and concentrated, and the residue was purified by silica gel column chromatography (developing solvent: ethyl acetate/hexane), thereby obtaining methyl 4-ethynyl-3,5-difluorobenzimidate (172 mg).
LCMS (ESI) m/z 196 $[M+H]^+$

Step 2: Synthesis of Reference Example Compound 58

The title compound was obtained as in step 4 of Reference Example 1, except that methyl 4-ethynyl-3,5-difluorobenzimidate was used in place of 2-ethynyl-1,3-difluorobenzene.
$^1$H-NMR ($CD_3OD$) δ: 8.27 (1H, s), 7.76 (1H, s), 7.69 (2H, d, J = 7.8 Hz), 5.88 (1H, d, J = 6.6 Hz), 4.85-4.78 (1H, m), 4.33-4.31 (1H, m), 4.27-4.24 (1H, m), 3.94 (3H, s), 3.42-3.33 (2H, m). LCMS (ESI) m/z 539 $[M+H]^+$.

Reference Example 59

4-Amino-7-[(2R,3R,4S,5R)-3,4-dihydroxy-5-[(sulfamoylamino)methyl]tetrahydrofuran-2-yl]-5-[2-(2-fluoro-6-methylsulfanyl-phenyl)ethynyl]pyrrolo[2,3-d]pyrimidine

Step 1: Synthesis of (2-ethynyl-3-fluorophenyl)(methyl)sulfane

The title compound was obtained as in step 1 of Reference Example 51, except that 2-fluoro-6-(methylthio)benzaldehyde was used in place of 2-fluoro-6-methoxybenzaldehyde.
$^1$H-NMR ($CDCl_3$) δ: 7.32-7.25 (1H, m), 6.94 (1H, d, J = 8.1 Hz), 6.89-6.64 (1H, m), 3.70 (1H, s), 2.51 (3H, s).

Step 2: Synthesis of Reference Example Compound 59

The title compound was obtained as in step 4 of Reference Example 1, except that (2-ethynyl-3-fluorophenyl)(methyl)sulfane was used in place of 2-ethynyl-1,3-difluorobenzene.
$^1$H-NMR ($CD_3OD$) δ: 8.24 (1H, s), 7.65 (1H, s), 7.37-7.31 (1H, m), 7.11 (1H, d, J = 8.0 Hz), 6.99-6.94 (1H, m), 5.88 (1H,

d, J = 6.8 Hz), 4.85-4.78 (1H, m), 4.33-4.31 (1H, m), 4.27-4.24 (1H, m), 3.42-3.33 (2H, m), 2.56 (3H, s). LCMS (ESI) m/z 509 $[M+H]^+$.

Reference Example 60

4-Amino-7-[(2R,3R,4S,5R)-3,4-dihydroxy-5-[(sulfamoylamino)methyl]tetrahydrofuran-2-yl]-5-[2-(2-fluoro-6-propoxyphenyl)ethynyl]pyrrolo[2,3-d]pyrimidine

Step 1: Synthesis of 2-fluoro-6-propoxybenzaldehyde

The title compound was obtained as in step 1 of Reference Example 55, except that 1-iodopropane was used in place of iodoethane.
$^1$H-NMR ($CDCl_3$) δ: 10.48 (1H, s), 7.46 (1H, dt, J = 6.3, 8.5 Hz), 6.76 (1H, d, J = 8.5 Hz), 6.71 (1H, dd, J = 10.4, 8.5 Hz), 4.04 (2H, t, J = 6.3 Hz), 1.93-1.83 (2H, m), 1.56 (1H, s), 1.07 (3H, t, J = 7.3 Hz). LCMS (ESI) m/z 183 $[M+H]^+$

Step 2: Synthesis of 2-ethynyl-1-fluoro-3-propoxybenzene

The title compound was obtained as in step 1 of Reference Example 51, except that 2-fluoro-6-propoxybenzaldehyde was used in place of 2-fluoro-6-methoxybenzaldehyde.
$^1$H-NMR ($CDCl_3$) δ: 7.27-7.20 (1H, m), 6.72-6.63 (2H, m), 4.01 (2H, t, J = 6.5 Hz), 3.49 (1H, s), 1.81-1.91 (2H, m), 1.07 (3H, t, J = 8.0 Hz).

Step 3: Synthesis of Reference Example Compound 60

The title compound was obtained as in step 4 of Reference Example 1, except that 2-ethynyl-1-fluoro-3-propoxybenzene was used in place of 2-ethynyl-1,3-difluorobenzene.
$^1$H-NMR ($CD_3OD$) δ: 8.24 (1H, s), 7.57 (1H, s), 7.34-7.28 (1H, m), 6.88 (1H, d, J = 8.0 Hz), 6.80-6.74 (1H, m), 5.86 (1H, d, J = 6.8 Hz), 4.85-4.78 (1H, m), 4.33-4.31 (1H, m), 4.27-4.24 (1H, m), 4.13 (2H, t, J = 6.7 Hz), 3.42-3.33 (2H, m), 1.92-1.82 (2H, m), 1.07 (3H, t, 7.4 Hz). LCMS (ESI) m/z 521 $[M+H]^+$.

Reference Example 61

4-Amino-7-[(2R,3R,4S,5R)-3,4-dihydroxy-5-[(sulfamoylamino)methyl]tetrahydrofuran-2-yl]-5-[2-[2-fluoro-6-(2,2,2-trifluoroethoxy)phenyl]ethynyl]pyrrolo[2,3-d]pyrimidine

Step 1: Synthesis of 2-fluoro-6-(2,2,2-trifluoroethoxy)benzaldehyde

The title compound was obtained as in step 1 of Reference Example 55, except that 1,1,1-trifluoro-2-iodoethane was used in place of iodoethane.
$^1$H-NMR ($CDCl_3$) δ: 10.45 (1H, s), 7.53 (1H, dt, J = 6.1, 8.6 Hz), 6.89 (1H, dd, J = 9.8, 8.6 Hz), 6.77 (1H, d, J = 8.6 Hz), 4.48 (2H, q, J = 8.0 Hz). LCMS (ESI) m/z 223 $[M+H]^+$

Step 2: Synthesis of 2-ethynyl-1-fluoro-3-(2,2,2-trifluoroethoxy)benzene

The title compound was obtained as in step 1 of Reference Example 51, except that 2-fluoro-6-(2,2,2-trifluoroethoxy)benzaldehyde was used in place of 2-fluoro-6-methoxybenzaldehyde.
$^1$H-NMR ($CDCl_3$) δ: 7.32-7.24 (1H, m), 6.88-6.81 (1H, m), 6.72 (1H, d, J = 8.0 Hz), 4.45 (2H, q, J = 8.0 Hz), 3.53 (1H, s).

Step 3: Synthesis of Reference Example Compound 61

The title compound was obtained as in step 4 of Reference Example 1, except that 2-ethynyl-1-fluoro-3-(2,2,2-trifluoroethoxy)benzene was used in place of 2-ethynyl-1,3-difluorobenzene.
$^1$H-NMR ($CD_3OD$) δ: 8.24 (1H, s), 7.60 (1H, s), 7.39-7.33 (1H, m), 6.98 (1H, d, J = 8.5 Hz), 6.93-6.89 (1H, m), 5.86 (1H, d, J = 7.1 Hz), 4.84-4.82 (1H, m), 4.76 (2H, q, J = 8.4 Hz), 4.33-4.29 (1H, m), 4.27-4.25 (1H, m), 3.40-3.37 (2H, m). LCMS (ESI) m/z 561 $[M+H]^+$.

Reference Example 62

4-Amino-7-[(2R,3R,4S,5R)-3,4-dihydroxy-5-[(sulfamoylamino)methyl]tetrahydrofuran-2-yl]-5-[2-(2-ethoxy-6-methoxyphenyl)ethynyl]pyrrolo[2,3-d]pyrimidine

Step 1: Synthesis of 2-ethoxy-6-methoxybenzaldehyde

The title compound was obtained as in step 1 of Reference Example 55, except that 2-hydroxy-6-methoxybenzaldehyde was used in place of 2-fluoro-6-hydroxybenzaldehyde.
$^1$H-NMR ($CDCl_3$) δ: 10.53 (1H, s), 7.42 (1H, t, J = 8.5 Hz), 6.56 (2H, d, J = 8.5 Hz), 4.15-4.08 (2H, q, J = 7.0 Hz), 3.90 (3H, s), 1.46 (3H, t, J = 7.0 Hz). LCMS (ESI) m/z 181 $[M+H]^+$ Step 2: Synthesis of 1-ethoxy-2-ethynyl-3-methoxybenzene

The title compound was obtained as in step 1 of Reference Example 51, except that 2-ethoxy-6-methoxybenzaldehyde was used in place of 2-fluoro-6-methoxybenzaldehyde.
$^1$H-NMR ($CDCl_3$) δ: 7.23 (1H, t, J = 8.4 Hz), 6.55-6.50 (2H, m), 4.12 (2H, q, J = 7.0 Hz), 3.90 (3H, s), 3.53 (1H, s), 1.46 (3H, t, J = 7.0 Hz). LCMS (ESI) m/z 177 $[M+H]^+$

Step 3: Synthesis of Reference Example Compound 62

The title compound was obtained as in step 4 of Reference Example 1, except that 1-ethoxy-2-ethynyl-3-methoxybenzene was used in place of 2-ethynyl-1,3-difluorobenzene.
$^1$H-NMR ($CD_3OD$) δ: 8.22 (1H, s), 7.48 (1H, s), 7.27-7.23 (1H, m), 6.67-6.64 (2H, m), 5.85 (1H, d, J = 6.5 Hz), 4.88-4.80 (1H, m), 4.32-4.30 (1H, m), 4.26-4.23 (1H, m), 4.18 (2H, q, J = 7.0 Hz), 3.91 (3H, s), 3.42-3.31 (2H, m), 1.45 (3H, t, J = 7.0 Hz). LCMS (ESI) m/z 519 $[M+H]^+$.

Reference Example 63

8-[2-[4-Amino-7-[(2R,3R,4S,5R)-3,4-dihydroxy-5-[(sulfamoylamino)methyl]tetrahydrofuran-2-yl]pyrrolo[2.3-d]pyrimidin-5-yl]ethynyl]-7-methoxyquinoline

Step 1: Synthesis of 8-ethynyl-7-methoxyquinoline

The title compound was obtained as in step 1 of Reference Example 51, except that 7-methoxyquinoline-8-carbaldehyde was used in place of 2-fluoro-6-methoxybenzaldehyde.
$^1$H-NMR ($CDCl_3$) δ: 9.03 (1H, dd, J = 4.2, 1.8 Hz), 8.12 (1H, dd, J = 8.3, 1.8 Hz), 7.83 (1H, d, J = 9.2 Hz), 7.35 (1H, d, J = 9.2 Hz), 7.33 (1H, dd, J = 8.3, 4.2 Hz), 4.10 (3H, s), 3.87 (1H, s). LCMS (ESI) m/z 184 $[M+H]^+$

Step 2: Synthesis of Reference Example Compound 63

The title compound was obtained as in step 4 of Reference Example 1, except that 8-ethynyl-7-methoxyquinoline was used in place of 2-ethynyl-1,3-difluorobenzene.
$^1$H-NMR ($CD_3OD$) δ: 8.92 (1H, dd, J = 4.3, 1.7 Hz), 8.29 (1H, dd, J = 8.3, 1.7 Hz), 8.22 (1H, s), 7.92 (1H, d, J = 9.0 Hz), 7.59 (1H, s), 7.54 (1H, d, J = 9.0 Hz), 7.43 (1H, dd, J = 8.3, 4.3 Hz), 5.89 (1H, d, J = 6.8 Hz), 4.61-4.59 (1H, m), 4.33 (1H, dd, J = 5.4, 2.7 Hz), 4.29-4.26 (1H, m), 4.11 (3H, s), 3.44-3.34 (2H, m). LCMS (ESI) m/z 526 $[M+H]^+$.

Reference Example 64

8-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine

Step 1: Synthesis of 7-fluoro-4-methyl-2,3-dihydro-1,4-benzoxazine

7-Fluoro-2,3-dihydro-1,4-benzoxazine (1.50 g, 9.79 mmol) was dissolved in N,N-dimethylformamide (15 mL), and potassium carbonate (2.98 g, 21.5 mmol) and methyl iodide (1.67 g, 11.8 mmol) were added thereto at room temperature. After the resulting mixture was stirred at room temperature for 3 days, water (60 mL) and ethyl acetate (60 mL) were sequentially added thereto to partition the mixture into an aqueous layer and an organic layer, followed by separation of each layer. The aqueous layer was extracted with ethyl acetate (60 mL), and the obtained organic layers were combined. The combined organic layer was sequentially washed with water (60 mL) and saturated saline (60 mL), and dried over anhydrous sodium sulfate, followed by distilling off the solvent. The residue was purified by silica gel

column chromatography (developing solvent: ethyl acetate/hexane), thereby obtaining 7-fluoro-4-methyl-2,3-dihydro-1,4-benzoxazine (1.09 g) as a light-yellow oil.
$^1$H-NMR ($CDCl_3$) δ: 6.58-6.51 (3H, m), 4.31 (2H, t, J = 4.4 Hz), 3.20 (2H, t, J = 4.4 Hz), 2.84 (3H, s). LCMS (ESI) m/z 168.1 $[M+H]^+$

Step 2: Synthesis of 7-fluoro-4-methyl-2,3-dihydro-1,4-benzoxazine-8-carbaldehyde

7-Fluoro-4-methyl-2,3-dihydro-1,4-benzoxazine (1.07 g, 6.40 mmol) was dissolved in tetrahydrofuran (17.1 mL), and n-butyllithium (a 1.6 M hexane solution, 4.83 mL) was added thereto dropwise with stirring at -78°C. After the mixture was stirred at -78°C for 3 hours, N,N-dimethylformamide (702 mg, 9.60 mmol) was added thereto dropwise, and the temperature was increased to 0°C. A saturated aqueous ammonium chloride solution (40 mL) and ethyl acetate (40 mL) were sequentially added thereto, followed by separation of each layer. The aqueous layer was extracted twice with ethyl acetate (40 mL), and the obtained organic layers were combined. The combined organic layer was sequentially washed with a saturated aqueous ammonium chloride solution (100 mL), water (100 mL), and saturated saline (100 mL), and dried over anhydrous sodium sulfate, followed by distilling off the solvent. The residue was purified by silica gel column chromatography (developing solvent: ethyl acetate/hexane), thereby obtaining 7-fluoro-4-methyl-2,3-dihydro-1,4-benzoxazine-8-carbaldehyde (1.13 g) as a bright yellow solid.
$^1$H-NMR ($CDCl_3$) δ: 10.38 (1H, d, J = 2.2 Hz), 6.78-6.73 (1H, m), 6.62-6.57 (1H, m), 4.45-4.42 (2H, m), 3.28-3.25 (2H, m), 2.87 (3H, d, J = 1.8 Hz). LCMS (ESI) m/z 196.2 $[M+H]^+$

Step 3: Synthesis of 8-ethynyl-7-fluoro-4-methyl-2,3-dihydro-1,4-benzoxazine

The title compound was obtained as in step 1 of Reference Example 51, except that 7-fluoro-4-methyl-2,3-dihydro-1,4-benzoxazine-8-carbaldehyde was used in place of 2-fluoro-6-methoxybenzaldehyde.
$^1$H-NMR ($CDCl_3$) δ: 6.59-6.56 (2H, m), 4.43 (2H, t, J = 4.4 Hz), 3.49 (1H, s), 3.22 (2H, t, J = 4.4 Hz), 2.84 (3H, s). LCMS (ESI) m/z 192.4 $[M+H]^+$

Step 4: Synthesis of Reference Example Compound 64

The title compound was obtained as in step 4 of Reference Example 1, except that 8-ethynyl-7-fluoro-4-methyl-2,3-dihydro-1,4-benzoxazine was used in place of 2-ethynyl-1,3-difluorobenzene.
$^1$H-NMR (DMSO-$D_6$) δ: 8.17 (1H, s), 7.88 (1H, s), 7.38 (1H, dd, J = 7.3, 4.8 Hz), 6.78-6.69 (2H, m), 6.60 (2H, s), 5.91 (1H, d, J = 7.3 Hz), 5.40 (1H, s), 5.24 (1H, s), 4.58 (1H, t, J = 6.2 Hz), 4.43 (2H, t, J = 4.2 Hz), 4.12-4.07 (1H, m), 4.07-4.02 (1H, m), 3.26 (2H, t, J = 4.2 Hz), 3.23-3.16 (1H, m), 3.15-3.07 (1H, m), 2.82 (3H, s). LCMS (ESI) m/z 534.3 $[M+H]^+$.

Step 5: Synthesis of 8-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine hydrochloride

The title compound hydrochloride was obtained as in step 5 of Reference Example 1.
$^1$H-NMR (DMSO-$D_6$) δ: 8.47 (1H, s), 8.23 (1H, s), 6.82-6.76 (2H, m), 6.06 (1H, d, J = 6.8 Hz), 4.49-4.45 (3H, m), 4.12 (1H, dd, J = 5.2, 2.8 Hz), 4.05 (1H, dt, J = 5.6, 2.8 Hz), 3.28 (2H, t, J = 4.4 Hz), 3.23 (1H, dd, J = 14.0, 6.0 Hz), 3.13 (1H, dd, J = 14.0, 6.0 Hz), 2.84 (3H, s). LCMS (ESI) m/z 534.3 $[M+H]^+$

Reference Example 65

4-Amino-7-[(2R,3R,4S,5R)-3,4-dihydroxy-5-[(sulfamoylamino)methyl]tetrahydrofuran-2-yl]-5-[2-(2,4-dimethoxy-3-pyridyl)ethynyl]pyrrolo[2,3-d]pyrimidine

Step 1: Synthesis of 3-ethynyl-2,4-dimethoxypyridine

The title compound was obtained as in step 1 of Reference Example 51, except that 2,4-dimethoxynicotinaldehyde was used in place of 2-fluoro-6-methoxybenzaldehyde.
$^1$H-NMR ($CDCl_3$) δ: 8.05 (1H, d, J = 6.1 Hz), 6.54 (1H, d, J = 6.1 Hz), 4.02 (3H, s), 3.95 (3H, s), 3.58 (1H, s). LCMS (ESI) m/z 164 $[M+H]^+$

Step 2: Synthesis of Reference Example Compound 65

The title compound was obtained as in step 4 of Reference Example 1, except that 3-ethynyl-2,4-dimethoxypyridine was used in place of 2-ethynyl-1,3-difluorobenzene.
$^1$H-NMR (DMSO-$D_6$) δ: 8.18 (1H, s), 8.08 (1H, d, J = 6.1 Hz), 7.85 (1H, d, J = 2.0 Hz), 7.35 (1H, s), 6.89 (1H, d, J = 6.1 Hz), 6.59 (2H, s), 5.91 (1H, d, J = 7.1 Hz), 4.58 (1H, t, J = 5.7 Hz), 4.11-4.08 (1H, m), 4.06-4.03 (1H, m), 3.95 (6H, d, J = 2.7 Hz), 3.35 (2H, s), 3.22-3.10 (2H, m). LCMS (ESI) m/z 506 $[M+H]^+$.

Reference Example 66

4-Amino-7-[(2R,3R,4S,5R)-3,4-dihydroxy-5-[(sulfamoylamino)methyl]tetrahydrofuran-2-yl]-5-[2-(2-ethylsulfanyl-6-fluoro-phenyl)ethynyl]pyrrolo[2,3-d]pyrimidine

Step 1: Synthesis of 2-(ethylthio)-6-fluorobenzaldehyde

Ethyl (3-fluorophenyl)sulfane (2.0 g, 12.8 mmol) was dissolved in tetrahydrofuran (30 mL), and n-butyllithium (a 2.69 M hexane solution, 5.71 mL) was added thereto dropwise while being stirred with cooling at -78°C. After the resulting mixture was stirred at -78°C for 30 minute, N,N-dimethylformamide (2.95 mL, 38.4 mmol) was added thereto, followed by stirring for 1 hour. A saturated aqueous ammonium chloride solution was added to the reaction liquid, followed by extraction with ethyl acetate. After the solvent was distilled off, the residue was purified by column chromatography (developing solvent: hexane/ethyl acetate), thereby obtaining 2-(ethylthio)-6-fluorobenzaldehyde (430 mg, 18%) as a light-yellow solid.
$^1$H-NMR ($CDCl_3$) δ: 10.52 (1H, s), 7.51-7.46 (1H, m), 7.13 (1H, d, J = 8.8 Hz), 6.91 (1H, t, J = 8.8 Hz), 2.99 (2H, q, J = 7.3 Hz), 1.42 (3H, t, J = 7.3 Hz).

Step 2: Synthesis of ethyl(2-ethynyl-3-fluorophenyl)sulfane

The title compound was obtained as in step 1 of Reference Example 51, except that 2-(ethylthio)-6-fluorobenzaldehyde was used in place of 2-fluoro-6-methoxybenzaldehyde.
$^1$H-NMR ($CDCl_3$) δ: 7.30-7.24 (1H, m), 7.03 (1H, d, J = 8.1 Hz), 6.89 (1H, dd, J = 8.8, 8.1 Hz), 3.71 (1H, s), 3.03 (2H, q, J = 7.3 Hz), 1.40 (3H, t, J = 7.3 Hz).

Step 3: Synthesis of Reference Example Compound 66

The title compound was obtained as in step 4 of Reference Example 1, except that ethyl (2-ethynyl-3-fluorophenyl)sulfane was used in place of 2-ethynyl-1,3-difluorobenzene.
$^1$H-NMR (DMSO-$D_6$) δ: 8.16 (1H, s), 7.95 (1H, s), 7.91 (1H, d, J = 7.0 Hz), 7.46 (1H, t, J = 7.7 Hz), 7.41-7.32 (2H, m), 7.22 (1H, d, J = 8.1 Hz), 7.12 (1H, t, J = 8.8 Hz), 6.57 (2H, s), 5.91 (1H, d, J = 7.0 Hz), 5.37 (1H, brs), 5.21 (1H, brs), 4.57 (1H, brs), 4.09-4.06 (1H, brm), 4.04-4.01 (1H, brm), 3.23-3.05 (2H, m), 1.28 (3H, t, J = 7.0 Hz). LCMS (ESI) m/z 523 $[M+H]^+$.

Reference Example 67

4-Amino-5-[2-[2,6-difluoro-4-(triazol-2-ylmethoxy)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-((2H-1,2,3-triazol-2-yl)methoxy)-2,6-difluorobenzaldehyde

The title compound was obtained as in step 1 of Reference Example 55, except that 2,6-difluoro-4-hydroxybenzaldehyde was used in place of 2-fluoro-6-hydroxybenzaldehyde, and that 2-(chloromethyl)triazole was used in place of iodoethane.
$^1$H-NMR ($CDCl_3$) δ: 10.21 (1H, s), 7.76 (2H, s), 6.88 (2H, d, J = 10.0 Hz), 6.30 (2H, s). LCMS (ESI) m/z 240 $[M+H]^+$ Step 2: Synthesis of 2-((4-ethynyl-3,5-difluorophenoxy)methyl)-2H-1,2,3-triazole

The title compound was obtained as in step 1 of Reference Example 51, except that 4-((2H-1,2,3-triazol-2-yl)methoxy)-2,6-difluorobenzaldehyde was used in place of 2-fluoro-6-methoxybenzaldehyde.
$^1$H-NMR ($CDCl_3$) δ: 7.74 (2H, s), 7.26 (2H, s), 6.82 (1H, d, J = 8.5 Hz), 6.24 (1H, s), 3.43 (1H, s). LCMS (ESI) m/z 236 $[M+H]^+$

Step 3: Synthesis of Reference Example Compound 67

The title compound was obtained as in step 4 of Reference Example 1, except that 2-((4-ethynyl-3,5-difluorophenoxy)methyl)-2H-1,2,3-triazole was used in place of 2-ethynyl-1,3-difluorobenzene.

$^1$H-NMR (DMSO-$D_6$) δ: 8.17 (1H, s), 7.99 (2H, s), 7.96 (1H, s), 7.36-7.31 (1H, brs), 7.19 (2H, d, J = 9.5 Hz), 6.61-6.57 (2H, s), 6.52 (2H, s), 5.91 (1H, d, J = 7.1 Hz), 5.40 (1H, d, J = 6.6 Hz), 5.23 (1H, d, J = 4.1 Hz), 4.59-4.54 (1H, m), 4.10-4.06 (1H, m), 4.05-4.02 (1H, m), 3.22-3.16 (1H, m), 3.10-3.06 (1H, m). LCMS (ESI) m/z 578 $[M+H]^+$.

Reference Example 68

4-Amino-7-[(2R,3R,4S,5R)-3,4-dihydroxy-5-[(sulfamoylamino)methyl]tetrahydrofuran-2-yl]-5-[2-[2-(ethylamino)-6-fluoro-phenyl]ethynyl]pyrrolo[2,3-d]pyrimidine

Step 1: Synthesis of 2-(ethylamino)-6-fluorobenzaldehyde

The title compound was obtained as in step 1 of Reference Example 23, except that 2,6-difluorobenzaldehyde and ethylamine were used in place of 2-ethynyl-1,3,5-trifluorobenzene and morpholine.
$^1$H-NMR ($CDCl_3$) δ: 10.28 (1H, s), 8.66 (1H, brs), 7.35-7.29 (1H, m), 6.45 (1H, d, J = 8.1 Hz), 6.27 (1H, dd, J = 11.4, 8.1 Hz), 3.32-3.25 (2H, m), 1.33 (3H, t, J = 7.3 Hz). LRMS (ESI) m/z 168 $[M+H]^+$ Step 2: Synthesis of N-ethyl-2-ethynyl-3-fluoroaniline
The title compound was obtained as in step 1 of Reference Example 51, except that 2-(ethylamino)-6-fluorobenzaldehyde was used in place of 2-fluoro-6-methoxybenzaldehyde.
$^1$H-NMR ($CDCl_3$) δ: 7.13 (1H, dd, J = 14.7, 7.7 Hz), 6.38-6.34 (2H, m), 4.62-4.56 (1H, brm), 3.62 (1H, s), 3.21 (2H, dq, J = 7.3, 6.6 Hz), 1.29 (3H, t, J = 7.3 Hz). LRMS (ESI) m/z 164 $[M+H]^+$

Step 3: Synthesis of Reference Example Compound 68

The title compound was obtained as in step 4 of Reference Example 1, except that N-ethyl-2-ethynyl-3-fluoroaniline was used in place of 2-ethynyl-1,3-difluorobenzene.
$^1$H-NMR ($CD_3OD$) δ: 8.22 (1H, s), 7.55 (1H, s), 7.26 (1H, ddd, J = 8.4, 8.4, 7.0 Hz), 6.82 (1H, d, J = 8.4 Hz), 6.74 (1H, t, J = 8.4 Hz), 5.87 (1H, d, J = 6.6 Hz), 4.79 (1H, t, J = 6.6 Hz), 4.30 (1H, dd, J = 5.5, 2.6 Hz), 4.23-4.17 (2H, m), 4.19 (1H, q, J = 7.0 Hz), 3.56-3.54 (2H, m), 1.46 (3H, t, J = 7.0 Hz). LCMS (ESI) m/z 506 $[M+H]^+$.

Reference Example 69

4-Amino-5-[2-(2,4-difluoro-3-pyridyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 3-ethynyl-2,4-difluoropyridine

The title compound was obtained as in step 1 of Reference Example 51, except that 2,4-difluoronicotinaldehyde was used in place of 2-fluoro-6-methoxybenzaldehyde.
$^1$H-NMR (ACETONE-$D_6$) δ: 8.38 (1H, dd, J = 8.2, 5.7 Hz), 7.44-7.40 (1H, m), 4.46 (1H, s). LCMS (ESI) m/z 140 $[M+H]^+$
Step 2: Synthesis of Reference Example Compound 69
The title compound was obtained as in step 4 of Reference Example 1, except that 3-ethynyl-2,4-difluoropyridine was used in place of 2-ethynyl-1,3-difluorobenzene.
$^1$H-NMR ($CD_3OD$) δ: 8.25 (1H, s), 8.18 (1H, dd, J = 8.2, 5.8 Hz), 7.73 (1H, s), 7.27 (1H, dd, J = 8.2, 5.8 Hz), 5.87 (1H, d, J = 6.6 Hz), 4.82 (1H, d, J = 5.9 Hz), 4.32 (1H, dd, J = 5.5, 2.6 Hz), 4.26 (1H, q, J = 3.1 Hz), 3.43-3.33 (2H, m). LCMS (ESI) m/z 482 $[M+H]^+$.

Reference Example 70

4-Amino-7-[(2R,3R,4S,5R)-3,4-dihydroxy-5-[(sulfamoylamino)methyl]tetrahydrofuran-2-yl]-5-[2-(2-ethylsulfonyl-6-fluoro-4-pyrrolidin-1-yl-phenyl)ethynyl]pyrrolo[2,3-d]pyrimidine

Step 1: Synthesis of 2,6-difluoro-4-(pyrrolidin-1-yl)benzaldehyde

The title compound was obtained as in step 1 of Reference Example 23, except that 2,4,6-trifluorobenzaldehyde and pyrrolidine were used in place of 2-ethynyl-1,3,5-trifluorobenzene and morpholine.
$^1$H-NMR ($CDCl_3$) δ: 10.04 (1H, s), 6.01 (2H, d, J = 12.8 Hz), 3.35-3.32 (4H, m), 2.07-2.03 (4H, m). LCMS (ESI) m/z 212 $[M+H]^+$ Step 2: Synthesis of 2-(ethylthio)-6-fluoro-4-(pyrrolidin-1-yl)benzaldehyde
2,6-Difluoro-4-(pyrrolidin-1-yl)benzaldehyde (3.7 g, 18 mmol) was dissolved in N,N-dimethylformamide (37 mL),

and sodium ethanethiolate (1.6 g, 18 mmol) was added thereto, followed by stirring at room temperature for 30 minutes. Ethyl acetate, water, and a saturated aqueous sodium hydrogen carbonate solution were added to the reaction solution, and the aqueous layer was extracted with ethyl acetate. The organic layer was washed with water and a saturated sodium chloride aqueous solution, and dried over sodium sulfate, followed by distilling off the solvent. The residue was purified by silica gel column chromatography (developing solvent: chloroform), thereby obtaining 2-(ethylthio)-6-fluoro-4-(pyrrolidin-1-yl)benzaldehyde (4.4 g) as a white solid.
$^1$H-NMR ($CDCl_3$) δ: 10.18 (1H, s), 6.10 (1H, d, J = 2.0 Hz), 5.98 (1H, dd, J = 14.3, 2.0 Hz), 3.39-3.35 (4H, m), 2.91 (2H, q, J = 7.3 Hz), 2.07-2.04 (4H, m), 1.41 (3H, t, J = 7.3 Hz). LCMS (ESI) m/z 254 $[M+H]^+$

Step 3: Synthesis of 2-(ethylsulfonyl)-6-fluoro-4-(pyrrolidin-1-yl)benzaldehyde

2-(Ethylthio)-6-fluoro-4-(pyrrolidin-1-yl)benzaldehyde (79 mg, 0.31 mmol) was dissolved in dichloromethane (1.6 mL), and 3-chloroperbenzoic acid (110 mg, 0.65 mmol) was added thereto in an ice bath, followed by stirring for 2 hours in an ice bath. 3-chloroperbenzoic acid (56 mg, 0.32 mmol) was added to the resulting mixture in an ice bath, followed by stirring for 1 hour in an ice bath. Thereafter, 3-chloroperbenzoic acid (10 mg, 0.058 mmol) was further added thereto in an ice bath, followed by stirring for 30 minutes in an ice bath. Chloroform, water, and a saturated aqueous sodium hydrogen carbonate solution were added in an ice bath to the reaction solution, and the aqueous layer was extracted with chloroform. The organic layer was washed with a saturated sodium chloride aqueous solution, and dried over sodium sulfate, followed by distilling off the solvent. The residue was purified by silica gel column chromatography (developing solvent: ethyl acetate/hexane), thereby obtaining 2-(ethylsulfonyl)-6-fluoro-4-(pyrrolidin-1-yl)benzaldehyde (54 mg, 0.19 mmol, 62%) as a yellow solid.
$^1$H-NMR ($CDCl_3$) δ: 10.28 (1H, s), 7.19 (1H, d, J = 2.6 Hz), 6.33 (1H, dd, J = 13.9, 2.6 Hz), 3.62 (2H, q, J = 7.5 Hz), 3.50-3.40 (4H, m), 2.11-2.08 (4H, m), 1.31 (3H, t, J = 7.5 Hz). LCMS (ESI) m/z 286 $[M+H]^+$

Step 4: Synthesis of 1-(3-(ethylsulfonyl)-4-ethynyl-5-fluorophenyl)pyrrolidine

The title compound was obtained as in step 1 of Reference Example 51, except that 2-(ethylsulfonyl)-6-fluoro-4-(pyrrolidin-1-yl)benzaldehyde was used in place of 2-fluoro-6-methoxybenzaldehyde.
$^1$H-NMR ($CDCl_3$) δ: 7.01 (1H, d, J = 2.3 Hz), 6.38 (1H, dd, J = 11.9, 2.3 Hz), 3.63 (1H, s), 3.49 (2H, q, J = 7.4 Hz), 3.36-3.32 (4H, m), 2.07-2.04 (4H, m), 1.27 (3H, t, J = 7.4 Hz). LCMS (ESI) m/z 282 $[M+H]^+$

Step 5: Synthesis of Reference Example Compound 70

The title compound was obtained as in step 4 of Reference Example 1, except that 1-(3-(ethylsulfonyl)-4-ethynyl-5-fluorophenyl)pyrrolidine was used in place of 2-ethynyl-1,3-difluorobenzene.
$^1$H-NMR ($CD_3OD$) δ: 8.22 (1H, s), 7.61 (1H, s), 7.01 (1H, d, J = 2.3 Hz), 6.66 (1H, dd, J = 12.4, 2.3 Hz), 5.86 (1H, d, J = 7.1 Hz), 4.85-4.81 (1H, m), 4.32-4.30 (1H, m), 4.27-4.24 (1H, m), 3.49 (2H, q, J = 7.3 Hz), 3.40-3.36 (4H, m), 3.31-3.30 (2H, m), 2.10-2.07 (4H, m), 1.25 (3H, t, J = 7.3 Hz). LCMS (ESI) m/z 624 [M+H].

Reference Example 71

4-Amino-7-[(2R,3R,4S,5R)-3,4-dihydroxy-5-[(sulfamoylamino)methyl]tetrahydrofuran-2-yl]-5-[2-(3-fluoro-5-methoxy-4-pyridyl)ethynyl]pyrrolo[2,3-d]pyrimidine

Step 1: Synthesis of 4-ethynyl-3-fluoro-5-methoxypyridine

The title compound was obtained as in step 1 of Reference Example 51, except that 3-fluoro-5-methoxyisonicotinaldehyde was used in place of 2-fluoro-6-methoxybenzaldehyde.
$^1$H-NMR ($CDCl_3$) δ: 8.21-8.17 (2H, m), 4.03 (3H, s), 3.69 (1H, s). LCMS (ESI) m/z 152 $[M+H]^+$

Step 2: Synthesis of Reference Example Compound 71

The title compound was obtained as in step 4 of Reference Example 1, except that 4-ethynyl-3-fluoro-5-methoxypyridine was used in place of 2-ethynyl-1,3-difluorobenzene.
$^1$H-NMR (DMSO-$D_6$) δ: 8.38 (1H, d, J = 7.8 Hz), 8.37 (1H, s), 8.20 (1H, s), 8.05 (1H, s), 7.32-7.29 (1H, brs), 6.58 (2H, s), 5.93 (1H, d, J = 6.8 Hz), 5.41-5.38 (1H, brs), 5.23-5.21 (1H, brs), 4.59-4.57 (1H, m), 4.08-4.04 (2H, m), 4.07 (3H, s), 3.22-3.18 (1H, m), 3.12-3.08 (1H, m). LCMS (ESI) m/z 494 [M+H].

Reference Example 72

4-Amino-7-[(2R,3R,4S,5R)-3,4-dihydroxy-5-[(sulfamoylamino)methyl]tetrahydrofuran-2-yl]-5-[2-[2-ethylsulfonyl-6-fluoro-4-[(3R)-3-hydroxypyrrolidin-1-yl]phenyl]ethynyl]pyrrolo[2,3-d]pyrimidine

Step 1: Synthesis of 2,6-difluoro-4-[(3R)-3-hydroxypyrrolidin-1-yl]benzaldehyde

The title compound was obtained as in step 1 of Reference Example 23, except that 2,4,6-trifluorobenzaldehyde and (R)-pyrrolidin-3-ol were used in place of 2-ethynyl-1,3,5-trifluorobenzene and morpholine.
$^1$H-NMR ($CDCl_3$) δ: 10.05 (1H, s), 6.03 (2H, d, J = 12.4 Hz), 4.70-4.66 (1H, m), 3.60-3.53 (2H, m), 3.48-3.42 (1H, m), 3.36-3.31 (1H, m), 2.21-2.12 (2H, m), 1.79 (1H, d, J = 3.9 Hz). LCMS (ESI) m/z 228 $[M+H]^+$

Step 2: Synthesis of 2-ethylsulfanyl-6-fluoro-4-[(3R)-3-hydroxypyrrolidin-1-yl]benzaldehyde

The title compound was obtained as in step 2 of Reference Example 70, except that 2,6-difluoro-4-[(3R)-3-hydroxypyrrolidin-1-yl]benzaldehyde was used in place of 2,6-difluoro-4-(pyrrolidin-1-yl)benzaldehyde.
$^1$H-NMR ($CDCl_3$) δ: 10.19 (1H, s), 6.11 (1H, d, J = 2.2 Hz), 6.00 (1H, dd, J = 14.1, 2.2 Hz), 4.69-4.66 (1H, m), 3.63-3.57 (2H, m), 3.50-3.45 (1H, m), 3.41-3.35 (1H, m), 2.91 (2H, q, J = 7.4 Hz), 2.21-2.14 (2H, m), 1.77 (1H, d, J = 4.1 Hz), 1.41 (3H, t, J = 7.4 Hz). LCMS (ESI) m/z 270 $[M+H]^+$

Step 3: Synthesis of 2-ethylsulfonyl-6-fluoro-4-[(3R)-3-hydroxypyrrolidin-1-yl]benzaldehyde

The title compound was obtained as in step 3 of Reference Example 70, except that 2-ethylsulfanyl-6-fluoro-4-[(3R)-3-hydroxypyrrolidin-1-yl]benzaldehyde was used in place of 2-(ethylthio)-6-fluoro-4-(pyrrolidin-1-yl)benzaldehyde.
$^1$H-NMR ($CDCl_3$) δ: 10.30 (1H, s), 7.20 (1H, d, J = 2.4 Hz), 6.36 (1H, dd, J = 14.0, 2.4 Hz), 4.73-4.71 (1H, m), 3.67-3.42 (4H, m), 3.46-3.42 (1H, m), 3.35-3.32 (1H, m), 2.21-2.17 (2H, m), 1.76 (1H, d, J = 3.4 Hz), 1.32 (3H, t, J = 7.6 Hz). LCMS (ESI) m/z 302 $[M+H]^+$

Step 4: Synthesis of (3R)-1-(3-(ethylsulfonyl)-4-ethynyl-5-fluorophenyl)pyrrolidin-3-ol

The title compound was obtained as in step 1 of Reference Example 51, except that 2-ethylsulfonyl-6-fluoro-4-[(3R)-3-hydroxypyrrolidin-1-yl]benzaldehyde was used in place of 2-fluoro-6-methoxybenzaldehyde.
$^1$H-NMR ($CDCl_3$) δ: 7.03 (1H, d, J = 2.4 Hz), 6.41 (1H, dd, J = 11.7, 2.4 Hz), 4.69-4.67 (1H, m), 3.65 (1H, s), 3.61-3.43 (5H, m), 3.35 (1H, d, J = 11.0 Hz), 2.23-2.12 (2H, m), 1.69 (1H, d, J = 3.7 Hz), 1.27 (3H, t, J = 7.4 Hz). LCMS (ESI) m/z 298 $[M+H]^+$ Step 5: Synthesis of Reference Example Compound 72

The title compound was obtained as in step 4 of Reference Example 1, except that (3R)-1-(3-(ethylsulfonyl)-4-ethynyl-5-fluorophenyl)pyrrolidin-3-ol was used in place of 2-ethynyl-1,3-difluorobenzene.
$^1$H-NMR ($CD_3OD$) δ: 8.23 (1H, s), 7.62 (1H, s), 7.01 (1H, d, J = 2.3 Hz), 6.67 (1H, dd, J = 12.2, 2.3 Hz), 5.87 (1H, d, J = 7.1 Hz), 4.85-4.79 (1H, m), 4.58-4.54 (1H, m), 4.32-4.30 (1H, m), 4.26-4.24 (1H, m), 3.58-3.43 (6H, m), 3.42-3.33 (2H, m), 2.22-2.14 (1H, m), 2.12-2.05 (1H, m), 1.25 (3H, t, J = 7.4 Hz). LCMS (ESI) m/z 640 [M+H].

Reference Example 73

4-Amino-5-[2-(2-chloro-6-fluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-chloro-2-ethynyl-3-fluorobenzene

The title compound was obtained as in step 1 of Reference Example 51, except that 2-chloro-6-fluorobenzaldehyde was used in place of 2-fluoro-6-methoxybenzaldehyde.
$^1$H-NMR ($CDCl_3$) δ: 7.29-7.21 (2H, m), 7.05-7.00 (1H, m), 3.61 (1H, s) .

Step 2: Synthesis of Reference Example Compound 73

The title compound was obtained as in step 4 of Reference Example 1, except that 1-chloro-2-ethynyl-3-fluorobenzene was used in place of 2-ethynyl-1,3-difluorobenzene.
$^1$H-NMR ($CD_3OD$) δ: 8.23 (1H, s), 7.67 (1H, s), 7.37-7.30 (2H, m), 7.18-7.13 (1H, m), 5.87 (1H, d, J = 7.1 Hz), 4.85-4.82

(1H, m), 4.33-4.31 (1H, m), 4.27-4.25 (1H, m), 3.43-3.34 (2H, m). LCMS (ESI) m/z 497 [M+H].

Reference Example 74

4-[4-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3,5-difluoro-phenyl]-1,1-dioxo-1,4-thiazinane

Step 1: Synthesis of 4-(4-ethynyl-3,5-difluorophenyl)thiomorpholine-1,1-dioxide

The title compound was obtained as in step 3 of Reference Example 70, except that 4-(4-ethynyl-3,5-difluorophenyl)thiomorpholine was used in place of 2-(ethylthio)-6-fluoro-4-(pyrrolidin-1-yl)benzaldehyde.
$^1$H-NMR ($CDCl_3$) δ: 6.40 (2H, d, J = 10.2 Hz), 3.92-3.88 (4H, m), 3.42 (1H, s), 3.11-3.06 (4H, m). LCMS (ESI) m/z 272 $[M+H]^+$ Step 2: Synthesis of Reference Example Compound 74

The title compound was obtained as in step 4 of Reference Example 1, except that 4-(4-ethynyl-3,5-difluorophenyl)thiomorpholine-1,1-dioxide was used in place of 2-ethynyl-1,3-difluorobenzene.
$^1$H-NMR (DMSO-$D_6$) δ: 8.18 (1H, s), 7.90 (1H, s), 7.36-7.32 (1H, m), 6.95 (2H, d, J = 11.4 Hz), 6.60 (2H, s), 5.92 (1H, d, J = 7.0 Hz), 5.39 (1H, d, J = 6.2 Hz), 5.24-5.22 (1H, m), 4.60-4.55 (1H, m), 4.12-4.08 (1H, m), 4.06-4.03 (1H, m), 3.95-3.89 (4H, m), 3.25-3.17 (1H, m), 3.17-3.09 (5H, m). LCMS (ESI) m/z 614 $[M+H]^+$.

Reference Example 75

4-Amino-7-[(2R,3R,4S,5R)-3,4-dihydroxy-5-[(sulfamoylamino)methyl]tetrahydrofuran-2-yl]-5-[2-[2-ethylsulfonyl-6-fluoro-4-[(3R)-3-fluoropyrrolidin-1-yl]phenyl]ethynyl]pyrrolo[2,3-d]pyrimidine

Step 1: Synthesis of 2,6-difluoro-4-[(3R)-fluoropyrrolidin-1-yl]benzaldehyde

The title compound was obtained as in step 1 of Reference Example 23, except that 2,4,6-trifluorobenzaldehyde and (R)-3-fluoropyrrolidine were used in place of 2-ethynyl-1,3,5-trifluorobenzene and morpholine.
$^1$H-NMR ($CDCl_3$) δ: 10.07 (1H, s), 6.04 (2H, d, J = 12.5 Hz), 5.47-5.32 (1H, m), 3.64-3.51 (4H, m), 2.50-2.41 (1H, m), 2.28-2.08 (1H, m). LCMS (ESI) m/z 230 $[M+H]^+$

Step 2: Synthesis of 2-(ethylthio)-6-fluoro-4-[(3R)-3-fluoropyrrolidin-1-yl]benzaldehyde

The title compound was obtained as in step 2 of Reference Example 70, except that 2,6-difluoro-4-[(3R)-fluoropyrrolidin-1-yl]benzaldehyde was used in place of 2,6-difluoro-4-(pyrrolidin-1-yl)benzaldehyde.
$^1$H-NMR ($CDCl_3$) δ: 10.21 (1H, s), 6.11 (1H, d, J = 1.8 Hz), 6.01 (1H, dd, J = 13.9, 1.8 Hz), 5.47-5.33 (1H, m), 3.70-3.52 (4H, m), 2.92 (2H, q, J = 7.3 Hz), 2.49-2.40 (1H, m), 2.28-2.09 (1H, m), 1.41 (3H, t, J = 7.3 Hz). LCMS (ESI) m/z 272 $[M+H]^+$ Step 3: Synthesis of 2-(ethylsulfonyl)-6-fluoro-4-[(3R)-3-fluoropyrrolidin-1-yl]benzaldehyde

The title compound was obtained as in step 3 of Reference Example 70, except that 2-(ethylthio)-6-fluoro-4-[(3R)-3-fluoropyrrolidin-1-yl]benzaldehyde was used in place of 2-(ethylthio)-6-fluoro-4-(pyrrolidin-1-yl)benzaldehyde.
$^1$H-NMR ($CDCl_3$) δ: 10.31 (1H, s), 7.21 (1H, d, J = 2.3 Hz), 6.38 (1H, dd, J = 13.7, 2.3 Hz), 5.51-5.35 (1H, m), 3.74-3.60 (6H, m), 2.53-2.45 (1H, m), 2.29-2.14 (1H, m), 1.32 (3H, t, J = 7.3 Hz). LCMS (ESI) m/z 304 $[M+H]^+$

Step 4: Synthesis of (3R)-1-(3-ethylsulfonyl-4-ethynyl-5-fluorophenyl)-3-fluoro-pyrrolidine

The title compound was obtained as in step 1 of Reference Example 51, except that 2-(ethylsulfonyl)-6-fluoro-4-[(3R)-3-fluoropyrrolidin-1-yl]benzaldehyde was used in place of 2-fluoro-6-methoxybenzaldehyde.
$^1$H-NMR ($CDCl_3$) δ: 7.03 (1H, d, J = 2.4 Hz), 6.42 (1H, dd, J = 11.7, 2.4 Hz), 5.47-5.33 (1H, m), 3.66-3.64 (2H, m), 3.61-3.46 (5H, m), 2.49-2.40 (1H, m), 2.29-2.11 (1H, m), 1.27 (3H, t, J = 7.5 Hz). LCMS (ESI) m/z 300 $[M+H]^+$

Step 5: Synthesis of Reference Example Compound 75

The title compound was obtained as in step 4 of Reference Example 1, except that (3R)-1-(3-ethylsulfonyl-4-ethynyl-5-fluoro-phenyl)-3-fluoro-pyrrolidine was used in place of 2-ethynyl-1,3-difluorobenzene.
$^1$H-NMR ($CD_3OD$) δ: 8.20 (1H, s), 7.60 (1H, s), 6.99 (1H, d, J = 2.6 Hz), 6.66 (1H, dd, J = 12, 2.6 Hz), 5.86 (1H, d, J = 7.0 Hz), 5.47-5.34 (1H, m), 4.81-4.78 (1H, m), 4.31-4.29 (1H, m), 4.25-4.23 (1H, m), 3.70-3.44 (6H, m), 3.41-3.32 (2H, m), 2.42-2.15 (2H, m), 1.24 (3H, t, J = 7.3 Hz). LCMS (ESI) m/z 642 [M+H].

Reference Example 76

4-Amino-5-[2-(5-benzyloxypyrimidin-2-yl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 5-(benzyloxy)-2-bromopyrimidine

2-Bromopyrimidin-5-ol (200 mg, 1.1 mmol) was dissolved in a liquid mixture of tetrahydrofuran (1 mL) and N,N-dimethylformamide (1 mL). Then, potassium carbonate (170 mg, 1.3 mmol) and benzyl bromide (0.15 mL, 1.3 mmol) were added thereto at room temperature, followed by stirring at room temperature for 6 hours. The reaction solution was partitioned with ethyl acetate and water, and the organic layer was washed with water and saturated saline. The resulting product was dried over sodium sulfate, followed by filtration and concentration, and the residue was purified by silica gel column chromatography (developing solvent: ethyl acetate/hexane), thereby obtaining 5-(benzyloxy)-2-bromopyrimidine (200 mg, 0.75 mmol, 66%) as a white powder.
$^1$H-NMR ($CDCl_3$) δ: 8.31 (2H, s), 7.45-7.36 (5H, m), 5.15 (2H, s). LCMS (ESI) m/z 265 $[M+H]^+$

Step 2: Synthesis of 5-(benzyloxy)-2-((triisopropylsilyl)ethynyl)pyrimidine

5-(Benzyloxy)-2-bromopyrimidine (200 mg, 0.75 mmol), ethynyltriisopropylsilane (0.34 mL, 1.5 mmol), bis(triphenylphosphine)palladium (II) dichloride (53 mg, 0.075 mmol), copper iodide (14 mg, 0.075 mmol), and diisopropylethylamine (0.26 mL, 1.5 mmol) were suspended in tetrahydrofuran (2 mL). The reaction solution was stirred at 70°C for 1 hour and 30 minutes, and filtered through a celite bed, followed by distilling off the solvent. The residue was purified by silica gel column chromatography (developing solvent: ethyl acetate/hexane), thereby obtaining 5-(benzyloxy)-2-((triisopropylsilyl)ethynyl)pyrimidine (120 mg, 0.32 mmol, 43%) as a yellow oil.
$^1$H-NMR ($CDCl_3$) δ: 8.42 (2H, s), 7.43-7.36 (5H, m), 5.18 (2H, s), 1.17-1.13 (21H, m). LCMS (ESI) m/z 367 $[M+H]^+$

Step 3: Synthesis of 5-(benzyloxy)-2-ethynylpyrimidine

5-(Benzyloxy)-2-((triisopropylsilyl)ethynyl)pyrimidine (120 mg, 0.32 mmol) was dissolved in tetrahydrofuran (1 mL), and a tetrabutylammonium fluoride solution (1 M tetrahydrofuran solution, 0.38 mL, 0.38 mmol) was added thereto at room temperature, followed by stirring at room temperature for 30 minutes.
After the solvent was distilled off, the residue was purified by silica gel column chromatography (developing solvent: ethyl acetate/hexane), thereby obtaining 5-(benzyloxy)-2-ethynylpyrimidine (45 mg, 0.21 mmol, 67%) as a yellow powder.
$^1$H-NMR ($CDCl_3$) δ: 8.43 (2H, s), 7.44-7.38 (5H, m), 5.19 (2H, s), 3.04 (1H, s). LCMS (ESI) m/z 211 $[M+H]^+$

Step 4: Synthesis of Reference Example Compound 76

The title compound was obtained as in step 4 of Reference Example 1, except that 5-(benzyloxy)-2-ethynylpyrimidine was used in place of 2-ethynyl-1,3-difluorobenzene.
$^1$H-NMR ($CD_3OD$) δ: 8.57 (2H, s), 8.26 (1H, s), 7.75 (1H, s), 7.49-7.33 (5H, m), 5.87 (1H, d, J = 6.8 Hz), 5.30 (2H, s), 4.84-4.79 (1H, m), 4.32-4.27 (1H, m), 4.26-4.24 (1H, m), 3.40-3.35 (2H, m). LCMS (ESI) m/z 553 $[M+H]^+$.

Reference Example 77

4-Amino-5-[2-(4-benzyloxy-2-fluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of ((4-(benzyloxy)-2-fluorophenyl)ethynyl)trimethylsilane

The title compound was obtained as in step 2 of Reference Example 76, except that 4-(benzyloxy)-2-fluoro-1-iodobenzene and ethynyltrimethylsilane were used in place of 5-(benzyloxy)-2-bromopyrimidine and ethynyltriisopropylsilane.
$^1$H-NMR ($CDCl_3$) δ: 7.43-7.33 (6H, m), 6.77 (1H, dd, J = 10.4, 2.8 Hz), 6.70-6.67 (1H, m), 5.04 (2H, s), 0.25 (9H, s).

Step 2: Synthesis of 4-(benzyloxy)-1-ethynyl-2-fluorobenzene

The title compound was obtained as in step 3 of Reference Example 76, except that 4((4-(benzyloxy)-2-fluorophenyl)ethynyl)trimethylsilane was used in place of 5-(benzyloxy)-2-((triisopropylsilyl)ethynyl)pyrimidine.

$^1$H-NMR ($CDCl_3$) δ: 7.43-7.29 (6H, m), 6.66-6.79 (2H, m), 5.04 (2H, s), 3.25 (1H, s).

Step 3: Synthesis of Reference Example Compound 77

The title compound was obtained as in step 4 of Reference Example 1, except that 4-(benzyloxy)-1-ethynyl-2-fluorobenzene was used in place of 2-ethynyl-1,3-difluorobenzene.
$^1$H-NMR ($CD_3OD$) δ: 8.22 (1H, s), 7.56 (1H, s), 7.40-7.26 (6H, m), 6.87-6.84 (2H, m), 5.85 (1H, d, J = 6.8 Hz), 5.12 (2H, s), 4.83-4.81 (1H, m), 4.33-4.29 (1H, m), 4.27-4.25 (1H, m), 3.40-3.35 (2H, m). LCMS (ESI) m/z 569 $[M+H]^+$.

Reference Example 78

4-Amino-5-[2-[4-(benzylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of N-benzyl-4-((triisopropylsilyl)ethynyl)aniline

The title compound was obtained as in step 2 of Reference Example 76, except that N-benzyl-4-iodoaniline and ethynyltriisopropylsilane were used in place of 5-(benzyloxy)-2-bromopyrimidine and ethynyltriisopropylsilane.
$^1$H-NMR ($CDCl_3$) δ: 7.37-7.25 (7H, m), 6.53 (2H, d, J = 8.8 Hz), 4.34 (2H, s), 1.12-1.05 (21H, m). LCMS (ESI) m/z 364 $[M+H]^+$ Step 2: Synthesis of N-benzyl-4-ethynylaniline

The title compound was obtained as in step 3 of Reference Example 76, except that N-benzyl-4-((triisopropylsilyl)ethynyl)aniline was used in place of 5-(benzyloxy)-2-((triisopropylsilyl)ethynyl)pyrimidine.
$^1$H-NMR ($CDCl_3$) δ: 7.39-7.25 (7H, m), 6.55 (2H, d, J = 8.0 Hz), 4.35 (2H, s), 4.28-4.19 (1H, brs), 2.96 (1H, s). LCMS (ESI) m/z 208 $[M+H]^+$

Step 3: Synthesis of Reference Example Compound 78

The title compound was obtained as in step 4 of Reference Example 1, except that N-benzyl-4-ethynylaniline was used in place of 2-ethynyl-1,3-difluorobenzene.
$^1$H-NMR ($CD_3OD$) δ: 8.25 (1H, s), 7.60 (1H, s), 7.36-7.20 (7H, m), 6.60 (2H, d, J = 8.8 Hz), 5.93 (1H, d, J = 6.6 Hz), 4.73-4.70 (1H, m), 4.35 (2H, s), 4.31-4.29 (1H, m), 4.25-4.22 (1H, m), 3.40-3.35 (2H, m). LCMS (ESI) m/z 550 $[M+H]^+$.

Reference Example 79

2-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3-fluorobenzamide

Step 1: Synthesis of 3-fluoro-2-((trimethylsilyl)ethynyl)benzamide

The title compound was obtained as in step 2 of Reference Example 76, except that 3-fluoro-2-iodobenzamide and ethynyltrimethylsilane were used in place of 5-(benzyloxy)-2-bromopyrimidine and ethynyltriisopropylsilane.
$^1$H-NMR ($CDCl_3$) δ: 7.96 (1H, d, J = 7.8 Hz), 7.83-7.55 (1H, brs), 7.45-7.40 (1H, m), 7.28-7.22 (1H, m), 6.19-5.72 (1H, brs), 0.31 (9H, s). LCMS (ESI) m/z 236 $[M+H]^+$

Step 2: Synthesis of 2-ethynyl-3-fluorobenzamide

The title compound was obtained as in step 3 of Reference Example 76, except that 3-fluoro-2-((trimethylsilyl)ethynyl)benzamide was used in place of 5-(benzyloxy)-2-((triisopropylsilyl)ethynyl)pyrimidine.
$^1$H-NMR ($CDCl_3$) δ: 7.89-7.86 (1H, m), 7.46 (1H, dt, J = 5.4, 8.0 Hz), 7.30-7.21 (2H, m), 6.29-5.45 (1H, brs), 3.77 (1H, s). LCMS (ESI) m/z 164 $[M+H]^+$

Step 3: Synthesis of Reference Example Compound 79

The title compound was obtained as in step 4 of Reference Example 1, except that 2-ethynyl-3-fluorobenzamide was used in place of 2-ethynyl-1,3-difluorobenzene.
$^1$H-NMR ($CD_3OD$) δ: 8.23 (1H, s), 7.66 (1H, s), 7.47-7.41 (2H, m), 7.35-7.30 (1H, m), 5.87 (1H, d, J = 6.8 Hz), 4.82-4.75 (1H, m), 4.32-4.29 (1H, m), 4.26-4.24 (1H, m), 3.40-3.35 (2H, m). LCMS (ESI) m/z 506 $[M+H]^+$.

Reference Example 80

4-Amino-5-[2-(3,5-difluoro-2-methoxy-4-pyridyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 4-ethynyl-3,5-difluoro-2-methoxypyridine

2,3,5-Trifluoro-4-iodopyridine (500 mg, 1.93 mmol) was dissolved in methanol (3 mL), and sodium methoxide (a 5 M methanol solution, 1.15 mL) was added thereto at room temperature, followed by stirring at room temperature overnight. The reaction solution was partitioned between ethyl acetate and water, and the organic layer was separated and sequentially washed with water and saturated brine. The solvent was distilled off, and the residue was purified by silica gel column chromatography (developing solvent: ethyl acetate/hexane), thereby obtaining the target product (376 mg) as an oil mixture of 4-ethynyl-3,5-difluoro-2-methoxypyridine and 4-ethynyl-3-fluoro-2,5-dimethoxypyridine.

The synthesis was performed as in steps 2 and 3 of Reference Example 76 by using the obtained mixture, followed by separation and purification by silica gel column chromatography (developing solvent: ethyl acetate/hexane) to give the title compound.
$^1$H-NMR ($CDCl_3$) δ: 7.87 (1H, s), 4.02 (3H, s), 3.73 (1H, s).

Step 2: Synthesis of Reference Example Compound 80

The title compound was obtained as in step 4 of Reference Example 1, except that 4-ethynyl-3,5-difluoro-2-methoxypyridine was used in place of 2-ethynyl-1,3-difluorobenzene.
$^1$H-NMR (DMSO-$D_6$) δ: 8.20 (1H, s), 8.19 (1H, s), 8.14 (1H, s), 7.32-7.29 (1H, m), 6.59 (2H, s), 5.94 (1H, d, J = 6.6 Hz), 5.41 (1H, d, J = 6.6 Hz), 5.24 (1H, d, J = 4.4 Hz), 4.58 (1H, ddd, J = 7.3, 6.2, 4.4 Hz), 4.11-4.04 (2H, m), 3.96 (3H, s).
LCMS (ESI) m/z 512 $[M+H]^+$.

Reference Example 81

4-Amino-7-[(2R,3R,4S,5R)-3,4-dihydroxy-5-[(sulfamoylamino)methyl]tetrahydrofuran-2-yl]-5-[2-(3-fluoro-2,5-dimethoxy-4-pyridyl)ethynyl]pyrrolo[2,3-d]pyrimidine

Step 1: Synthesis of 4-ethynyl-3-fluoro-2,5-dimethoxypyridine

In accordance with step 1 of Reference Example 80, the title compound was obtained.
$^1$H-NMR ($CDCl_3$) δ: 7.76 (1H, s), 4.01 (3H, s), 3.97 (3H, s), 3.66 (1H, s).

Step 2: Synthesis of Reference Example Compound 81

The title compound was obtained as in step 4 of Reference Example 1, except that 4-ethynyl-3-fluoro-2,5-dimethoxypyridine was used in place of 2-ethynyl-1,3-difluorobenzene.
$^1$H-NMR ($CD_3OD$) δ: 8.26 (1H, s), 7.83 (1H, s), 7.75 (1H, s), 5.89 (1H, d, J = 6.6 Hz), 4.85-4.82 (3H, m), 4.34-4.32 (1H, m), 4.29-4.26 (1H, m), 4.04 (3H, s), 3.99 (3H, s), 3.70-3.67 (1H, m), 3.58-3.56 (1H, m), 3.42 (1H, dd, J = 12.5, 4.4 Hz), 3.37 (1H, dd, J = 12.5, 3.7 Hz). LCMS (ESI) m/z 524 $[M+H]^+$.

Reference Example 82

4-Amino-7-[(2R,3R,4S,5R)-3,4-dihydroxy-5-[(sulfamoylamino)methyl]tetrahydrofuran-2-yl]-5-[2-(2-ethylsulfanylphenyl)ethynyl]pyrrolo[2,3-d]pyrimidine

Step 1: Synthesis of 1-ethylsulfanyl-2-ethynyl-benzene

The title compound was obtained as in step 2 and step 3 of Reference Example 76, except that 1-bromo-2-ethylsulfanyl-benzene was used in place of 5-(benzyloxy)-2-bromopyrimidine.
$^1$H-NMR ($CDCl_3$) δ: 7.49 (1H, dd, J = 7.5, 1.3 Hz), 7.33-7.25 (2H, m), 7.12 (1H, dt, J = 1.5, 7.3 Hz), 3.47 (1H, s), 3.01 (2H, q, J = 7.5 Hz), 1.37 (3H, dd, J = 9.5, 5.1 Hz).

Step 2: Synthesis of Reference Example Compound 82

The title compound was obtained as in step 4 of Reference Example 1, except that 1-ethylsulfanyl-2-ethynylbenzene was used in place of 2-ethynyl-1,3-difluorobenzene.
$^1$H-NMR (DMSO-$D_6$) δ: 8.18 (1H, s), 7.91 (1H, s), 7.52 (1H, dd, J = 7.7, 1.1 Hz), 7.44-7.36 (3H, m), 7.23 (1H, dt, J = 1.5, 7.3 Hz), 6.61 (2H, s), 5.93 (1H, d, J = 7.0 Hz), 5.41 (1H, d, J = 6.6 Hz), 5.25 (1H, d, J = 4.4 Hz), 4.59 (1H, q, J = 6.2 Hz), 4.12-4.03 (2H, m), 3.23-3.19 (1H, m), 3.13 (1H, dd, J = 8.1, 5.1 Hz), 3.05 (2H, q, J = 7.5 Hz), 1.29 (3H, t, J = 7.3 Hz). LCMS (ESI) m/z 505.3 $[M+H]^+$.

Reference Example 83

4-Amino-7-[(2R,3R,4S,5R)-3,4-dihydroxy-5-[(sulfamoylamino)methyl]tetrahydrofuran-2-yl]-5-[2-(1,1-dioxo-3,4-dihydro-2H-thiochromen-8-yl)ethynyl]pyrrolo[2,3-d]pyrimidine

Step 1: Synthesis of triisopropyl(thiochroman-8-ylethynyl)silane

The title compound was obtained as in step 2 of Reference Example 76, except that 8-iodothiochroman was used in place of 5-(benzyloxy)-2-bromopyrimidine.
$^1$H-NMR ($CDCl_3$) δ: 7.27-7.25 (1H, m), 6.97-6.93 (1H, m), 6.89 (1H, t, J = 7.6 Hz), 3.07-3.04 (2H, m), 2.80 (2H, t, J = 6.1 Hz), 2.12-2.06 (2H, m), 1.26-1.04 (21H, m).

Step 2: Synthesis of 8-ethynyl thiochroman

The title compound was obtained as in step 3 of Reference Example 76, except that triisopropyl(thiochroman-8-ylethynyl)silane was used in place of 5-(benzyloxy)-2-((triisopropylsilyl)ethynyl)pyrimidine.
$^1$H-NMR ($CDCl_3$) δ: 7.31-7.26 (1H, m), 7.02-6.98 (1H, m), 6.92 (1H, t, J = 7.6 Hz), 3.45 (1H, s), 3.09-3.06 (2H, m), 2.82 (2H, t, J = 6.1 Hz), 2.13-2.07 (2H, m).

Step 3: Synthesis of 8-ethynyl thiochroman 1,1-dioxide

1,4-Dioxane (1 mL) and water (0.50 mL) were added to 8-ethynyl thiochroman (59 mg, 0.34 mmol). Then, oxone (420 mg, 0.68 mmol) was added thereto under ice-cooling, and the resulting mixture was allowed to warm to room temperature with stirring overnight. The reaction solution was partitioned at room temperature with the addition of ethyl acetate, a saturated aqueous sodium hydrogen carbonate solution, and water, and the aqueous layer was extracted with ethyl acetate. The organic layer was washed with saturated saline, and dried over sodium sulfate, followed by filtration and concentration. The residue was purified by silica gel column chromatography (developing solvent: ethyl acetate/hexane), thereby obtaining the title compound (51 mg, 0.25 mmol, 73%) as a white solid.
$^1$H-NMR ($CDCl_3$) δ: 7.55 (1H, d, J = 7.8 Hz), 7.38 (1H, t, J = 7.8 Hz), 7.21 (1H, dd, J = 7.8, 1.0 Hz), 3.59 (1H, s), 3.44-3.41 (2H, m), 3.01 (2H, t, J = 6.2 Hz), 2.48-2.42 (2H, m). LCMS (ESI) m/z 207 $[M+H]^+$

Step 4: Synthesis of Reference Example Compound 83

The title compound was obtained as in step 4 of Reference Example 1, except that 8-ethynyl thiochroman 1,1-dioxide was used in place of 2-ethynyl-1,3-difluorobenzene.
$^1$H-NMR ($CD_3OD$) δ: 8.22 (1H, s), 7.69 (1H, s), 7.57 (1H, d, J = 7.6 Hz), 7.48 (1H, t, J = 7.6 Hz), 7.30 (1H, d, J = 7.6 Hz), 5.87 (1H, d, J = 6.8 Hz), 4.83-4.79 (1H, m), 4.32-4.30 (1H, m), 4.27-4.25 (1H, m), 3.54-3.51 (2H, m), 3.43-3.33 (2H, m), 3.07 (2H, t, J = 6.0 Hz), 2.43-2.38 (2H, m). LCMS (ESI) m/z 549 [M+H].

Reference Example 84

2-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-3-fluoro-benzenesulfonamide

Step 1: Synthesis of 3-fluoro-2-((trimethylsilyl)ethynyl)benzenesulfonamide

The title compound was obtained as in step 2 of Reference Example 76, except that 3-fluoro-2-iodobenzenesulfonamide and ethynyltrimethylsilane were used in place of 5-(benzyloxy)-2-bromopyrimidine and ethynyltriisopropylsilane.

$^1$H-NMR ($CDCl_3$) δ: 7.85 (1H, d, J = 7.8 Hz), 7.45-7.41 (1H, m), 7.31-7.27 (1H, m), 5.24 (2H, s), 0.33 (9H, s). LCMS (ESI) m/z 272 $[M+H]^+$

Step 2: Synthesis of 2-ethynyl-3-fluorobenzenesulfonamide

The title compound was obtained as in step 3 of Reference Example 76, except that 3-fluoro-2-((trimethylsilyl)ethynyl)benzenesulfonamide was used in place of 5-(benzyloxy)-2-((triisopropylsilyl)ethynyl)pyrimidine.
$^1$H-NMR ($CDCl_3$) δ: 7.88 (1H, d, J = 7.8 Hz), 7.52-7.47 (1H, m), 7.37-7.31 (1H, m), 5.21 (2H, s), 3.90 (1H, s). LCMS (ESI) m/z 200 $[M+H]^+$

Step 3: Synthesis of Reference Example Compound 84

The title compound was obtained as in step 4 of Reference Example 1, except that 2-ethynyl-3-fluorobenzenesulfonamide was used in place of 2-ethynyl-1,3-difluorobenzene.
$^1$H-NMR ($CD_3OD$) δ: 8.30 (1H, s), 7.85 (1H, d, J = 7.8 Hz), 7.74 (1H, s), 7.54-7.49 (1H, m), 7.46-7.41 (1H, m), 5.88 (1H, d, J = 6.8 Hz), 4.84-4.81 (1H, m), 4.33-4.30 (1H, m), 4.27-4.25 (1H, m), 3.43-3.33 (2H, m). LCMS (ESI) m/z 542 [M+H].

Reference Example 85

4-Amino-5-[2-(2-cyano-6-fluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 3-fluoro-2-((trimethylsilyl)ethynyl)benzonitrile

The title compound was obtained as in step 2 of Reference Example 76, except that 3-fluoro-2-iodobenzonitrile and ethynyltrimethylsilane were used in place of 5-(benzyloxy)-2-bromopyrimidine and ethynyltriisopropylsilane.
$^1$H-NMR ($CDCl_3$) δ: 7.46-7.44 (1H, m), 7.41-7.35 (1H, m), 7.33-7.29 (1H, m), 0.31 (9H, s). LCMS (ESI) m/z 218 $[M+H]^+$

Step 2: Synthesis of 2-ethynyl-3-fluorobenzonitrile

The title compound was obtained as in step 3 of Reference Example 76, except that 3-fluoro-2-((trimethylsilyl)ethynyl)benzonitrile was used in place of 5-(benzyloxy)-2-((triisopropylsilyl)ethynyl)pyrimidine.
$^1$H-NMR ($CDCl_3$) δ: 7.51 (1H, dd, J = 7.7, 1.5 Hz), 7.49-7.44 (1H, m), 7.39-7.35 (1H, m), 3.72 (1H, s).

Step 3: Synthesis of Reference Example Compound 85

The title compound was obtained as in step 4 of Reference Example 1, except that 2-ethynyl-3-fluorobenzonitrile was used in place of 2-ethynyl-1,3-difluorobenzene.
$^1$H-NMR ($CD_3OD$) δ: 8.27 (1H, s), 7.79 (1H, s), 7.66-7.64 (1H, m), 7.57-7.53 (2H, m), 5.88 (1H, d, J = 6.8 Hz), 4.84-4.80 (1H, m), 4.33-4.31 (1H, m), 4.28-4.26 (1H, m), 3.45-3.35 (2H, m). LCMS (ESI) m/z 488 [M+H].

Reference Example 86

4-Amino-5-[2-[2-(cyclopropylmethoxy)-6-fluoro-phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine

Step 1: Synthesis of 1-(cyclopropylmethoxy)-3-fluoro-2-iodobenzene

3-Fluoro-2-iodophenol (200 mg, 0.84 mmol) was dissolved in tetrahydrofuran (2 mL). Then, cyclopropylmethanol (0.14 mL, 1.7 mmol) and triphenylphosphine (440 mg, 1.7 mmol) were added thereto at room temperature, and diisopropyl azodicarboxylate (0.33 ml, 1.7 mmol) was added thereto in an ice bath, followed by stirring for 2 hours in an ice bath. The reaction solution was concentrated, and the residue was purified by silica gel column chromatography (developing solvent: ethyl acetate/hexane), thereby obtaining 1-(cyclopropylmethoxy)-3-fluoro-2-iodobenzene (192 mg) as a yellow oil.
$^1$H-NMR ($CDCl_3$) δ: 7.26-7.19 (1H, m), 6.72-6.67 (1H, m), 6.57 (1H, d, J = 8.3 Hz), 3.91 (2H, d, J = 6.6 Hz), 1.33-1.28 (1H, m), 0.68-0.63 (2H, m), 0.44-0.41 (2H, m).

Step 2: Synthesis of ((2-(cyclopropylmethoxy)-6-fluorophenyl)ethynyl)trimethylsilane

The title compound was obtained as in step 2 of Reference Example 76, except that 1-(cyclopropylmethoxy)-3-fluoro-2-iodobenzene and ethynyltrimethylsilane were used in place of 5-(benzyloxy)-2-bromopyrimidine and ethynyltriisopropylsilane.
$^1$H-NMR ($CDCl_3$) δ: 7.17 (1H, dt, J = 6.6, 8.4 Hz), 6.69-6.64 (1H, m), 6.61 (1H, d, J = 8.5 Hz), 3.91 (2H, d, J = 6.3 Hz), 1.31-1.24 (1H, m), 0.64-0.60 (2H, m), 0.45-0.41 (2H, m), 0.28 (9H, s).

Step 3: Synthesis of 1-(cyclopropylmethoxy)-2-ethynyl-3-fluorobenzene

The title compound was obtained as in step 3 of Reference Example 76, except that ((2-(cyclopropylmethoxy)-6-fluorophenyl)ethynyl)trimethylsilane was used in place of 5-(benzyloxy)-2-((triisopropylsilyl)ethynyl)pyrimidine.
$^1$H-NMR ($CDCl_3$) δ: 7.24 (1H, dt, J = 6.6, 8.4 Hz), 6.72 (1H, t, J = 8.4 Hz), 6.67 (1H, J = 8.4 Hz), 3.93 (2H, d, J = 6.6 Hz), 3.52 (1H, s), 1.36-1.30 (1H, m), 0.68-0.63 (2H, m), 0.43-0.39 (2H, m).

Step 4: Synthesis of Reference Example Compound 86

The title compound was obtained as in step 4 of Reference Example 1, except that 1-(cyclopropylmethoxy)-2-ethynyl-3-fluorobenzene was used in place of 2-ethynyl-1,3-difluorobenzene.
$^1$H-NMR ($CD_3OD$) δ: 8.25 (1H, s), 7.59 (1H, s), 7.33-7.27 (1H, m), 6.88 (1H, d, J = 8.5 Hz), 6.79 (1H, t, J = 8.5 Hz), 5.88 (1H, d, J = 7.0 Hz), 4.86-4.83 (1H, m), 4.34-4.32 (1H, m), 4.28-4.26 (1H, m), 4.01 (2H, d, J = 7.0 Hz), 3.44-3.34 (2H, m), 1.40-1.32 (1H, m), 0.73-0.68 (2H, m), 0.43 (2H, m). LCMS (ESI) m/z 533 [M+H].

Reference Example 87

4-Amino-7-[(2R,3R,4S,5R)-3,4-dihydroxy-5-[(sulfamoylamino)methyl]tetrahydrofuran-2-yl]-5-[2-[4-(3-pyridylmethoxy)phenyl]ethynyl]pyrrolo[2,3-d]pyrimidine

Step 1: Synthesis of tert-butyl N-(((3aR,4R,6R,6aR)-6-(4-amino-5-((trimethylsilyl)ethynyl)-7H-pyrrolo[2,3-d]pyrimidin-7-yl)-2,2-dimethyltetrahydrofuro[3,4-d][1,3]dioxol-4-yl)methyl)sulfamoyl carbamate

tert-Butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1, 3]dioxol-4-yl)methyl)sulfamoyl carbamate (500 mg, 0.82 mmol), ethynyltrimethylsilane (240 mg, 2.5 mmol), bis(triphenylphosphine)palladium (II) dichloride (58 mg, 0.082 mmol), copper iodide (16 mg, 0.082 mmol), and diisopropylethylamine (0.28 mL, 1.6 mmol) were suspended in tetrahydrofuran (5 mL). The reaction solution was stirred at 70°C overnight and filtered through a celite bed, followed by distilling off the solvent. The residue was purified by silica gel column chromatography (developing solvent: methanol/chloroform), thereby obtaining the title compound (339 mg, 0.58 mmol, 71%) as a yellow amorphous substance.
$^1$H-NMR ($CDCl_3$) δ: 9.41 (1H, brs), 8.50 (1H, s), 7.16 (1H, s), 5.98 (2H, brs), 5.63 (1H, d, J = 4.6 Hz), 5.25-5.21 (1H, m), 5.07-5.03 (1H, m), 4.51-4.49 (1H, m), 3.59-3.50 (2H, m), 1.59 (3H, s), 1.43 (9H, s), 1.34 (3H, s), 0.26 (9H, s). LCMS (ESI) m/z 581 $[M+H]^+$

Step 2: Synthesis of tert-butyl N-(((3aR,4R,6R,6aR)-6-(4-amino-5-ethynyl-7H-pyrrolo[2,3-d]pyrimidin-7-yl)-2,2-dimethyltetrahydrofuro[3,4-d][1,3]dioxol-4-yl)methyl)sulfamoyl carbamate

tert-Butyl N-(((3aR,4R,6R,6aR)-6-(4-amino-5-((trimethylsilyl)ethynyl)-7H-pyrrolo[2,3-d]pyrimidin-7-yl)-2,2-dimethyltetrahydrofuro[3,4-d][1,3]dioxol-4-yl)methyl)sulfamoyl carbamate (339 mg, 0.58 mmol) was dissolved in tetrahydrofuran (6.8 mL), and a tetrabutylammonium fluoride solution (a 1 M tetrahydrofuran solution, 0.70 mL, 0.70 mmol) was added thereto at room temperature, followed by stirring at room temperature for 25 minutes. After the solvent was distilled off, the residue was purified by silica gel column chromatography (developing solvent: methanol/chloroform), thereby obtaining the title compound (260 mg, 0.51 mmol, 88%) as a yellow powder.
$^1$H-NMR ($CDCl_3$) δ: 9.25 (1H, brs), 8.51 (1H, s), 7.20 (1H, s), 6.11 (2H, brs), 5.65 (1H, d, J = 4.6 Hz), 5.29-5.25 (1H, m), 5.10-5.06 (1H, m), 4.52-4.49 (1H, m), 3.66-3.53 (2H, m), 3.25 (1H, s), 1.61 (3H, s), 1.44 (9H, s), 1.35 (3H, s). LCMS (ESI) m/z 509 $[M+H]^+$ Step 3: Synthesis of Reference Example Compound 87

tert-Butyl N-(((3aR,4R,6R,6aR)-6-(4-amino-5-ethynyl-7H-pyrrolo[2,3-d]pyrimidin-7-yl)-2,2-dimethyltetrahydrofuro[3,4-d][1,3]dioxol-4-yl)methyl)sulfamoyl carbamate (20 mg, 0.039 mmol), 3-((4-iodophenoxy)methyl)pyridine (24 mg, 0.079 mmol), bis(triphenylphosphine)palladium (II) dichloride (3 mg, 0.0043 mmol), copper iodide (1 mg, 0.0053 mmol), and diisopropylethylamine (0.013 mL, 0.079 mmol) were suspended in tetrahydrofuran (0.30 mL). The reaction solution

was stirred at 70°C overnight, and a mixed solution (0.60 mL) of trifluoroacetic acid/water = 4/1 was added thereto at room temperature, followed by stirring at room temperature overnight. After the solvent was distilled off, the residue was purified by basic silica gel column chromatography (developing solvent: methanol/chloroform), thereby obtaining the title compound (2.1 mg, 0.0039 mmol, 10%) as a yellow powder.
$^1$H-NMR ($CD_3OD$) δ: 8.65-8.60 (1H, m), 8.55-8.50 (1H, m), 8.24 (1H, s), 7.95-7.90 (1H, m), 7.50-7.40 (4H, m), 7.05-7.00 (2H, brs), 5.84-5.82 (1H, brs), 5.19 (2H, s), 4.80-4.70 (1H, m), 4.35-4.20 (2H, m), 3.40-3.30 (2H, m). LCMS (ESI) m/z 552 $[M+H]^+$.

Reference Example 88

4-Amino-7-[(2R,3R,4S,5R)-3,4-dihydroxy-5-[(sulfamoylamino)methyl]tetrahydrofuran-2-yl]-5-[2-(3-fluoro-2-pyridyl)ethynyl]pyrrolo[2,3-d]pyrimidine

The title compound was obtained as in step 3 of Reference Example 87, except that 3-fluoro-2-iodopyridine was used in place of 3-((4-iodophenoxy)methyl)pyridine.
$^1$H-NMR ($CD_3OD$) δ: 8.43-8.39 (1H, m), 8.26 (1H, s), 7.78 (1H, s), 7.78-7.71 (1H, m), 7.50-7.46 (1H, m), 5.88 (1H, d, J = 7.1 Hz), 4.90-4.60 (1H, m), 4.33-4.31 (1H, m), 4.28-4.25 (1H, m), 3.40-3.31 (2H, m). LCMS (ESI) m/z 464 $[M+H]^+$.

Reference Example 89

4-Amino-7-[(2R,3R,4S,5R)-3,4-dihydroxy-5-[(sulfamoylamino)methyl]tetrahydrofuran-2-yl]-5-[2-(2-methylsulfanylphenyl)ethynyl]pyrrolo[2,3-d]pyrimidine

The title compound was obtained as in step 3 of Reference Example 87, except that (2-iodophenyl)(methyl)sulfane was used in place of 3-((4-iodophenoxy)methyl)pyridine.
$^1$H-NMR ($CD_3OD$) δ: 8.24 (1H, s), 7.62 (1H, s), 7.48-7.46 (1H, m), 7.37-7.34 (2H, m), 7.22-7.16 (1H, m), 5.86 (1H, d, J = 8.0 Hz), 4.83-4.81 (1H, m), 4.33-4.29 (1H, m), 4.27-4.25 (1H, m), 3.40-3.35 (2H, m), 2.55 (3H, s). LCMS (ESI) m/z 491 $[M+H]^+$.

Reference Example 90

2-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]benzenesulfonamide

The title compound was obtained as in step 3 of Reference Example 87, except that 2-iodobenzenesulfonamide was used in place of 3-((4-iodophenoxy)methyl)pyridine.
$^1$H-NMR ($CD_3OD$) δ: 8.23 (1H, s), 8.02 (1H, dd, J = 8.0, 4.0 Hz), 7.74 (1H, dd, J = 7.8, 4.0 Hz), 7.71 (1H, s), 7.85-7.56 (1H, m), 7.53-7.49 (1H, m), 5.87 (1H, d, J = 6.8 Hz), 4.83-4.81 (1H, m), 4.33-4.29 (1H, m), 4.27-4.25 (1H, m), 3.40-3.35 (2H, m). LCMS (ESI) m/z 524 $[M+H]^+$.

Reference Example 91

3-[[4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]phenoxy]methyl]benzamide

The title compound was obtained as in step 3 of Reference Example 87, except that 3-((4-iodophenoxy)methyl)benzamide was used in place of 3-((4-iodophenoxy)methyl)pyridine.
$^1$H-NMR ($CD_3OD$) δ: 8.23 (1H, s), 7.98 (1H, s), 7.84 (1H, d, J = 8.1 Hz), 7.65 (1H, d, J = 8.0 Hz), 7.54 (1H, s), 7.54-7.49 (1H, m), 7.48 (2H, d, J = 8.0 Hz), 7.05 (2H, d, J = 8.0 Hz), 5.85 (1H, d, J = 7.1 Hz), 5.19 (2H, s), 4.83-4.81 (1H, m), 4.33-4.29 (1H, m), 4.27-4.25 (1H, m), 3.40-3.35 (2H, m). LCMS (ESI) m/z 594 $[M+H]^+$.

Reference Example 92

4-[2-[4-Amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]isoquinoline

The title compound was obtained as in step 3 of Reference Example 87, except that 4-iodoisoquinoline was used in place of 3-((4-iodophenoxy)methyl)pyridine.

$^1$H-NMR ($CD_3OD$) δ: 9.20 (1H, s), 8.69 (1H, s), 8.34 (1H, d, J = 8.3 Hz), 8.26 (1H, s), 8.16 (1H, d, J = 8.3 Hz), 7.95-7.90 (1H, m), 7.81 (1H, s), 7.80-7.74 (1H, m), 5.92 (1H, d, J = 6.8 Hz), 4.87-4.81 (1H, m), 4.36-4.33 (1H, m), 4.29-4.25 (1H, m), 3.43-3.39 (2H, m). LCMS (ESI) m/z 496 [M+H]$^+$.

Reference Example 93

4-Amino-7-[(1R,2S,3R,4R)-2,3-dihydroxy-4-[(sulfamoylamino)methyl]cyclopentyl]-5-[2-(1-naphthyl)ethynyl]pyrrolo[2,3-d]pyrimidine

Step 1: Synthesis of ((3aR,4R,6R,6aS)-6-amino-2,2-dimethyltetrahydro-3aH-cyclopenta[d][1,3]dioxol-4-yl)methanol and (4aR,6R,7S,7aR)-6-amino-2,2-dimethylhexahydrocyclopenta[d][1,3]dioxin-7-ol

(1R,2S,3R,5R)-3-Amino-5-(hydroxymethyl)cyclopentane-1,2-diol hydrochloride (40 g, 218 mmol) was dissolved at room temperature in methanol (400 mL) and 2,2-dimethoxypropane (54 mL, 436 mmol), and methanesulfonic acid(14 mL, 218 mmol) was added thereto dropwise in an ice bath with stirring so as to keep the internal temperature at 7°C or lower. After the mixture was stirred in an ice bath for 5 minutes and at room temperature overnight, triethylamine (122 mL, 872 mmol) was added thereto dropwise in an ice bath so as to keep the internal temperature at 10°C or lower. After the mixture was stirred in an ice bath for 5 minutes and at room temperature for 30 minutes, the solvent was distilled off under reduced pressure, thereby obtaining the title compound as a crude isomeric mixture (102 g).
LCMS (ESI) m/z 188 [M+H]$^+$

Step 2: Synthesis of ((3aR,4R,6R,6aS)-6-(4-chloro-7H-pyrrolo[2,3-d]pyrimidin-7-yl)-2,2-dimethyltetrahydro-3aH-cyclopenta[d][1,3]dioxol-4-yl)methanol and (4aR,6R,7S,7aR)-6-(4-chloro-7H-pyrrolo[2,3-d]pyrimidin-7-yl)-2,2-dimethylhexahydrocyclopenta[d][1,3]dioxin-7-ol

The crude isomeric mixture (102 g) of ((3aR,4R,6R,6aS)-6-amino-2,2-dimethyltetrahydro-3aH-cyclopenta[d][1,3]dioxol-4-yl)methanol and (4aR,6R,7S,7aR)-6-amino-2,2-dimethylhexahydrocyclopenta[d][1,3]dioxin-7-ol obtained in step 1 of Reference Example 93, and 2-(4,6-dichloropyrimidin-5-yl)acetaldehyde (46 g, 240 mmol) was suspended in 2-butanol (400 mL). Then, triethylamine (61 mL, 436 mmol) was added thereto at room temperature, and the reaction solution was stirred at 80°C overnight. After the reaction solvent was distilled off under reduced pressure, the residue was partitioned with the addition of ethyl acetate and an aqueous sodium hydrogen carbonate solution, followed by extraction with ethyl acetate. The organic layer was washed with saturated saline, and dried over sodium sulfate, followed by distilling off the solvent, thereby obtaining the title compound as a crude isomeric mixture (72 g).
LCMS (ESI) m/z 324 [M+H]$^+$

Step 3: Synthesis of ((3aR,4R,6R,6aS)-6-(4-chloro-7H-pyrrolo[2,3-d]pyrimidin-7-yl)-2,2-dimethyltetrahydro-3aH-cyclopenta[d][1,3]dioxol-4-yl)methanol

The crude isomeric mixture (72 g) of ((3aR,4R,6R,6aS)-6-(4-chloro-7H-pyrrolo[2,3-d]pyrimidin-7-yl)-2,2-dimethyltetrahydro-3aH-cyclopenta[d][1,3]dioxol-4-yl)methanol and (4aR,6R,7S,7aR)-6-(4-chloro-7H-pyrrolo[2,3-d]pyrimidin-7-yl)-2,2-dimethylhexahydrocyclopenta[d][1,3]dioxin-7-ol, obtained in step 2 of Reference Example 93, was suspended in acetone (720 mL), and methanesulfonic acid (14.2 mL, 218 mmol) was added thereto in an ice bath. After the reaction solution was stirred in an ice bath for 40 minutes and at room temperature overnight, triethylamine (122 mL, 872 mmol) was added thereto dropwise in an ice bath so as to keep the internal temperature at 10°C or lower. After the mixture was stirred in an ice bath for 10 minutes and at room temperature for 30 minutes, the solvent was distilled off under reduced pressure. The residue was partitioned with the addition of ethyl acetate and a sodium bicarbonate solution, followed by extraction with ethyl acetate. The organic layer was washed with saturated saline, and dried over sodium sulfate. The solvent was distilled off, thereby giving a crude product of the title compound (77 g) as a brown oil.
$^1$H-NMR ($CDCl_3$) δ: 8.63 (1H, s), 7.33 (1H, d, J = 3.7 Hz), 6.63 (1H, d, J = 3.7 Hz), 5.03-4.95 (2H, m), 4.73-4.70 (1H, m), 3.90-3.86 (1H, m), 3.83-378 (1H, m), 2.52-2.43 (2H, m), 2.38-2.32 (1H, m), 2.18-2.16 (1H, m), 1.60 (3H, s), 1.32 (3H, s). LCMS (ESI) m/z 324 [M+H]$^+$

Step 4: Synthesis of 7-((3aS,4R,6R,6aR)-6-(((tertbutyldimethylsilyl)oxy)methyl)-2,2-dimethyltetrahydro-3aH-cyclopenta[d][1,3]dioxol-4-yl)-4-chloro-7H-pyrrolo[2,3-d]pyrimidine

The crude product (77 g), i.e., ((3aR,4R,6R,6aS)-6-(4-chloro-7H-pyrrolo[2,3-d]pyrimidin-7-yl)-2,2-dimethyltetrahydro-3aH-cyclopenta[d][1,3]dioxol-4-yl)methanol, obtained in step 3 of Reference Example 93 was dissolved in N,N-dimethylformamide (770 mL). Then, imidazole (37 g, 545 mmol) was added thereto at room temperature, and tert-butyldimethylchlorosilicane (58 g, 382 mmol) was added in a water bath (25°C), followed by stirring for 40 minutes in a water bath (29°C). The reaction solution was partitioned with the addition of ethyl acetate (800 mL) and water (800 mL) in an ice bath, and the organic layer was washed with water (400 mL). After the aqueous layer (about 300 mL) was removed, saturated saline (100 mL) was added to the organic layer and washed, followed by further washing with saturated saline (400 mL). After the resulting product was dried over sodium sulfate, the solvent was distilled off under reduced pressure, and the residue was purified by silica gel column chromatography (developing solvent: ethyl acetate/hexane), thereby obtaining the title compound (79 g, four steps: 83%) as a yellow oil.
$^1$H-NMR ($CDCl_3$) δ: 8.62 (1H, s), 7.31 (1H, d, J = 3.7 Hz), 6.63 (1H, d, J = 3.7 Hz), 5.09-5.04 (1H, m), 4.88 (1H, t, J = 6.3 Hz), 4.67-4.64 (1H, m), 3.82-3.74 (2H, m), 2.39-2.37 (3H, m), 1.59 (3H, s), 1.31 (3H, s), 0.93 (9H, s), 0.087 (3H, s), 0.074 (3H, s). LCMS (ESI) m/z 438 $[M+H]^+$

Step 5: Synthesis of 7-((3aS,4R,6R,6aR)-6-(((tertbutyldimethylsilyl)oxy)methyl)-2,2-dimethyltetrahydro-3aH-cyclopenta[d][1,3]dioxol-4-yl)-4-chloro-5-iodo-7H-pyrrolo [2,3-d]pyrimidine

7-((3aS,4R,6R,6aR)-6-(((tertButyldimethylsilyl)oxy)methyl)-2,2-dimethyltetrahydro-3aH-cyclopenta[d] [1,3]dioxol-4-yl)-4-chloro-7H-pyrrolo[2,3-d]pyrimidine (79 g, 180 mmol) was dissolved in N,N-dimethylformamide (790 mL), and N-iodosuccinimide (45 g, 198 mmol) was added thereto at room temperature, followed by stirring at 50°C for 11 hours and at room temperature for 12 hours. Ethyl acetate (600 mL), a saturated sodium hydrogen sulphite solution (300 mL), and water (600 mL) were added in an ice bath to the reaction solution, followed by stirring at room temperature for 10 minutes. After partition, the organic layer was sequentially washed with a liquid mixture of water (600 mL) and saturated saline (100 mL), and saturated saline (500 mL). After the resulting product was dried over sodium sulfate, the solvent was distilled off under reduced pressure, thereby giving a crude product of the title compound (95 g) as a brown oil.
$^1$H-NMR ($CDCl_3$) δ: 8.61 (1H, s), 7.46 (1H, s), 5.08-5.04 (1H, m), 4.85 (1H, t, J = 6.3 Hz), 4.64-4.62 (1H, m), 3.81-3.73 (2H, m), 2.42-2.32 (3H, m), 1.58 (3H, s), 1.30 (3H, s), 0.94 (9H, s), 0.095 (3H, s), 0.082 (3H, s). LCMS (ESI) m/z 564 $[M+H]^+$

Step 6: Synthesis of ((3aR,4R,6R,6aS)-6-(4-chloro-5-iodo-7H-pyrrolo[2,3-d]pyrimidin-7-yl)-2,2-dimethyltetrahydro-3aH-cyclopenta[d][1,3]dioxol-4-yl)methanol

The crude product (95 g), i.e., 7-((3aS,4R,6R,6aR)-6-(((tert-butyldimethylsilyl)oxy)methyl)-2,2-dimethyltetrahydro-3aH-cyclopenta[d] [1,3]dioxol-4-yl)-4-chloro-5-iodo-7H-pyrrolo[2,3-d]pyrimidine, obtained in step 3 of Reference Example 93 was dissolved in tetrahydrofuran (950 mL), and a tetrabutylammonium fluoride solution (1 M tetrahydrofuran solution, 180 mL) was added thereto at room temperature, followed by stirring at room temperature for 1 hour. The resulting mixture was partitioned in an ice bath with the addition of ethyl acetate (500 mL), water (500 mL), and a saturated sodium bicarbonate solution (300 mL), and the aqueous layer was extracted with ethyl acetate (500 mL). The organic layer was washed with saturated saline (500 mL), and dried over sodium sulfate, followed by distilling off the solvent under reduced pressure. The residue was purified by silica gel column chromatography (developing solvent: ethyl acetate/hexane), thereby obtaining the title compound (70 g, two steps: 86%) as a light-green amorphous substance.
$^1$H-NMR ($CDCl_3$) δ: 8.62 (1H, s), 7.48 (1H, s), 5.05-4.99 (1H, m), 4.91 (1H, t, J = 6.5 Hz), 4.71-4.68 (1H, m), 3.89-3.85 (1H, m), 3.82-3.78 (1H, m), 2.50-2.45 (2H, m), 2.31-2.27 (1H, m), 1.90-1.88 (1H, m), 1.59 (3H, s), 1.31 (3H, s). LCMS (ESI) m/z 450 $[M+H]^+$

Step 7: Synthesis of ((3aR,4R,6R,6aS)-6-(4-amino-5-iodo-7H-pyrrolo[2,3-d]pyrimidin-7-yl)-2,2-dimethyltetrahydro-3aH-cyclopenta[d][1,3]dioxol-4-yl)methanol

((3aR,4R,6R,6aS)-6-(4-Chloro-5-iodo-7H-pyrrolo[2,3-d]pyrimidin-7-yl)-2,2-dimethyltetrahydro-3aH-cyclopenta[d][1,3]dioxol-4-yl)methanol (70 g) was dissolved in 1,4-dioxane (350 mL), and 28% aqueous ammonia (350 mL) was added thereto at room temperature, followed by stirring at 100°C overnight in a pressure-resistant container. After the solvent was distilled off under reduced pressure, the resulting product was suspended in water (700 mL), followed by stirring at room temperature overnight. The precipitate was collected by filtration and washed with water (420 mL), followed by drying to give the title compound (67 g, 99%) as a light-brown powder.
$^1$H-NMR ($CDCl_3$) δ: 8.26 (1H, s), 7.12 (1H, s), 5.69-5.67 (2H, brs), 4.94 (1H, t, J = 6.2 Hz), 4.90-4.84 (1H, m), 4.72-4.70 (1H, m), 3.89-3.85 (1H, m), 3.81-3.77 (1H, m), 3.06-3.04 (1H, brs), 2.58-2.46 (2H, m), 2.35-2.27 (1H, m), 1.59 (3H, s), 1.33 (3H, s) LCMS (ESI) m/z 431 $[M+H]^+$.

Step 8: Synthesis of 7-((3aS,4R,6R,6aR)-6-(aminomethyl)-2,2-dimethyltetrahydro-3aH-cyclopenta[d][1,3]dioxol-4-yl)-5-iodo-7H-pyrrolo[2,3-d]pyrimidin-4-amine

((3aR,4R,6R,6aS)-6-(4-Amino-5-iodo-7H-pyrrolo[2,3-d]pyrimidin-7-yl)-2,2-dimethyltetrahydro-3aH-cyclopenta[d][1,3]dioxol-4-yl)methanol (51 g, 118 mmol) and phthalimide (35 g, 236 mmol) were dissolved in tetrahydrofuran (1000 mL). Then, triphenylphosphine (93 g, 354 mmol) was added thereto with stirring under ice-cooling. After triphenylphosphine was dissolved, diisopropyl azodicarboxylate (70 mL, 354 mmol) was added thereto dropwise with stirring under ice-cooling. After the reaction solution was stirred for 30 minutes under ice-cooling, the resulting product was stirred at room temperature for 90 minutes, and the reaction solution was distilled off under reduced pressure. Ethanol (750 mL), hydrazine monohydrate (25 mL, 519 mmol), and water (150 mL) were added to the residue, followed by stirring at 80°C overnight. After the solvent was distilled off under reduced pressure, the resulting product was partitioned with the addition of chloroform, water, and a saturated aqueous sodium hydrogen carbonate solution. The aqueous layer was then separated and extracted with chloroform. All of the organic layers were combined and dried over sodium sulfate, followed by distilling off the solvent. The residue was purified by basic silica gel column chromatography (developing solvent: methanol/chloroform), thereby obtaining the title compound (52 g, 89 wt%) as a yellow amorphous substance.
$^1$H-NMR ($CDCl_3$) $\delta$: 8.27 (1H, s), 7.13 (1H, s), 5.62-5.60 (2H, brs), 4.96-4.90 (2H, m), 4.56 (1H, t, J = 6.0 Hz), 2.90 (2H, d, J = 6.6 Hz), 2.46-2.40 (1H, m), 2.27-2.21 (1H, m), 2.17-2.08 (1H, m), 1.58 (3H, s), 1.32 (3H, s). LCMS (ESI) m/z 430 $[M+H]^+$.

Step 9: Synthesis of tert-butyl N-(((3aR,4R,6R,6aS)-6-(4-amino-5-iodo-7H-pyrrolo[2,3-d]pyrimidin-7-yl)-2,2-dimethyltetrahydro-3aH-cyclopenta[d][1,3]dioxol-4-yl)methyl)sulfamoyl carbamate

7-((3aS,4R,6R,6aR)-6-(Aminomethyl)-2,2-dimethyltetrahydro-3aH-cyclopenta[d][1,3]dioxol-4-yl)-5-iodo-7H-pyrrolo[2,3-d]pyrimidin-4-amine (51.5 g, 89 wt%, ca. 107 mmol) obtained in step 8 of Reference Example 93 was dissolved in acetonitrile (515 mL). Then, 1-aza-4-azoniabicyclo[2.2.2]octan-4-ylsulfonyl(tert-butoxycarbonyl)azanido: 1,4-diazabicyclo[2.2.2]octane monohydrochloride (Reference: Organic Letters, 2012, 10, 2626-2629) (70.5 g, 160 mmol) was added thereto at room temperature. After the reaction solution was stirred at room temperature for 30 minutes, water (1030 mL) and a saturated aqueous ammonium chloride solution (258 mL) were added to the reaction solution, followed by stirring at room temperature for 5 hours. The precipitate was collected by filtration and washed with water (1545 mL), followed by drying to give the title compound (57.1 g, 88%) as a yellowish white powder.
$^1$H-NMR ($CDCl_3$) $\delta$: 8.33 (1H, s), 7.05 (1H, s), 6.62-6.60 (1H, brs), 5.73-5.71 (2H, brs), 4.99 (1H, t, J = 5.9 Hz), 4.78-4.72 (1H, m), 4.64-4.62 (1H, m), 3.36-3.32 (1H, m), 3.24-3.25 (1H, m), 2.57-2.46 (2H, m), 2.38-2.32 (1H, m), 1.56 (3H, s), 1.48 (9H, s), 1.29 (3H, s). LCMS (ESI) m/z 609 $[M+H]^+$.

Step 10: Synthesis of Reference Example Compound 93

tert-Butyl N-(((3aR,4R,6R,6aS)-6-(4-amino-5-iodo-7H-pyrrolo[2,3-d]pyrimidin-7-yl)-2,2-dimethyltetrahydro-3aH-cyclopenta[d][1,3]dioxol-4-yl)methyl)sulfamoyl carbamate (20 mg, 0.033 mmol), 1-ethynylnaphthalene (10 mg, 0.066 mmol), bis(triphenylphosphine)palladium (II) dichloride (3 mg, 0.0043 mmol), copper iodide (1 mg, 0.0053 mmol), and diisopropylethylamine (0.011 mL, 0.066 mmol) were suspended in tetrahydrofuran (0.30 mL). After the reaction solution was stirred at 70°C for 1 hour, a mixed solution (0.60 mL) of trifluoroacetic acid/water = 4/1 was added thereto at room temperature, followed by stirring at room temperature overnight. After the solvent was distilled off, the residue was purified by silica gel column chromatography (developing solvent: methanol/chloroform), thereby obtaining the title compound (8.2 mg, 50%) as a yellow powder.
$^1$H-NMR ($CD_3OD$) $\delta$: 8.36 (1H, d, J = 8.5 Hz), 8.17 (1H, s), 7.93-7.89 (2H, m), 7.78-7.75 (1H, m), 7.74 (1H, s), 7.64-7.47 (3H, m), 5.02-4.95 (1H, m), 4.47-4.44 (1H, m), 4.05-4.03 (1H, m), 3.30-3.16 (2H, m), 2.52-2.44 (1H, m), 2.37-2.28 (1H, m), 1.90-1.82 (1H, m). LCMS (ESI) m/z 493 $[M+H]^+$.

Reference Example 94

4-Amino-7-[(1R,2S,3R,4R)-2,3-dihydroxy-4-[(sulfamoylamino)methyl]cyclopentyl]-5-[2-(2,6-dimethoxyphenyl)ethynyl]pyrrolo[2,3-d]pyrimidine

The title compound was obtained as in step 10 of Reference Example 93, except that 2-ethynyl-1,3-dimethoxybenzene was used in place of 1-ethynylnaphthalene.
$^1$H-NMR ($CD_3OD$) $\delta$: 8.12 (1H, s), 7.52 (1H, s), 7.27 (1H, t, J = 8.5 Hz), 6.68 (2H, d, J = 8.5 Hz), 4.95-4.86 (1H, m), 4.40-4.35 (1H, m), 4.05-3.95 (1H, m), 3.91 (6H, s), 3.30-3.13 (2H, m), 2.50-2.40 (1H, m), 2.34-2.28 (1H, m), 1.85-1.73 (1H, m). LCMS (ESI) m/z 503 $[M+H]^+$.

Reference Example 95

4-Amino-7-[(1R,2S,3R,4R)-2,3-dihydroxy-4-[(sulfamoylamino)methyl]cyclopentyl]-5-[2-(2-fluoro-6-methoxyphenyl)ethynyl]pyrrolo[2,3-d]pyrimidine

The title compound was obtained as in step 10 of Reference Example 93, except that 2-ethynyl-1-fluoro-3-methoxybenzene was used in place of 1-ethynylnaphthalene.
$^1$H-NMR ($CD_3OD$) δ: 8.13 (1H, s), 7.59 (1H, s), 7.34-7.28 (1H, m), 6.88 (1H, d, J = 8.5 Hz), 6.78-6.74 (1H, m), 4.97-4.86 (1H, m), 4.43-4.39 (1H, m), 4.03-4.00 (1H, m), 3.96 (3H, s), 3.30-3.23 (1H, m), 3.22-3.14 (1H, m), 2.50-2.40 (1H, m), 2.34-2.28 (1H, m), 1.85-1.73 (1H, m). LCMS (ESI) m/z 491 $[M+H]^+$.

Reference Example 96

8-[2-[4-Amino-7-[(1R,2S,3R,4R)-2,3-dihydroxy-4-[(sulfamoylamino)methyl]cyclopentyl]pyrrolo[2,3-d]pyrimidin-5-yl]ethynyl]quinoline

The title compound was obtained as in step 10 of Reference Example 93, except that 8-ethynylquinoline was used in place of 1-ethynylnaphthalene.
$^1$H-NMR ($CD_3OD$) δ: 9.04-9.02 (1H, m), 8.40-8.35 (1H, m), 8.16 (1H, s), 7.94-7.85 (2H, m), 7.63 (1H, s), 7.61-7.57 (2H, m), 5.00-4.91 (1H, m), 4.46-4.42 (1H, m), 4.05-4.00 (1H, m), 3.30-3.14 (2H, m), 2.50-2.38 (1H, m), 2.34-2.28 (1H, m), 1.87-1.81 (1H, m). LCMS (ESI) m/z 494 $[M+H]^+$.

Reference Example 97

4-Amino-7-[(1R,2S,3R,4R)-2,3-dihydroxy-4-[(sulfamoylamino)methyl]cyclopentyl]-5-[2-(2-ethoxy-6-fluorophenyl)ethynyl]pyrrolo[2,3-d]pyrimidine

Step 1: The title compound was obtained as in step 10 of Reference Example 93, except that 1-ethoxy-2-ethynyl-3-fluorobenzene was used in place of 1-ethynylnaphthalene.

$^1$H-NMR ($CD_3OD$) δ: 8.14 (1H, s), 7.60 (1H, s), 7.32-7.26 (1H, m), 6.86 (1H, d, J = 8.5 Hz), 6.79-6.74 (1H, m), 5.00-4.91 (1H, m), 4.45-4.42 (1H, m), 4.23 (2H, q, J = 7.1 Hz), 4.05-4.00 (1H, m), 3.30-3.24 (1H, m), 3.21-3.15 (1H, m), 2.50-2.40 (1H, m), 2.34-2.28 (1H, m), 1.87-1.81 (1H, m), 1.47 (3H, t, J = 7.1 Hz). LCMS (ESI) m/z 505 $[M+H]^+$.

Step 2: Synthesis of 4-amino-7-[(1R,2S,3R,4R)-2,3-dihydroxy-4-[(sulfamoylamino)methyl]cyclopentyl]-5-[2-(2-ethoxy-6-fluorophenyl)ethynyl]pyrrolo[2,3-d]pyrimidine hydrochloride

The title compound hydrochloride was obtained in accordance with step 5 of Reference Example 1.
$^1$H-NMR ($CD_3OD$) δ: 8.32 (1H, s), 7.95 (1H, s), 7.38-7.34 (1H, m), 6.93 (1H, d, J = 8.5 Hz), 6.84-6.80 (1H, m), 5.13-5.09 (1H, m), 4.41-4.37 (1H, m), 4.27 (2H, q, J = 7.0 Hz), 4.02-4.00 (1H, m), 3.28-3.23 (1H, m), 3.19-3.13 (1H, m), 2.47-2.42 (1H, m), 2.32-2.80 (1H, m), 1.87-1.79 (1H, m), 1.48 (3H, t, J = 7.0 Hz). LCMS (ESI) m/z 505 $[M+H]^+$

Reference Example 98

4-Amino-7-[(1R,2S,3R,4R)-2,3-dihydroxy-4-[(sulfamoylamino)methyl]cyclopentyl]-5-[2-(2-fluoro-6-methylsulfanyl-phenyl)ethynyl]pyrrolo[2,3-d]pyrimidine

The title compound was obtained as in step 10 of Reference Example 93, except that 2-ethynyl-1-fluoro-3-methylsulfanyl-benzene was used in place of 1-ethynylnaphthalene.
$^1$H-NMR ($CD_3OD$) δ: 8.15 (1H, s), 7.67 (1H, s), 7.35-7.30 (1H, m), 7.10 (1H, d, J = 7.8 Hz), 6.98-6.92 (1H, m), 5.00-4.91 (1H, m), 4.45-4.40 (1H, m), 4.05-4.00 (1H, m), 3.30-3.24 (1H, m), 3.21-3.15 (1H, m), 2.56 (3H, s), 2.50-2.41 (1H, m), 2.34-2.28 (1H, m), 1.87-1.78 (1H, m). LCMS (ESI) m/z 507 $[M+H]^+$.

Reference Example 99

4-Amino-7-[(1R,2S,3R,4R)-2,3-dihydroxy-4-[(sulfamoylamino)methyl]cyclopentyl]-5-[2-(2-ethylsulfanyl-6-fluoro-phenyl)ethynyl]pyrrolo[2,3-d]pyrimidine

The title compound was obtained as in step 10 of Reference Example 93, except that 1-ethylsulfanyl-2-ethynyl-3-fluoro-benzene was used in place of 1-ethynylnaphthalene.
$^1$H-NMR (DMSO-$D_6$) δ: 8.15 (1H, s), 7.93 (1H, s), 7.42-7.36 (1H, m), 7.23 (1H, d, J = 8.8 Hz), 7.13 (1H, t, J = 8.8 Hz), 6.65 (1H, dd, J = 6.6, 5.9 Hz), 6.53 (2H, brs), 4.90 (1H, ddd, J = 10.3, 9.9, 8.4 Hz), 4.71 (1H, brs), 4.24 (1H, dd, J = 8.8, 5.5 Hz), 3.79 (1H, dd, J = 5.5, 3.7 Hz), 3.10-3.05 (1H, m), 3.09 (2H, q, J = 7.3 Hz), 2.96-2.89 (1H, m), 2.22 (1H, dt, J = 13.2, 8.1 Hz), 2.15-2.06 (1H, m), 1.61-1.53 (1H, m), 1.30 (3H, t, J = 7.3 Hz). LCMS (ESI) m/z 521 $[M+H]^+$.

Reference Example 100

4-Amino-7-[(1R,2S,3R,4R)-2,3-dihydroxy-4-[(sulfamoylamino)methyl]cyclopentyl]-5-[2-(2-ethoxy-6-methoxyphenyl)ethynyl]pyrrolo[2,3-d]pyrimidine

The title compound was obtained as in step 10 of Reference Example 93, except that 1-ethoxy-2-ethynyl-3-methoxybenzene was used in place of 1-ethynylnaphthalene.
$^1$H-NMR (DMSO-$D_6$) δ: 8.12 (1H, s), 7.72 (1H, s), 7.27 (1H, t, J = 8.4 Hz), 6.72-6.65 (3H, m), 6.53 (2H, s), 4.92-4.85 (2H, m), 4.68 (1H, d, J = 4.4 Hz), 4.25-4.20 (1H, m), 4.16 (2H, q, J = 7.0 Hz), 3.87 (3H, s), 3.82-3.78 (1H, m), 3.12-3.06 (1H, m), 2.96-2.90 (1H, m), 2.23-2.18 (1H, m), 2.15-2.07 (1H, m), 1.61-1.53 (1H, m), 1.37 (3H, t, J = 7.0 Hz). LCMS (ESI) m/z 517.3 $[M+H]^+$.

Reference Example 101

4-[4-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-3,5-difluoro-phenyl]morpholine

The title compound was obtained as in step 10 of Reference Example 93, except that 4-(4-ethynyl-3,5-difluorophenyl)morpholine was used in place of 1-ethynylnaphthalene.
$^1$H-NMR ($CD_3OD$) δ: 8.14 (1H, s), 7.59 (1H, s), 6.63 (2H, d, J = 11.5 Hz), 4.97-4.93 (1H, m), 4.41 (1H, dd, J = 8.4, 5.7 Hz), 4.01 (1H, dd, J = 5.7, 3.8 Hz), 3.80 (4H, t, J = 4.9 Hz), 3.27-3.11 (6H, m), 2.48-2.40 (1H, m), 2.35-2.25 (1H, m), 1.84-1.76 (1H, m). LCMS (ESI) m/z 564 $[M+H]^+$.

Reference Example 102

4-Amino-5-[2-[2,6-difluoro-4-(1-piperidyl)phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 1-(4-ethynyl-3,5-difluorophenyl)piperidine

The title compound was obtained as in step 1 of Reference Example 23, except that piperidine was used in place of morpholine.
$^1$H-NMR ($CDCl_3$) δ: 6.34 (2H, d, J = 11.4 Hz), 3.37 (1H, s), 3.26-3.20 (4H, m), 1.68-1.60 (6H, m). LCMS (ESI) m/z 222 $[M+H]^+$ Step 2: Synthesis of Reference Example Compound 102

The title compound was obtained as in step 10 of Reference Example 93, except that 1-(4-ethynyl-3,5-difluorophenyl)piperidine was used in place of 1-ethynylnaphthalene.
$^1$H-NMR ($CD_3OD$) δ: 8.13 (1H, s), 7.57 (1H, s), 6.54 (2H, d, J = 11.7 Hz), 4.98-4.91 (1H, m), 4.40 (1H, dd, J = 8.4, 5.6 Hz), 4.01 (1H, dd, J = 5.6, 3.8 Hz), 3.31-3.30 (4H, m), 3.28-3.14 (2H, m), 2.48-2.39 (1H, m), 2.35-2.26 (1H, m), 1.85-1.75 (1H, m), 1.70-1.63 (6H, m). LCMS (ESI) m/z 562 $[M+H]^+$.

Reference Example 103

4-Amino-5-[2-[2,6-difluoro-4-(4-methylpiperazin-1-yl)phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 1-(4-ethynyl-3,5-difluorophenyl)-4-methylpiperazine

The title compound was obtained as in step 1 of Reference Example 23, except that 4-methylpiperazine was used in place of morpholine.
$^1$H-NMR ($CDCl_3$) δ: 6.36 (2H, d, J = 11.0 Hz), 3.39 (1H, s), 3.27-3.23 (4H, m), 2.54-2.50 (4H, m), 2.34 (3H, s). LCMS (ESI) m/z 237 $[M+H]^+$

Step 2: Synthesis of Reference Example Compound 103

The title compound was obtained as in step 10 of Reference Example 93, except that 1-(4-ethynyl-3,5-difluorophenyl)-4-methylpiperazine was used in place of 1-ethynylnaphthalene.
$^1$H-NMR ($CD_3OD$) δ: 8.23 (1H, s), 7.76 (1H, s), 6.76 (2H, d, J = 10.6 Hz), 5.06-4.97 (1H, m), 4.40 (1H, dd, J = 8.6, 5.6 Hz), 4.01 (1H, dd, J = 5.6, 3.7 Hz), 3.49-3.11 (10H, m), 2.97 (3H, s), 2.48-2.40 (1H, m), 2.34-2.26 (1H, m), 1.87-1.77 (1H, m). LCMS (ESI) m/z 577 $[M+H]^+$.

Reference Example 104

4-Amino-5-[2-[2,6-difluoro-4-(pyrazol-1-ylmethoxy)phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 4-((1H-pyrazol-1-yl)methoxy)-2,6-difluorobenzaldehyde

The title compound was obtained as in step 1 of Reference Example 55, except that 2,6-difluoro-4-hydroxybenzaldehyde and 1-(chloromethyl)pyrazole hydrochloride were used in place of 2-fluoro-6-hydroxybenzaldehyde and iodoethane.
$^1$H-NMR ($CDCl_3$) δ: 10.20 (1H, s), 7.65-7.62 (2H, m), 6.81 (2H, d, J = 10.0 Hz), 6.39 (1H, t, J = 2.1 Hz), 6.05 (2H, s). LCMS (ESI) m/z 239 $[M+H]^+$

Step 2: Synthesis of 1-((4-ethynyl-3,5-difluorophenoxy)methyl)-1H-pyrazole

The title compound was obtained as in step 1 of Reference Example 51, except that 4-((1H-pyrazol-1-yl)methoxy)-2,6-difluorobenzaldehyde was used in place of 2-fluoro-6-methoxybenzaldehyde.
$^1$H-NMR ($CDCl_3$) δ: 7.64-7.60 (2H, m), 6.73 (2H, d, J = 8.8 Hz), 6.37 (1H, t, J = 2.2 Hz), 5.99 (2H, s), 3.42 (1H, s). LCMS (ESI) m/z 235 $[M+H]^+$

Step 3: Synthesis of Reference Example Compound 104

The title compound was obtained as in step 10 of Reference Example 93, except that 1-((4-ethynyl-3,5-difluorophenoxy)methyl)-1H-pyrazole was used in place of 1-ethynylnaphthalene.
$^1$H-NMR ($DMSO-D_6$) δ: 8.14 (1H, s), 8.04 (1H, d, J = 2.0 Hz), 7.91 (1H, s), 7.61 (1H, d, J = 1.5 Hz), 7.15 (2H, d, J = 9.5 Hz), 6.64 (1H, t, J = 6.3 Hz), 6.51 (2H, s), 6.37-6.36 (1H, m), 6.18 (2H, s), 4.91-4.86 (2H, m), 4.69-4.66 (1H, brs), 4.23-4.19 (1H, m), 3.80-3.77 (1H, brs), 3.09-3.04 (1H, m), 2.95-2.88 (1H, m), 2.26-2.18 (1H, m), 2.14-2.10 (1H, m), 1.59-1.51 (1H, m). LCMS (ESI) m/z 575 $[M+H]^+$.

Reference Example 105

4-Amino-5-[2-(2,6-difluoro-4-pyrrolidin-1-yl-phenyl)ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 1-(4-ethynyl-3,5-difluorophenyl)pyrrolidine

The title compound was obtained as in step 1 of Reference Example 23, except that pyrrolidine was used in place of morpholine.

$^1$H-NMR ($CDCl_3$) δ: 6.02 (2H, d, J = 10.6 Hz), 3.38 (1H, s), 3.28-3.22 (4H, m), 2.04-2.00 (4H, m). LCMS (ESI) m/z 208 $[M+H]^+$ Step 2: Synthesis of Reference Example Compound 105

The title compound was obtained as in step 10 of Reference Example 93, except that 1-(4-ethynyl-3,5-difluorophenyl)pyrrolidine was used in place of 1-ethynylnaphthalene.
$^1$H-NMR ($CD_3OD$) δ: 8.13 (1H, s), 7.54 (1H, s), 6.21 (2H, d, J = 11.0 Hz), 4.40 (1H, dd, J = 8.6, 5.7 Hz), 4.00 (1H, t, J = 4.6 Hz), 3.67-3.62 (1H, m), 3.29-3.05 (6H, m), 2.48-2.39 (1H, m), 2.35-2.25 (1H, m), 2.08-2.02 (4H, m), 1.85-1.75 (1H, m). LCMS (ESI) m/z 548 $[M+H]^+$.

Reference Example 106

4-Amino-7-[(1R,2S,3R,4R)-2,3-dihydroxy-4-[(sulfamoylamino)methyl]cyclopentyl]-5-[2-[2-ethoxy-6-fluoro-4-(1-piperidyl)phenyl]ethynyl]pyrrolo[2,3-d]pyrimidine

Step 1: Synthesis of 1-(3-ethoxy-4-ethynyl-5-fluorophenyl)piperidine

The title compound was obtained as in step 1 of Reference Example 34, except that 1-(4-ethynyl-3,5-difluorophenyl)piperidine was used in place of 4-(4-ethynyl-3,5-difluorophenyl)morpholine.
$^1$H-NMR ($CDCl_3$) δ: 6.24 (1H, dd, J = 12.8, 2.2 Hz), 6.18-6.16 (1H, m), 4.12 (2H, q, J = 7.0 Hz), 3.42 (1H, s), 3.27-3.22 (4H, m), 1.73-1.60 (6H, m), 1.48 (3H, t, J = 7.0 Hz). LCMS (ESI) m/z 248 $[M+H]^+$

Step 2: Synthesis of Reference Example Compound 106

The title compound was obtained as in step 10 of Reference Example 93, except that 1-(3-ethoxy-4-ethynyl-5-fluorophenyl)piperidine was used in place of 1-ethynylnaphthalene.
$^1$H-NMR ($CD_3OD$) δ: 8.13 (1H, s), 7.50 (1H, s), 6.33-6.30 (2H, m), 4.97-4.90 (1H, m), 4.39 (1H, dd, J = 8.4, 5.5 Hz), 4.17 (2H, q, J = 7.1 Hz), 4.00 (1H, dd, J = 5.7, 3.8 Hz), 3.29-3.13 (6H, m), 2.47-2.40 (1H, m), 2.34-2.26 (1H, m), 1.84-1.75 (1H, m), 1.71-1.61 (6H, m), 1.45 (3H, t, J = 7.1 Hz). LCMS (ESI) m/z 588 $[M+H]^+$.

Reference Example 107

4-Amino-5-[2-(4-benzyloxy-2,6-difluoro-phenyl)ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 4-(benzyloxy)-2,6-difluorobenzaldehyde

The title compound was obtained as in step 1 of Reference Example 55, except that 2,6-difluoro-4-hydroxybenzaldehyde and benzylbromide were used in place of 2-fluoro-6-hydroxybenzaldehyde and iodoethane.
$^1$H-NMR ($CDCl_3$) δ: 10.20 (1H, s), 7.49-7.36 (5H, m), 6.57 (2H, d, J = 10.5 Hz), 5.11 (2H, s). LCMS (ESI) m/z 249 $[M+H]^+$

Step 2: Synthesis of 5-(benzyloxy)-2-ethynyl-1,3-difluorobenzene

The title compound was obtained as in step 1 of Reference Example 51, except that 4-(benzyloxy)-2,6-difluorobenzaldehyde was used in place of 2-fluoro-6-methoxybenzaldehyde.
$^1$H-NMR ($CDCl_3$) δ: 7.41-7.36 (5H, m), 6.54 (2H, d, J = 9.0 Hz), 5.04 (2H, s), 3.42 (1H, s). LCMS (ESI) m/z 245 $[M+H]^+$

Step 3: Synthesis of Reference Example Compound 107

The title compound was obtained as in step 10 of Reference Example 93, except that 5-(benzyloxy)-2-ethynyl-1,3-difluorobenzene was used in place of 1-ethynylnaphthalene.
$^1$H-NMR ($CD_3OD$) δ: 8.15 (1H, s), 7.63 (1H, s), 7.45-7.34 (5H, m), 6.78 (2H, d, J = 9.5 Hz), 5.13 (2H, s), 4.92-4.88 (1H, m), 4.43-4.40 (1H, m), 4.02-3.98 (1H, m), 3.30-3.13 (2H, m), 2.46-2.40 (1H, m), 2.31-2.28 (1H, m), 1.83-1.78 (1H, m). LCMS (ESI) m/z 585 $[M+H]^+$.

Reference Example 108

3-[4-[2-[4-Amino-7-[(1R,2S,3R,4R)-2,3-dihydroxy-4-[(sulfamoylamino)methyl]cyclopentyl]pyrrolo[2,3-d]pyrimidin-5-yl]ethynyl]-3-ethoxy-5-fluoro-phenyl]-8-oxa-3-azabicyclo[3,2,1]octane

Step 1: Synthesis of 3-(4-ethynyl-3,5-difluorophenyl)-8-oxa-3-azabicyclo[3.2.1]octane

The title compound was obtained as in step 1 of Reference Example 23, except that 8-oxa-3-azabicyclo[3.2.1]octane was used in place of morpholine.
$^1$H-NMR ($CDCl_3$) δ: 6.28 (2H, dd, J = 13.9, 2.9 Hz), 4.52-4.49 (2H, m), 3.40 (1H, s), 3.26 (2H, d, J = 11.3 Hz), 3.08 (2H, dd, J = 11.3, 2.7 Hz), 2.04-1.96 (2H, m), 1.89-1.82 (2H, m). LCMS (ESI) m/z 250 $[M+H]^+$

Step 2: Synthesis of 3-(3-ethoxy-4-ethynyl-5-fluorophenyl)-8-oxa-3-azabicyclo[3.2.1]octane

The title compound was obtained as in step 1 of Reference Example 34, except that 3-(4-ethynyl-3,5-difluorophenyl)-8-oxa-3-azabicyclo[3.2.1]octane was used in place of 4-(4-ethynyl-3,5-difluorophenyl)morpholine.
$^1$H-NMR ($CDCl_3$) δ: 6.12 (1H, dd, J = 12.6, 2.4 Hz), 6.03 (1H, s), 4.51-4.47 (2H, m), 4.09 (2H, q, J = 7.0 Hz), 3.40 (1H, s), 3.28 (2H, d, J = 11.3 Hz), 3.06 (2H, dd, J = 11.3, 2.7 Hz), 2.03-1.92 (2H, m), 1.92-1.84 (2H, m), 1.46 (3H, t, J = 7.0 Hz). LCMS (ESI) m/z 276 $[M+H]^+$

Step 3: Synthesis of Reference Example Compound 108

The title compound was obtained as in step 10 of Reference Example 93, except that 3-(3-ethoxy-4-ethynyl-5-fluorophenyl)-8-oxa-3-azabicyclo[3.2.1]octane was used in place of 1-ethynylnaphthalene.
$^1$H-NMR ($CD_3OD$) δ: 8.12 (1H, s), 7.49 (1H, s), 6.29 (1H, d, J = 1.8 Hz), 6.25 (1H, s), 4.94-4.90 (1H, m), 4.47 (2H, s), 4.39 (1H, dd, J = 8.4, 5.9 Hz), 4.18 (2H, q, J = 7.1 Hz), 4.00 (1H, dd, J = 5.7, 3.8 Hz), 3.45 (2H, d, J = 11.0 Hz), 3.26 (1H, dd, J = 12.8, 6.2 Hz), 3.17 (1H, dd, J = 12.6, 6.8 Hz), 2.97 (2H, dd, J = 11.5, 2.4 Hz), 2.48-2.39 (1H, m), 2.35-2.25 (1H, m), 2.00-1.87 (4H, m), 1.84-1.74 (1H, m), 1.45 (3H, t, J = 7.0 Hz). LCMS (ESI) m/z 616 $[M+H]^+$.

Reference Example 109

4-Amino-5-[2-(2,3-dihydrobenzothiophen-7-yl)ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 2,3-dihydrobenzo[b]thiophene-7-carbaldehyde

7-Bromo-2,3-dihydrobenzo[b]thiophene (200 mg, 0.93 mmol) was dissolved in tetrahydrofuran (2 mL), and an n-butyllithium solution (1.6 M hexane solution, 1.4 mL, 2.2 mmol) was added thereto at -78°C, followed by stirring at -78°C for 30 minutes. Thereafter, N,N-dimethylformamide (0.22 mL, 2.8 mmol) was added thereto at -78 °C, followed by stirring at -78°C for 20 minutes and at room temperature for 1 hour. A saturated aqueous ammonium chloride solution, water, and ethyl acetate were added to the reaction solution, and the aqueous layer was extracted with ethyl acetate. The organic layer was washed with a saturated sodium chloride aqueous solution, and dried over sodium sulfate, followed by distilling off the solvent. The residue was purified by silica gel column chromatography (developing solvent: ethyl acetate/hexane), thereby obtaining the title compound (74 mg, 49%) as a yellow oil.
$^1$H-NMR ($CDCl_3$) δ: 10.05 (1H, s), 7.62 (1H, d, J = 7.5 Hz), 7.39 (1H, dd, J = 7.5, 1.0 Hz), 7.18 (1H, t, J = 7.5 Hz), 3.42-3.37 (2H, m), 3.31 (2H, t, J = 7.6 Hz). LCMS (ESI) m/z 165 $[M+H]^+$

Step 2: Synthesis of 7-ethynyl-2,3-dihydrobenzo[b]thiophene

The title compound was obtained as in step 1 of Reference Example 51, except that 2,3-dihydrobenzo[b]thiophene-7-carbaldehyde was used in place of 2-fluoro-6-methoxybenzaldehyde.
$^1$H-NMR ($CDCl_3$) δ: 7.25 (1H, d, J = 7.6 Hz), 7.15 (1H, dd, J = 7.6, 1.0 Hz), 6.96 (1H, t, J = 7.6 Hz), 3.40-3.31 (5H, m).

Step 3: Synthesis of Reference Example Compound 109

The title compound was obtained as in step 10 of Reference Example 93, except that 7-ethynyl-2,3-dihydrobenzo[b]thiophene was used in place of 1-ethynylnaphthalene.
$^1$H-NMR ($CD_3OD$) δ: 8.15 (1H, s), 7.60 (1H, s), 7.23 (1H, d, J = 7.6 Hz), 7.20-7.17 (1H, m), 7.02 (1H, dd, J = 7.6, 7.6

Hz), 4.97-4.88 (1H, m), 4.43-4.40 (1H, m), 4.02-4.00 (1H, m), 3.44-3.34 (4H, m), 3.29-3.23 (1H, m), 3.20-3.15 (1H, m), 2.48-2.41 (1H, m), 2.32-2.28 (1H, m), 1.86-1.78 (1H, m). LCMS (ESI) m/z 501 [M+H].

Reference Example 110

8-[4-[2-[4-Amino-7-[(1R,2S,3R,4R)-2,3-dihydroxy-4-[(sulfamoylamino)methyl]cyclopentyl]pyrrolo[2,3-d]pyrimidin-5-yl]ethynyl]-3-ethoxy-5-fluoro-phenyl]-3-oxa-8-azabicyclo[3,2,1]octane

Step 1: Synthesis of 8-(4-ethynyl-3,5-difluorophenyl)-3-oxa-8-azabicyclo[3.2.1]octane

The title compound was obtained as in step 1 of Reference Example 23, except that 3-oxa-8-azabicyclo[3.2.1]octane was used in place of morpholine.
$^1$H-NMR ($CDCl_3$) δ: 6.23 (2H, dd, J = 13.6, 3.3 Hz), 4.01-3.98 (2H, m), 3.81 (2H, d, J = 11.2 Hz), 3.53 (2H, d, J = 11.2 Hz), 3.39 (1H, s), 2.16-2.01 (4H, m). LCMS (ESI) m/z 250 $[M+H]^+$

Step 2: Synthesis of 8-(3-ethoxy-4-ethynyl-5-fluorophenyl)-3-oxa-8-azabicyclo[3.2.1]octane

The title compound was obtained as in step 1 of Reference Example 34, except that 8-(4-ethynyl-3,5-difluorophenyl)-3-oxa-8-azabicyclo[3.2.1]octane was used in place of 4-(4-ethynyl-3,5-difluorophenyl)morpholine.
$^1$H-NMR ($CDCl_3$) δ: 6.08 (1H, dd, J = 12.1, 2.2 Hz), 6.00 (1H, s), 4.08 (2H, q, J = 7.0 Hz), 4.01 (2H, d, J = 2.6 Hz), 3.85 (2H, d, J = 11.0 Hz), 3.52 (2H, d, J = 11.0 Hz), 3.39 (1H, d, J = 5.9 Hz), 2.13-2.00 (4H, m), 1.45 (3H, t, J = 7.0 Hz). LCMS (ESI) m/z 276 $[M+H]^+$

Step 3: Synthesis of Reference Example Compound 110

The title compound was obtained as in step 10 of Reference Example 93, except that 8-(3-ethoxy-4-ethynyl-5-fluorophenyl)-3-oxa-8-azabicyclo[3.2.1]octane was used in place of 1-ethynylnaphthalene.
$^1$H-NMR ($CD_3OD$) δ: 8.13 (1H, s), 7.50 (1H, s), 6.29 (1H, d, J = 2.2 Hz), 6.25 (1H, d, J = 2.2 Hz), 4.97-4.92 (1H, m), 4.39 (1H, dd, J = 8.4, 5.8 Hz), 4.21-4.14 (4H, m), 4.01 (1H, dd, J = 5.8, 3.8 Hz), 3.83 (2H, d, J = 10.8 Hz), 3.52 (2H, d, J = 10.8 Hz), 3.30-3.12 (2H, m), 2.49-2.39 (1H, m), 2.35-2.26 (1H, m), 2.10-1.99 (4H, m), 1.85-1.75 (1H, m), 1.45 (3H, t, J = 7.0 Hz). LCMS (ESI) m/z 616 $[M+H]^+$.

Reference Example 111

4-Amino-7-[(1R,2S,3R,4R)-2,3-dihydroxy-4-[(sulfamoylamino)methyl]cyclopentyl]-5-[2-[2-methylsulfanyl-4-(1-piperidyl)phenyl]ethynyl]pyrrolo[2,3-d]pyrimidine

Step 1: Synthesis of 4-fluoro-2-(methylthio)benzaldehyde

Sodium thiomethoxide (680 mg, 9.6 mmol) was suspended in toluene (10 mL), and 2,4-difluorobenzaldehyde (1 g, 7.0 mmol) was added thereto, followed by stirring at 80°C for 2 days. Ethyl acetate and water were added to the reaction solution. Then, the organic layer was washed with a saturated aqueous sodium hydrogen carbonate solution and saturated saline, and dried over sodium sulfate, followed by distilling off the solvent. The residue was purified by silica gel column chromatography (developing solvent: ethyl acetate/hexane), thereby obtaining the title compound (499 mg, 42%) as a white solid.
$^1$H-NMR ($CDCl_3$) δ: 10.15 (1H, s), 7.82 (1H, dd, J = 8.3, 6.1 Hz), 7.01 (1H, dd, J = 10.1, 2.3 Hz), 6.95 (1H, dt, J = 2.3, 8.3 Hz), 2.49 (3H, s). LCMS (ESI) m/z 171 $[M+H]^+$

Step 2: Synthesis of 2-(methylthio)-4-(piperidin-1-yl)benzaldehyde

4-Fluoro-2-(methylthio)benzaldehyde (200 mg, 1.2 mmol) and potassium carbonate (220 mg, 1.6 mmol) were suspended in dimethylsulfoxide (2 mL), and piperidine (0.16 ml, 1.6 mmol) was added thereto at room temperature, followed by stirring at 80°C for 2 hours and 30 minutes. Ethyl acetate and water were added at room temperature to the reaction solution, and the aqueous layer was extracted with ethyl acetate. The organic layer was washed with a saturated sodium chloride aqueous solution, and dried over sodium sulfate, followed by distilling off the solvent. The residue was purified by silica gel column chromatography (developing solvent: ethyl acetate/hexane), thereby obtaining the title compound (345 mg) as a yellow oil.
$^1$H-NMR ($CDCl_3$) δ: 9.99 (1H, s), 7.64 (1H, d, J = 8.7 Hz), 6.68 (1H, dd, J = 8.7, 2.3 Hz), 6.64 (1H, d, J = 2.3 Hz), 3.43-3.40

(4H, m), 2.47 (3H, s), 1.80-1.70 (6H, m). LCMS (ESI) m/z 236 $[M+H]^+$

Step 3: Synthesis of 1-(4-ethynyl-3-(methylthio)phenyl)piperidine

The title compound was obtained as in step 1 of Reference Example 51, except that 2-(methylthio)-4-(piperidin-1-yl)benzaldehyde was used in place of 2-fluoro-6-methoxybenzaldehyde.
$^1H$-NMR ($CDCl_3$) $\delta$: 7.32 (1H, d, J = 8.6 Hz), 6.68 (1H, d, J = 2.5 Hz), 6.63 (1H, dd, J = 8.6, 2.5 Hz), 3.35 (1H, s), 3.24-3.21 (4H, m), 2.49 (3H, s), 1.72-1.58 (6H, m). LCMS (ESI) m/z 232 $[M+H]^+$

Step 4: Synthesis of Reference Example Compound 111

The title compound was obtained as in step 10 of Reference Example 93, except that 1-(4-ethynyl-3-(methylthio)phenyl)piperidine was used in place of 1-ethynylnaphthalene.
$^1H$-NMR ($CD_3OD$) $\delta$: 8.13 (1H, s), 7.53 (1H, s), 7.29 (1H, d, J = 8.8 Hz), 6.81 (1H, d, J = 2.3 Hz), 6.75 (1H, dd, J = 8.8, 2.3 Hz), 4.96-4.87 (1H, m), 4.42-4.38 (1H, m), 4.02-4.00 (1H, m), 3.29-3.15 (6H, m), 2.52 (3H, s), 2.48-2.40 (1H, m), 2.28-2.32 (1H, m), 1.85-1.77 (1H, m), 1.72-1.61 (6H, m). LCMS (ESI) m/z 572 [M+H].

Reference Example 112

4-[4-[2-[4-Amino-7-[(1R,2S,3R,4R)-2,3-dihydroxy-4-[(sulfamoylamino)methyl]cyclopentyl]pyrrolo[2,3-d]pyrimidin-5-yl]ethynyl]-3-ethoxy-5-fluoro-phenyl]morpholine

The title compound was obtained as in step 10 of Reference Example 93, except that 4-(3-ethoxy-4-ethynyl-5-fluorophenyl)morpholine was used in place of 1-ethynylnaphthalene.
$^1H$-NMR (DMSO-$D_6$) $\delta$: 8.12 (1H, s), 7.73 (1H, s), 6.65 (1H, t, J = 6.0 Hz), 6.56-6.39 (4H, m), 4.91-4.84 (2H, m), 4.69 (1H, d, J = 4.4 Hz), 4.23-4.16 (2H, m), 3.79 (1H, dd, J = 8.4, 4.4 Hz), 3.74-3.68 (4H, m), 3.26-3.21 (4H, m), 3.12-3.04 (1H, m), 2.98-2.88 (1H, m), 2.27-2.17 (1H, m), 2.15-2.07 (2H, m), 1.60-1.51 (1H, m), 1.36 (3H, t, J = 7.0 Hz). LCMS (ESI) m/z 590 $[M+H]^+$.

Reference Example 113

4-Amino-7-[(1R,2S,3R,4R)-2,3-dihydroxy-4-[(sulfamoylamino)methyl]cyclopentyl]-5-[2-(4-methoxy-2-methylsulfonyl-phenyl)ethynyl]pyrrolo[2,3-d]pyrimidine

Step 1: Synthesis of triisopropyl((4-methoxy-2-(methylsulfonyl)phenyl)ethynyl)silane

The title compound was obtained as in step 2 of Reference Example 76, except that 1-bromo-2-methanesulfonyl-4-methoxybenzene was used in place of 5-(benzyloxy)-2-bromopyrimidine.
$^1H$-NMR ($CDCl_3$) $\delta$: 7.61-7.59 (2H, m), 7.06 (1H, dd, J = 8.5, 2.7 Hz), 3.88 (3H, s), 3.33 (3H, s), 1.19-1.12 (21H, m). LCMS (ESI) m/z 367 $[M+H]^+$

Step 2: Synthesis of 1-ethynyl-4-methoxy-2-(methylsulfonyl)benzene

The title compound was obtained as in step 3 of Reference Example 76, except that triisopropyl((4-methoxy-2-(methylsulfonyl)phenyl)ethynyl)silane was used in place of 5-(benzyloxy)-2-((triisopropylsilyl)ethynyl)pyrimidine.
$^1H$-NMR ($CDCl_3$) $\delta$: 7.64-7.62 (2H, m), 7.09 (1H, dd, J = 8.5, 2.7 Hz), 3.90 (3H, s), 3.53 (1H, s), 3.31 (3H, s). LCMS (ESI) m/z 211 $[M+H]^+$

Step 3: Synthesis of Reference Example Compound 113

The title compound was obtained as in step 10 of Reference Example 93, except that 1-ethynyl-4-methoxy-2-(methylsulfonyl)benzene was used in place of 1-ethynylnaphthalene.
$^1H$-NMR ($CD_3OD$) $\delta$: 8.16 (1H, s), 7.72 (1H, s), 7.70 (1H, d, J = 8.6 Hz), 7.57 (1H, d, J = 2.8 Hz), 7.26 (1H, dd, J = 8.6, 2.8 Hz), 4.99-4.91 (1H, m), 4.44-4.40 (1H, m), 4.03-4.01 (1H, m), 3.91 (3H, s), 3.30 (3H, s), 3.39-3.24 (1H, m), 3.21-3.16 (1H, m), 2.50-2.42 (1H, m), 2.35-2.28 (1H, m), 1.87-1.79 (1H, m). LCMS (ESI) m/z 551 [M+H].

Reference Example 114

4-Amino-7-[(1R,2S,3R,4R)-2,3-dihydroxy-4-[(sulfamoylamino)methyl]cyclopentyl]-5-[2-(2-ethylsulfonyl-6-fluoro-4-pyrrolidin-1-yl-phenyl)ethynyl]pyrrolo[2,3-d]pyrimidine

The title compound was obtained as in step 10 of Reference Example 93, except that 1-(3-(ethylsulfonyl)-4-ethynyl-5-fluorophenyl)pyrrolidine was used in place of 1-ethynylnaphthalene.
$^1$H-NMR ($CD_3OD$) δ: 8.13 (1H, s), 7.62 (1H, s), 6.99 (1H, d, J = 2.6 Hz), 6.64 (1H, dd, J = 12.4, 2.6 Hz), 4.98-4.90 (1H, m), 4.43-4.39 (1H, m), 4.02-4.00 (1H, m), 3.49 (2H, q, J = 7.4 Hz), 3.39-3.34 (4H, m), 3.28-3.24 (1H, m), 3.20-3.15 (1H, m), 2.48-2.40 (1H, m), 2.34-2.28 (1H, m), 2.09-2.06 (4H, m), 1.85-1.77 (1H, m), 1.24 (3H, t, J = 7.4 Hz). LCMS (ESI) m/z 622 [M+H].

Reference Example 115

4-Amino-5-[2-[2,6-difluoro-4-[(3R)-3-fluoropyrrolidin-1-yl]phenyl]ethynyl]-7-[(1R,2S,3R,4R)-2,3-dihydroxy-4-[(sulfamoylamino)methyl]cyclopentyl]pyrrolo[2,3-d]pyrimidine

The title compound was obtained as in step 10 of Reference Example 93, except that (3R)-1-(4-ethynyl-3,5-difluorophenyl)-3-fluoropyrrolidine was used in place of 1-ethynylnaphthalene.
$^1$H-NMR ($CD_3OD$) δ: 8.12 (1H, s), 7.54 (1H, s), 6.25 (2H, d, J = 10.6 Hz), 5.38 (1H, d, J = 53.2 Hz), 5.04-4.95 (1H, m), 4.40 (1H, dd, J = 8.4, 5.9 Hz), 4.01 (1H, dd, J = 5.5, 3.7 Hz), 3.64-3.10 (6H, m), 2.49-2.38 (1H, m), 2.37-2.26 (2H, m), 2.22-2.12 (1H, m), 1.84-1.75 (1H, m). LCMS (ESI) m/z 566 $[M+H]^+$.

Reference Example 116

4-Amino-5-[2-[2,6-difluoro-4-[(3S)-3-fluoropyrrolidin-1-yl]phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of (3S)-1-(4-ethynyl-3,5-difluorophenyl)-3-fluoropyrrolidine

The title compound was obtained as in step 1 of Reference Example 23, except that (S)-3-fluoropyrrolidine was used in place of morpholine.
$^1$H-NMR ($CDCl_3$) δ: 6.06 (2H, d, J = 10.3 Hz), 5.38 (1H, d, J = 53.5 Hz), 3.62-3.39 (5H, m), 2.47-2.36 (1H, m), 2.28-2.07 (1H, m). LCMS (ESI) m/z 226 $[M+H]^+$

Step 2: Synthesis of Reference Example Compound 116

The title compound was obtained as in step 10 of Reference Example 93, except that (3S)-1-(4-ethynyl-3,5-difluorophenyl)-3-fluoropyrrolidine was used in place of 1-ethynylnaphthalene.
$^1$H-NMR ($CD_3OD$) δ: 8.11 (1H, s), 7.53 (1H, s), 6.22 (2H, d, J = 10.6 Hz), 5.38 (1H, d, J = 53.2 Hz), 4.93-4.90 (1H, m), 4.42-4.37 (1H, m), 4.01 (1H, dd, J = 5.5, 4.0 Hz), 3.62-3.15 (6H, m), 2.49-2.40 (1H, m), 2.34-2.27 (2H, m), 2.22-2.08 (1H, m), 1.84-1.76 (1H, m). LCMS (ESI) m/z 566 $[M+H]^+$.

Reference Example 117

4-Amino-7-[(1R,2S,3R,4R)-2,3-dihydroxy-4-[(sulfamoylamino)methyl]cyclopentyl]-5-[2-(1,1-dioxo-2,3-dihydrobenzothiophen-7-yl)ethynyl]pyrrolo[2,3-d]pyrimidine

Step 1: Synthesis of 7-ethynyl-2,3-dihydrobenzo[b]thiophene 1,1-dioxide

The title compound was obtained as in step 3 of Reference Example 70, except that 7-ethynyl-2,3-dihydrobenzo[b]thiophene was used in place of 2-(ethylthio)-6-fluoro-4-(pyrrolidin-1-yl)benzaldehyde.
$^1$H-NMR ($CDCl_3$) δ: 7.57-7.51 (2H, m), 7.36 (1H, d, J = 7.1 Hz), 3.57-3.52 (3H, m), 3.35 (2H, t, J = 7.2 Hz). LCMS (ESI) m/z 193 $[M+H]^+$

Step 2: Synthesis of Reference Example Compound 117

The title compound was obtained as in step 10 of Reference Example 93, except that 7-ethynyl-2,3-dihydrobenzo[b]thiophene 1,1-dioxide was used in place of 1-ethynylnaphthalene.
$^1$H-NMR ($CD_3OD$) δ: 8.15 (1H, s), 7.74 (1H, s), 7.64 (1H, t, J = 7.6 Hz), 7.58 (1H, d, J = 7.6 Hz), 7.45 (1H, d, J = 7.6 Hz), 5.00-4.89 (1H, m), 4.44-4.40 (1H, m), 4.03-4.00 (1H, m), 3.62 (2H, t, J = 7.1 Hz), 3.41 (2H, t, J = 7.1 Hz), 3.22-3.11 (2H, m), 2.48-2.42 (1H, m), 2.32-2.26 (1H, m), 1.87-1.77 (1H, m). LCMS (ESI) m/z 533 [M+H].

Reference Example 118

4-Amino-5-[2-[4-(azetidin-1-yl)-2,6-difluoro-phenyl]ethynyl]-7-[(1R,2S,3R,4R)-2,3-dihydroxy-4-[(sulfamoylamino)methyl]cyclopentyl]pyrrolo[2,3-d]pyrimidine

The title compound was obtained as in step 10 of Reference Example 93, except that 1-(4-ethynyl-3,5-difluorophenyl)azetidine was used in place of 1-ethynylnaphthalene.
$^1$H-NMR ($CD_3OD$) δ: 8.14 (1H, s), 7.56 (1H, s), 6.03 (2H, d, J = 10.0 Hz), 4.99-4.89 (1H, m), 4.40 (1H, dd, J = 8.5, 5.6 Hz), 4.01 (1H, dd, J = 5.6, 3.7 Hz), 3.94 (4H, t, J = 7.4 Hz), 3.28-3.09 (2H, m), 2.46-2.36 (3H, m), 2.34-2.24 (1H, m), 1.85-1.72 (1H, m). LCMS (ESI) m/z 534 $[M+H]^+$.

Reference Example 119

4-Amino-5-[2-[2,6-difluoro-4-(4-hydroxy-1-piperidyl)phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of 1-(4-ethynyl-3,5-difluorophenyl)piperidin-4-ol

The title compound was obtained as in step 1 of Reference Example 23, except that piperidin-4-ol was used in place of morpholine.
$^1$H-NMR ($CDCl_3$) δ: 6.36 (2H, d, J = 11.2 Hz), 3.93-3.87 (1H, m), 3.58 (2H, dt, J = 13.0, 4.9 Hz), 3.40 (1H, s), 3.06-3.00 (2H, m), 2.21-2.10 (1H, m), 1.99-1.91 (2H, m), 1.65-1.55 (2H, m). LCMS (ESI) m/z 238 $[M+H]^+$

Step 2: Synthesis of Reference Example Compound 119

The title compound was obtained as in step 10 of Reference Example 93, except that 1-(4-ethynyl-3,5-difluorophenyl)piperidin-4-ol was used in place of 1-ethynylnaphthalene.
$^1$H-NMR ($CD_3OD$) δ: 8.19 (1H, s), 7.57 (1H, s), 6.57 (2H, d, J = 11.5 Hz), 4.98-4.89 (1H, m), 4.40 (1H, t, J = 6.3 Hz), 4.01 (1H, dd, J = 5.5, 3.8 Hz), 3.85-3.78 (1H, m), 3.70-3.65 (2H, m), 3.30-3.14 (2H, m), 3.08-3.00 (2H, m), 2.49-2.40 (1H, m), 2.36-2.25 (1H, m), 1.97-1.88 (2H, m), 1.85-1.75 (1H, m), 1.60-1.50 (2H, m). LCMS (ESI) m/z 578 $[M+H]^+$.

Reference Example 120

4-Amino-5-[2-[2,6-difluoro-4-[(3R)-3-hydroxypyrrolidin-1-yl]phenyl]ethynyl]-7-[(1R,2S,3R,4R)-2,3-dihydroxy-4-[(sulfamoylamino)methyl]cyclopentyl]pyrrolo[2,3-d]pyrimidine

The title compound was obtained as in step 10 of Reference Example 93, except that (3R)-1-(4-ethynyl-3,5-difluorophenyl)-pyrrolidin-3-ol was used in place of 1-ethynylnaphthalene.
$^1$H-NMR ($CD_3OD$) δ: 8.22 (1H, s), 7.55 (1H, s), 6.20 (2H, d, J = 11.0 Hz), 4.98-4.90 (1H, m), 4.55-4.51 (1H, m), 4.43-4.36 (1H, m), 4.01 (1H, dd, J = 5.5, 3.8 Hz), 3.51-3.42 (2H, m), 3.40-3.33 (1H, m), 3.27-3.14 (3H, m), 2.48-2.40 (1H, m), 2.34-2.26 (1H, m), 2.20-2.09 (1H, m), 2.08-2.00 (1H, m), 1.84-1.75 (1H, m). LCMS (ESI) m/z 564 $[M+H]^+$.

Reference Example 121

4-Amino-5-[2-[2,6-difluoro-4-[(3S)-3-hydroxypyrrolidin-1-yl]phenyl]ethynyl]-7-[(1R,2S,3R,4R)-2,3-dihydroxy-4-[(sulfamoylamino)methyl]cyclopentyl]pyrrolo[2,3-d]pyrimidine

Step 1: Synthesis of (3S)-1-(4-ethynyl-3,5-difluorophenyl)-pyrrolidin-3-ol

The title compound was obtained as in step 1 of Reference Example 23, except that (S)-pyrrolidin-3-ol was used in place of morpholine.
$^1$H-NMR ($CDCl_3$) δ: 6.03 (2H, d, J = 10.5 Hz), 4.64-4.60 (1H, m), 3.51-3.43 (2H, m), 3.39 (1H, s), 3.34 (1H, dt, J = 3.2, 9.0 Hz), 3.22 (1H, d, J = 10.7 Hz), 2.22-2.12 (1H, m), 2.12-2.05 (1H, m). LCMS (ESI) m/z 224 $[M+H]^+$

Step 2: Synthesis of Reference Example Compound 121

The title compound was obtained as in step 10 of Reference Example 93, except that (3S)-1-(4-ethynyl-3,5-difluorophenyl)-pyrrolidin-3-ol was used in place of 1-ethynylnaphthalene.
$^1$H-NMR ($CD_3OD$) δ: 8.23 (1H, s), 7.55 (1H, s), 6.20 (2H, d, J = 10.7 Hz), 4.97-4.89 (1H, m), 4.54-4.49 (1H, m), 4.46-4.35 (1H, m), 4.03-3.96 (1H, m), 3.51-3.41 (2H, m), 3.40-3.33 (1H, m), 3.27-3.12 (3H, m), 2.48-2.39 (1H, m), 2.35-2.25 (1H, m), 2.19-2.09 (1H, m), 2.08-2.00 (1H, m), 1.84-1.74 (1H, m). LCMS (ESI) m/z 564 $[M+H]^+$.

Reference Example 122

8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine

Step 1: The title compound was obtained as in step 10 of Reference Example 93, except that 8-ethynyl-7-fluoro-4-methyl-2,3-dihydro-1,4-benzoxazine was used in place of 1-ethynylnaphthalene.

$^1$H-NMR (DMSO-$D_6$) δ: 8.17-8.12 (2H, m), 7.84 (1H, s), 6.77-6.64 (3H, m), 6.53 (2H, s), 4.88 (1H, dd, J = 18.7, 8.4 Hz), 4.43 (2H, t, J = 4.2 Hz), 4.21 (1H, dd, J = 8.6, 5.3 Hz), 3.80 (1H, dd, J = 5.1, 3.3 Hz), 3.25 (2H, t, J = 4.2 Hz), 3.12-3.04 (1H, m), 2.96-2.89 (1H, m), 2.82 (3H, s), 2.24-2.19 (1H, m), 2.15-2.08 (1H, m), 1.56 (1H, dd, J = 20.9, 11.0 Hz). LCMS (ESI) m/z 532.3 $[M+H]^+$.

Step 2: Synthesis of 8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine hydrochloride

The title compound hydrochloride was obtained as in step 5 of Reference Example 1.
$^1$H-NMR (DMSO-$D_6$) δ: 8.45 (1H, s), 8.24 (1H, s), 6.81-6.74 (2H, m), 4.98 (1H, dd, J = 19.1, 8.8 Hz), 4.47 (2H, t, J = 4.2 Hz), 4.20 (1H, dd, J = 9.0, 5.3 Hz), 3.80 (1H, dd, J = 5.1, 2.9 Hz), 3.27 (2H, t, J = 4.4 Hz), 3.08 (1H, dd, J = 12.8, 6.6 Hz), 2.93 (1H, dd, J = 12.5, 7.3 Hz), 2.84 (3H, s), 2.29-2.22 (1H, m), 2.17-2.10 (1H, m), 1.58 (1H, dd, J = 20.7, 10.8 Hz). LCMS (ESI) m/z 532.4 $[M+H]^+$

Reference Example 123

4-Amino-7-[(1R,2S,3R,4R)-2,3-dihydroxy-4-[(sulfamoylamino)methyl]cyclopentyl]-5-[2-(1,1-dioxo-3,4-dihydro-2H-thiochromen-8-yl)ethynyl]pyrrolo[2,3-d]pyrimidine

The title compound was obtained as in step 10 of Reference Example 93, except that 8-ethynyl thiochroman 1,1-dioxide was used in place of 1-ethynylnaphthalene.
$^1$H-NMR ($CD_3OD$) δ: 8.16 (1H, s), 7.73 (1H, s), 7.57 (1H, d, J = 7.7 Hz), 7.50 (1H, t, J = 7.7 Hz), 7.31 (1H, d, J = 7.7 Hz), 5.03-4.89 (1H, m), 4.44-4.40 (1H, m), 4.04-4.02 (1H, m), 3.56-3.53 (2H, m), 3.30-3.25 (1H, m), 3.22-3.17 (1H, m), 3.09 (2H, t, J = 6.0 Hz), 2.50-2.39 (3H, m), 2.36-2.27 (1H, m), 1.87-1.79 (1H, m). LCMS (ESI) m/z 547 [M+H].

Reference Example 124

4-Amino-5-[2-(2,6-difluorophenyl)ethynyl]-7-[(2R,4S,5R)-4-hydroxy-5-[(sulfamoylamino)methyl]tetrahydrofuran-2-yl]pyrrolo[2,3-d]pyrimidine

Step 1: Synthesis of (2R,3S,5R)-5-(4-amino-5-iodo-7H-pyrrolo[2,3-d]pyrimidin-7-yl)-2-(aminoethyl)tetrahydrofuran-3-ol

The title compound was obtained as in step 2 of Reference Example 1, except that (2R,3S,5R)-5-(4-amino-5-iodo-7H-pyrrolo[2,3-d]pyrimidin-7-yl)-2-(hydroxymethyl)tetrahydrofuran-3-ol was used in place of [(3aR,4R,6R,6aR)-4-(4-amino-5-iodo-pyrrolo[2,3-d]pyrimidin-7-yl)-2,2-dimethyl-3a,4,6,6a-tetrahydrofuro[3,4-d][1,3]dioxol-6-yl]methanol.
$^1$H-NMR ($CD_3OD$) δ: 8.09 (1H, s), 7.48 (1H, s), 6.52 (1H, t, J = 7.0 Hz), 4.37 (1H, dt, J = 6.6, 4.0 Hz), 3.89 (1H, dt, J = 7.0, 4.0 Hz), 2.91 (1H, dd, J = 13.2, 4.0 Hz), 2.83 (1H, dd, J = 13.2, 7.0 Hz), 2.66-2.59 (1H, m), 2.33 (1H, ddd, J = 13.9, 7.0, 3.7 Hz). LCMS (ESI) m/z 376 $[M+H]^+$

Step 2: Synthesis of tert-butyl-N-[[(2R,3S,5R)-5-(4-amino-5-iodo-pyrrolo[2,3-d]pyrimidin-7-yl)-3-hydroxy-tetrahydrofuran-2-yl]methyl sulfamoyl]carbamate

The title compound was obtained as in step 3 of Reference Example 1, except that (2R,3S,5R)-5-(4-amino-5-iodo-7H-pyrrolo[2,3-d]pyrimidin-7-yl)-2-(aminoethyl)tetrahydrofuran-3-ol was used in place of 7-((3aR,4R,6R,6aR)-6-(aminomethyl)-2,2-dimethyltetrahydrofuro[3,4-d][1,3]dioxol-4-yl)-5-iodo-7H-pyrrolo[2,3-d]pyrimidin-4-amine.
$^1$H-NMR (DMSO-$D_6$) δ: 10.84 (1H, s), 8.09 (1H, s), 7.61 (1H, s), 6.76-6.61 (2H, brm), 6.35 (1H, dd, J = 8.8, 5.9 Hz), 5.33 (1H, d, J = 4.0 Hz), 4.28-4.26 (1H, brm), 3.90-3.87 (1H, m), 3.16-3.10 (2H, m), 2.59-2.52 (1H, m), 2.11-2.06 (1H, m), 1.36 (9H, s). LCMS (ESI) m/z 555 $[M+H]^+$

Step 3: Synthesis of Reference Example Compound 124

tert-Butyl-N-[[(2R,3S,5R)-5-(4-amino-5-iodo-pyrrolo[2,3-d]pyrimidin-7-yl)-3-hydroxy-tetrahydrofuran-2-yl]methyl sulfamoyl]carbamate (40 mg, 0.072 mmol), 2-ethynyl-1,3-difluorobenzene (15 mg, 0.11 mmol), bis(triphenylphosphine)palladium (II) dichloride (10 mg, 0.014 mmol), copper iodide (2.7 mg, 0.014 mmol), and diisopropylethylamine (0.030 mL, 0.18 mmol) were suspended in tetrahydrofuran (0.5 mL). The reaction solution was stirred at 70°C overnight. Thereafter, trifluoroacetic acid (0.5 mL) was added thereto at room temperature, followed by stirring at room temperature overnight. After the solvent was distilled off, the residue was purified by silica gel column chromatography (developing solvent: methanol/chloroform), thereby obtaining the title compound (1.2 mg, 4%) as a white powder.
$^1$H-NMR (DMSO-$D_6$) δ: 8.18 (1H, s), 8.00 (1H, s), 7.53-7.47 (1H, m), 7.29-7.24 (1H, m), 7.18-7.15 (1H, m), 6.58 (2H, s), 6.43 (1H, dd, J = 8.8, 5.6 Hz), 5.35 (1H, d, J = 4.1 Hz), 4.39-4.35 (1H, brm), 3.98-3.94 (1H, brm), 3.18-3.03 (2H, brm), 2.66-2.59 (1H, m). LCMS (ESI) m/z 465 $[M+H]^+$.

Reference Example 125

4-Amino-7-[(2R,4S,5R)-4-hydroxy-5-[(sulfamoylamino)methyl]tetrahydrofuran-2-yl]-5-[2-(1-naphthyl)ethynyl]pyrrolo[2,3-d]pyrimidine

The title compound was obtained as in step 3 of Reference Example 124, except that 1-ethynylnaphthalene was used in place of 2-ethynyl-1,3-difluorobenzene.
$^1$H-NMR ($CD_3OD$) δ: 8.39 (1H, d, J = 8.4 Hz), 8.35 (1H, s), 8.07 (1H, s), 7.96 (1H, brs), 7.94 (1H, brs), 7.85 (1H, d, J = 7.3 Hz), 7.67-7.46 (3H, m), 6.59 (1H, dd, J = 7.7, 6.2 Hz), 4.62-4.59 (1H, m), 4.18-4.15 (1H, m), 3.41-3.34 (2H, m), 2.77 (1H, ddd, J = 13.9, 7.7, 6.2 Hz), 2.45 (1H, ddd, J = 13.9, 6.2, 2.9 Hz). LCMS (ESI) m/z 479 $[M+H]^+$.

Reference Example 126

4-Amino-7-[(2R,4S,5R)-4-hydroxy-5-[(sulfamoylamino)methyl]tetrahydrofuran-2-yl]pyrrolo[2,3-d]pyrimidin-5-yl]-2-(o-tolyl)thiazole

The title compound was obtained as in Reference Example 2, except that tert-butyl-N-[[(2R,3S,5R)-5-(4-amino-5-iodo-pyrrolo[2,3-d]pyrimidin-7-yl)-3-hydroxy-tetrahydrofuran-2-yl]methyl sulfamoyl]carbamate was used in place of tert-butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1,3]dioxol-4-yl)methyl)sulfamoyl carbamate.
$^1$H-NMR ($CD_3OD$) δ: 8.37 (1H, s), 8.26 (1H, s), 8.15 (1H, s), 8.04-8.01 (1H, m), 7.66 (1H, d, J = 7.7 Hz), 7.49-7.35 (3H, m), 6.68 (1H, dd, J = 7.7, 6.2 Hz), 4.61-4.58 (1H, m), 4.16 (1H, dt, J = 5.9, 3.3 Hz), 3.44 (1H, dd, J = 13.6, 4.0 Hz), 3.39 (1H, dd, J = 13.6, 4.8 Hz), 2.72-2.65 (1H, m), 2.55 (3H, s), 2.47 (1H, ddd, J = 13.6, 6.2, 3.3 Hz). LCMS (ESI) m/z 502 $[M+H]^+$.

Reference Example 127

4-Amino-5-[2-(2-ethoxy-6-fluoro-phenyl)ethynyl]-7-[(2R,4S,5R)-4-hydroxy-5-[(sulfamoylamino)methyl]tetrahydrofuran-2-yl]pyrrolo[2,3-d]pyrimidine

The title compound was obtained as in step 3 of Reference Example 124, except that 1-ethoxy-2-ethynyl-3-fluorobenzene was used in place of 2-ethynyl-1,3-difluorobenzene.
$^1$H-NMR (DMSO-$D_6$) δ: 8.17 (1H, s), 7.88 (1H, s), 7.37 (1H, ddd, J = 8.8, 8.4, 7.0 Hz), 7.19 (1H, dd, J = 7.0, 5.5 Hz), 6.97 (1H, d, J = 8.4 Hz), 6.91 (1H, t, J = 8.8 Hz), 6.57 (2H, s), 6.42 (1H, dd, J = 8.4, 5.5 Hz), 5.37 (1H, d, J = 4.0 Hz), 4.37 (1H, brs), 4.22 (2H, q, J = 7.0 Hz), 3.99-3.93 (1H, m), 3.22-3.04 (2H, m), 2.67-2.61 (1H, m), 2.17 (1H, dd, J = 13.6, 5.5 Hz), 1.37 (3H, t, J = 7.0 Hz). LCMS (ESI) m/z 491 $[M+H]^+$.

Reference Example 128

4-Amino-5-[2-(2,6-difluorophenyl)ethynyl]-7-[(1R,4R,5S)-4,5-dihydroxy-3-[(sulfamoylamino)methyl]cyclopent-2-en-1-yl]pyrrolo[2,3-d]pyrimidine

Step 1: Synthesis of ((3aS,4R,6aR)-6-(((tert-butyldiphenylsilyl)oxy)methyl)-2,2-dimethyl-4,6a-dihydro-3aH-cyclopenta[d][1,3]dioxol-4-ol

(3aR,6aR)-6-(((tert-Butyldiphenylsilyl)oxy)methyl)-2,2-dimethyl-3aH-cyclopenta[d][1,3]dioxol-4(6aH)-one (1.0 g, 2.36 mmol) and cerium chloride heptahydrate (881 mg, 2.36 mmol) were suspended in methanol (5 mL), and sodium borohydride (92%, 146 mg, 3.54 mmol) was added thereto with stirring at 0°C. After the mixture was stirred at 0°C for 2 hours, water (20 mL) was added thereto. Acetic acid was then added thereto until the pH of the reaction liquid became about 5. After the reaction liquid was partitioned between ethyl acetate and water, the organic layer was separated. The obtained organic layer was sequentially washed with a saturated aqueous ammonium chloride solution and saturated saline, and dried over magnesium sulfate, followed by distilling off the solvent. The residue was purified by silica gel column chromatography (developing solvent: ethyl acetate/hexane), thereby obtaining the title compound (960 mg) as a colorless oil.
$^1$H-NMR ($CDCl_3$) $\delta$: 7.72-7.66 (4H, m), 7.45-7.37 (6H, m), 5.85 (1H, s), 4.88 (1H, d, J = 5.5 Hz), 4.76 (1H, dd, J = 6.2, 5.9 Hz), 4.58-4.55 (1H, brm), 4.39 (1H, d, J = 14.7 Hz), 4.29 (1H, d, J = 14.7 Hz), 1.37 (3H, s), 1.34 (3H, s), 1.08 (9H, s). LCMS (ESI) m/z 425 $[M+H]^+$

Step 2: Synthesis of 7-((3aS,4R,6aR)-6-(((tert-butyldiphenylsilyl)oxy)methyl)-2,2-dimethyl-4,6a-dihydro-3aH-cyclopenta[d][1,3]dioxol-4-yl)-4-chloro-5-iodo-7H-pyrrolo[2,3-d]pyrimidine

(3aS,4R,6aR)-6-(((tert-Butyldiphenylsilyl)oxy)methyl)-2,2-dimethyl-4,6a-dihydro-3aH-cyclopenta[d][1,3]dioxol-4-ol (960 mg, 2.26 mmol), 4-chloro-5-iodo-7H-pyrrolo[2,3-d]pyrimidine (632 mg, 2.26 mmol), and triphenylphosphine (889 mg, 3.39 mmol) were dissolved in tetrahydrofuran (7 mL). Then, diisopropyl azodicarboxylate (667 μL, 3.39 mmol) was added thereto dropwise with stirring at 0°C. After the reaction liquid was stirred at room temperature for 14 hours, the reaction liquid was concentrated, and the residue was purified by silica gel column chromatography (developing solvent: ethyl acetate/hexane), thereby obtaining the title compound (1.33 g) as a colorless amorphous substance.
$^1$H-NMR ($CDCl_3$) $\delta$: 8.69 (1H, s), 7.72-7.69 (4H, m), 7.49-7.39 (6H, m), 7.15 (1H, s), 5.88-5.86 (1H, brm), 5.84-5.82 (1H, brm), 5.21 (1H, d, J = 5.5 Hz), 4.56 (1H, d, J = 5.5 Hz), 4.49 (2H, d, J = 14.7 Hz), 1.44 (3H, s), 1.31 (3H, s), 1.11 (9H, s). LCMS (ESI) m/z 687 $[M+H]^+$

Step 3: Synthesis of ((3aR,6R,6aS)-6-(4-amino-5-iodo-7H-pyrrolo[2,3-d]pyrimidin-7-yl)-2,2-dimethyl-6,6a-dihydro-3aH-cyclopenta[d][1,3]dioxol-4-yl)methanol

7-((3aS,4R,6aR)-6-(((tert-Butyldiphenylsilyl)oxy)methyl)-2,2-dimethyl-4,6a-dihydro-3aH-cyclopenta[d][1,3]dioxol-4-yl)-4-chloro-5-iodo-7H-pyrrolo[2,3-d]pyrimidine (16.2 g, 23.6 mmol) was suspended in 1,4-dioxane (50 mL) and aqueous ammonia (28%, 50 mL), followed by heating with stirring at 100°C for 24 hours in a pressure-resistant container. After the solvent was distilled off, tetrahydrofuran (50 mL) was added to the residue, and tetrabutylammonium fluoride (a 1 M tetrahydrofuran solution, 47 mL) was added thereto with stirring at room temperature, followed by stirring at room temperature overnight. After the reaction solution was partitioned between ethyl acetate and water, the aqueous layer was separated, and extracted with ethyl acetate. The organic layers were combined and washed with saturated saline, followed by distilling off the solvent. The residue was purified by silica gel column chromatography (developing solvent: methanol/chloroform), thereby obtaining the title compound (7.4 g) as a milky-white solid.
$^1$H-NMR (DMSO-$D_6$) $\delta$: 8.12 (1H, s), 7.18 (1H, s), 6.63 (2H, brs), 5.63-5.61 (1H, brm), 5.59-5.59 (1H, brm), 5.29 (1H, d, J = 5.5 Hz), 5.06 (1H, dd, J = 5.7, 5.5 Hz), 4.49 (1H, d, J = 5.9 Hz), 4.13 (2H, d, J = 5.9 Hz), 1.36 (3H, s), 1.25 (3H, s). LCMS (ESI) m/z 429 $[M+H]^+$

Step 4: Synthesis of tert-butyl(((3aR,6R,6aS)-6-(4-amino-5-iodo-7H-pyrrolo[2,3-d]pyrimidin-7-yl)-2,2-dimethyl-6,6a-dihydro-3aH-cyclopenta[d][1,3]dioxol-4-yl)methyl)(sulfamoyl)carbamate

((3aR, 6R,6aS)-6-(4-Amino-5-iodo-7H-pyrrolo[2,3-d]pyrimidin-7-yl)-2,2-dimethyl-6,6a-dihydro-3aH-cyclopenta[d][1,3]dioxol-4-yl)methanol (100 mg, 0.233 mmol), tert-butyl sulfamoylcarbamate (60 mg, 0.30 mmol), and triphenylphosphine (92 mg, 0.35 mmol) were dissolved in tetrahydrofuran (1 mL). Then, diisopropyl azodicarboxylate (69 μL, 0.35 mmol) was added thereto dropwise with stirring at 0°C. After the reaction liquid was stirred for 3 hours under ice-cooling, methanol (1 mL) was added thereto, followed by stirring at room temperature for 10 minutes. The reaction liquid

was concentrated, and the residue was purified by silica gel column chromatography (developing solvent: methanol/chloroform), thereby obtaining the title compound (60 mg) as a colorless amorphous substance.
$^1$H-NMR ($CDCl_3$) δ: 8.22 (1H, s), 6.91 (1H, s), 6.36-6.07 (4H, brm), 5.81 (1H, brs), 5.69 (1H, brs), 5.32 (1H, d, J = 5.9 Hz), 4.66-4.52 (3H, m), 1.55 (9H, s), 1.48 (3H, s), 1.34 (3H, s). LCMS (ESI) m/z 607 $[M+H]^+$

Step 5: Synthesis of Reference Example Compound 128

tert-Butyl (((3aR,6R,6aS)-6-(4-amino-5-iodo-7H-pyrrolo[2,3-d]pyrimidin-7-yl)-2,2-dimethyl-6,6a-dihydro-3aH-cyclopenta[d][1,3]dioxol-4-yl)methyl)(sulfamoyl)carbamate (60 mg, 0.099 mmol), 2-ethynyl-1,3-difluorobenzene (27.3 mg, 0.198 mmol), bis(triphenylphosphine)palladium (II) dichloride (3.5 mg, 0.005 mmol), copper iodide (1 mg, 0.0053 mmol), and diisopropylethylamine (0.034 mL, 0.19 mmol) were suspended in tetrahydrofuran (0.7 mL). After the reaction solution was stirred at 50°C for 1 hour, the reaction liquid was concentrated, and the residue was roughly purified by silica gel column chromatography (developing solvent: methanol/chloroform). The obtained residue was dissolved at room temperature in acetonitrile (0.5 mL) and concentrated hydrochloric acid (0.2 mL), followed by stirring at room temperature overnight. After the reaction liquid was concentrated, the residue was purified by basic silica gel column chromatography (developing solvent: methanol/chloroform), thereby obtaining the title compound (5.7 mg, 12%) as a white solid.
$^1$H-NMR ($CD_3OD$) δ: 8.16 (1H, s), 7.56 (1H, s), 7.45-7.38 (1H, m), 7.11-7.05 (2H, m), 5.94 (1H, brs), 5.63 (1H, d, J = 5.5 Hz), 4.68 (1H, d, J = 5.5 Hz), 4.27 (1H, t, J = 5.5 Hz), 3.95 (1H, d, J = 16.5 Hz), 3.87 (1H, d, J = 16.5 Hz). LCMS (ESI) m/z 477 $[M+H]^+$.

Reference Example 129

4-Amino-7-[(1R,4R,5S)-4,5-dihydroxy-3-[(sulfamoylamino)methyl]cyclopent-2-en-1-yl]-5-[2-(2-ethoxy-6-fluoro-phenyl)ethynyl]pyrrolo[2,3-d]pyrimidine

The title compound was obtained as in step 5 of Reference Example 128, except that 1-ethoxy-2-ethynyl-3-fluorobenzene was used in place of 2-ethynyl-1,3-difluorobenzene.
$^1$H-NMR ($CD_3OD$) δ: 8.14 (1H, s), 7.45 (1H, s), 7.30 (1H, ddd, J = 8.8, 8.4, 6.6 Hz), 6.88 (1H, d, J = 8.4 Hz), 6.78 (1H, t, J = 8.8 Hz), 5.94 (1H, brs), 5.61 (1H, d, J = 4.8 Hz), 4.68 (1H, d, J = 5.5 Hz), 4.27 (1H, dd, J = 5.5, 4.8 Hz), 4.24 (2H, q, J = 7.0 Hz), 3.94 (1H, d, J = 16.1 Hz), 3.91 (1H, d, J = 16.1 Hz), 1.48 (3H, t, J = 7.0 Hz). LCMS (ESI) m/z 503 $[M+H]^+$.

Reference Example 130

4-Amino-7-[(1R,4R,5S)-4,5-dihydroxy-3-[(sulfamoylamino)methyl]cyclopent-2-en-l-yl]-5-[2-(2-fluoro-6-methylsulfanyl-phenyl)ethynyl]pyrrolo[2,3-d]pyrimidine

The title compound was obtained as in step 5 of Reference Example 128, except that 2-ethynyl-1-fluoro-3-methanesulfanyl-benzene was used in place of 2-ethynyl-1,3-difluorobenzene.
$^1$H-NMR (DMSO-$D_6$) δ: 8.17 (1H, s), 7.62 (1H, s), 7.41 (1H, ddd, J = 8.8, 8.8, 6.2 Hz), 7.15 (1H, d, J = 8.8 Hz), 7.11 (1H, t, J = 8.8 Hz), 6.80 (1H, t, J = 5.7 Hz), 6.64 (2H, s), 5.74 (1H, s), 5.56 (1H, brs), 5.10 (1H, d, J = 6.2 Hz), 5.01 (1H, d, J = 6.2 Hz), 4.48 (1H, t, J = 6.2 Hz), 4.11-4.06 (1H, m), 3.73-3.59 (2H, m), 2.55 (3H, s). LCMS (ESI) m/z 505 $[M+H]^+$.

Reference Example 131

[(2R,3S,4R,5R)-5-[4-amino-5-(2-phenylethynyl)pyrrolo[2,3-d]pyrimidin-7-yl]-3,4-dihydroxy-tetrahydrofuran-2-yl]methyl sulfamate

Step 1: Synthesis of ((3aR,4R,6R,6aR)-6-(4-amino-5-(phenylethynyl)-7H-pyrrolo[2,3-d]pyrimidin-7-yl)-2,2-dimethyltetrahydrofuro[3,4-d][1,3]dioxol-4-yl)methanol

[(3aR,4R,6R,6aR)-4-(4-Amino-5-iodo-pyrrolo[2,3-d]pyrimidin-7-yl)-2,2-dimethyl-3a,4,6,6a-tetrahydrofuro[3,4-d][1,3]dioxol-6-yl]methanol (1 g, 2.3 mmol), phenylacetylene (354 mg, 3.5 mmol), bis(triphenylphosphine)palladium (II) dichloride (161 mg, 0.23 mmol), and copper iodide (44 mg, 0.23 mmol) were suspended in tetrahydrofuran (10 mL), Then, nitrogen purging was performed, and after diisopropylethylamine (0.78 mL, 4.6 mmol) was added thereto, the reaction solution was stirred at 70°C for 2 hours. The reaction solution was filtered through a celite bed and washed with chloroform, followed by distilling off the solvent. The residue was purified by silica gel column chromatography (developing solvent: ethyl acetate/hexane), thereby obtaining the title compound (800 mg, 85%) as a yellow amorphous substance.

$^1$H-NMR ($CDCl_3$) δ: 8.27 (1H, s), 7.51-7.49 (2H, m), 7.39-7.36 (3H, m), 7.27-7.25 (1H, m), 6.52 (1H, d, J = 10.7 Hz), 5.85-5.72 (2H, brs), 5.72 (1H, d, J = 5.1 Hz), 5.24 (1H, t, J = 5.5 Hz), 5.12-5.09 (1H, m), 4.52-4.50 (1H, brs), 4.00-3.96 (1H, m), 3.83-3.76 (1H, m), 1.64 (3H, s), 1.37 (3H, s). LCMS (ESI) m/z 407 [M+H]$^+$

Step 2: Synthesis of ((3aR,4R,6R,6aR)-6-(4-amino-5-(phenylethynyl)-7H-pyrrolo[2,3-d]pyrimidin-7-yl)-2,2-dimethyltetrahydrofuro[3,4-d] [1,3]dioxol-4-yl)methyl sulfamate

((3aR,4R,6R,6aR)-6-(4-Amino-5-(phenylethynyl)-7H-pyrrolo[2,3-d]pyrimidin-7-yl)-2,2-dimethyltetrahydrofuro[3,4-d][1,3]dioxol-4-yl)methanol (50 mg, 0.12 mmol) was dissolved in acetonitrile (0.5 mL). Then, triethylamine (0.084 mL, 0.59 mmol) was added thereto at room temperature, and sulfamoyl chloride (0.5 M acetonitrile solution, 0.27 mL) was added thereto in an ice bath. After the resulting mixture was stirred for 40 minutes in an ice bath, the solvent was distilled off. Then, chloroform and an aqueous sodium hydrogen carbonate solution were added thereto, and the aqueous layer was extracted with a liquid mixture of chloroform/methanol = 5/1. The organic layer was washed with saturated saline and dried over sodium sulfate, followed by distilling off the solvent. The residue was purified by silica gel column chromatography (developing solvent: ethyl acetate/hexane), thereby obtaining the title compound (39 mg, 67%) as a yellow amorphous substance.
$^1$H-NMR ($CDCl_3$) δ: 8.29 (1H, s), 7.52-7.49 (2H, m), 7.37-7.36 (3H, m), 7.30 (1H, s), 6.08 (1H, d, J = 2.7 Hz), 5.72-5.68 (2H, brs), 5.35 (1H, dd, J = 6.3, 2.9 Hz), 5.13-5.11 (1H, m), 4.50-4.43 (3H, m), 1.62 (3H, s), 1.39 (3H, s). LCMS (ESI) m/z 486 [M+H]$^+$ Step 3: Synthesis of Reference Example Compound 131

((3aR,4R,6R,6aR)-6-(4-Amino-5-(phenylethynyl)-7H-pyrrolo[2,3-d]pyrimidin-7-yl)-2,2-dimethyltetrahydrofuro[3,4-d][1,3]dioxol-4-yl)methyl sulfamate (380 mg, 0.79 mmol) was dissolved in tetrahydrofuran (4 mL), and a mixed solution (9.5 mL) of trifluoroacetic acid/water = 4/1 was added thereto, followed by stirring at room temperature for 8 hours. After the solvent was distilled off, methanol was added, and the solvent was distilled off again. The residue was purified by basic silica gel column chromatography (developing solvent: methanol/chloroform), thereby obtaining the title compound (323 mg, 92%) as a white powder.
$^1$H-NMR ($CD_3OD$) δ: 8.15 (1H, s), 7.67 (1H, s), 7.55-7.52 (2H, m), 7.39-7.37 (3H, m), 6.22 (1H, d, J = 5.6 Hz), 4.90-4.80 (1H, m), 4.47-4.26 (4H, m). LCMS (ESI) m/z 446 [M+H]$^+$.

Reference Example 132

[(2R,3S,4R,5R)-5-[4-Amino-5-[2-(2,6-difluorophenyl)ethynyl]pyrrolo[2,3-d]pyrimidin-7-yl]-3,4-dihydroxy-tetrahydrofuran-2-yl]methyl sulfamate

The title compound was obtained as in Reference Example 131, except that 2-ethynyl-1,3-difluorobenzene was used in place of phenylacetylene.
$^1$H-NMR (DMSO-$D_6$) δ: 8.20 (1H, s), 7.97 (1H, s), 7.55-7.50 (1H, m), 7.31-7.25 (2H, m), 6.45-6.37 (2H, brs), 6.13 (1H, d, J = 5.9 Hz), 4.47-4.43 (1H, m), 4.28-4.23 (1H, m), 4.20-4.09 (5H, m). LCMS (ESI) m/z 482 [M+H]$^+$.

Reference Example 133

[(2R,3S,4R,5R)-5-[4-Amino-5-[2-(1-naphthyl)ethynyl]pyrrolo[2,3-d]pyrimidin-7-yl]-3,4-dihydroxy-tetrahydrofuran-2-yl]methyl sulfamate

The title compound was obtained as in Reference Example 131, except that 1-ethynylnaphthalene was used in place of phenylacetylene.
$^1$H-NMR ($CD_3OD$) δ: 8.41 (1H, d, J = 8.0 Hz), 8.19 (1H, s), 7.94-7.90 (2H, m), 7.86 (1H, s), 7.80 (1H, d, J = 4.0 Hz), 7.69-7.48 (3H, m), 6.29 (1H, d, J = 5.1 Hz), 4.87-4.80 (1H, m), 4.52-4.26 (4H, m). LCMS (ESI) m/z 496 [M+H]$^+$.

Reference Example 134

[(2R,3S,5R)-5-[4-Amino-5-(2-phenylethynyl)pyrrolo[2,3-d]pyrimidin-7-yl]-3-hydroxy-tetrahydrofuran-2-yl]methyl sulfamate

The title compound was obtained as in Reference Example 131, except that (2R,3S,5R)-5-(4-amino-5-iodo-7H-pyrrolo[2,3-d]pyrimidin-7-yl)-2-(hydroxymethyl)tetrahydrofuran-3-ol was used in place of [(3aR,4R,6R,6aR)-4-(4-amino-5-iodo-pyrrolo[2,3-d]pyrimidin-7-yl)-2,2-dimethyl-3a,4,6,6a-tetrahydrofuro[3,4-d][1,3]dioxol-6-yl]methanol.
$^1$H-NMR ($CD_3OD$) δ: 8.14 (1H, s), 7.67 (1H, s), 7.54-7.52 (2H, m), 7.39-7.36 (3H, m), 6.65 (1H, dd, J = 7.8, 6.1 Hz), 4.57-4.55 (1H, m), 4.32 (1H, dd, J = 11.0, 3.9 Hz), 4.29 (1H, dd, J = 11.0, 3.9 Hz), 4.18 (1H, dt, J = 3.2, 3.9 Hz), 2.60

(1H, ddd, J = 13.7, 7.8, 6.1 Hz), 2.39 (1H, ddd, J = 13.7, 6.1, 3.2 Hz). LCMS (ESI) m/z 430 $[M+H]^+$.

Reference Example 135

[(2R,3S,5R)-5-[4-Amino-5-(1-benzylpyrazol-4-yl)pyrrolo[2,3-d]pyrimidin-7-yl]-3-hydroxy-tetrahydrofuran-2-yl]methyl sulfamate

Step 1: Synthesis of (2R,3S,5R)-5-(4-amino-5-(1-benzyl-1H-pyrazol-4-yl)-7H-pyrrolo[2,3-d]pyrimidin-7-yl)-2-(hydroxymethyl)tetrahydrofuran-3-ol

(2R,3S,5R)-5-(4-Amino-5-iodo-7H-pyrrolo[2,3-d]pyrimidin-7-yl)-2-(hydroxymethyl)tetrahydrofuran-3-ol (100 mg, 0.265 mmol), tetrakis(triphenylphosphine)palladium(0) (15.3 mg, 0.013 mmol), and 1-benzyl-4-(4,4,5,5-tetramethyl-1,3,2-dioxaborolan-2-yl)-1H-pyrazole (98 mg, 0.34 mmol) were suspended in a 2 M aqueous sodium carbonate solution (0.66 mL) and 1,2-dimethoxyethane (2 mL), followed by stirring at 100°C for 3 hours. The reaction solution was partitioned between ethyl acetate and water, and the organic layer was washed with water, followed by concentration. The residue was purified by silica gel column chromatography (developing solvent: methanol/chloroform), thereby obtaining the target product (66 mg, 61%) as a yellow oil.
$^1$H-NMR ($CDCl_3$) δ: 8.18 (1H, brs), 7.59 (1H, s), 7.44 (1H, s), 7.38-7.32 (3H, m), 7.27-7.23 (2H, m), 6.90 (1H, s), 6.24 (1H, dd, J = 8.8, 5.5 Hz), 5.42-5.38 (2H, m), 5.32 (2H, s), 4.71 (1H, d, J = 4.0 Hz), 4.15 (1H, s), 3.91 (1H, d, J = 12.5 Hz), 3.75 (1H, d, J = 12.5 Hz), 3.05-2.98 (1H, m), 2.25 (1H, dd, J = 12.8, 5.5 Hz). LRMS (ESI) m/z 407 $[M+H]^+$

Step 2: Synthesis of Reference Example Compound 135

(2R,3S,5R)-5-(4-amino-5-(1-benzyl-1H-pyrazol-4-yl)-7H-pyrrolo[2,3-d]pyrimidin-7-yl)-2-(hydroxymethyl)tetrahydrofuran-3-ol (53 mg, 0.13 mmol) was dissolved in acetonitrile (1 mL). Then, 1-aza-4-azoniabicyclo[2.2.2]octan-4-ylsulfonyl(tert-butoxycarbonyl)azanido: 1,4-diazabicyclo[2.2.2]octane monohydrochloride (Reference: Organic Letters, 2012, 10, 2626-2629) (114 mg, 0.26 mmol) was added thereto at room temperature. After the reaction solution was stirred at 40°C overnight, trifluoroacetic acid (0.3 mL) was added to the reaction liquid, followed by stirring at room temperature overnight. The reaction solution was concentrated, and the residue was purified by silica gel column chromatography (developing solvent: methanol/chloroform), thereby obtaining the title compound (4.7 mg, 7%) as a milky-white solid.
$^1$H-NMR ($CD_3OD$) δ: 8.12 (1H, s), 7.88 (1H, s), 7.69 (1H, s), 7.41 (1H, s), 7.37-7.28 (5H, m), 6.71 (1H, t, J = 7.0 Hz), 5.39 (2H, s), 4.57-4.52 (1H, brm), 4.29-4.28 (2H, brm), 4.18-4.15 (1H, brm), 2.62-2.55 (1H, m), 2.39-2.35 (1H, m). LCMS (ESI) m/z 486 $[M+H]^+$.

Reference Example 136

[(1R,2R,3S,4R)-4-[4-Amino-5-(1-benzylpyrazol-4-yl)pyrrolo[2,3-d]pyrimidin-7-yl]-2,3-dihydroxy-cyclopentyl]methyl sulfamate

The title compound was obtained as in Reference Example 135, except that ((3aR,4R,6R,6aS)-6-(4-amino-5-iodo-7H-pyrrolo[2,3-d]pyrimidin-7-yl)-2,2-dimethyltetrahydro-3aH-cyclopenta[d][1,3]dioxol-4-yl)methanol was used in place of (2R,3S,5R)-5-(4-amino-5-iodo-7H-pyrrolo[2,3-d]pyrimidin-7-yl)-2-(hydroxymethyl)tetrahydrofuran-3-ol.
$^1$H-NMR ($CD_3OD$) δ: 8.10 (1H, s), 7.85 (1H, s), 7.66 (1H, s), 7.37-7.21 (6H, m), 5.39 (2H, s), 5.07-4.98 (1H, m), 4.32 (1H, dd, J = 8.2, 5.7 Hz), 4.25 (2H, d, J = 4.9 Hz), 4.05 (1H, dd, J = 5.7, 3.5 Hz), 2.48-2.34 (2H, m), 1.82-1.76 (1H, m). LCMS (ESI) m/z 500 [M+H]+.

Reference Example 137

[(2R,3S,5R)-5-[4-Amino-5-[1-[(3,4-dimethylphenyl)methyl]pyrazol-4-yl]pyrrolo[2,3-d]pyrimidin-7-yl]-3-hydroxy-tetrahydrofuran-2-yl]methyl sulfamate

Step 1: Synthesis of 1-(3,4-dimethylbenzyl)-4-(4,4,5,5-tetramethyl-1,3,2-dioxaborolan-2-yl)-1H-pyrazole

4-(4,4,5,5-Tetramethyl-1,3,2-dioxaborolan-2-yl)-1H-pyrazole (1.0 g, 5.2 mmol), cesium carbonate (2.18 g, 6.7 mmol), and 3,4-dimethylbenzylchloride (0.98 mL, 6.7 mmol) were suspended in acetonitrile (10 mL). After the mixture was stirred at room temperature overnight, the solid was filtered off through celite, and the filtrate was concentrated. The residue was purified by basic silica gel column chromatography (developing solvent: ethyl acetate/hexane), thereby

obtaining the title compound (1.29 g, 80%) as a light-yellow oil.
$^1$H-NMR ($CDCl_3$) δ: 7.80 (1H, s), 7.63 (1H, s), 7.14-6.97 (3H, m), 5.21 (2H, s), 2.25 (3H, s), 2.25 (3H, s), 1.29 (12H, s).
LRMS (ESI) m/z 313 $[M+H]^+$

Step 2: Synthesis of Reference Example Compound 137

The title compound was obtained as in Reference Example 135, except that 1-(3,4-dimethylbenzyl)-4-(4,4,5,5-tetramethyl-1,3,2-dioxaborolan-2-yl)-1H-pyrazole was used in place of 1-benzyl-4-(4,4,5,5-tetramethyl-1,3,2-dioxaborolan-2-yl)-1H-pyrazole.
$^1$H-NMR ($CD_3OD$) δ: 8.12 (1H, s), 7.83 (1H, s), 7.66 (1H, s), 7.39 (1H, s), 7.11 (1H, d, J = 7.8 Hz), 7.09 (1H, s), 7.02 (1H, d, J = 7.8 Hz), 6.70 (1H, dd, J = 7.9, 6.2 Hz), 5.29 (2H, s), 4.57-4.52 (1H, m), 4.28 (2H, m), 4.16 (1H, m), 3.60 (1H, dd, J = 14.1, 7.1 Hz), 2.62-2.55 (1H, m), 2.40-2.34 (1H, m), 2.25 (3H, s), 2.23 (3H, s). LCMS (ESI) m/z 514 $[M+H]^+$.

Reference Example 138

[(1R,2S,4R)-4-[4-Amino-5-[1-[(3,4-dimethylphenyl)methyl]pyrazol-4-yl]pyrrolo[2,3-d]pyrimidin-7-yl]-2-hydroxy-cyclopentyl]methyl sulfamate

Step 1: Synthesis of (1S,2R,4R)-4-(4-amino-5-iodo-7H-pyrrolo[2,3-d]pyrimidin-7-yl)-2-(hydroxymethyl)cyclopentanol

The title compound was obtained as in step 2, step 5, and step 7 of Reference Example 93, except that (1S,2R,4R)-4-amino-2-(hydroxymethyl)cyclopentanol was used in place of ((3aR,4R,6R,6aS)-6-amino-2,2-dimethyltetrahydro-3aH-cyclopenta[d][1,3]dioxol-4-yl)methanol.
$^1$H-NMR (DMSO-$D_6$) δ: 8.06 (1H, s), 7.54 (1H, s), 6.64 (2H, brs), 5.23-5.14 (1H, m), 4.73 (1H, brs), 4.61 (1H, brs), 4.01 (1H, m), 3.48-3.44 (1H, m), 3.41-3.36 (1H, m), 2.23-2.16 (1H, m), 2.08-2.01 (1H, m), 1.96-1.85 (2H, m), 1.58-1.50 (1H, m). LCMS (ESI) m/z 375 $[M+H]^+$

Step 2: Synthesis of Reference Example Compound 138

The title compound was obtained as in Reference Example 135, except that (1S,2R,4R)-4-(4-amino-5-iodo-7H-pyrrolo[2,3-d]pyrimidin-7-yl)-2-(hydroxymethyl)cyclopentanol and 1-(3,4-dimethylbenzyl)-4-(4,4,5,5-tetramethyl-1,3,2-dioxaborolan-2-yl)-1H-pyrazole were used in place of (2R,3S,5R)-5-(4-amino-5-iodo-7H-pyrrolo[2,3-d]pyrimidin-7-yl)-2-(hydroxymethyl)tetrahydrofuran-3-ol and 1-benzyl-4-(4,4,5,5-tetramethyl-1,3,2-dioxaborolan-2-yl)-1H-pyrazole.
$^1$H-NMR ($CD_3OD$) δ: 8.10 (1H, s), 7.79 (1H, s), 7.64 (1H, s), 7.26 (1H, s), 7.10 (1H, d, J = 7.8 Hz), 7.09 (1H, s), 7.02 (1H, d, J = 7.8 Hz), 5.39-5.31 (1H, m), 5.29 (2H, s), 4.30-4.20 (3H, m), 2.53-2.46 (1H, m), 2.38-2.15 (3H, m), 2.24 (3H, s), 2.23 (3H, s), 1.87-1.79 (1H, m). LCMS (ESI) m/z 512 $[M+H]^+$.

Reference Example 139

[(2R,3S,5R)-5-[4-Amino-5-[2-(o-tolyl)thiazol-4-yl]pyrrolo[2,3-d]pyrimidin-7-yl]-3-hydroxy-tetrahydrofuran-2-yl]methyl sulfamate

The title compound was obtained as in Reference Example 135, except that 4-(4,4,5,5-tetramethyl-1,3,2-dioxaborolan-2-yl)-2-(o-tolyl)thiazole was used in place of 1-benzyl-4-(4,4,5,5-tetramethyl-1,3,2-dioxaborolan-2-yl)-1H-pyrazole.
$^1$H-NMR ($CD_3OD$) δ: 8.07 (1H, s), 8.04 (1H, s), 7.92 (1H, s), 7.67 (1H, d, J = 7.3 Hz), 7.42-7.36 (2H, m), 7.34-7.30 (1H, m), 6.75 (1H, t, J = 7.0 Hz), 4.62-4.59 (1H, m), 4.39 (1H, dd, J = 11.0, 3.2 Hz), 4.35 (1H, dd, J = 11.0, 3.2 Hz), 4.22 (1H, dd, J = 6.1, 3.2 Hz), 2.59-2.56 (1H, m), 2.56 (3H, s), 2.46-2.41 (1H, m). LCMS (ESI) m/z 503 $[M+H]^+$.

Reference Example 140

4-Amino-7-[(2R,3R,4S,5R)-3,4-dihydroxy-5-[(sulfamoylamino)methyl]tetrahydrofuran-2-yl]-5-[2-(2-ethylsulfonyl-6-fluoro-phenyl)ethynyl]pyrrolo[2,3-d]pyrimidine

Step 1: Synthesis of tert-butyl N-(((3aR,4R,6R,6aR)-6-(4-amino-5-((2-(ethylthio)-6-fluorophenyl)ethynyl)-7H-pyrrolo[2,3-d]pyrimidin-7-yl)-2,2-dimethyltetrahydrofuro[3,4-d][1,3]dioxol-4-yl)methyl)sulfamoylcarbamate

tert-Butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1,3]dioxol-4-yl)methyl)sulfamoylcarbamate (39 mg, 0.064 mmol), ethyl(2-ethynyl-3-fluorophenyl)sulfane (23 mg, 0.13 mmol), bis(triphenylphosphine)palladium (II) dichloride (4.5 mg, 0.0064 mmol), copper iodide (1.2 mg, 0.0064 mmol), and diisopropylethylamine (0.022 mL, 0.13 mmol) were suspended in tetrahydrofuran (1 mL). After the reaction solution was stirred at 50°C for 3 hours, the solvent was distilled off, and the residue was purified by silica gel column chromatography (developing solvent: methanol/chloroform), thereby obtaining the title compound (28 mg, 66%) as a yellow powder.

$^1$H-NMR ($CDCl_3$) δ: 9.15 (1H, d, J = 8.1 Hz), 8.52 (1H, s), 7.31 (1H, s), 7.24-7.20 (1H, m), 7.06 (1H, d, J = 8.1 Hz), 6.91 (1H, t, J = 8.1 Hz), 6.74-6.59 (2H, brm), 5.68 (1H, d, J = 4.8 Hz), 5.30 (1H, dd, J = 6.2, 4.8 Hz), 5.11 (1H, dd, J = 6.2, 2.2 Hz), 4.51 (1H, d, J = 2.2 Hz), 3.72-3.66 (1H, m), 3.60 (1H, d, J = 12.8 Hz), 3.02 (3H, q, J = 7.3 Hz), 1.61 (3H, s), 1.45 (9H, s), 1.37 (3H, t, J = 7.3 Hz), 1.35 (3H, s).

Step 2: Synthesis of Reference Example Compound 140

tert-Butyl N-(((3aR,4R,6R,6aR)-6-(4-amino-5-((2-(ethylthio)-6-fluorophenyl)ethynyl)-7H-pyrrolo[2,3-d]pyrimidin-7-yl-2,2-dimethyltetrahydrofuro[3,4-d][1,3]dioxol-4-yl)methyl)sulfamoylcarbamate (55 mg, 0.0829 mmol) was suspended with stirring under ice-cooling in 1,4-dioxane (0.5 mL) and water (0.5 mL) while oxone (102 mg, 0.166 mmol) was added thereto. After the reaction liquid was stirred at room temperature for 3 hours, the reaction solution was partitioned between ethyl acetate and water, and the organic layer was extracted. After the solvent was distilled off, acetonitrile (0.5 mL), water (0.1 mL), and trifluoroacetic acid (0.5 mL) were sequentially added to the residue, and the reaction solution was stirred at room temperature for 3 hours. The reaction liquid was concentrated, and the residue was purified by basic silica gel column chromatography (developing solvent: methanol/chloroform), thereby obtaining the title compound (20 mg) as a light-yellow solid.
$^1$H-NMR (DMSO-$D_6$) δ: 8.16 (1H, s), 8.04 (1H, s), 7.82 (2H, d, J = 8.1 Hz), 7.77 (2H, t, J = 8.1 Hz), 7.70 (1H, dd, J = 8.1, 5.5 Hz), 7.35 (1H, dd, J = 7.7, 4.8 Hz), 6.58 (2H, s), 5.92 (1H, d, J = 7.0 Hz), 5.38 (1H, d, J = 6.2 Hz), 5.22 (1H, brs), 4.62-4.58 (1H, m), 4.09-4.07 (1H, brm), 4.05-4.02 (1H, m), 3.49 (2H, q, J = 7.3 Hz), 3.22-3.17 (1H, m), 3.14-3.06 (1H, m), 1.14 (3H, t, J = 7.3 Hz). LCMS (ESI) m/z 555 $[M+H]^+$.

Reference Example 141

4-Amino-5-[2-(4-benzyloxy-2-methylsulfonyl-phenyl)ethynyl]-7-[(1R,2S,3R,4R)-2,3-dihydroxy-4-[(sulfamoylamino)methyl]cyclopentyl]pyrrolo[2,3-d]pyrimidine

Step 1: (5-(Benzyloxy)-2-iodophenyl) (methyl)sulfane

The title compound was obtained as in step 1 of Reference Example 76, except that 4-iodo-3-(methylsulfanyl)phenol was used in place of 2-bromopyrimidin-5-ol.
$^1$H-NMR ($CDCl_3$) δ: 7.63 (1H, d, J = 8.6 Hz), 7.43-7.34 (5H, m), 6.74 (1H, d, J = 2.9 Hz), 6.51 (1H, dd, J = 8.6, 2.8 Hz), 5.06 (2H, s), 3.63 (3H, s). LCMS (ESI) m/z 356 $[M+H]^+$

Step 2: Synthesis of (5-(benzyloxy)-2-ethynylphenyl)(methyl)sulfane

The title compound was obtained as in step 2 and step 3 of Reference Example 76, except that (5-(benzyloxy)-2-iodophenyl)(methyl)sulfane was used in place of 5-(benzyloxy)-2-bromopyrimidine.
$^1$H-NMR ($CDCl_3$) δ: 7.41-7.34 (6H, m), 6.76 (1H, d, J = 2.3 Hz), 6.69 (1H, dd, J = 8.4, 2.3 Hz), 5.08 (2H, s), 3.39 (1H, s), 2.45 (3H, s). LCMS (ESI) m/z 255 $[M+H]^+$

Step 3: Synthesis of Reference Example Compound 141

The title compound was obtained as in Reference Example 140, except that (5-(benzyloxy)-2-ethynylphenyl)(methyl)sulfane was used in place of ethyl(2-ethynyl-3-fluorophenyl)sulfane.
$^1$H-NMR ($CD_3OD$) δ: 8.20 (1H, s), 7.82 (1H, s), 7.73 (1H, d, J = 8.4 Hz), 7.67 (1H, d, J = 2.8 Hz), 7.47 (2H, d, J = 6.8 Hz), 7.42-7.32 (4H, m), 5.23 (2H, s), 5.05-4.95 (1H, m), 4.43-4.39 (1H, m), 4.03-4.00 (1H, m), 3.30 (3H, s), 3.27-3.12 (2H, m), 2.50-2.42 (1H, m), 2.32-2.28 (1H, m), 1.87-1.79 (1H, m). LCMS (ESI) m/z 627 [M+H].

Reference Example 142

4-Amino-7-[(1R,2S,3R,4R)-2,3-dihydroxy-4-[(sulfamoylamino)methyl]cyclopentyl]-5-[2-(2-ethylsulfonyl-6-fluoro-phenyl)ethynyl]pyrrolo[2,3-d]pyrimidine

The title compound was obtained as in Reference Example 140, except that tert-butyl N-(((3aR,4R,6R,6aS)-6-(4-amino-5-iodo-7H-pyrrolo[2,3-d]pyrimidin-7-yl)-2,2-dimethyltetrahydro-3aH-cyclopenta[d][1,3]dioxol-4-yl)methyl)sulfamoylcarbamate was used in place of tert-butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1,3]dioxol-4-yl)methyl)sulfamoylcarbamate.
$^1$H-NMR (DMSO-$D_6$) $\delta$: 8.13 (1H, s), 7.99 (1H, s), 7.81 (1H, d, J = 8.1 Hz), 7.76 (1H, t, J = 8.1 Hz), 7.68 (1H, dd, J = 8.1, 5.1 Hz), 6.63 (1H, t, J = 6.2 Hz), 6.50 (2H, s), 4.89 (2H, d, J = 7.0 Hz), 4.67 (1H, d, J = 4.4 Hz), 4.27-4.22 (1H, m), 4.08 (1H, q, J = 5.3 Hz), 3.79-3.76 (1H, m), 3.49 (2H, q, J = 7.3 Hz), 3.10-3.03 (1H, m), 2.94-2.87 (1H, m), 2.24-2.17 (1H, m), 2.12-2.06 (1H, m), 1.60-1.52 (1H, m), 1.14 (3H, t, J = 7.3 Hz). LCMS (ESI) m/z 553 $[M+H]^+$.

Reference Example 143

4-Amino-5-[2-(2-ethylsulfonyl-6-fluoro-phenyl)ethynyl]-7-[(2R,4S,5R)-4-hydroxy-5-[(sulfamoylamino)methyl]tetrahydrofuran-2-yl]pyrrolo[2,3-d]pyrimidine

The title compound was obtained as in Reference Example 140, except that tert-butyl N-[[(2R,3S,5R)-5-(4-amino-5-iodo-pyrrolo[2,3-d]pyrimidin-7-yl)-3-hydroxy-tetrahydrofuran-2-yl]methyl sulfamoyl]carbamate was used in place of tert-butyl N-(((3aR,4R,6R,6aR)-6-(4-amino-5-iodo-7H-pyrrolo[2,3-d]pyrimidin-7-yl)-2,2-dimethyltetrahydrofuro[3,4-d][1,3]dioxol-4-yl)methyl)sulfamoylcarbamate.
$^1$H-NMR (DMSO-$D_6$) $\delta$: 8.18 (1H, s), 8.03 (1H, s), 7.87-7.83 (1H, m), 7.80-7.75 (1H, m), 7.73-7.68 (1H, m), 7.16 (1H, t, J = 5.9 Hz), 6.56 (2H, s), 6.43 (1H, dd, J = 8.7, 5.7 Hz), 4.37 (1H, brs), 3.97 (1H, dt, J = 2.0, 4.6 Hz), 3.50 (2H, q, J = 7.4 Hz), 3.20-3.14 (1H, m), 3.12-3.05 (1H, m), 2.71-2.62 (1H, m), 2.21-2.16 (1H, m), 1.15 (3H, t, J = 7.4 Hz). LCMS (ESI) m/z 539 $[M+H]^+$.

Comparative Example

N-[(1S)-1-indanyl]-7-[(1R)-3$\alpha$-hydroxy-4$\alpha$-(sulfamoyloxymethyl)cyclopentyl]-7H-pyrrolo[2,3-d]pyrimidin-4-amine (MLN4924) was obtained by performing the synthesis in accordance with the method disclosed in Patent Document 1.

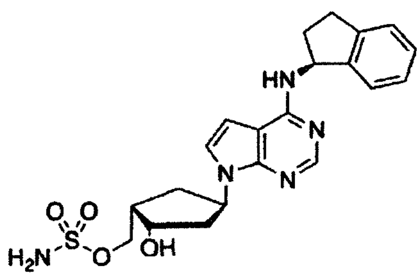

The following tables show the structural formulae of the Reference Example compounds of the present application.

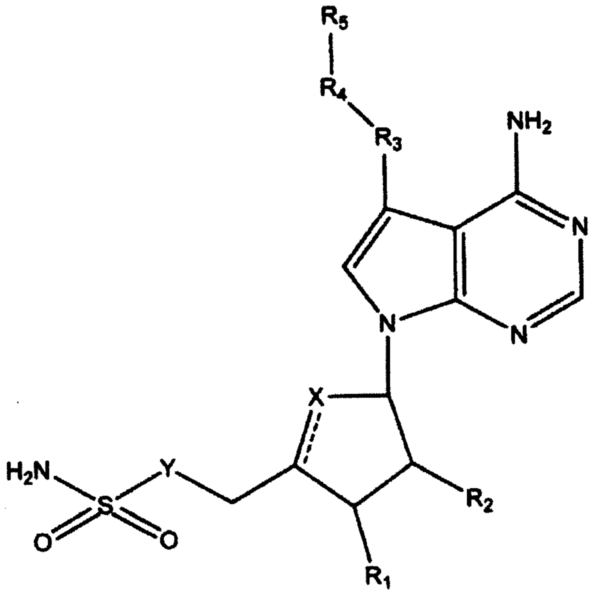

(A)

Table 1

| | 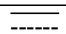 | X | Y | $R_1$ | $R_2$ | $R_3$ | $R_4$ | $R_5$ |
|---|---|---|---|---|---|---|---|---|
| Reference Example 1 | single | O | NH | OH | OH |  | bond | 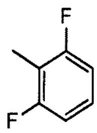 |
| Reference Example 2 | single | O | NH | OH | OH | 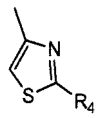 | bond | 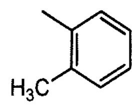 |
| Reference Example 3 | single | O | NH | OH | OH |  | bond | 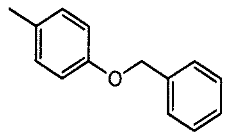 |
| Reference Example 4 | single | O | NH | OH | OH |  | bond | 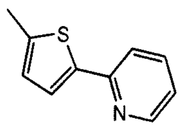 |
| Reference Example 5 | single | O | NH | OH | OH | 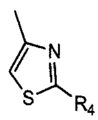 | bond | 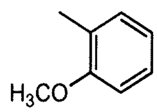 |
| Reference Example 6 | single | O | NH | OH | OH |  | bond | 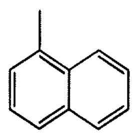 |
| Reference Example 7 | single | O | NH | OH | OH |  | $-CH_2-$ | 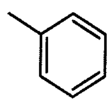 |

(continued)

| | 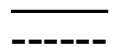 | X | Y | $R_1$ | $R_2$ | $R_3$ | $R_4$ | $R_5$ |
|---|---|---|---|---|---|---|---|---|
| Reference Example 8 | single | O | NH | OH | OH |  | bond | 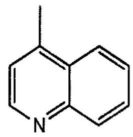 |
| Reference Example 9 | single | O | NH | OH | OH |  | bond | 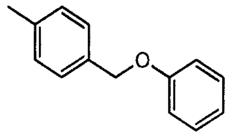 |
| Reference Example 10 | single | O | NH | OH | OH |  | 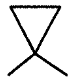 | 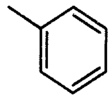 |

Table 2

| | 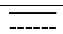 | X | Y | $R_1$ | $R_2$ | $R_3$ | $R_4$ | $R_5$ |
|---|---|---|---|---|---|---|---|---|
| Reference Example 11 | single | O | NH | OH | OH |  | bond | 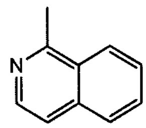 |
| Reference Example 12 | single | O | NH | OH | OH | 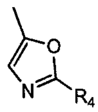 | bond | 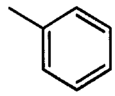 |
| Reference Example 13 | single | O | NH | OH | OH |  | bond | 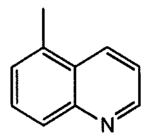 |
| Reference Example 14 | single | O | NH | OH | OH |  | bond | 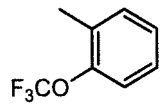 |
| Reference Example 15 | single | O | NH | OH | OH |  | bond | 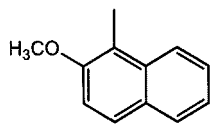 |
| Reference Example 16 | single | O | NH | OH | OH |  | bond | 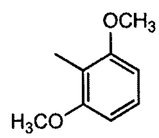 |
| Reference Example 17 | single | O | NH | OH | OH |  | bond | 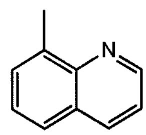 |

(continued)

| | 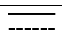 | X | Y | $R_1$ | $R_2$ | $R_3$ | $R_4$ | $R_5$ |
|---|---|---|---|---|---|---|---|---|
| Reference Example 18 | single | O | NH | OH | OH |  | bond | 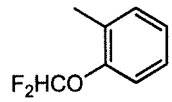 |
| Reference Example 19 | single | O | NH | OH | OH |  | bond | 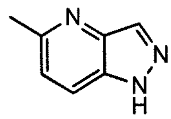 |
| Reference Example 20 | single | O | NH | OH | OH |  | bond | 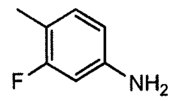 |

Table 3

| | 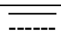 | X | Y | $R_1$ | $R_2$ | $R_3$ | $R_4$ | $R_5$ |
|---|---|---|---|---|---|---|---|---|
| Reference Example 21 | single | O | NH | OH | OH |  | bond | 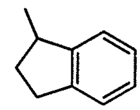 |
| Reference Example 22 | single | O | NH | OH | OH |  | bond | 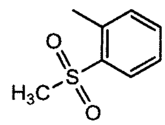 |
| Reference Example 23 | single | O | NH | OH | OH |  | bond | 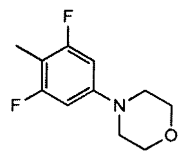 |
| Reference Example 24 | single | O | NH | OH | OH |  | bond | 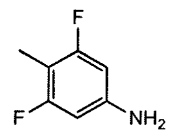 |
| Reference Example 25 | single | O | NH | OH | OH |  | bond | 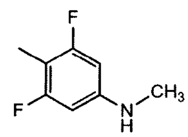 |
| Reference Example 26 | single | O | NH | OH | OH |  | bond | 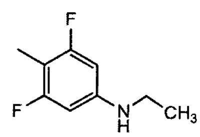 |
| Reference Example 27 | single | O | NH | OH | OH |  | bond | 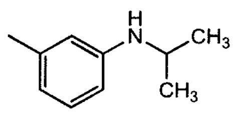 |
| Reference Example 28 | single | O | NH | OH | OH |  | bond | 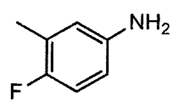 |

(continued)

| | 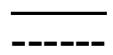 | X | Y | $R_1$ | $R_2$ | $R_3$ | $R_4$ | $R_5$ |
|---|---|---|---|---|---|---|---|---|
| Reference Example 29 | single | O | NH | OH | OH |  | bond | 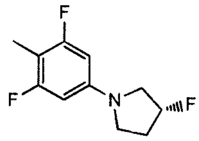 |
| Reference Example 30 | single | O | NH | OH | OH |  | bond | 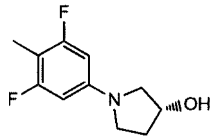 |

Table 4

| | 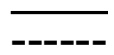 | X | Y | $R_1$ | $R_2$ | $R_3$ | $R_4$ | $R_5$ |
|---|---|---|---|---|---|---|---|---|
| Reference Example 31 | single | O | NH | OH | OH |  | $-CH_2-$ | 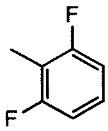 |
| Reference Example 32 | single | O | NH | OH | OH |  | bond | 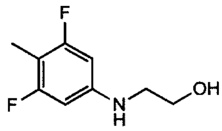 |
| Reference Example 33 | single | O | NH | OH | OH |  | bond | 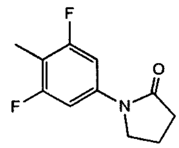 |
| Reference Example 34 | single | O | NH | OH | OH |  | bond | 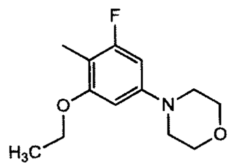 |
| Reference Example 35 | single | O | NH | OH | OH |  | bond | 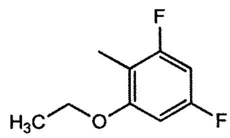 |
| Reference Example 36 | single | O | NH | OH | OH |  | $-CH_2-$ | 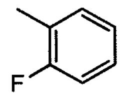 |
| Reference Example 37 | single | O | NH | OH | OH |  | bond | 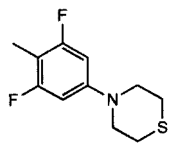 |

(continued)

| | | X | Y | $R_1$ | $R_2$ | $R_3$ | $R_4$ | $R_5$ |
|---|---|---|---|---|---|---|---|---|
| Reference Example 38 | single | O | NH | OH | OH |  | bond | 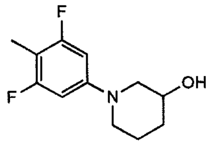 |
| Reference Example 39 | single | O | NH | OH | OH |  | bond | 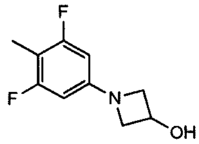 |
| Reference Example 40 | single | O | NH | OH | OH |  | bond | 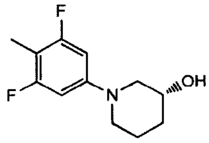 |

Table 5

| | | X | Y | $R_1$ | $R_2$ | $R_3$ | $R_4$ | $R_5$ |
|---|---|---|---|---|---|---|---|---|
| Reference Example 41 | single | O | NH | OH | OH |  | bond | 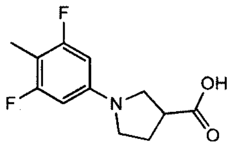 |
| Reference Example 42 | single | O | NH | OH | OH |  | bond | 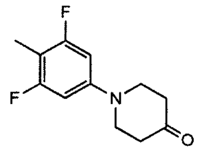 |
| Reference Example 43 | single | O | NH | OH | OH |  | bond | 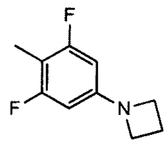 |
| Reference Example 44 | single | O | NH | OH | OH |  | bond | 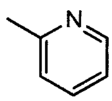 |
| Reference Example 45 | single | O | NH | OH | OH |  | bond | 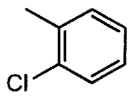 |
| Reference Example 46 | single | O | NH | OH | OH |  | bond | 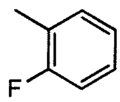 |
| Reference Example 47 | single | O | NH | OH | OH |  | bond | 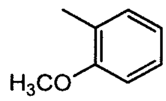 |

(continued)

| | | X | Y | $R_1$ | $R_2$ | $R_3$ | $R_4$ | $R_5$ |
|---|---|---|---|---|---|---|---|---|
| Reference Example 48 | single | O | NH | OH | OH |  | bond | 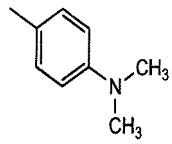 |
| Reference Example 49 | single | O | NH | OH | OH |  | bond | 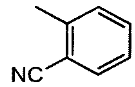 |
| Reference Example 50 | single | O | NH | OH | OH |  | $-CH_2-$ | 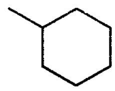 |

Table 6

| | | X | Y | $R_1$ | $R_2$ | $R_3$ | $R_4$ | $R_5$ |
|---|---|---|---|---|---|---|---|---|
| Reference Example 51 | single | O | NH | OH | OH |  | bond | 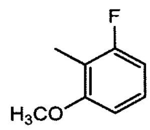 |
| Reference Example 52 | single | O | NH | OH | OH |  | bond | 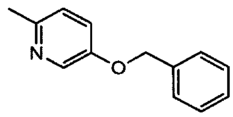 |
| Reference Example 53 | single | O | NH | OH | OH |  | bond | 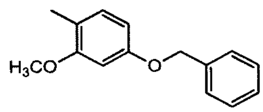 |
| Reference Example 54 | single | O | NH | OH | OH |  | bond | 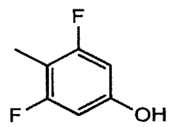 |
| Reference Example 55 | single | O | NH | OH | OH |  | bond | 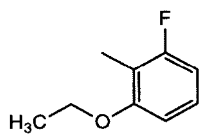 |
| Reference Example 56 | single | O | NH | OH | OH |  | bond | 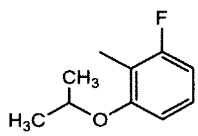 |
| Reference Example 57 | single | O | NH | OH | OH |  | bond | 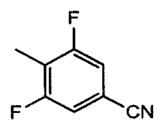 |

(continued)

| | 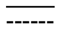 | X | Y | $R_1$ | $R_2$ | $R_3$ | $R_4$ | $R_5$ |
|---|---|---|---|---|---|---|---|---|
| Reference Example 58 | single | O | NH | OH | OH |  | bond | 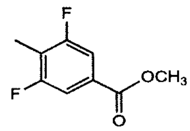 |
| Reference Example 59 | single | O | NH | OH | OH |  | bond | 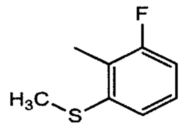 |
| Reference Example 60 | single | O | NH | OH | OH |  | bond | 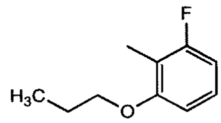 |

Table 7

| | 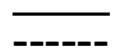 | X | Y | $R_1$ | $R_2$ | $R_3$ | $R_4$ | $R_5$ |
|---|---|---|---|---|---|---|---|---|
| Reference Example 61 | single | O | NH | OH | OH |  | bond | 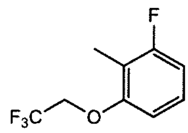 |
| Reference Example 62 | single | O | NH | OH | OH |  | bond | 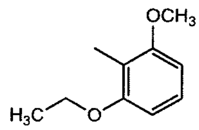 |
| Reference Example 63 | single | O | NH | OH | OH |  | bond | 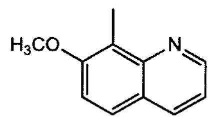 |
| Reference Example 64 | single | O | NH | OH | OH |  | bond | 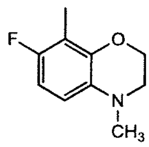 |
| Reference Example 65 | single | O | NH | OH | OH |  | bond | 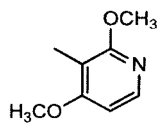 |
| Reference Example 66 | single | O | NH | OH | OH |  | bond | 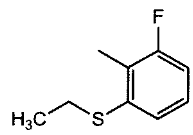 |

(continued)

| | | X | Y | $R_1$ | $R_2$ | $R_3$ | $R_4$ | $R_5$ |
|---|---|---|---|---|---|---|---|---|
| Reference Example 67 | single | O | NH | OH | OH |  | bond | 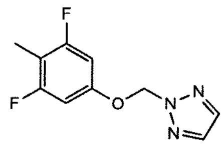 |
| Reference Example 68 | single | O | NH | OH | OH |  | bond | 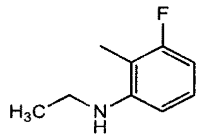 |
| Reference Example 69 | single | O | NH | OH | OH |  | bond | 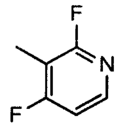 |
| Reference Example 70 | single | O | NH | OH | OH |  | bond | 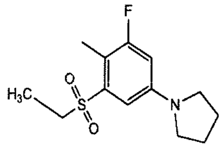 |

Table 8

| | | X | Y | $R_1$ | $R_2$ | $R_3$ | $R_4$ | $R_5$ |
|---|---|---|---|---|---|---|---|---|
| Reference Example 71 | single | O | NH | OH | OH |  | bond | 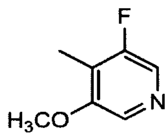 |
| Reference Example 72 | single | O | NH | OH | OH |  | bond | 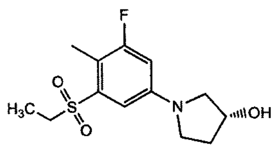 |
| Reference Example 73 | single | O | NH | OH | OH |  | bond | 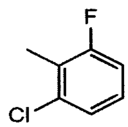 |
| Reference Example 74 | single | O | NH | OH | OH |  | bond | 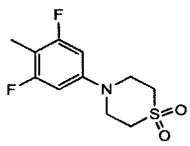 |
| Reference Example 75 | single | O | NH | OH | OH |  | bond | 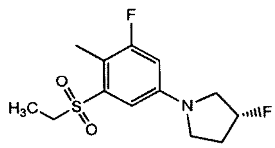 |

(continued)

| | | X | Y | $R_1$ | $R_2$ | $R_3$ | $R_4$ | $R_5$ |
|---|---|---|---|---|---|---|---|---|
| Reference Example 76 | single | O | NH | OH | OH |  | bond | 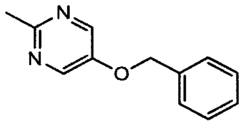 |
| Reference Example 77 | single | O | NH | OH | OH |  | bond | 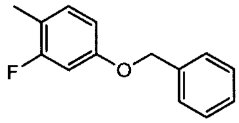 |
| Reference Example 78 | single | O | NH | OH | OH |  | bond | 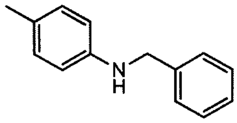 |
| Reference Example 79 | single | O | NH | OH | OH |  | bond | 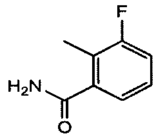 |
| Reference Example 80 | single | O | NH | OH | OH |  | bond | 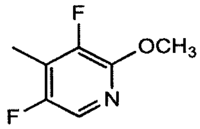 |

Table 9

| | | X | Y | $R_1$ | $R_2$ | $R_3$ | $R_4$ | $R_5$ |
|---|---|---|---|---|---|---|---|---|
| Reference Example 81 | single | O | NH | OH | OH |  | bond | 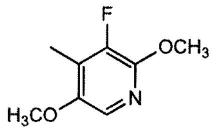 |
| Reference Example 82 | single | O | NH | OH | OH |  | bond | 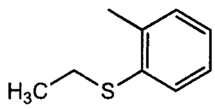 |
| Reference Example 83 | single | O | NH | OH | OH |  | bond | 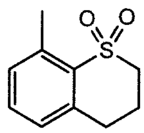 |
| Reference Example 84 | single | O | NH | OH | OH |  | bond | 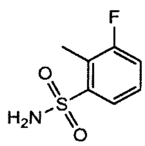 |
| Reference Example 85 | single | O | NH | OH | OH |  | bond | 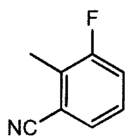 |

(continued)

| | | X | Y | $R_1$ | $R_2$ | $R_3$ | $R_4$ | $R_5$ |
|---|---|---|---|---|---|---|---|---|
| Reference Example 86 | single | O | NH | OH | OH |  | bond | 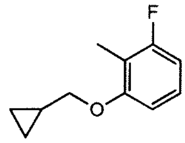 |
| Reference Example 87 | single | O | NH | OH | OH |  | bond | 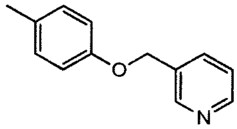 |
| Reference Example 88 | single | O | NH | OH | OH |  | bond | 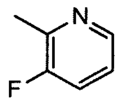 |
| Reference Example 89 | single | O | NH | OH | OH |  | bond | 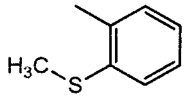 |
| Reference Example 90 | single | O | NH | OH | OH |  | bond | 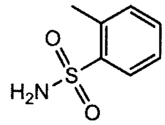 |

Table 10

| | | X | Y | $R_1$ | $R_2$ | $R_3$ | $R_4$ | $R_5$ |
|---|---|---|---|---|---|---|---|---|
| Reference Example 91 | single | O | NH | OH | OH |  | bond | 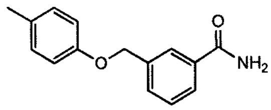 |
| Reference Example 92 | single | O | NH | OH | OH |  | bond | 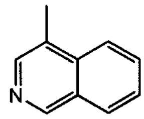 |
| Reference Example 93 | single | C | NH | OH | OH |  | bond | 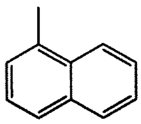 |
| Reference Example 94 | single | C | NH | OH | OH |  | bond | 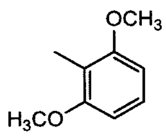 |
| Reference Example 95 | single | C | NH | OH | OH |  | bond | 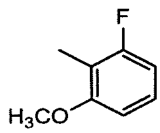 |

(continued)

| | | X | Y | $R_1$ | $R_2$ | $R_3$ | $R_4$ | $R_5$ |
|---|---|---|---|---|---|---|---|---|
| Reference Example 96 | single | C | NH | OH | OH |  | bond | 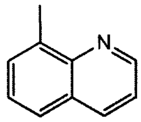 |
| Reference Example 97 | single | C | NH | OH | OH |  | bond | 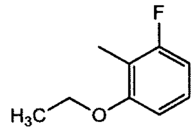 |
| Reference Example 98 | single | C | NH | OH | OH |  | bond | 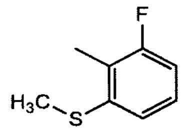 |
| Reference Example 99 | single | C | NH | OH | OH |  | bond | 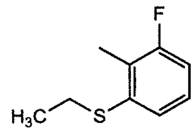 |
| Reference Example 100 | single | C | NH | OH | OH |  | bond | 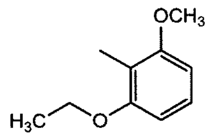 |

Table 11

| | | X | Y | $R_1$ | $R_2$ | $R_3$ | $R_4$ | $R_5$ |
|---|---|---|---|---|---|---|---|---|
| Reference Example 101 | single | C | NH | OH | OH |  | bond | 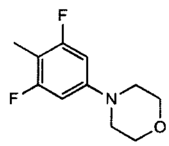 |
| Reference Example 102 | single | C | NH | OH | OH |  | bond | 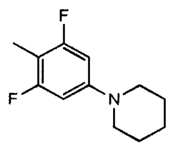 |
| Reference Example 103 | single | C | NH | OH | OH |  | bond | 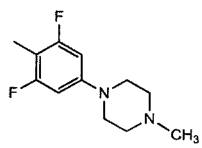 |
| Reference Example 104 | single | C | NH | OH | OH |  | bond | 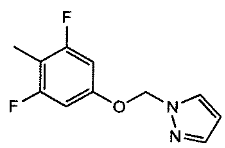 |

(continued)

| | 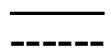 | X | Y | $R_1$ | $R_2$ | $R_3$ | $R_4$ | $R_5$ |
|---|---|---|---|---|---|---|---|---|
| Reference Example 105 | single | C | NH | OH | OH |  | bond | 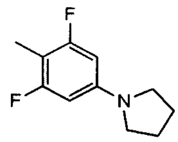 |
| Reference Example 106 | single | C | NH | OH | OH |  | bond | 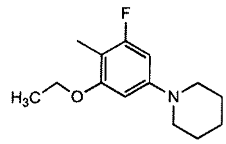 |
| Reference Example 107 | single | C | NH | OH | OH |  | bond | 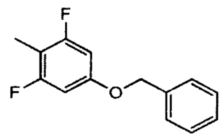 |
| Reference Example 108 | single | C | NH | OH | OH |  | bond | 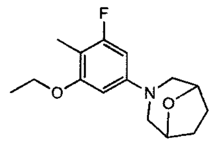 |
| Reference Example 109 | single | C | NH | OH | OH |  | bond | 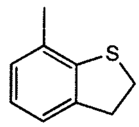 |
| Reference Example 110 | single | C | NH | OH | OH |  | bond | 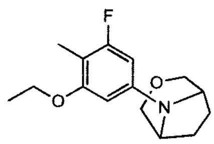 |

Table 12

| | 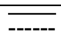 | X | Y | $R_1$ | $R_2$ | $R_3$ | $R_4$ | $R_5$ |
|---|---|---|---|---|---|---|---|---|
| Reference Example 111 | single | C | NH | OH | OH |  | bond | 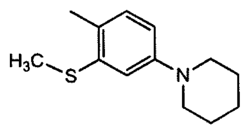 |
| Reference Example 112 | single | C | NH | OH | OH |  | bond | 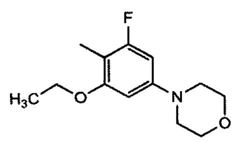 |
| Reference Example 113 | single | C | NH | OH | OH |  | bond | 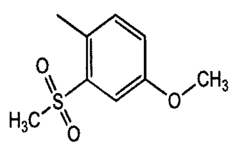 |

(continued)

| | 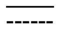 | X | Y | $R_1$ | $R_2$ | $R_3$ | $R_4$ | $R_5$ |
|---|---|---|---|---|---|---|---|---|
| Reference Example 114 | single | C | NH | OH | OH |  | bond | 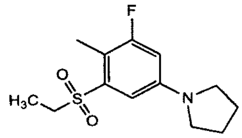 |
| Reference Example 115 | single | C | NH | OH | OH |  | bond | 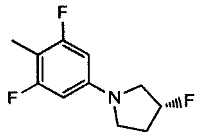 |
| Reference Example 116 | single | C | NH | OH | OH |  | bond | 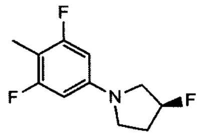 |
| Reference Example 117 | single | C | NH | OH | OH |  | bond | 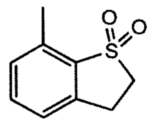 |
| Reference Example 118 | single | C | NH | OH | OH |  | bond | 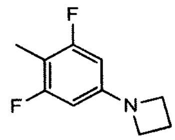 |
| Reference Example 119 | single | C | NH | OH | OH |  | bond | 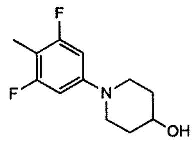 |
| Reference Example 120 | single | C | NH | OH | OH |  | bond | 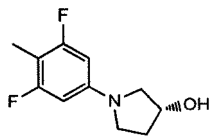 |

Table 13

| | 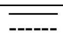 | X | Y | $R_1$ | $R_2$ | $R_3$ | $R_4$ | $R_5$ |
|---|---|---|---|---|---|---|---|---|
| Reference Example 121 | single | C | NH | OH | OH |  | bond | 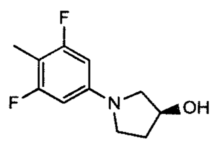 |
| Reference Example 122 | single | C | NH | OH | OH |  | bond | 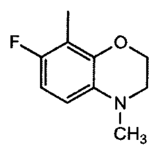 |

(continued)

| | ------ | X | Y | $R_1$ | $R_2$ | $R_3$ | $R_4$ | $R_5$ |
|---|---|---|---|---|---|---|---|---|
| Reference Example 123 | single | C | NH | OH | OH |  | bond | 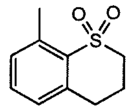 |
| Reference Example 124 | single | O | NH | OH | H |  | bond | 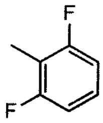 |
| Reference Example 125 | single | O | NH | OH | H |  | bond | 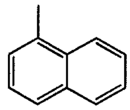 |
| Reference Example 126 | single | O | NH | OH | H | 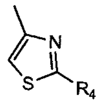 | bond | 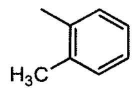 |
| Reference Example 127 | single | O | NH | OH | H |  | bond | 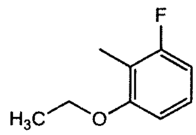 |
| Reference Example 128 | double | C | NH | OH | OH |  | bond | 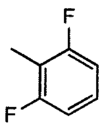 |
| Reference Example 129 | double | C | NH | OH | OH |  | bond | 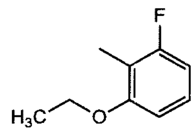 |
| Reference Example 130 | double | C | NH | OH | OH |  | bond | 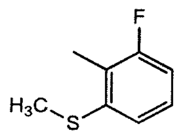 |

Table 14

| | ------ | X | Y | $R_1$ | $R_2$ | $R_3$ | $R_4$ | $R_5$ |
|---|---|---|---|---|---|---|---|---|
| Reference Example 131 | single | O | O | OH | OH |  | bond | 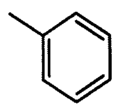 |
| Reference Example 132 | single | O | O | OH | OH |  | bond | 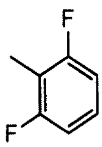 |

(continued)

| | ------ | X | Y | $R_1$ | $R_2$ | $R_3$ | $R_4$ | $R_5$ |
|---|---|---|---|---|---|---|---|---|
| Reference Example 133 | single | O | O | OH | OH |  | bond | 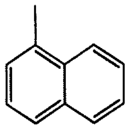 |
| Reference Example 134 | single | O | O | OH | H |  | bond | 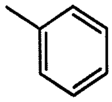 |
| Reference Example 135 | single | O | O | OH | H | 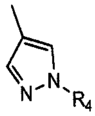 | $-CH_2-$ | 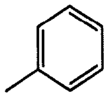 |
| Reference Example 136 | single | C | O | OH | OH | 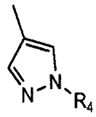 | $-CH_2-$ | 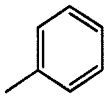 |
| Reference Example 137 | single | O | O | OH | H | 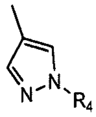 | $-CH_2-$ | 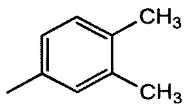 |
| Reference Example 138 | single | C | O | OH | H | 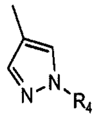 | $-CH_2-$ | 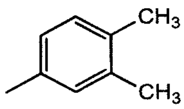 |
| Reference Example 139 | single | O | O | OH | H | 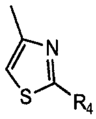 | bond | 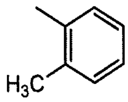 |
| Reference Example 140 | single | O | NH | OH | OH |  | bond | 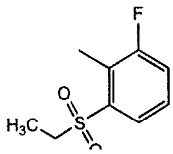 |

Table 15

| | ------ | X | Y | $R_1$ | $R_2$ | $R_3$ | $R_4$ | $R_5$ |
|---|---|---|---|---|---|---|---|---|
| Reference Example 141 | single | C | NH | OH | OH |  | bond | 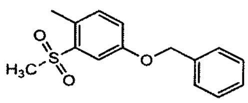 |
| Reference Example 142 | single | C | NH | OH | OH |  | bond | 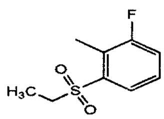 |

(continued)

| | ------ | X | Y | $R_1$ | $R_2$ | $R_3$ | $R_4$ | $R_5$ |
|---|---|---|---|---|---|---|---|---|
| Reference Example 143 | single | O | NH | OH | H |  | bond | 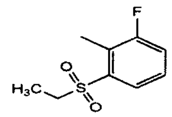 |

Test Example 1: Nedd8 conjugation inhibitory activity

A purified NAE (heterodimer of APPBP1 and UBA3) solution was prepared in the following manner. The human APPBP1 gene (NCBI Reference Sequence number: NM_003905) region corresponding to amino acids 1 to 534 of human APPBP1 protein (NCBI Reference Sequence number: NP_003896, full length: 534 amino acids) was inserted in pBacPAK9 (produced by Clontech) to construct a plasmid pBacPAK9-APPBP1 for expressing APPBP1 full-length protein having a His tag and a TEV protease-recognition sequence at the N-terminus. Next, the human UBA3 gene (NCBI Reference Sequence number: NM_003968) region corresponding to amino acids 1 to 463 of human UBA3 protein (NCBI Reference Sequence number: NP_003959, full length: 463 amino acids) was inserted in pBacPAK9 to construct a plasmid pBacPAK9-UBA3 for expressing UBA3 full-length protein. The pBacPAK9-APPBP1 or pBacPAK9-UBA3, and BacPAK6 DNA were cotransfected into insect cells (Sf9, produced by Clontech) to produce a recombinant baculovirus containing APPBP1 or UBA3 gene. The APPBP1 gene recombinant baculovirus was mixed with the UBA3 gene recombinant baculovirus, and the resulting mixture was used to infect Sf9 cells. The baculovirus-infected Sf9 cells were incubated at 28°C with shaking for 72 hours in Grace's Insect Medium (produced by Gibco), and the collected cells were suspended in a lysis buffer (50 mM Tris-HCl, 200 mM NaCl, and 10% glycerol (pH 7.4)), followed by sonication. The sonicated cell solution was centrifuged (40,000 x g, for 30 minutes) to obtain the supernatant as a crude extract. The crude extract was fractionated on a HisTrap HP column (produced by GE Healthcare) and a TALON Superflow column (produced by Clontech), followed by the addition of a TEV protease. Then, a His-tag cleavage reaction was performed at 4°C overnight. The resulting solution was subjected to TALON Superflow column chromatography, and the unadsorbed fraction was collected. This fraction was applied to a HiLoad 16/60 Superdex 75 prep grade column equilibrated with 50 mM Tris-HCl, 200 mM NaCl, and 10% glycerol (pH 7.4), and fractionated. A fraction containing an APPBP1/UBA3 complex was concentrated to obtain a purified NAE solution. The purification above was performed entirely at 4°C. The purified NAE solution was stored at -80°C until use.

A purified GST-UBC12 solution was prepared in the following manner. The human UBC12 gene (NCBI Reference Sequence number: NM_003969) region corresponding to amino acids 1 to 183 of human UBC12 protein (NCBI Reference Sequence number: NP_003960, full length: 183 amino acids) was inserted in pGEX-4T-2 (produced by GE Healthcare) to construct a plasmid pGEX-UBC12 for expressing UBC12 full-length protein having a GST tag at the N-terminus. The pGEX-UBC12 was introduced into Escherichia coli (BL21 (DE3), produced by Stratagene), followed by culture at 37°C for 2 hours in the presence of 1 mM isopropyl-beta-D-thiogalactopyranoside (produced by Sigma-Aldrich). The collected Escherichia coli was suspended in PBS, followed by sonication. The sonicated cell solution was centrifuged (40,000 x g, for 5 minutes) to obtain the supernatant as a crude extract. A Glutathione Sepharose 4B carrier (produced by GE Healthcare) was added to the crude extract, and eluted with 50 mM Tris-HCl (pH 7.9), 150 mM NaCl, and a 10 mM reduced glutathione solution, followed by dialysis with 50 mM HEPES (pH 7.5) and a 0.05% BSA solution to obtain a purified GST-UBC12 solution. The purified GST-UBC12 solution was divided and stored at -80°C until use.

The Nedd8 conjugation inhibitory activity was measured using an AlphaScreen assay system. Each of the purified NAE solution and GST-UBC12 solution was diluted with an assay buffer (50 mM HEPES (pH 7.5), 5 mM $MgCl_2$, 1 mM DTT, 0.05% BSA) and added to a 384-well plate (#3673, produced by Corning) containing the test compound. After reaction at room temperature for 30 minutes, a solution obtained by diluting ATP and Biotin-Nedd8 (produced by Boston Biochem) with the assay buffer was added thereto, followed by reaction for 90 minutes.

Detection mix (50 mM HEPES (pH 7.5), 0.05% BSA, 0.04 mg/mL anti-GST Acceptor beads, 0.04 mg/mL Streptavidin Donor beads) (#6760603M, produced by Perkin Elmer) was added to each well in the same amount as that of the reaction solution. Then, after reaction in a dark place at room temperature for 1 hour, the fluorescence intensity was measured using an EnVision (produced by Perkin Elmer) multilabel plate reader. The Neddylation inhibition rate (%) achieved by the compound of Reference Examples was determined by the equation (equation A) below using the fluorescence signal of a test compound-free group as a positive control, and the fluorescence signal of the test compound- and ATP-free group as a negative control. The concentration at which the Nedd8 conjugation was reduced with the addition of each compound to 50% of the control was calculated ($IC_{50}$ ($\mu$M)) and used as a relative index of the Nedd8 conjugation inhibitory activity.

```
Inhibition rate (%) = 100 - (T-B)/(C-B) x 100 (equation A)
```

T: Signal in a well to which a test compound was added
C: Signal in a well to which a test compound was not added
B: Signal in a well to which a test compound and ATP were not added

Table 16 below show the results.

Table 16

| | IC50 (μM) | | IC50 (μM) | | IC50 (μM) | | IC50 (μM) |
|---|---|---|---|---|---|---|---|
| Reference Example 1 | ≤0.0030 | Reference Example 42 | 0.0043 | Reference Example 75 | ≤0.0030 | Reference Example 114 | ≤0.0030 |
| Reference Example 2 | 0.0071 | Reference Example 43 | 0.0076 | Reference Example 76 | 0.0051 | Reference Example 115 | 0.004 |
| Reference Example 8 | 0.029 | Reference Example 44 | 0.029 | Reference Example 79 | 0.0032 | Reference Example 116 | 0.0051 |
| Reference Example 10 | 0.024 | Reference Example 46 | 0.013 | Reference Exemple 80 | 0.014 | Reference Example 117 | 0.0043 |
| Reference Example 11 | 0.0078 | Reference Example 47 | 0.0093 | Reference Example 81 | 0.0056 | Reference Example 118 | 0.0068 |
| Reference Exanple 16 | 0.0096 | Reference Example 49 | 0.018 | Reference Example 82 | 0.01 | Reference Example 119 | 0.0042 |
| Reference Example 17 | 0.0054 | Reference Example 51 | 0.0035 | Referance Example 83 | 0.019 | Reference Example 120 | 0.0043 |
| Reference Example 18 | 0.027 | Reference Example 52 | 0.008 | Reference Example 84 | 0.0097 | Reference Example 121 | 0.0062 |
| Reference Example 19 | 0.028 | Reference Example 54 | 0.0045 | Reference Example 86 | 0.0054 | Reference Example 122 | 0.02 |
| Reference Example 20 | 0.0091 | Reference Example 55 | ≤0.0030 | Reference Example 88 | 0.018 | Reference Example 123 | 0.0091 |
| Reference Example 21 | 0.015 | Reference Example 56 | 0.0053 | Reference Example 89 | 0.011 | Reference Example 127 | 0.019 |
| Reference Example 22 | ≤0.0030 | Reference Example 57 | 0.0058 | Reference Example 90 | 0.0056 | Reference Example 128 | 0.0033 |
| Reference Example 23 | ≤0.0030 | Reference Example 58 | ≤0.0030 | Reference Example 91 | 0.018 | Reference Example 129 | ≤0.0030 |
| Reference Example 24 | ≤0.0030 | Reference Example 59 | 0.0085 | Reference Example | 0.021 | Reference Example 130 | 0.004 |
| Reference Example 25 | 0.0048 | Reference Example 60 | 0.0085 | Reference Example 95 | 0.013 | Reference Example 131 | ≤0.0030 |
| Reference Example 26 | 0.012 | Reference Example 61 | 0.0038 | Reference Example 96 | 0.0092 | Reference Example 132 | ≤0.0030 |
| Reference Example 29 | 0.0071 | Reference Example 62 | 0.016 | Reference Example 97 | 0.0088 | Reference Example 133 | 0.025 |
| Reference Example 30 | 0.027 | Reference Example 63 | 0.011 | Reference Example 98 | 0.0083 | Reference Example 134 | 0.018 |

(continued)

| | IC50 (μM) | | IC50 (μM) | | IC50 (μM) | | IC50 (μM) |
|---|---|---|---|---|---|---|---|
| Reference Example 33 | ≤0.0030 | Reference Example 64 | 0.0094 | Reference Example 99 | 0.019 | Reference Example 136 | 0.012 |
| Reference Example 34 | 0.024 | Reference Example 66 | 0.0071 | Reference Example 101 | 0.0076 | Reference Example 137 | 0.019 |
| Reference Example 35 | 0.0041 | Reference Example 67 | 0.0085 | Reference Example 103 | 0.013 | Reference Example 138 | 0.0065 |
| Reference Example 37 | 0.011 | Reference Example 69 | 0.015 | Reference Example 104 | 0.0089 | Reference Example 139 | 0.017 |
| Reference Example 38 | 0.0035 | Reference Example 70 | 0.013 | Reference Example 106 | 0.028 | Reference Example 142 | 0.009 |
| Reference Example 39 | ≤0.0030 | Reference Example 71 | 0.014 | Reference Example 110 | 0.013 | Reference Example 143 | 0.024 |
| Reference Example 40 | ≤0.0030 | Reference Example 72 | 0.0044 | Reference Example 112 | 0.015 | Comp. Example | 0.033 |
| Reference Example 41 | ≤0.0030 | Reference Example 74 | ≤0.0030 | Reference Example 113 | ≤0.0030 | | |

These results show that the compounds of Reference Examples exhibited excellent Nedd8 conjugation inhibitory activity, compared with the Comparative Example.

Test Example 2: Cell growth inhibition 1

The ability of test compounds to inhibit cell growth was determined by quantifying ATP from viable cells using a CellTiter-Glo™ Luminescent Cell Viability Assay (#G7573, produced by Promega, Inc.). The human acute T lymphoblastic leukemia CCRF-CEM cell lines (distributed by Dainippon Pharmaceutical Co., Ltd. (currently Sumitomo Dainippon Pharma Co., Ltd.)) were seeded in a 96-well plate (#165305, produced by Thermo Scientific Nunc) at a concentration of 1,000 cells/100 μL medium per well. The cells were cultured in a 5% $CO_2$ incubator at 37°C overnight, and the test compound was added thereto, followed by culture for another 72 hours. A CellTiter-Glo™ Luminescent Cell Viability Assay reagent in an amount equal to the medium was added to each well, stirred for 5 minutes on a shaker under shading conditions, and then left to stand at room temperature for about 30 minutes. The luminescence intensity was measured using a microplate reader (EnSpire™ Multimode Plate Reader, produced by PerkinElmer Japan Co., Ltd.), and used as an index of the number of viable cells in each well. The cell growth inhibition rate (%) achieved by the compound was determined by the following equation (equation B) by using the luminescence intensity of a drug untreated group (control) as a control. The concentration at which the number of cells are reduced with the addition of each compound to 50% of the control was calculated ($IC_{50}$ (μM)).

$$\text{Inhibition rate (\%)} = (C-T)/C \times 100 \quad \text{(equation B)}$$

T: Luminescence intensity in a well to which a test compound was added
C: Luminescence intensity in a well to which a test substance was not added

Table 17 below shows the results.

Table 17

| | CCRF-CEM | | CCRF-CEM |
|---|---|---|---|
| | IC50 (μM) | | IC50 (μM) |
| Reference Example 1 | 0.0062 | Reference Example 66 | 0.0043 |

(continued)

| | CCRF-CEM | | CCRF-CEM |
|---|---|---|---|
| | IC50 (μM) | | IC50 (μM) |
| Reference Example 24 | 0.0027 | Reference Example 70 | 0.015 |
| Reference Example 25 | 0.0017 | Reference Example 71 | 0.045 |
| Reference Example 29 | 0.004 | Reference Example 72 | 0.088 |
| Reference Example 31 | 0.6 | Reference Example 73 | 0.071 |
| Reference Example 32 | 2.5 | Reference Example 74 | 0.16 |
| Reference Example 33 | 0.037 | Reference Example 83 | 0.056 |
| Reference Example 34 | 0.0061 | Reference Example 84 | 0.23 |
| Reference Example 35 | 0.002 | Reference Example 85 | 0.038 |
| Reference Example 36 | 0.25 | Reference Example 86 | 0.0055 |
| Reference Example 37 | 0.006 | Reference Example 97 | 0.0031 |
| Reference Example 38 | 0.014 | Reference Example 98 | 0.0073 |
| Reference Example 39 | 0.0078 | Reference Example 120 | 0.035 |
| Reference Example 40 | 0.019 | Reference Example 122 | 0.0088 |
| Reference Example 43 | 0.0027 | Reference Example 123 | 0.097 |
| Reference Example 55 | 0.0022 | Reference Example 129 | 0.0021 |
| Reference Example 60 | 0.0051 | Reference Example 130 | 0.0032 |
| Reference Example 61 | 0.003 | Reference Example 132 | 0.0018 |
| Reference Example 64 | 0.0058 | Comp. Example | 0.089 |

These results indicate that the compounds inhibited the growth of human leukemia CCRF-CEM cell lines.

Test Example 3: Cell growth inhibition 2

The ability of test compounds to inhibit cell growth was determined as in Test Example 2, except that the cell lines, the culture plate, and the number of cells seeded per well were as shown in Table 18. For the culture plate, a 384-well plate (#3571, produced by Corning) was used.

Table 19 below shows the results.

Table 18

| Cell Name | Origin | Obtained from | Plate | Seeding number (per 1 well) (cells/μL medium) |
|---|---|---|---|---|
| HCT116 | Colon cancer | ATCC | 384 wells | 250 cells/20 μL |
| Capan-1 | Pancreatic cancer | ATCC | 384 wells | 500 cells/20 μL |
| A-427 | Lung cancer | ATCC | 384 wells | 250 cells/20 μL |
| MDA-MB-453 | Breast cancer | ATCC | 384 wells | 500 cells/20 μL |

(continued)

| Cell Name | Origin | Obtained from | Plate | Seeding number (per 1 well) (cells/μL medium) |
|---|---|---|---|---|
| LNCaP.FGC | Prostate cancer | Dainippon Pharmaceutical Co., Ltd. (currently Sumitomo Dainippon Pharma Co., Ltd.) | 384 wells | 500 cells/20 μL |
| SJCRH30 | Osteo- and soft tissue sarcoma | ATCC | 384 wells | 250 cells/20 μL |
| U266B1 | Multiple myeloma | ATCC | 384 wells | 3000 cells/20 μL |
| A-431 | Skin cancer | ATCC | 384 wels | 400 cells/20 μL |
| MV-4-11 | Acute leukemia | ATCC | 384 wells | 400 cells/20 μL |
| DB | B-cell lymphoma | ATCC | 384 wells | 1500 cells/20 μL |

Table 19

| | HCT116 | Capan-1 | A-427 | MDA-MB-453 | LNCaP.FGC |
|---|---|---|---|---|---|
| Reference Examples | IC50 (μM) | IC50 (μM) | IC50 (μM) | IC50 (μM) | IC50 (μM) |
| 1 | 0.016 | 0.73 | 0.022 | 0.46 | 0.65 |
| 25 | 0.0064 | 0.38 | 0.0054 | 0.48 | 0.5 |
| 55 | 0.012 | 0.87 | 0.007 | 0.094 | 0.26 |
| 130 | 0.012 | 0.21 | 0.0064 | 0.032 | 0.46 |
| 29 | 0.014 | 0.56 | 0.049 | 0.64 | 0.28 |
| 122 | 0.018 | 0.89 | 0.022 | 0.076 | 0.51 |
| 64 | 0.014 | 1.2 | 0.091 | 0.14 | 0.68 |
| Comp. Example | 0.089 | 1.5 | 0.18 | 1.9 | 0.41 |

| | SJCRH30 | U266B1 | A-431 | MV-4-11 | DB |
|---|---|---|---|---|---|
| Reference Examples | IC50 (μM) | IC50 (μM) | IC50 (μM) | IC50 (μM) | IC50 (μM) |
| 1 | >10 | 0.11 | 0.73 | 0.021 | 0.26 |
| 25 | 5.1 | 0.22 | 0.12 | 0.011 | 0.12 |
| 55 | 0.55 | 0.031 | 0.48 | 0.0067 | 0.061 |
| 130 | 0.17 | 0.024 | 0.16 | 0.025 | 0.13 |
| 29 | 6.6 | 0.42 | 0.56 | 0.02 | 0.15 |
| 122 | 0.13 | 0.27 | 1.6 | 0.038 | 0.12 |
| 64 | 0.56 | 0.11 | 0.71 | 0.032 | 0.11 |
| Comp. Example | >10 | 1.4 | 0.75 | 0.11 | 0.56 |

According to the above results, these compounds inhibited the growth of human colon cancer HCT116 cell line, human pancreatic cancer Capan-1 cell line, human lung cancer A-427 cell line, human breast cancer MDA-MB-453 cell line, human prostate cancer LNCAP.FGC cell line, human osteo- and soft tissue sarcoma SJCRH30 cell line, human multiple myeloma U266B1 cell line, human skin cancer A-431 cell line, human acute leukemia MV-4-11 cell line, and human diffuse large B-cell lymphoma DB cell line.

Example 1: Potentiation of antitumor effect of proteasome inhibitor

(1) Cell growth inhibition test

Table 20 shows the tumor cell lines and their culture medium used. All cell lines were obtained from ATCC, Sumitomo Dainippon Pharma Co., Ltd., or Health Science Research Resources Bank. TMD8 was obtained from Tokyo Medical and Dental University (Leuk Res.2006 Nov;30 (11):1385-90.).

[Table 20]

Table 20

| Cell Line | Medium | Number of Seeded Cells [cells/ 20 μL/well] |
|---|---|---|
| U266B1 | ATCC formulated RPMI-1640 Medium (containing 15% fetal bovine serum) | 3000 |
| MM.1S | ATCC formulated RPMI-1640 Medium (containing 10% fetal bovine serum) | 4000 |
| MOLT-4 | ATCC formulated RPMI-1640 Medium (containing 10% fetal bovine serum) | 250 |
| NCI-H1975 | ATCC formulated RPMI-1640 Medium (containing 10% fetal bovine serum) | 250 |
| SNU-16 | ATCC formulated RPMI-1640 Medium (containing 10% fetal bovine serum) | 400 |
| HOS | MEM medium (containing 2 mM L-glutamine, 0.1 mM NEAA, 1 mM Napyruvate, and 10% fetal bovine serum) | 250 |
| A549 | DMEM medium (containing 10% fetal bovine serum) | 250 |
| A431 | ATCC formulated DMEM medium (containing 10% fetal bovine serum) | 400 |
| TMD8 | αMEM medium (containing 10% fetal bovine serum) | 400 |
| VERC-RCW | MEM medium (containing 10% fetal bovine serum) | 400 |
| HCT116 | McCoy's 5A medium (containing 10% fetal bovine serum) | 250 |
| HL-60 | 1MDM medium (containing 20% fetal bovine serum) | 400 |
| MV4-11 | 1MDM medium (containing 10% fetal bovine serum) | 250 |

Cells were seeded in a 384-well culture plate (#3571, Corning Incorporated) at 20 μL/well under the following conditions. The cells seeded in the plate were cultured in an incubator set at 5% $CO_2$ and 37°C. On the following day the seeding, a novel pyrrolopyrimidine compound (Reference Example Compound 55) was adjusted to prepare medicinal solutions of 8 different concentrations in geometric progression (including 0 nM) by serial dilution with an RPMI-1640 medium, and other compounds having an antitumor effect were also adjusted to prepare medicinal solutions of 10 different concentrations in geometric progression (including 0 nM) by serial dilution with an RPMI-1640 medium. These were mixed to prepare 80 solutions of different compound concentrations. These mixed compounds were added to the plate at 5 μL/well, and further cultured at 37°C in 5% $CO_2$ for 3 days. After 3 days, CellTiter-Glo™ (Promaga) was added thereto at 25 μL/well and the chemiluminescence was measured with a plate reader.

(2) Analysis method

The potentiating effect of a combined use of drugs was evaluated by a known method (a method disclosed in Trends Pharmacol.Sci.4, 450-454, 1983, and Pharmacol Rev. 2006; 58 (3) : 621-81).

Specifically, cells treated with a single drug of the novel pyrrolopyrimidine compound or other compounds

having an antitumor effect were compared with control cells to determine a standardized cell survival rate T/C (Treatment/Control Ratio, %). From each of the determined T/C value, the half maximal inhibitory concentration ($IC_{50}$) of each drug was determined by a curve fitting regression analysis tool using a regression analysis tool XLfit 5.3 (IDBS Ltd.).

The ratio of $IC_{50}$ for each single drug ($IC_{50}$ ratio) was determined by equation I.

$IC_{50}$ Ratio = [$IC_{50}$ of Novel Pyrrolopyrimidine Compound]:[$IC_{50}$ of another compound having an antitumor effect] (Equation I)

The $IC_{50}$ ratio determined by calculation is not equal to the concentration ratio of the novel pyrrolopyrimidine compound to another compound having an antitumor effect that have been mixed and actually used in an experiment. Thus, of the mixed compounds having various concentration ratios actually used in experiments, a line of concentration ratios close to the $IC_{50}$ ratio was identified. From the T/C ratio of the line of the concentration ratios, a half maximal inhibitory concentration ($ED_{50}$) in use of both the pyrrolopyrimidine compound and another compounds having an antitumor effect was determined, and then, a combination index (CI) for that $ED_{50}$ was determined. The CI value was determined using a median effect analysis software CalcuSyn 2.0 (CalcuSyn, Inc.), which calculates the median-effect principle.

As details are shown in Table 21, the determined CI values were evaluated as follows: a CI value significantly exceeding 1 is antagonistic, a CI value substantially equal to 1 is additive, and a CI value significantly less than 1 is synergistic (Table 21). The comprehensive evaluation was made based on the result of lower potentiating action among the results from treatment with compounds mixed at a concentration ratio close to their $IC_{50}$ ratio.

[Table 21]

Table 21

| Details of Combination Index (CI) | |
|---|---|
| CI Range | Details (Determination) |
| <0.1 | Very strong synergism |
| 0.1-0.3 | Strong synergism |
| 0.3-0.7 | Synergism |
| 0.7-0.85 | Moderate synergism |
| 0.85-0.9 | Slight synergism |
| 0.9-1.10 | Additive |
| 1.10-1.20 | Slight antagonism |
| 1.20-1.45 | Moderate antagonism |
| 1.45-3.3 | Antagonism |
| 3.3-10 | Strong antagonism |
| >10 | Very strong antagonism |

(3) Combined use with a proteasome inhibitor

A cell growth inhibition test was performed using a novel pyrrolopyrimidine compound (Reference Example Compound 55) with highest concentrations, other compounds having an antitumor effect with highest doses, and tumor cell lines shown in Table 22. Table 23 shows the $IC_{50}$ ratios, CI values, and evaluation results.

The results indicated that the use of the novel pyrrolopyrimidine compound in combination with a proteasome inhibitor synergistically enhances the antitumor effect.

[Table 22]

Table 22

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Bortezomib | U266B1 | 5000 | 1000 |
| Carfilzomib | U266B1 | 5000 | 750 |
| Ixazomib | HL-60 | 300 | 1000 |

[Table 23]

Table 23

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Bortezomib | 0.05 | 1:0.02<br>1:0.07 | 0.56<br>0.51 | Synergism |
| Carfilzomib | 0.30 | 1:015<br>1:045 | 0.64<br>0.66 | Synergism |
| Ixazomib | 19 | 1:10<br>1:30 | 0.82<br>0.94 | Additive |

Example 2: Potentiation of antitumor effect of tyrosine kinase inhibitor

In the same manner as in Example 1, a cell growth inhibition test was performed using the novel pyrrolopyrimidine compound with highest concentrations, other compounds having an antitumor effect with highest doses, and tumor cell lines shown in Table 24. Table 25 shows the $IC_{50}$ ratios, CI values, and evaluation results. In the following Examples 2 to 15, Reference Example Compound 55 was used as a novel pyrrolopyrimidine compound as in Example 1.

The results indicate that the use of the novel pyrrolopyrimidine compound in combination with an ALK inhibitor, a HER family inhibitor (an EGFR inhibitor and HER2 inhibitor), a CBR-ABL inhibitor, an FLT3 inhibitor, a VEGFR inhibitor, a c-kit inhibitor, a Janus kinase inhibitor, or a multi-kinase inhibitor additively or synergistically enhances the antitumor effect.

[Table 24]

Table 24

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Alectinib | MM.1S | 100 | 100000 |
| Crizotinib | MM.1S | 100 | 100000 |
| Afatinib | A549 | 10000 | 50000 |
| Erlotinib | A549 | 10000 | 100000 |

(continued)

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Gefitinib | A431 | 10000 | 50000 |
| Osimertinib | NCI-H1975 | 100000 | 10000 |
| Lapatinib | A431 | 10000 | 100000 |
| ARRY380 | A431 | 10000 | 40000 |
| Dasatinib | HCT116 | 1000 | 100000 |
| Imatinib | A549 | 10000 | 100000 |
| Quizartinib | MOLT-4 | 300 | 100000 |
| Gilteritinib | MOLT-4 | 500 | 10000 |
| Sunitinib | SNU-16 | 10000 | 100000 |
| Regorafenib | SNU-16 | 10000 | 50000 |
| Lenvatinib | SNU-16 | 10000 | 100000 |
| Pazopanib | SNU-16 | 10000 | 100000 |
| Crenolanib | MV4-11 | 300 | 10000 |
| Masitinib | MOLT-4 | 300 | 10000 |
| Ponatinib | MOLT-4 | 300 | 10000 |
| Ruxolitinib | MV4-11 | 300 | 10000 |

[Table 25]

Table 25

| Other Compounds Having an Antitumor Effect | $IC_{50}$ Ratio | Novel Pyrrolopyrimidine Compound: Other Compounds Having an Antitumor Effect | CI | Comprehensive Evaluation |
|---|---|---|---|---|
| Alectinib | 4592 | 1:3000<br>1:9000 | 0.58<br>0.59 | Synergism |
| Crizotinib | 94 | 1:37<br>1:111 | 0.87<br>0.74 | Slight synergism |
| Afatinib | 3 | 1:1.7<br>1:5 | 0.70<br>0.48 | Synergism |
| Erlotinib | >110 | 1:10<br>1:30 | 0.54<br>0.45 | Synergism |
| Gefitinib | 11 | 1:5<br>1:15 | 0.33<br>0.32 | Synergism |
| Osimertinib | 0.02 | 1:0.01<br>1:0.03 | 0.96<br>0.99 | Additive |
| Lapatinib | 46 | 1:10<br>1:30 | 0.15<br>0.19 | Strong synergism |
| ARRY380 | 65 | 1:36 | 0.34 | Synergism |

(continued)

| Other Compounds Having an Antitumor Effect | $IC_{50}$ Ratio | Novel Pyrrolopyrimidine Compound: Other Compounds Having an Antitumor Effect | CI | Comprehensive Evaluation |
|---|---|---|---|---|
| Dasatinib | 298 | 1:300<br>1:900 | 0.83<br>0.96 | Additive |
| Imatinib | 23 | 1:10<br>1:30 | 0.31<br>0.35 | Synergism |
| Quizartinib | 286 | 1:111<br>1:333 | 0.39<br>0.44 | Synergism |
| Gilteritinib | 33 | 1:20<br>1:60 | 0.96<br>0.88 | Additive |
| Sunitinib | 5 | 1:3.3<br>1:10 | 0.86<br>0.91 | Additive |
| Regorafenib | 17 | 1:15<br>1:45 | 0.88<br>1.06 | Additive |
| Lenvatinib | 4 | 1:3.3<br>1:10 | 0.44<br>0.63 | Synergism |
| Pazopanib | 91 | 1:30<br>1:90 | 0.71<br>0.52 | Slight synergism |

[Table 26]

| | | | | |
|---|---|---|---|---|
| Crenolanib | 4 | 1:3.7<br>1:11 | 0.91<br>0.83 | Additive |
| Masitinib | 70 | 1:33<br>1:100 | 0.31<br>0.20 | Synergism |
| Ponatinib | 11 | 1:3.7<br>1:11 | 0.46<br>0.36 | Synergism |
| Ruxolitinib | 483 | 1:300 | 0.82 | Moderate synergism |
| * For ARRY380, the CI value was calculated only at 1:36.<br>* For Ruxolitinib, the CI value was calculated only at 1:300. | | | | |

Example 3: Potentiation of antitumor effect of serine-threonine kinase inhibitor

A cell growth inhibition test was performed in the same manner as in Example 1 except for the use of the novel pyrrolopyrimidine compound with highest concentrations, other compounds having an antitumor effect with highest doses, and tumor cell lines shown in Table 26. Table 27 shows the $IC_{50}$ ratios, CI values, and evaluation results.

The results indicate that the use of the novel pyrrolopyrimidine compound in combination with a PI3K inhibitor, an Akt inhibitor, a mTOR inhibitor, or a PLK inhibitor synergistically enhances the antitumor effect.

[Table 27]

Table 26

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Everolimus | VMRC-RCW | 5000 | 10000 |
| Rapamycin | A549 | 10000 | 1000 |
| AZD5363 | VMRC-RCW | 5000 | 100000 |
| MK2206 | A549 | 10000 | 10000 |
| Idelalisib | TMD-8 | 300 | 10000 |
| Duvelisib | TMD-8 | 300 | 10000 |
| Volasertib | HL-60 | 300 | 10000 |

[Table 28]

Table 27

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Everolimus | 6 | 1:2<br>1:6 | 0.32<br>0.37 | Synergism |
| Rapamycin | 0.1 | 1:0.03<br>1:0.1 | 0.62<br>0.56 | Synergism |
| AZD5363 | 67 | 1:60<br>1:180 | 0.33<br>0.38 | Synergism |
| MK2206 | 5 | 1:3<br>1:9 | 0.31<br>0.35 | Synergism |
| Idelalisib | 23 | 1:11<br>1:33 | 0.57<br>0.64 | Synergism |
| Duvelisib | 2 | 1:1.2<br>1:3.7 | 0.42<br>0.43 | Synergism |
| Volasertib | 14 | 1:11<br>1:33 | 0.95<br>0.82 | Additive |
| Duvelisib | 2 | 1:1.2<br>1:3.7 | 0.42<br>0.43 | Synergism |
| Volasertib | 14 | 1:11<br>1:33 | 0.95<br>0.82 | Additive |

Example 4: Potentiation of antitumor effect of PARP inhibitor

A cell growth inhibition test was performed in the same manner as in Example 1 except for the use of the novel pyrrolopyrimidine compound with a highest concentration, another compound having an antitumor effect with highest dose, and a tumor cell line shown in Table 28. Table 29 shows the $IC_{50}$ ratio, CI value, and evaluation result.

The result indicates that the use of the novel pyrrolopyrimidine compound in combination with a PARP inhibitor synergistically enhances the antitumor effect.

[Table 29]

Table 28

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Olaparib | A549 | 10000 | 100000 |

[Table 30]

Table 29

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Olaparib | 21 | 1:10<br>1:30 | 0.61<br>0.69 | Synergism |

Example 5: Potentiation of antitumor effect of steroid

A cell growth inhibition test was performed in the same manner as in Example 1 except for the use of the novel pyrrolopyrimidine compound with highest concentrations, other compounds having an antitumor effect with highest doses shown, and tumor cell lines shown in Table 30. Table 31 shows the $IC_{50}$ ratios, CI values, and evaluation results.

The results indicate that the use of the novel pyrrolopyrimidine compound in combination with steroid synergistically enhances the antitumor effect.

[Table 31]

Table 30

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Prednisolone | MM.1S | 1000 | 10000 |
| Dexamethasone | MM.1S | 1000 | 1000 |

[Table 32]

Table 31

| Other Compounds Having an Antitumor Effect | $IC_{50}$ Ratio | Novel Pyrrolopyrimidine Compound: Another Compound Having an Antitumor Effect | CI | Comprehensive Evaluation |
|---|---|---|---|---|
| Prednisolone | 8.3 | 1:3.3 1:10 | 0.54<br>0.44 | Synergism |
| Dexamethasone | 0.4 | 1:0.3<br>1:1 | 0.51<br>0.39 | Synergism |

Example 6: Potentiation of antitumor effect of immunomodulator

A cell growth inhibition test was performed in the same manner as in Example 1 except for the use of the novel pyrrolopyrimidine compound with highest concentrations, other compounds having an antitumor effect with highest doses, and tumor cell lines shown in Table 32. Table 33 shows the $IC_{50}$ ratios, CI values, and evaluation results.
The results indicate that the use of the novel pyrrolopyrimidine compound in combination with an immunomodulator synergistically enhances the antitumor effect.

[Table 33]

Table 32

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Lenalidomide | MM.1S | 1000 | 10000 |
| Pomalidomide | MM.1S | 1000 | 1000 |
| Thalidomide | MM.1S | 1000 | 100000 |

[Table 34]

Table 33

| Other Compounds Having an Antitumor Effect | $IC_{50}$ Ratio | Novel Pyrrolopyrimidine Compound: Other Compounds Having an Antitumor Effect | CI | Comprehensive Evaluation |
|---|---|---|---|---|
| Lenalidomide | 14 | 1:10<br>1:30 | 0.75<br>0.69 | Moderate synergism |
| Pomalidomide | 3 | 1:1<br>1:3 | 0.81<br>0.66 | Moderate synergism |
| Thalidomide | 6603 | 1:900 | 0.63 | Synergism |
| * For thalidomide, the CI value was calculated only at 1:900. | | | | |

Example 7: Potentiation of antitumor effect of Exportin-1 Inhibitor

A cell growth inhibition test was performed in the same manner as in Example 1 except for the use of the novel pyrrolopyrimidine compound with a highest concentration, another compound having an antitumor effect with a highest dose, and a tumor cell line shown in Table 34. Table 35 shows the $IC_{50}$ ratio, CI value, and evaluation result.
The result indicates that the use of the novel pyrrolopyrimidine compound in combination with an exportin-1 inhibitor synergistically enhances the antitumor effect.

[Table 35]

Table 34

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| KPT-330 | HCT116 | 300 | 10000 |

[Table 36]

Table 35

| Other Compounds Having an Antitumor Effect | $IC_{50}$ Ratio | Novel Pyrrolopyrimidine Compound: Other Compounds Having an Antitumor Effect | CI | Comprehensive Evaluation |
|---|---|---|---|---|
| KPT-330 | 21 | 1:11<br>1:33 | 0.69<br>0.75 | Moderate synergism |

Example 8: Potentiation of antitumor effect of BTK Inhibitor

A cell growth inhibition test was performed in the same manner as in Example 1 except for the use of the novel pyrrolopyrimidine compound with a highest concentration, another compound having an antitumor effect with a highest dose, and a tumor cell line shown in Table 36. Table 37 shows the $IC_{50}$ ratio, CI value, and evaluation result.
The result indicates that the use of the novel pyrrolopyrimidine compound in combination with a BTK inhibitor synergistically enhances the antitumor effect.

[Table 37]

Table 36

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Ibrutinib | MV-4-11 | 100 | 100000 |

[Table 38]

Table 37

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Ibrutinib | 140 | 1:111<br>1:333 | 0.70<br>0.72 | Moderate synergism |

Example 9: Potentiation of antitumor effect of BCL-2 inhibitor

A cell growth inhibition test was performed in the same manner as in Example 1 except for the use of the novel pyrrolopyrimidine compound with a highest concentration, another compound having an antitumor effect with highest dose, and a tumor cell line shown in Table 38. Table 39 shows the $IC_{50}$ ratio, CI value, and evaluation result.
The result indicates that the use of the novel pyrrolopyrimidine compound in combination with a BCL-2 inhibitor synergistically enhances the antitumor effect.

[Table 39]

Table 38

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| ABT-199 | MOLT-4 | 500 | 10000 |

[Table 40]

Table 39

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| ABT-199 | 119 | 1:60<br>1:180 | 0.37<br>0.40 | Synergism |

Example 10: Potentiation of antitumor effect of HDAC inhibitor

A cell growth inhibition test was performed in the same manner as in Example 1 except for the use of the novel pyrrolopyrimidine compound with highest concentrations, other compounds having an antitumor effect with highest doses, and tumor cell lines shown in Table 40. Table 41 shows the $IC_{50}$ ratios, CI values, and evaluation results.

The results indicate that the use of the novel pyrrolopyrimidine compound in combination with a HDAC inhibitor additively enhances the antitumor effect.

[Table 41]

Table 40

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Panobinostat | U266B1 | 5000 | 10000 |
| Vorinostat | U266B1 | 5000 | 10000 |

[Table 42]

Table 41

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Panobinostat | 0.08 | 1:0.07<br>1:0.22 | 0.90<br>0.61 | Additive |
| Vorinostat | 4.4 | 1:2<br>1:6 | 0.91<br>0.88 | Additive |

Example 11: Potentiation of antitumor effect of antimetabolite

A cell growth inhibition test was performed in the same manner as in Example 1 except for the use of the novel

pyrrolopyrimidine compound with highest concentrations, other compounds having an antitumor effect with highest doses, and tumor cell lines shown in Table 42. Table 43 shows the $IC_{50}$ ratios, CI values, and evaluation results.

The results indicate that the use of the novel pyrrolopyrimidine compound in combination with an antimetabolite additively or synergistically enhances the antitumor effect.

[Table 43]

Table 42

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Fluorouracil | A431 | 10000 | 50000 |
| Gemcitabine | A431 | 10000 | 200 |
| Cytarabine | HCT116 | 1000 | 10000 |
| 6-Mercaptopurine | MM.1S | 100 | 10000 |
| Pemetrexed | HCT116 | 1000 | 50000 |
| Trifluridine | A431 | 10000 | 10000 |

[Table 44]

Table 43

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Fluorouracil | 0.8 | 1:0.56<br>1:1.67 | 0.44<br>0.20 | Synergism |
| Gemcitabine | 0.01 | 1:0.007<br>1:0.02 | 0.70<br>0.49 | Synergism |
| Cytarabine | 48 | 1:30<br>1:90 | 0.52<br>0.66 | Synergism |
| 6-Mercaptopurine | 169 | 1:100<br>1:300 | 0.61<br>0.80 | Moderate synergism |
| Pemetrexed | 34 | 1:17<br>1:50 | 0.73<br>0.55 | Moderate synergism |
| Trifluridine | 0.5 | 1:0.33<br>1:1 | 1.06<br>0.74 | Additive |

Example 12: Potentiation of antitumor effect of platinum-based antitumor agent (platinum complex)

A cell growth inhibition test was performed in the same manner as in Example 1 except for the use of the novel pyrrolopyrimidine compound with highest concentrations, other compounds having an antitumor effect with highest doses, and tumor cell lines shown in Table 44. Table 45 shows the $IC_{50}$ ratios, CI values, and evaluation results.

The results indicate that the use of the novel pyrrolopyrimidine compound in combination with a platinum-based antitumor agent (platinum complex) synergistically enhances the antitumor effect.

[Table 45]

Table 44

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Cisplatin | HCT116 | 1000 | 10000 |
| Carboplatin | A549 | 10000 | 500000 |
| Oxaliplatin | HL-60 | 500 | 50000 |

[Table 46]

Table 45

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Cisplatin | 343 | 1:333<br>1:1000 | 0.44<br>0.72 | Moderate synergism |
| Carboplatin | 38 | 1:17<br>1:50 | 0.41<br>0.87 | Slight synergism |
| Oxaliplatin | 132 | 1:100<br>1:300 | 0.65<br>0.70 | Synergism |

Example 13: Potentiation of antitumor effect of microtubule inhibitor

A cell growth inhibition test was performed in the same manner as in Example 1 except for the use of the novel pyrrolopyrimidine compound with highest concentrations, other compounds having an antitumor effect with highest doses, and tumor cell lines shown in Table 46. Table 47 shows the $IC_{50}$ ratios, CI values, and evaluation results.

The results indicate that the use of the novel pyrrolopyrimidine compound in combination with a microtubule inhibitor additively enhances the antitumor effect.

[Table 47]

Table 46

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Eribulin | A549 | 10000 | 1000 |
| Paclitaxel | A549 | 10000 | 5000 |

[Table 48]

Table 47

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Eribulin | 0.01 | 1:0.01<br>1:0.03 | 1.02<br>0.89 | Additive |
| Paclitaxel | 0.04 | 1:0.02<br>1:0.06 | 0.98<br>0.77 | Additive |

Example 14: Potentiation of antitumor effect of alkylating agent

A cell growth inhibition test was performed in the same manner as in Example 1 except for the use of the novel pyrrolopyrimidine compound with highest concentrations, other compounds having an antitumor effect with highest doses, and tumor cell lines shown in Table 48. Table 49 shows the $IC_{50}$ ratios, CI values, and evaluation results.

The results indicate that the use of the novel pyrrolopyrimidine compound in combination with an alkylating agent additively or synergistically enhances the antitumor effect.

[Table 49]

Table 48

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Trabectedin | HOS | 1000 | 10000 |
| Ifosfamide | MV4-11 | 100 | 100000 |
| Dacarbazine | MV4-11 | 100 | 100000 |

[Table 50]

Table 49

| Other Compounds Having an Antitumor Effect | $IC_{50}$ Ratio | Novel Pyrrolopyrimidine Compound: Another Compound Having an Antitumor Effect | CI | Comprehensive Evaluation |
|---|---|---|---|---|
| Trabectedin | 0.2 | 1:0.1<br>1:0.3 | 0.83<br>1.01 | Additive |
| Ifosfamide | 6315 | 1:3000<br>1:9000 | 0.57<br>0.60 | Synergism |
| Dacarbazine | 6582 | 1:3000<br>1:9000 | 0.92<br>0.95 | Additive |

Example 15: Potentiation of antitumor effect of anthracycline-based antitumor agent

A cell growth inhibition test was performed in the same manner as in Example 1 except for the use of the novel pyrrolopyrimidine compound with highest concentrations, other compounds having an antitumor effect with highest doses, and tumor cell lines shown in Table 50. Table 51 shows the $IC_{50}$ ratios, CI values, and evaluation results.

The results indicate that the use of the novel pyrrolopyrimidine compound in combination with an anthracycline-based antitumor agent synergistically enhances the antitumor effect.

[Table 51]

Table 50

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| Doxorubicin | HOS | 10000 | 50000 |
| Pixantrone | MM.1S | 100 | 10000 |

[Table 52]

Table 51

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| Doxorubicin | 2 | 1:1.7<br>1:5 | 0.77<br>0.61 | Moderate synergism |
| Pixantrone | 8.4 | 1:3.7<br>1:11 | 0.73<br>0.84 | Moderate synergism |

Example 16: Potentiation of tumor growth inhibitory effect on tumor cells transplanted into mice by combinational treatment with bortezomib

Human multiple myeloma (MM) KMS-26 cell lines were washed in PBS and suspended in 50% PBS and 50% Martigel basement membrane matrix (#356237, Corning Incorporated) to give a concentration of $1\times10^8$ cells/mL. 0.1 mL of this cell suspension was subcutaneously implanted into the right chest of 6-week-old CB17/Icr-Prkdc$^{scid}$/CrlCrlj mice (Charles River, Japan) .

The length and the width of the implanted tumor and the body weight were measured. The tumor volume (TV) was calculated using the following equation (equation C). The unit for the length and width is mm.

$$TV\ (mm^3) = \{(length) \times (width)^2\}/2 \quad (equation\ C)$$

When the TV reached 100 to 310 $mm^3$, the animals were assigned to different groups in accordance with a stratified randomization procedure. This day was determined to be day 1, the TV on this day was determined to be TV1, and the body weight on this day was determined to be BW1. The test ended on day 15.

A necessary amount of the test compound was weighed to prepare a solution to administer to the mice. Mice with tumors (6 mice/group) were intravenously administered with hydrochloride of Reference Example Compound 55 in an amount of 100 mg/kg/day (on a free-base dose basis) once a week (day 1 and 8) or twice a week (day 1, 4, 8, and 11) or intravenously administered with bortezomib in an amount of 1 mg/kg/day twice a week (day 1, 4, 8, and 11). A group for combinational treatment was administered with a combination of the reference example compound and bortezomib, and whether the tumor growth inhibitory effect was increased by the combinational treatment was evaluated. The control group was administered with the vehicle for Reference Example Compound 55.

Thereafter, the length and the width of the tumor and the body weight (BWn) on day n were measured over time in the same manner, and the TVn of each mouse was calculated. Further, the relative tumor volume (RTVn) was calculated based on the TVn of each mouse using the following equation (equation D); the treatment/control (T/Cn) value (%) on day n was calculated based on the mean RTVn of each drug administration group using the following equation (equation E); and the body weight change rate (BWCn) was calculated using the following equation (equation F).

$$RTVn = TVn/TV1 \quad (equation\ D)$$

T/Cn(%) = (mean RTV of each drug administration group on day n)/(mean RTV of the control group on day n) × 100 (equation E)

BWCn (%) = (BWn-BW1)/BW1×100 (equation F)

An RTV15 comparison using Dunnett's test revealed that the RTV of the group administered with the reference example compound was significantly lower than that of the control group. Further, a comparison using Aspin-Welch's t-test revealed that the RTV15 of the group treated with the combination of the reference example compound in an amount of 100 mg/kg/day twice a week and bortezomib in an amount of 1 mg/kg/day twice a week was significantly lower than the RTV15 of the groups treated with their respective single drugs. Fig. 1 shows the changes in RTV of this test.

Moreover, the RTV 15 of the group treated with the combination of the reference example compound in an amount of 100 mg/kg/day once a week and bortezomib in an amount of 1 mg/kg/day twice a week was also significantly lower than the RTV15 of the groups treated with their respective single drugs. Table 52 shows the results.

The change in average body weight of the group of combinational treatment on the last day of the test showed no enhanced toxicity compared with the groups treated with their respective single drugs.

The results indicated that the use of the reference example compound in combination enhances the tumor growth inhibitory effect of bortezomib.

[Table 53]

Table 52

| Group | Dose (mg/kg/day) | Administration Day | Number of Mice | RTV15 Mean±SD | T/C15 (%) | BWC15 (%) Mean±SD |
|---|---|---|---|---|---|---|
| Control | - | 1, 8 | 6 | 10.4±5.0 | 100 | 4.4±12.8 |
| Bortezomib | 1 | 1, 4, 8, 11 | 6 | 6.9±3.2 | 66 | 1.3±3.2 |
| Reference Example Compound | 100 | 1, 8 | 6 | 4.3±1.0 | 42 | 2.3±3.3 |
| Reference Example Compound | 100 | 1, 4, 8, 11 | 6 | 3.1±0.8 | 30 | 6.0±4.4 |
| Bortezomib /Reference Example Compound | 1/100 | 1, 4, 8, 11/1, 8 | 6 | 2.0±0.6 | 20 | 2.0±6.2 |
| Bortezomib /Reference Example Compound | 1/100 | 1, 4, 8, 11/1, 4, 8, 11 | 6 | 0.6±0.2 | 6 | -5.8±2.6 |

Example 17: Potentiation of tumor growth inhibitory effect on tumor cells transplanted into mice by combinational treatment with gilteritinib

In accordance with the disclosure of Example 16, human acute myelogenous leukemia (AML) MV-4-11 cell lines were transplanted into 6-week old BALB/cAJcl-nu/nu mice (CLEA Japan,Inc.). When the TV reached 80 to 220 $mm^3$, the animals were assigned to different groups in accordance with a stratified randomization procedure. This day was determined to be day 1, the TV on this day was determined to be TV1, and the body weight on this day was determined to be BW1. The test ended on day 15.

Mice with tumors were intravenously administered with hydrochloride of Reference Example Compound 55 in an amount of 50 mg/kg/day or 100 mg/kg/day (both on a free-base dose basis) once a week (day 1 and 8) or orally administered with gilteritinib in an amount of 3 mg/kg/day daily. A group for combinational treatment was administered with a combination of the reference example compound and gilteritinib, and whether the tumor growth inhibitory effect on human tumors was increased by the combinational treatment with gilteritinib was evaluated. The control group was administered with the vehicle for the reference example compound.

An RTV15 comparison using Dunnett's test revealed that the RTV of the group administered with reference

example compound and the group administered with gilteritinib was significantly lower than that of the control group. Further, a comparison using Aspin-Welch's t-test revealed that the RTV15 of the group treated with the combination of the reference example compound in an amount of 50 mg/kg/day once a week and gilteritinib in an amount of 3 mg/kg/day daily was significantly lower than the RTV15 of the groups treated with their respective single drugs. Fig. 2 shows the changes in RTV of this test.

Moreover, the RTV 15 of the group treated with the combination of the reference example compound in an amount of 100 mg/kg/day once a week (day 1, 8) and gilteritinib in an amount of 3 mg/kg/day daily was also significantly lower than the RTV15 of the groups treated with their respective single drugs. Table 53 shows the results.

The change in average body weight of the group of combinational treatment on the last day of the test showed no enhanced toxicity compared with the groups treated with their respective single drugs.

The results indicated that the use of the reference example compound in combination enhances the tumor growth inhibitory effect of gilteritinib.

[Table 54]

Table 53

| Group | Dose (mg/kg/day) | Administration Day | Number of Mice | RTV15 Mean±SD | T/C15 (%) | BWC15 (%) Mean±SD |
|---|---|---|---|---|---|---|
| Control | - | 1, 8 | 6 | 4.5±0.8 | 100 | 9.8±3.0 |
| Gilteritinib | 3 | 1-14 | 6 | 1.9±0.5 | 41 | 5.8±3.0 |
| Reference Example Compound | 50 | 1, 8 | 6 | 3.3±0.7 | 74 | 8.1±3.8 |
| Reference Example Compound | 100 | 1, 8 | 6 | 2.0±0.5 | 46 | 9.1±2.2 |
| Gilteritinib /Reference Example Compound | 3/50 | 1-14/1, 8 | 6 | 0.9±0.4 | 21 | 9.6±3.4 |
| Gilteritinib /Reference Example Compound | 3/100 | 1-14/1, 8 | 6 | 0.8±0.3 | 18 | 6.2±1.0 |

Example 18: Potentiation of tumor growth inhibitory effect on tumor cells transplanted into mice by combinational treatment with doxorubicin

In accordance with the disclosure of Example 16, human clear cell sarcoma SU-CCS-1 cell lines were transplanted into 7-week old BALB/cAJcl-nu/nu mice (CLEA Japan,Inc.). When the TV reached 100 to 150 $mm^3$, the animals were assigned to different groups in accordance with a stratified randomization procedure. This day was determined to be day 0, the TV on this day was determined to be TV0, and the body weight on this day was determined to be BW0. The test ended on day 22.

Mice with tumors were intravenously administered with hydrochloride of Reference Example Compound 55 in an amount of 12.5 mg/kg/day or 25 mg/kg/day (both on a free-base dose basis) on day 1 and 8 or intravenously administered with doxorubicin hydrochloride in an amount of 8 mg/kg/day on day 1. A group for combinational treatment was administered with a combination of the reference example compound and doxorubicin hydrochloride, and whether the tumor growth inhibitory effect on human tumors was increased by the combinational treatment with doxorubicin hydrochloride was evaluated. The control group was untreated.

An RTV22 comparison using Dunnett's test revealed that the RTV of the group administered with the reference example compound and the group administered with doxorubicin hydrochloride was significantly lower than that of the control group. Further, a comparison using Aspin-Welch's t-test revealed that the RTV22 of the group treated with the combination of the reference example compound in an amount of 25 mg/kg/day on day 1 and 8 and doxorubicin hydrochloride in an amount of 8 mg/kg/day on day 1 was significantly lower than the RTV22 of the groups treated with their respective single drugs. Fig. 3 shows the changes in RTV of this test.

Moreover, the RTV22 of the group treated with the combination of the reference example compound in an amount of 12.5 mg/kg/day on day 1 and 8 and doxorubicin in an amount of 8 mg/kg/day on day 1 was also significantly

lower than the RTV22 of the groups treated with their respective single drugs. Table 54 shows the results.

The change in average body weight of the group of combinational treatment on the last day of the test showed no enhanced toxicity compared with the groups treated with their respective single drugs.

The results indicated that the use of the reference example compound in combination enhances the tumor growth inhibitory effect of doxorubicin.

[Table 55]

Table 54

| Group | Dose (mg/kg/day) | Administration Day | Number of Mice | RTV22 Mean+SD | T/C22 (%) | BWC22(%) Mean±SD |
|---|---|---|---|---|---|---|
| Control | - | - | 6 | 8.9±0.9 | 100 | 3.7±4.3 |
| Doxorubicin Hydrochloride | 8 | 1 | 6 | 6.6±0.8 | 74 | -0.3±2.6 |
| Reference Example Compound | 12.5 | 1, 8 | 6 | 3.7±0.8 | 42 | 5.0±5.3 |
| Reference Example Compound | 25 | 1, 8 | 6 | 2.2±0.4 | 25 | 4.1±4.1 |
| Doxorubicin Hydrochloride /Reference Example Compound | 8/12.5 | 1/1, 8 | 6 | 1.2±0.4 | 14 | -7.1±7.5 |
| Doxorubicin Hydrochloride /Reference Example Compound | 8/25 | 1/1, 8 | 6 | 0.6±0.2 | 7 | -9.6±6.9 |

Example 19: Potentiation of tumor growth inhibitory effect on tumor cells transplanted into mice by combinational treatment with rituximab 1

In accordance with the disclosure of Example 16, human diffuse large B-cell lymphoma (DLBCL) WILL-2 cell lines were transplanted into 6-week old CB17/Icr-Prkdc$^{scid}$/CrlCrlj mice (Charles River, Japan). When the TV reached 120 to 220 mm$^3$, the animals were assigned to different groups in accordance with a stratified randomization procedure. This day was determined to be day 1, the TV on this day was determined to be TV1, and the body weight on this day was determined to be BW1. The test ended on day 15.

Mice with tumors were intravenously administered with hydrochloride of Reference Example Compound 55 in an amount of 50 mg/kg/day or 100 mg/kg/day (both on a free-base dose basis) once a week (Day 1 and 8) or intravenously administered with rituximab in an amount of 10 mg/kg/day on day 1. A group for combinational treatment was administered with a combination of the reference example compound and rituximab, and whether the tumor growth inhibitory effect on human tumors was increased by the combinational treatment with rituximab was evaluated. The control group was administered with the vehicle for the reference example compound.

An RTV15 comparison using Dunnett's test revealed that the RTV of the group administered with the reference example compound and the group administered with rituximab was significantly lower than that of the control group. Further, a comparison using Aspin-Welch's t-test revealed that the RTV15 of the group treated with a combination of the reference example compound in an amount of 50 mg/kg/day once a week and rituximab in an amount of 10 mg/kg/day on day 1 was significantly lower than the RTV15 of the groups treated with their respective single drugs. Fig. 4 shows the changes in RTV of this test.

Moreover, the RTV15 of the group treated with the combination of the reference example compound in an amount of 100 mg/kg/day once a week and rituximab in an amount of 10 mg/kg/day on day 1 was also significantly lower than the RTV15 of the groups treated with their respective single drugs. Table 55 shows the results.

The change in average body weight of the group of combinational treatment on the last day of the test showed no enhanced toxicity compared with the groups treated with their respective single drugs.

The results indicated that the use of the reference example compound in combination enhances the tumor growth inhibitory effect of rituximab.

[Table 56]

Table 55

| Group | Dose (mg/kg/day) | Administration Day | Number of Mice | RTV15 Mean±SD | T/C15 (%) | BWC15(%) Mean±SD |
|---|---|---|---|---|---|---|
| Control | - | 1, 8 | 6 | 15.0±3.1 | 100 | 9.8±1.6 |
| Rituximab | 10 | 1 | 6 | 8.0±2.3 | 53 | 2.3±4.7 |
| Reference Example Compound | 50 | 1, 8 | 6 | 2.1±0.3 | 14 | 2.0±6.2 |
| Reference Example Compound | 100 | 1, 8 | 6 | 1.5±0.4 | 10 | 5.1±3.0 |
| Rituximab /Reference Example Compound | 1/50 | 1/1, 8 | 6 | 0.8±0.2 | 5 | 4.3±1.7 |
| Rituximab /Reference Example Compound | 1/100 | 1/1, 8 | 6 | 0.6±0.2 | 4 | 6.0±2.8 |

Example 20: Potentiation of tumor growth inhibitory effect on tumor cells transplanted into mice by combinational treatment with rituximab 2

In accordance with the disclosure of Example 16, human diffuse large B-cell lymphoma (DLBCL) OCI-LY18 cell lines were transplanted into 6-week old BALB/cAJcl-nu/nu mice (CLEA Japan, Inc.). When the TV reached 120 to 210 $mm^3$, the animals were assigned to different groups in accordance with a stratified randomization procedure. This day was determined to be day 1, the TV on this day was determined to be TV1, and the body weight on this day was determined to be BW1. The test ended on day 15.
Mice with tumors were intravenously administered with hydrochloride of Reference Example Compound 55 in an amount of 100 mg/kg/day (on a free-base dose basis) on day 1, 3, and 5 or twice a week (day 1, 4, 8, and 11) or intravenously administered or intravenously administered with rituximab in an amount of 10 mg/kg/day on day 1. A group for combinational treatment was administered with a combination of the reference example compound and rituximab, and whether the tumor growth inhibitory effect on human tumors was increased by the combinational treatment with rituximab was evaluated. The control group was administered with the vehicle for the reference example compound.
An RTV15 comparison using Dunnett's test revealed that the RTV of the group administered with the reference example compound and the group administered with rituximab was significantly lower than that of the control group. Further, a comparison using Aspin-Welch's t-test revealed that the RTV15 of the group treated with the combination of the reference example compound in an amount of 100 mg/kg/day on day 1, 3, and 5 and rituximab in an amount of 10 mg/kg/day on day 1 was significantly lower than the RTV15 of the groups treated with their respective single drugs. Fig. 5 shows the changes in RTV of this test.
Moreover, the RTV15 of the group treated with the combination of the reference example compound in an amount of 100 mg/kg/day twice a week and rituximab in an amount of 10 mg/kg/day on day 1 was also significantly lower than the RTV15 of the groups treated with their respective single drugs. Table 56 shows the results.
The change in average body weight of the group of combinational treatment on the last day of the test showed no enhanced toxicity compared with the groups treated with their respective single drugs.
The results indicated that the use of the reference example compound in combination intermediately enhances the tumor growth inhibitory effect of rituximab.

[Table 57]

Table 56

| Group | Dose (mg/kg/day) | Administration Day | Number of Mice | RTV15 Mean±SD | T/C15 (%) | BWC15 (%) Mean+SD |
|---|---|---|---|---|---|---|
| Control | - | 1, 4, 8, 11 | 6 | 10.9±2.8 | 100 | 12.2±2.6 |
| Rituximab | 10 | 1 | 6 | 5.2±1.8 | 48 | 9.8±4.2 |
| Reference Example Compound | 100 | 1, 3, 5 | 6 | 3.5±1.8 | 32 | 12.5±4.8 |
| Reference Example Compound | 100 | 1, 4, 8, 11 | 6 | 1.9±0.7 | 17 | 6.1±4.9 |
| Rituximab /Reference Example Compound | 1/100 | 1/1, 3, 5 | 6 | 0.6±0.2 | 5 | 9.0±3.0 |
| Rituximab /Reference Example Compound | 1/100 | 1/1, 4, 8, 11 | 6 | 0.4±0.1 | 4 | 0.7±3.5 |

Example 21: Potentiation of tumor growth inhibitory effect on tumor cells transplanted into mice by combinational treatment with azacytidine

In accordance with the disclosure of Example 16, human acute myelogenous leukemia (AML) THP-1 cell lines were transplanted into 7-weeks old BALB/cAJcl-nu/nu mice (CLEA Japan,Inc.). When the TV reached 90 to 180 $mm^3$, the animals were assigned to different groups in accordance with a stratified randomization procedure. This day was determined to be day 1, the TV on this day was determined to be TV1, and the body weight on this day was determined to be BW1. The test ended on day 22.

Mice with tumors were intravenously administered with hydrochloride of Reference Example Compound 55 in an amount of 12.5 mg/kg/day (on a free-base dose basis) twice a week (Day 1, 4, 8, 11, 15, and 18) or intraperitoneally administered with azacytidine in an amount of 20 mg/kg/day twice a week (day 1, 4, 8, 11, 15, and 18). A group for combinational treatment was administered with a combination of the reference example compound and azacytidine, and whether the tumor growth inhibitory effect on human tumors was increased by the combinational treatment with azacytidine was evaluated. The control group was administered with the vehicle for the reference example compound.

An RTV22 comparison using Dunnett's test revealed that the RTV of the group administered with the reference example compound in an amount of 12.5 mg/kg/day twice a week and the group administered with azacytidine in an amount of 20 mg/kg/day twice a week was significantly lower than that of the control group. Further, a comparison using Aspin-Welch's t-test revealed that the RTV22 of the group of combinational treatment was significantly lower than the RTV22 of the groups treated with their respective single drugs. Fig. 6 and Table 57 show the results of this test.

The change in average body weight of the group of combinational treatment on the last day of the test showed no enhanced toxicity compared with the groups treated with their respective single drugs.

The results indicated that the use of the reference example compound in combination intermediately enhances the tumor growth inhibitory effect of azacytidine.

[Table 58]

Table 57

| Group | Dose (mg/kg/day) | Administration Day | Number of Mice | RTV22 Mean±SD | T/C22 (%) | BWC22(%) Mean±SD |
|---|---|---|---|---|---|---|
| Control | - | 1, 4, 8, 11, 15, 18 | 6 | 19.6±6.6 | 100 | 17.2±3.9 |
| Azacytidine | 20 | 1, 4, 8, 11, 15, 18 | 6 | 8.8+2.0 | 45 | 3.6±4.5 |
| Reference Example Compound | 12.5 | 1, 4, 8, 11, 15, 18 | 6 | 7.1±2.3 | 36 | 12.7±2.3 |
| Azacytidine /Reference Example Compound | 20/12.5 | 1, 4, 8, 11, 15, 18/1, 4, 8, 11, 15, 18 | 6 | 1.7±0.5 | 8 | 4.4±5.6 |

Example 22: Potentiation of antitumor effect of IAP antagonist

A cell growth inhibition test was performed in the same manner as in Example 1 except for the use of the novel pyrrolopyrimidine compound with highest concentration, other compounds having an antitumor effect with highest concentration, and tumor cell lines shown in Table 58. Table 59 shows the $IC_{50}$ ratio, CI values, and evaluation results.

The result indicates that the use of the novel pyrrolopyrimidine compound in combination with an IAP antagonist synergistically enhances the antitumor effect.

[Table 59]

Table 58

| Other Compounds Having an Antitumor Effect | Tumor Cell Line | Novel Pyrrolopyrimidine Compound Highest Concentration (nM) | Other Compounds Having an Antitumor Effect Highest Concentration (nM) |
|---|---|---|---|
| GDC-0152 | HL-60 | 100 | 30000 |

[Table 60]

Table 59

| Other Compounds Having an Antitumor Effect | $IC_{50}$ Ratio | Novel Pyrrolopyrimidine Compound:Another Compound Having an Antitumor Effect | CI | Comprehensive Evaluation |
|---|---|---|---|---|
| GDC-0152 | 2122 | 1:90<br>1:270 | 0.45<br>0.62 | Synergism |

**Claims**

1. An antitumor effect potentiator for one or more other compounds having an antitumor effect, the antitumor effect potentiator comprising a compound represented by Formula (A) below or a salt thereof:

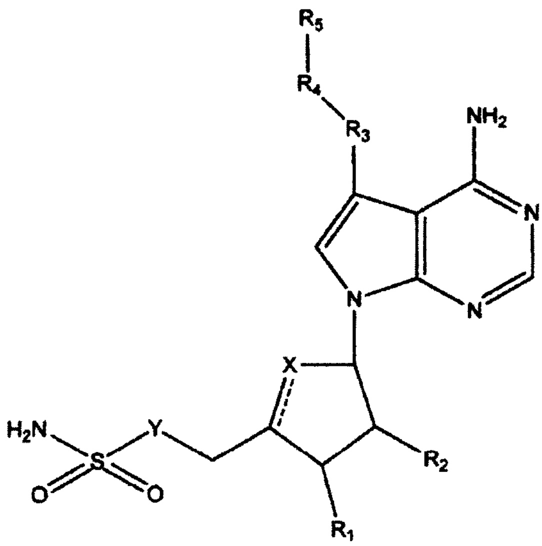

(A)

wherein:
------

is a single bond or a double bond;

X is -O-, $-CH_2-$, or -CH=;
Y is -NH- or -O-;
$R_1$ is hydrogen, fluorine, a hydroxy group, a cyano group, or an amino group;
$R_2$ is hydrogen, fluorine, a hydroxy group, a cyano group, or an amino group;
$R_3$ is a vinylene group, an ethynylene group, a C6-C14 arylene group, or a monocyclic or bicyclic heteroarylene group having at least one heteroatom selected from the group consisting of N, S, and O;
$R_4$ is a bond, a methylene group, or a C3-C7 cycloalkylidene group;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$;
$R_6$ is
halogen,
a hydroxy group,
a cyano group,
a C1-C6 alkyl group that may be substituted with one or more phenoxy groups,
a carbamoyl group,
a C1-C6 alkoxycarbonyl group,
a monocyclic or bicyclic unsaturated heterocycloalkyl group having at least one heteroatom selected from the group consisting of N, S, and O,
a monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group,
an amino group,
a mono- or di-(C1-C4 alkyl) amino group that may be substituted with one or more hydroxy groups or phenyl groups,
a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O,
a benzyloxy group that may be substituted with one or more carbamoyl groups,
a C1-C6 alkylthio group,
a C1-C6 alkylsulfonyl group, or
an aminosulfonyl group,
when a plurality of $R_6$ are present, the plurality of $R_6$ may be the same or different.

**2.** The antitumor effect potentiator according to claim 1, wherein in Formula (A),
$R_1$ is hydrogen, fluorine, or a hydroxy group;
$R_2$ is hydrogen, fluorine, or a hydroxy group; and
$R_3$ is an ethynylene group, or a monocyclic or bicyclic heteroarylene group having 1 to 4 of at least one kind of heteroatom selected from the group consisting of N, S, and O.

**3.** The antitumor effect potentiator according to claim 1 or 2, wherein in Formula (A),
$R_1$ is a hydroxy group;
$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$; and
$R_6$ is halogen; a hydroxy group; a cyano group; a C1-C6 alkyl group that may be substituted with one or more phenoxy groups; a carbamoyl group; a C1-C6 alkoxycarbonyl group; a monocyclic or bicyclic unsaturated heterocycloalkyl group having at least one heteroatom selected from the group consisting of N, S, and O; a monocyclic or bicyclic saturated heterocycloalkyl group that has at least one heteroatom selected from the group consisting of N, S, and O, and that may be substituted with one or more of halogen, hydroxy group, carboxyl group, or C1-C6 alkyl group; an amino group; a mono- or di-(C1-C4 alkyl) amino group that may be substituted with one or more hydroxy groups or phenyl groups; a C1-C6 alkoxy group that may be substituted with one or more of halogen, C3-C7 saturated cycloalkyl group, or monocyclic or bicyclic unsaturated heterocycloalkyl group having at least one

heteroatom selected from the group consisting of N, S, and O; a benzyloxy group that may be substituted with one or more carbamoyl groups; a C1-C4 alkylthio group; a C1-C4 alkylsulfonyl group; or an aminosulfonyl group (when a plurality of $R_6$ are present, the plurality of $R_6$ may be the same or different) .

**4.** The antitumor effect potentiator according to any one of claims 1 to 3, wherein, in Formula (A),
$R_1$ is a hydroxy group;
$R_2$ is hydrogen or a hydroxy group;
$R_3$ is an ethynylene group, or a monocyclic heteroarylene group having 2 of at least one kind of heteroatom selected from the group consisting of N, S, and O;
$R_5$ is a C3-C7 saturated cycloalkyl group that may have one or more $R_6$, a C6-C10 unsaturated cycloalkyl group that may have one or more $R_6$, or a monocyclic or bicyclic unsaturated heterocycloalkyl group that has at least one heteroatom selected from the group consisting of N, S, and O, and that may have one or more $R_6$; and
$R_6$ is fluorine; chlorine; a hydroxy group; a cyano group; a C1-C6 alkyl group that may be substituted with one or more phenoxy groups; a carbamoyl group; a C1-C6 alkoxycarbonyl group; a pyridinyl group that may have at least one member selected from the group consisting of halogen, hydroxy group and C1-C4 alkyl group as a substituent; an azetidinyl group; a hydroxyazetidinyl group; a thiomorpholinyl group; a dioxidothiomorpholinyl group; a methylpiperazinyl group; a hydroxypiperidinyl group; an oxopiperidinyl group; a piperidinyl group; a hydroxypyrrolidinyl group; an oxopyrrolidinyl group; a pyrrolidinyl group; a carboxylpyrrolidinyl group; a fluoropyrrolidinyl group; a morpholinyl group; a 9-oxa-3-azabicyclo[3.3.1]nonan-3-yl group; a 3-oxa-8-azabicyclo[3.2.1]octan-8-yl group; an amino group; a methylamino group; an ethylamino group; an isopropylamino group; a hydroxyethylamino group; a dimethylamino group; a phenylmethylamino group; a C1-C6 alkoxy group that may be substituted with one or more of halogen, C3-C7 saturated cycloalkyl group, or monocyclic or bicyclic unsaturated heterocycloalkyl group having at least one heteroatom selected from the group consisting of N, S, and O; a benzyloxy group that may be substituted with one or more carbamoyl groups; a C1-C4 alkylthio group; a C1-C4 alkylsulfonyl group; or an aminosulfonyl group (when a plurality of $R_6$ are present, the plurality of $R_6$ may be the same or different).

**5.** The antitumor effect potentiator according to any one of claims 1 to 4, wherein, in Formula (A),
$R_1$ is a hydroxy group;
$R_2$ is a hydroxy group;
$R_3$ is an ethynylene group;
$R_4$ is a bond;
$R_5$ is a C6-C10 unsaturated cycloalkyl group that may have one or more $R_6$; or a monocyclic or bicyclic unsaturated heterocycloalkyl group that has at least one heteroatom selected from the group consisting of N, S, and O, and that may have one or more $R_6$; and
$R_6$ is fluorine; chlorine; a hydroxy group; a cyano group; a methyl group; a 3-fluoropyrrolidinyl group; a morpholinyl group; a thiomorpholinyl group; a 3-hydroxyazetidinyl group; an azetidinyl group; an amino group; a N-methylamino group; a C1-C6 alkoxy group that may be substituted with one or more of either halogen or C3-C7 saturated cycloalkyl group; or a C1-C4 alkylthio group (when a plurality of $R_6$ are present, the plurality of $R_6$ may be the same or different).

**6.** The antitumor effect potentiator according to any one of claims 1 to 5, wherein in Formula (A),
Y is -NH-;
$R_1$ is a hydroxy group;
$R_2$ is a hydroxy group;
$R_3$ is an ethynylene group;
$R_4$ is a bond;
$R_5$ is a phenyl group or a naphthyl group that may have one or more $R_6$; or a monocyclic or bicyclic unsaturated heterocycloalkyl group that has at least one heteroatom selected from the group consisting of N, S, and O, and that may have one or more $R_6$;
$R_6$ is fluorine; a methyl group; 3-fluoropyrrolidinyl; 3-hydroxyazetidinyl; azetidinyl; an amino group; a N-methylamino group; a C1-C6 alkoxy group that may have one or more cyclopropyl groups; or a C1-C4 alkylthio group (when a plurality of $R_6$ are present, the plurality of $R_6$ may be the same or different) .

**7.** The antitumor effect potentiator according to any one of claims 1 to 6, wherein the compound represented by Formula (A) or a salt thereof is at least one member selected from the group consisting of:

4-amino-5-[2-(2,6-difluorophenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-(4-amino-2,6-difluoro-phenyl)ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-(methylamino)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[2,6-difluoro-4-[(3R)-3-fluoropyrrolidin-1-yl]phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-4,6-difluoro-phenyl)ethynyl]pyrrolo[2,3-d]pyrimidine;
4-amino-5-[2-[2,6-difluoro-4-(3-hydroxyazetidin-1-yl)phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-5-[2-[4-(azetidin-1-yl)-2,6-difluoro-phenyl]ethynyl]-7-[(2R,3R,4S,5R)-3,4-dihydroxy-5-[(sulfamoylamino)methyl]cyclopentyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-ethoxy-6-fluoro-phenyl)ethynyl]pyrrolo[2,3-d]pyrimidine;
4-amino-7-[(2R,3R,4S,5R)-3,4-dihydroxy-5-[(sulfamoylamino)methyl]tetrahydrofuran-2-yl]-5-[2-(2-fluoro-6-propoxy-phenyl)ethynyl]pyrrolo[2,3-d]pyrimidine;
8-[2-[4-amino-7-[(2R,3R,4S,5R)-3,4-dihydroxy-5-[(sulfamoylamino)methyl]tetrahydrofuran-2-yl]pyrrolo[2,3-d]pyrimidin-5-yl]ethynyl]-7-fluoro-4-methyl-2,3-dihydro-1,4-benzoxazine;
4-amino-7-[(2R,3R,4S,5R)-3,4-dihydroxy-5-[(sulfamoylamino)methyl]tetrahydrofuran-2-yl]-5-[2-(2-ethylsulfanyl-6-fluoro-phenyl)ethynyl]pyrrolo[2,3-d]pyrimidine;
4-amino-5-[2-[2-(cyclopropylmethoxy)-6-fluoro-phenyl]ethynyl]-7-[(2R,3R,4S,5R)-3,4-dihydroxy-5-[(sulfamoylamino)methyl]tetrahydrofuran-2-yl]pyrrolo[2,3-d]pyrimidine;
4-amino-7-[(1R,2S,3R,4R)-2,3-dihydroxy-4-[(sulfamoylamino)methyl]cyclopentyl]-5-[2-(2-fluoro-6-methylsulfanyl-phenyl)ethynyl]pyrrolo[2,3-d]pyrimidine;
8-[2-[4-amino-7-[(1R,2S,3R,4R)-2,3-dihydroxy-4-[(sulfamoylamino)methyl]cyclopentyl]pyrrolo[2,3-d]pyrimidin-5-yl]ethynyl]-7-fluoro-4-methyl-2,3-dihydro-1,4-benzoxazine;
4-amino-7-[(1R,4R,5S)-4,5-dihydroxy-3-[(sulfamoylamino)methyl]cyclopent-2-en-1-yl]-5-[2-(2-ethoxy-6-fluoro-phenyl)ethynyl]pyrrolo[2,3-d]pyrimidine; and
4-amino-7-[(1R,4R,5S)-4,5-dihydroxy-3-[(sulfamoylamino)methyl]cyclopent-2-en-1-yl]-5-[2-(2-fluoro-6-methylsulfanyl-phenyl)ethynyl]pyrrolo[2,3-d]pyrimidine; and salts of these compounds.

**8.** The antitumor effect potentiator according to any one of claims 1 to 7, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

**9.** The antitumor effect potentiator according to any one of claims 1 to 7, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

**10.** An antitumor agent comprising a combination of the compound represented by Formula (A) or a salt thereof according to any one of claims 1 to 7, and one or more other compounds having an antitumor effect.

**11.** The antitumor agent according to claim 10, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

**12.** The antitumor agent according to claim 10, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363,

MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

**13.** Use of the compound represented by Formula (A) or a salt thereof according to any one of claims 1 to 7 for potentiating the antitumor effect of one or more other compounds having an antitumor effect.

**14.** Use of the compound represented by Formula (A) or a salt thereof according to any one of claims 1 to 7 for producing an antitumor effect potentiator for one or more other compounds having an antitumor effect.

**15.** Use of the compound represented by Formula (A) or a salt thereof according to any one of claims 1 to 7 for producing an antitumor agent comprising a combination of the compound or a salt thereof (A) and one or more other compounds having an antitumor effect.

**16.** The use according to any one of claims 13 to 15, wherein the one or more other compounds having an antitumor effect are one or more members selected from the group consisting of kinase inhibitors, apoptosis inducers, nuclear receptor modulators, immunomodulators, nuclear export signal inhibitors, proteasome modulators, DNA-damaging agents, antimetabolites, platinum-based antitumor agents, microtubule inhibitors, alkylating agents, and anthracycline-based antitumor agents.

**17.** The use according to any one of claims 13 to 15, wherein the one or more other compounds having an antitumor effect are one or more members selected from the group consisting of bortezomib, carfilzomib, ixazomib, alectinib, crizotinib, afatinib, erlotinib, gefitinib, osimertinib, lapatinib, ARRY380, dasatinib, imatinib, quizartinib, gilteritinib, sunitinib, regorafenib, lenvatinib, pazopanib, crenolanib, masitinib, ponatinib, ruxolitinib, everolimus, rapamycin, AZD5363, MK2206, idelalisib, duvelisib, volasertib, olaparib, prednisolone, dexamethasone, lenalidomide, pomalidomide, thalidomide, KPT-330, ibrutinib, ABT-199, panobinostat, vorinostat, fluorouracil, gemcitabine, cytarabine, 6-mercaptopurine, pemetrexed, trifluridine, cisplatin, carboplatin, oxaliplatin, eribulin, paclitaxel, trabectedin, ifosfamide, dacarbazine, doxorubicin, pixantrone, rituximab, azacitidine, and GDC-0152.

**18.** A product as a combined preparation for use simultaneously, sequentially, or at one or more intervals upon prevention and/or treatment of a tumor, the product comprising the compound represented by Formula (A) or a salt thereof according to any one of claims 1 to 7 and one or more other compounds having an antitumor effect.

**19.** The compound represented by Formula (A) or a salt thereof according to any one of claims 1 to 7 for potentiating the antitumor effect of one or more other compounds having an antitumor effect.

**20.** A pharmaceutical composition comprising the compound represented by Formula (A) or a salt thereof according to any one of claims 1 to 7 and a pharmaceutical carrier, the pharmaceutical composition being for potentiating the antitumor effect of one or more other compounds having an antitumor effect.

**21.** An antitumor composition comprising the compound represented by Formula (A) or a salt thereof according to any one of claims 1 to 7, and one or more other compounds having an antitumor effect.

**22.** A method for treating a tumor, comprising administering to a patient the compound represented by Formula (A) or a salt thereof according to any one of claims 1 to 7, and one or more other compounds having an antitumor effect, in combination.

**23.** A method for potentiating the antitumor effect of one or more other compounds having an antitumor effect, the method comprising administering to a patient the compound represented by Formula (A) or a salt thereof according to any one of claims 1 to 7, and the one or more other compounds having an antitumor effect, in combination.

**24.** A combination of the compound represented by Formula (A) or a salt thereof according to any one of claims 1 to 7, and one or more other compounds having an antitumor effect, for tumor treatment.

Fig. 1

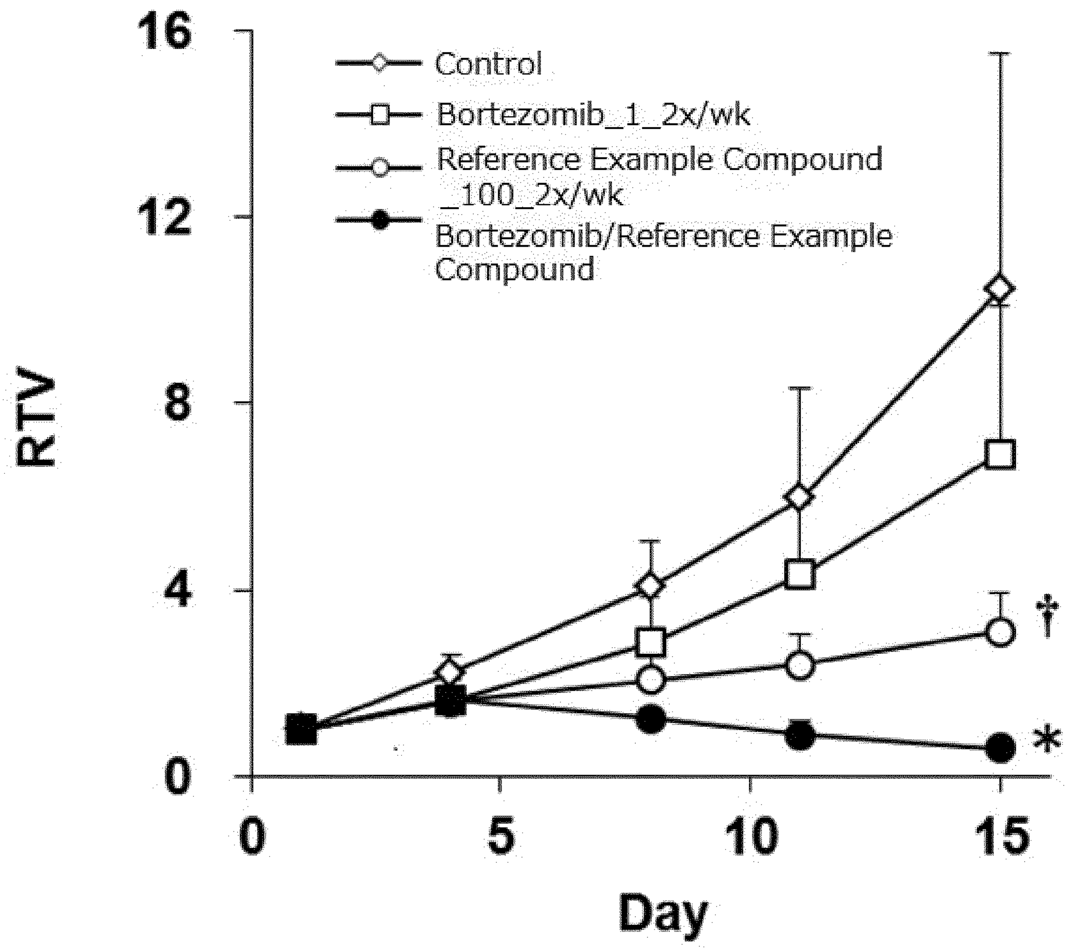


† P<0.05 vs. Control

* P<0.05 vs. Groups Administered with Single Drug

Fig. 2

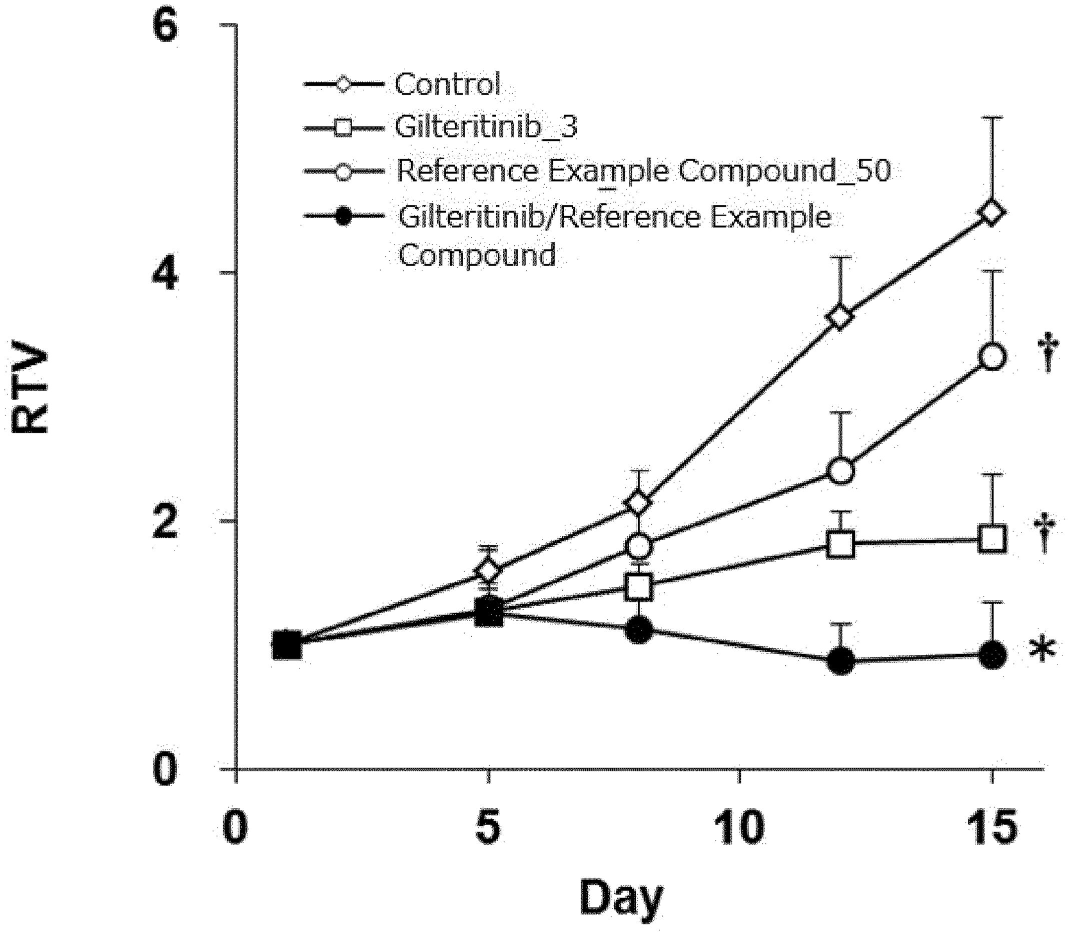


† P<0.05 vs. Control

* P<0.05 vs. Groups Administered with Single Drug

Fig. 3

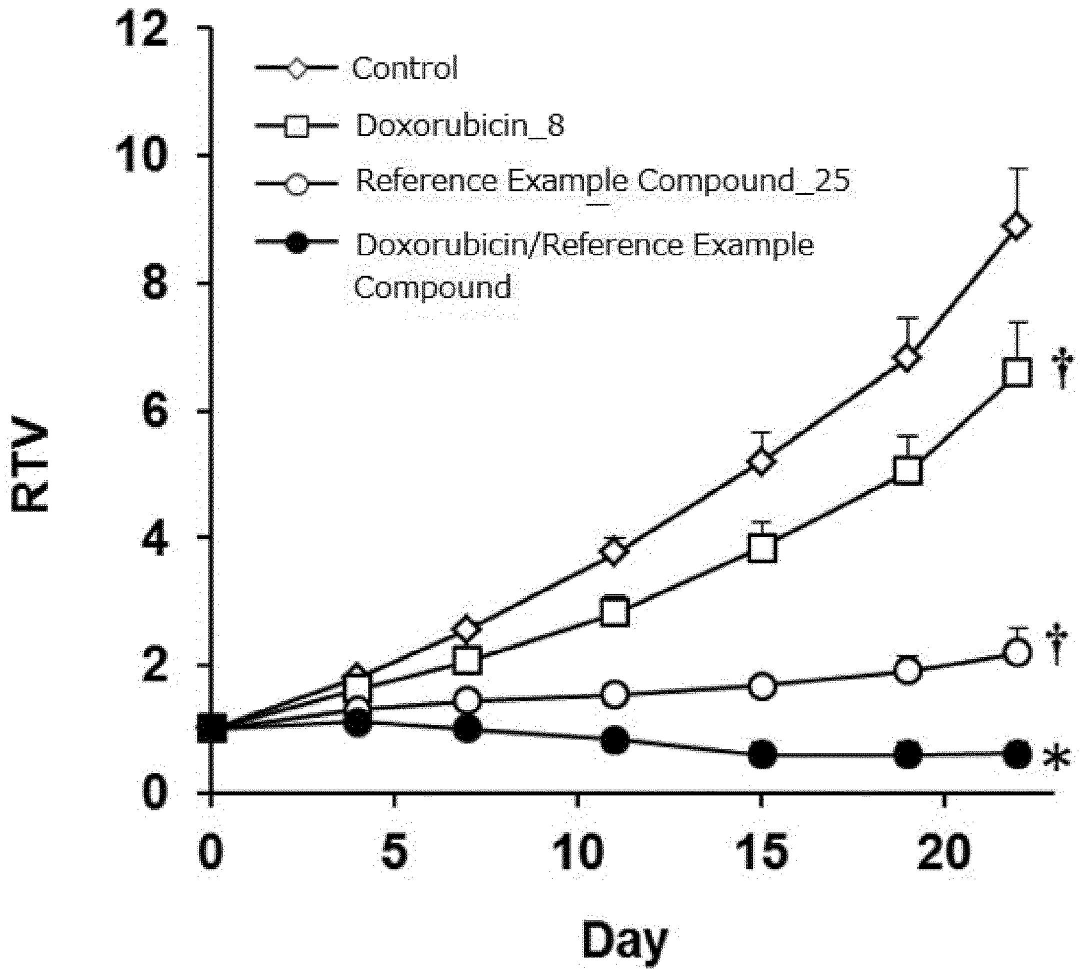


† P<0.05 vs. Control

* P<0.05 vs. Groups Administered with Single Drug

Fig. 4

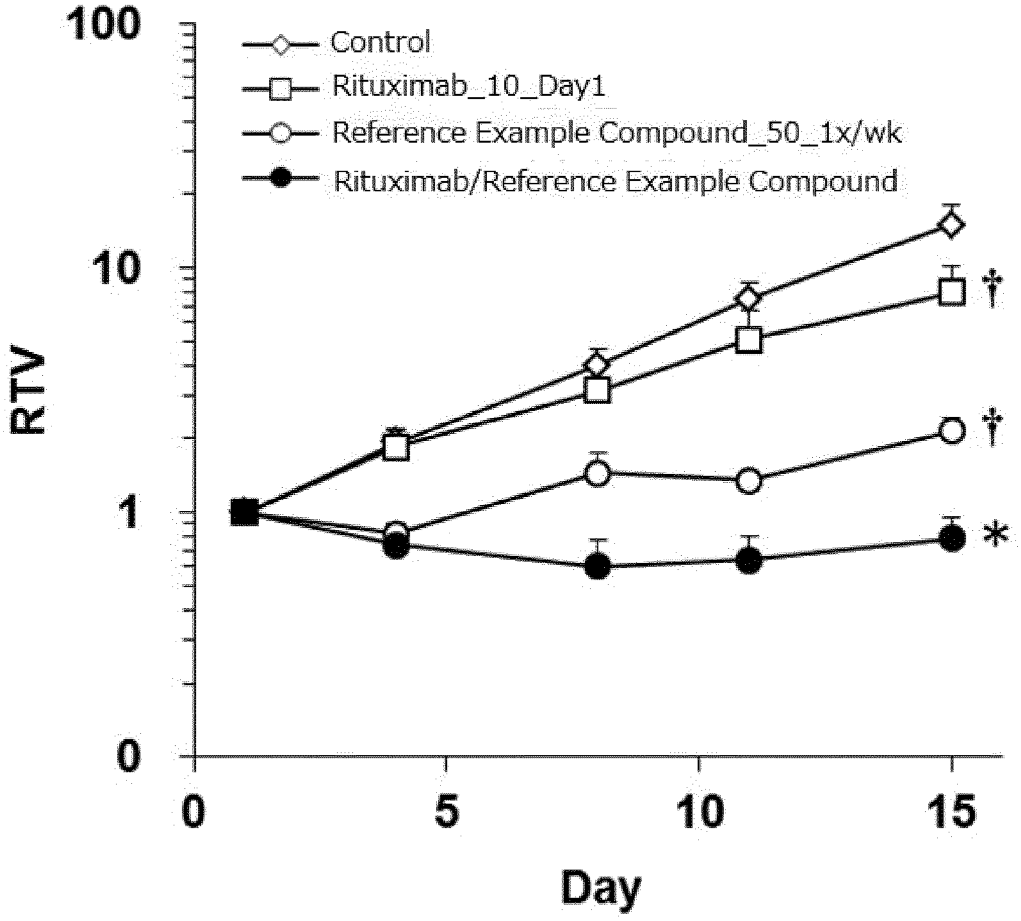


† P<0.05 vs. Control

* P<0.05 vs. Groups Administered with Single Drug

Fig. 5

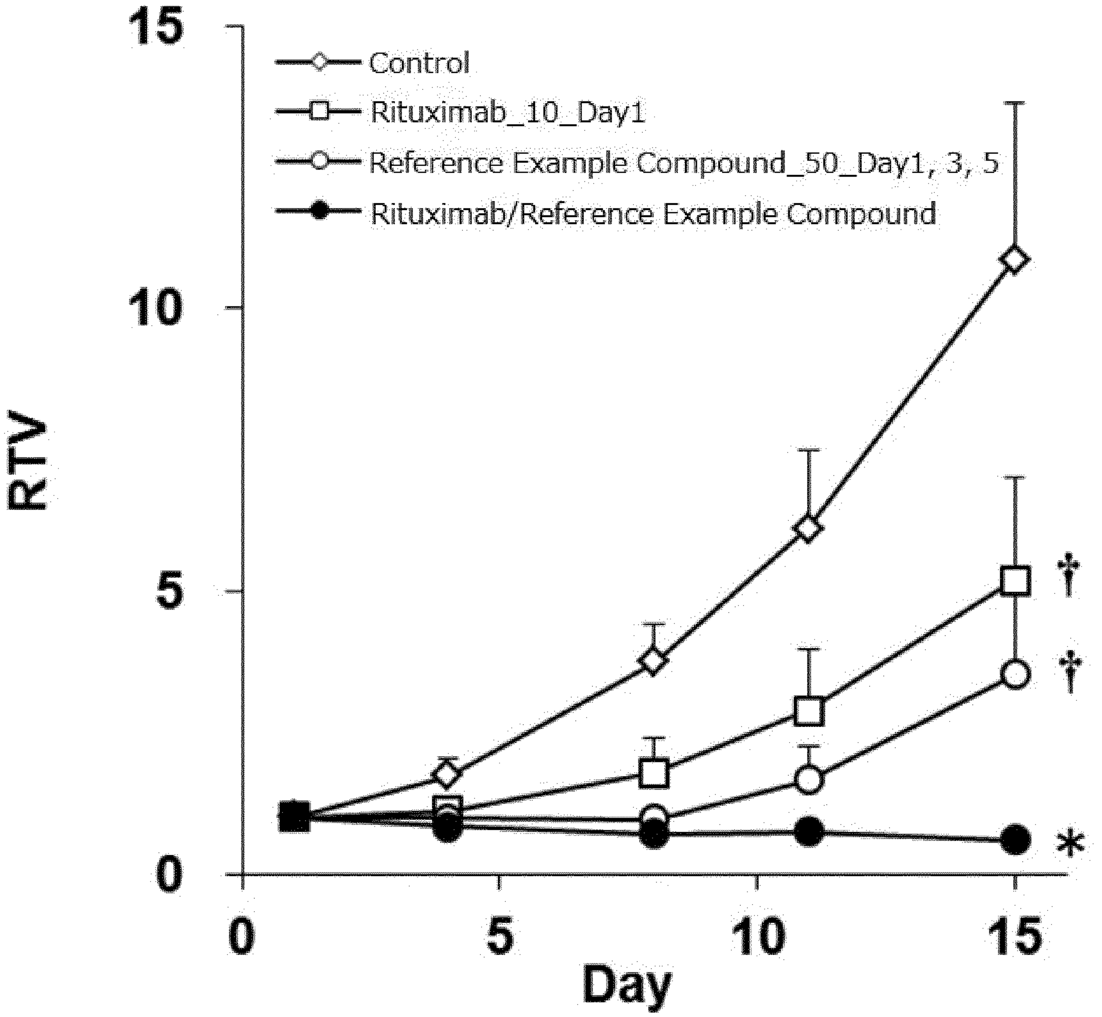


† P<0.05 vs. Control

* P<0.05 vs. Groups Administered with Single Drug

Fig. 6

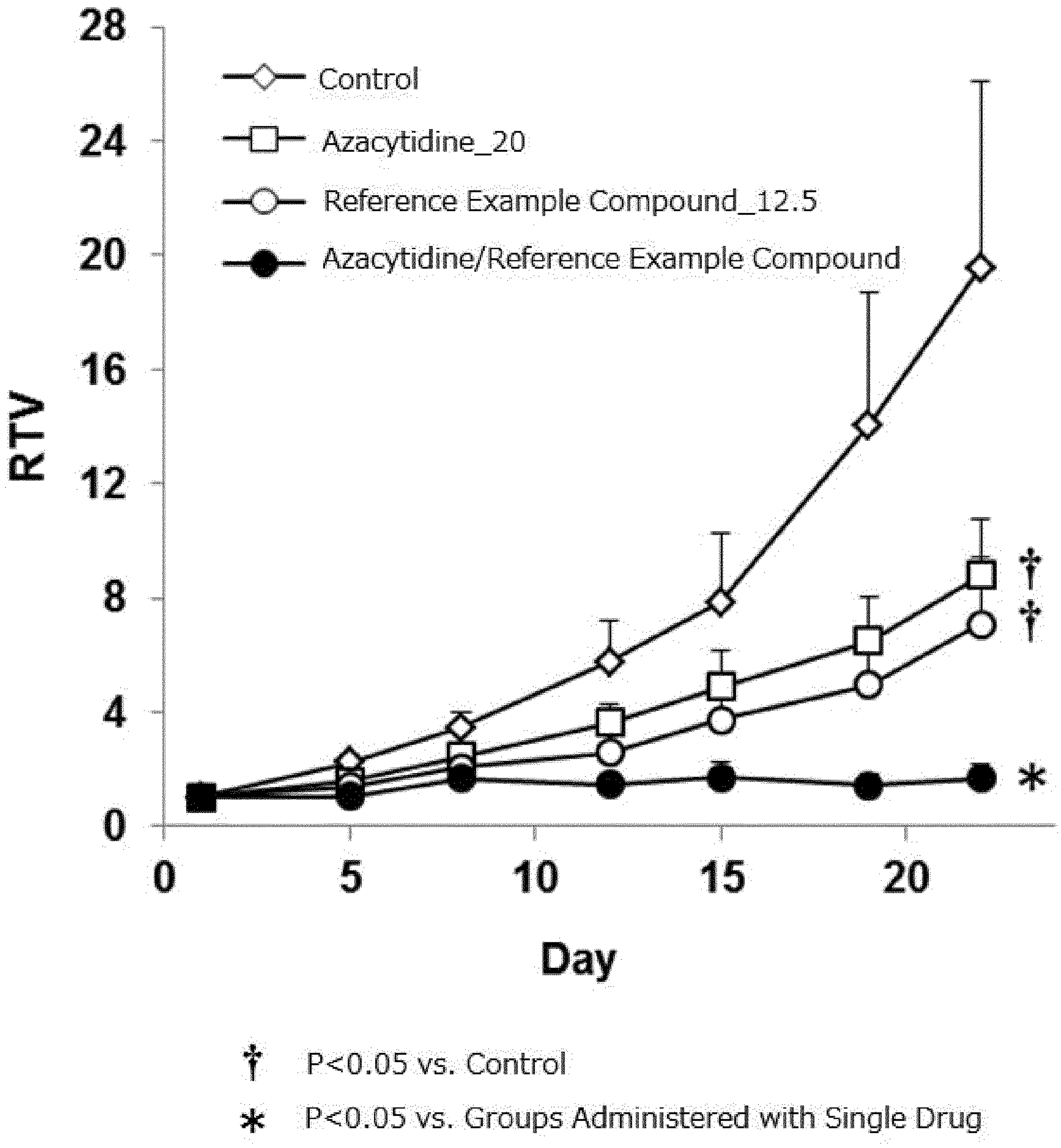
Control
Azacytidine_20
Reference Example Compound_12.5
Azacytidine/Reference Example Compound
RTV
Day
28
24
20
16
12
8
4
0
0
5
10
15
20
†
†
*
† P<0.05 vs. Control
* P<0.05 vs. Groups Administered with Single Drug

## INTERNATIONAL SEARCH REPORT

International application No.
PCT/JP2016/088473

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2008-530027 A (Millenium Pharmaceuticals, Inc.), 07 August 2008 (07.08.2008), claims; paragraphs [0016], [0033], [0037], [0039] to [0040], [0118] to [0135], [0191]; particularly, compounds I-131, I-135; example 139 & WO 2006/084281 A1 | 1-24 |
| Y | WO 2013/108809 A1 (Taiho Pharmaceutical Co., Ltd.), 25 July 2013 (25.07.2013), claims; compounds of the examples; particularly, compounds 38 to 50, 66 to 71 & US 2014/0343035 A1 | 1-24 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 January 2017 (20.01.17) | 07 February 2017 (07.02.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/088473

C (Continuation). DOCUMENTS CONSIDERED TO BE RELEVANT

| Category* | Citation of document, with indication, where appropriate, of the relevant passages | Relevant to claim No. |
|---|---|---|
| A | KIELKOWSKI, P. et al., 7-Aryl-7-deazaadenine 2'-Deoxyribonucleoside Triphosphates (dNTPs): Better Substrates for DNA Polym, Angewandte Chemie International Edition, 2014, Vol. 53, No. 29, pp. 7552-7555, Published online: 2014.05.30 | 1-24 |
| A | JP 2013-541587 A (Millenium Pharmaceuticals, Inc.), 14 November 2013 (14.11.2013), entire text & US 2012/0115892 A1 & WO 2012/061551 A1 | 1-24 |
| P,X | WO 2015/199136 A1 (Taiho Pharmaceutical Co., Ltd.), 30 December 2015 (30.12.2015), entire text & EP 3103802 A1 | 1-24 |

Form PCT/ISA/210 (continuation of second sheet) (January 2015)

INTERNATIONAL SEARCH REPORT

International application No.
PCT/JP2016/088473

Continuation of A. CLASSIFICATION OF SUBJECT MATTER
(International Patent Classification (IPC))

*A61K31/519*(2006.01)i, *A61K31/167*(2006.01)i, *A61K31/282*(2006.01)i, *A61K31/337*(2006.01)i, *A61K31/357*(2006.01)i, *A61K31/4045*(2006.01)i, *A61K31/4164*(2006.01)i, *A61K31/436*(2006.01)i, *A61K31/44*(2006.01)i, *A61K31/454*(2006.01)i, *A61K31/4545*(2006.01)i, *A61K31/47*(2006.01)i, *A61K31/4709*(2006.01)i, *A61K31/473*(2006.01)i, *A61K31/4747*(2006.01)i, *A61K31/497*(2006.01)i, *A61K31/502*(2006.01)i, *A61K31/5025*(2006.01)i, *A61K31/513*(2006.01)i, *A61K31/52*(2006.01)i, *A61K31/5377*(2006.01)i, *A61K31/538*(2006.01)i, *A61K31/541*(2006.01)i, *A61K31/553*(2006.01)i, *A61K31/573*(2006.01)i, *A61K31/675*(2006.01)i, *A61K31/704*(2006.01)i, *A61K31/7068*(2006.01)i, *A61K31/7072*(2006.01)i, *A61K33/24*(2006.01)i, *A61K45/00*(2006.01)i, *A61P35/00*(2006.01)i, *A61P43/00*(2006.01)i

(According to International Patent Classification (IPC) or to both national classification and IPC)

Continuation of B. FIELDS SEARCHED
Minimum documentation searched (International Patent Classification (IPC))

A61K31/519, A61K31/167, A61K31/282, A61K31/337, A61K31/357, A61K31/4045, A61K31/4164, A61K31/436, A61K31/44, A61K31/454, A61K31/4545, A61K31/47, A61K31/4709, A61K31/473, A61K31/4747, A61K31/497, A61K31/502, A61K31/5025, A61K31/513, A61K31/52, A61K31/5377, A61K31/538, A61K31/541, A61K31/553, A61K31/573, A61K31/675, A61K31/704, A61K31/7068, A61K31/7072,
A61K33/24, A61K45/00, A61P35/00, A61P43/00

Minimum documentation searched (classification system followed by classification symbols)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015250705 A
- JP 2016194889 A
- WO 2006084281 A
- WO 2012061551 A
- WO 2006102079 A


### Non-patent literature cited in the description


- *Nature Rev Mol Cell Biol,* 2009, vol. 10 (5), 319-331
- *Genes & Cancer,* vol. 1 (7), 690-699
- *Journal of Cellular Physiology,* 2000, vol. 183, 10-17
- *Nature,* 2009, vol. 458 (7239), 732-6
- *Mol Cancer Ther,* 2014, vol. 13 (6), 1-11
- *Mol Cancer Ther,* 2012, vol. 11 (4), 942-951
- *9th International ISSX Meeting Abstract,* 108
- *Israel Medical Association Journal,* April 2003, vol. 5, 260-263
- *Pharmacol Rev.,* 2006, vol. 58 (3), 621-81
- *BMC Complement Altern Med.,* 2013, vol. 13, 212
- *Anticancer Res,* 2005, vol. 25 (3B), 1909-17
- *Synthesis,* 1981, 1
- **T.W. GREENE.** Protective Groups in Organic Synthesis. John Wiley & Sons, 1981
- *Chemical Reviews,* 2007, vol. 107, 874
- *Chemical Reviews,* 1995, vol. 95, 2457
- *Tetrahedron Letters,* 1985, vol. 26 (16), 2001-2
- Iyakuhin no Kaihatsu [Development of Pharmaceuticals. Molecular Design. Hirokawa Shoten Co, 1990, vol. 7, 163-198
- *Organic Letters,* 2012, vol. 10, 2626-2629
- *Leuk Res.,* November 2006, vol. 30 (11), 1385-90
- *Trends Pharmacol.Sci.,* 1983, vol. 4, 450-454